# EUROPEAN PATENT APPLICATION

(11) **EP 3 960 875 A1**
(43) Date of publication of application: **02.03.2022**
(21) Application number: 20193288.6
(22) Date of filing: 28.08.2020
(51) Int. Cl.: C12Q 1/6886

(54) **PCR METHOD AND KIT FOR DETERMINING PATHWAY ACTIVITY**

(71) Applicant: Koninklijke Philips N.V., 5656 AG Eindhoven (NL)
(72) Inventor: DEN BIEZEN, Eveline Catharina Anna Clasina, 5656 AE Eindhoven (NL); VAN STRIJP, Arnoldina Margaretha Wilhelmina, 5656 AE Eindhoven (NL); VAN BRUSSEL, Anne Godefrida Catharina, 5656 AE Eindhoven (NL); WROBEL, Janneke, 5656 AE Eindhoven (NL); HOLTZER, Laurentius Henricus Franciscus Maria, 5656 AE Eindhoven (NL)
(74) Representative: Philips Intellectual Property & Standards

(57) **Abstract**

The present invention relates to a method for designing primers and probes suitable for determining one or more cellular signaling pathway activities in a sample. In particular WNT, HH, AR, ER, PR, TGFbeta, NFkB, STAT1/2, STAT3, PI3K-F0X0, Notch and MAPK-AP1,and wherein the printers and probes amplify and detect of the expression of three or more of the reference genes. The invention further relates to products comprising sets of primers and probes suitable for determining one or more cellular signaling pathway activity, and uses thereof.

## Description

### FIELD OF THE INVENTION

The subject matter described herein relates to cellular signaling pathway analysis based on gene expression data. More specifically the subject matter relates to PCR based methods to determine the expression levels of target genes which can be used for this purpose. The subject further relates to primers, probes and kits which are tailored for establishing the gene expression levels in a sample for the purpose of determining cellular signaling pathway activity.

### BACKGROUND OF THE INVENTION

Determining the activity of cellular signaling pathways in a sample is an emerging technology with many applications in diagnosis and prognostics, as well as biotechnological applications. It was found by the inventors that using a mathematical model, the cellular signaling pathway activity can be determined based on the expression levels of target genes of the cellular signaling pathway, as e.g. described in WO2013011479A2, WO2014102668A2, WO2015101635A1, WO2016062891A1, WO2017029215A1, WO2019068585A1, WO2019068562A1, WO2019068543A1 and WO2019120658A1 (each incorporated in its entirety by reference).

For many applications it would be desirable to determine the pathway activities of multiple cellular signaling pathways simultaneously. This means that many expression levels need to be determined in a sample at the same time. Namely, for each pathway preferably at least three target genes as well as at least three reference genes for normalization purposes. This simultaneous determination of expression levels could for example by done using Affymetrix Microarray technology, however this technology has the disadvantage that it is time consuming, expensive and cannot be performed in every lab due to the required equipment and specialized personnel.

The present invention aims, among other, to solve the above problems by the methods and products as defined in the appended claims.

### SUMMARY OF THE INVENTION

An alternative to e.g. a microarray based technology would be to use PCR based technology like qPCR, however the problem with such technology is that the used primers and probes often require different conditions for optimal performance, meaning many different reactions need to be performed. The present invention aims to overcome the above problems, among others, by narrowly defining the reaction conditions combined with the selection criteria for the primers and probes, and further by providing sets of primers and probes suitable for the designed reaction conditions which can be used to determine the expression levels of the different target genes which can be used to determining cellular signaling pathway activity. This allows the primers and probes to be used under the same reaction conditions (e.g. in the same multi-well plate or even as a multiplex).

Therefore, in a first aspect the present invention relates to a method for simultaneously determining the expression level of six or more genes in a sample, the method comprising simultaneously amplifying six or more gene products using a polymerase chain reaction to generate a plurality of amplification products, followed by the detection of the plurality of amplification products using a plurality of probes,
wherein the polymerase chain reaction uses, for each amplification product, a forward and a reverse primer which have the following characteristics:
- the forward and reverse primer have a GC content between 35% and 69%, preferably between 35% and 65%;
- the forward and reverse primer have a melting temperature between 50 and 71 degrees Celsius, preferably between 58 and 64 degrees Celsius;
- the forward and reverse primer have a length between 16 and 25 nucleotides, preferably between 17 and 24 nucleotides;
wherein the amplification products have a size between 60 and 240 base pairs, preferably between 65 and 150 base pairs, and preferably wherein the amplicon product is intron spanning,
wherein each of the probes used for detection of an amplification product comprises a binding part which is complementary to a part of the amplification product, the binding part further having the following characteristics:
- the binding part of the probe has a GC content between 35% and 69%, preferably between 40% and 60%;
- the binding part of the probe has a melting temperature between 56 and 72 degrees Celsius, preferably between 64 and 72 degrees Celsius;
- the binding part of the probe has a length between 17 and 31 nucleotides, preferably between 18 and 30 nucleotides;
- the binding part of the probe does not have a G at the 5' part,
wherein the expression levels are suitable for use in a method for determining one or more cellular signaling pathway activities selected from the group consisting of: WNT, HH, AR, ER, PR, PR, TGFbeta, NFkB, STAT1/2, STAT3, PI3K-FOXO, Notch, MAPK-AP1, and
wherein the primers and probes amplify and detect of the expression levels of three or more of the reference genes selected from: ACTB, ALAS1, B2M, EEF1A1 POLR2A, PUM1, RPLPO, TBP, TPT1 and TUBA1B, and
wherein the primers and probes further amplify and detect the expression levels of three or more target genes for one or more cellular signaling pathways selected from the group consisting of: AR, ER, PI3K-FOXO, MAPK-AP1, HH, Notch, TGFbeta, WNT, PR, NFkB, JAK-STAT1/2, JAK-STAT3,
wherein the three or more target genes for the ER cellular signaling pathway are selected from the group consisting of: AP1B1, CA12, CDH26, CELSR2, CTSD, ERBB2, ESR1, GREB1, HSPB1, IGFBP4, MYC, NRIP1, PDZK1, PGR, RARA, SGK3, SOD1, TFF1, WISP2, and XBP1;
the three or more target genes for the AR cellular signaling pathway are selected from the group consisting of: ABCC4, AR, CREB3L4, DHCR24, ELL2, FKBP5, GUCY1A3, KLK2, KLK3, LRIG1, NDRG1, NKX3.1 (also known as NKX3_1), PLAU, PMEPA1, PPAP2A, PRKACB 2, SGK1, and TMPRSS2;
the three or more target genes for the PI3K-FOXO cellular signaling pathway are selected from the group consisting of: AGRP, BCL2L11, BCL6, BNIP3, BTG1, CAT, CAV1, CCND1, CCNG2, CDKN1A, CDKN1B, ESR1,FBXO32, FOXO3, GADD45A, INSR, MXI1, SOD2, TNFSF10;
wherein the three or more target genes for the MAPK-AP1 cellular signaling pathway are selected from the group consisting of: BCL2L11, CCND1, DDIT3, EGFR, ENPP2, EZR, GLRX, MMP1, MMP3, MMP9, PLAU, PLAUR, PTGS2, SERPINE1, TIMP1, TP53, VEGFD, and VIM;
wherein the three or more target genes for the Notch cellular signaling pathway are selected from the group consisting of: CD44, EPHB3, FABP7, HES1, HES4, HES5, HEY1, HEY2, MYC, NOX1, NRARP, PIN1, PLXND1, and SOX9;
wherein the three or more target genes for the HH cellular signaling pathway are selected from the group consisting of: CFLAR, FOXM1, FYN, GLI1, HHIP, MYCN, NKX2-2, PTCH1, PTCH2, RAB34, SPP1, TCEA2, and TSC22D1;
wherein the three or more target genes for the TGFbeta cellular signaling pathway are selected from the group consisting of: ANGPTL4, CDKN1A, CTGF, GADD45A, GADD45B, ID1, IL11, JUNB, MMP2, MMP9, PDGFB, SERPINE1, SGK1, SKIL, SMAD4, SMAD7, SNAI1, TIMP1, and VEGFA;
wherein the three or more target genes for the WNT cellular signaling pathway are selected from the group consisting of: CEMIP, AXIN2, CD44, RNF43, MYC, TBX3, TDGF1, SOX9, ASCL2, CXCL8, SP5, ZNRF3, EPHB2, LGR5, EPHB3, KLF6, CCND1, DEFA6, and FZD7;
wherein the three or more target genes for the PR cellular signaling pathway are selected from the group consisting of: AGRP, BCL2L11, BCL6, BNIP3, BTG1, CAT, CAV1, CCND1, CCND2, CCNG2, CDKN1A, CDKN1B, ESR1, FASLG, FBXO32, GADD45A, INSR, MXI1, NOS3, PCK1, POMC, PPARGC1A, PRDX3, RBL2, SOD2 and TNFSF10;
wherein the three or more target genes for the NFkB cellular signaling pathway are selected from the group consisting of: BCL2L1, BIRC3, CCL2, CCL3, CCL4, CCL5, CCL20, CCL22, CX3CL1, CXCL1, CXCL2, CXCL3, ICAM1, IL1B, IL6, IL8, IRF1, MMP9, NFKB2, NFKBIA, NFKBIE, PTGS2, SELE, STAT5A, TNF, TNFAIP2, TNIP1 , TRAF1 , and VCAM1;
wherein the three or more target genes for the JAK-STAT1/2 cellular signaling pathway are selected from the group consisting of: APOL1, BID, CXCL9, GBP1, GNAZ, IFI6, IFIT2, IFITM1, IRF1, IRF7, IRF8, IRF9, ISG15, LGALS1, NCF4, NFAM1, OAS1, PDCD1, RAB36, RBX1, RFPL3, SAMM50, SMARCB1, SSTR3, ST13, STAT1, TAP1, TRMT1, UFD1L, USP18, and ZNRF3;
wherein the three or more target genes for the JAK-STAT3 cellular signaling pathway are selected from the group consisting of: AKT1, BCL2, BCL2L1, BIRC5, CCND1, CD274, CDKNIA, CRP, FGF2, FOS, FSCN1, FSCN2, FSCN3, HIFIA, HSP90AA1, HSP90AB1, HSP90B1, HSPA1A, HSPA1B, ICAM1, IFNG, IL10, JunB, MCL1, MMP1, MMP3, MMP9, MUC1, MYC, NOS2, POU2F1, PTGS2, SAA1, STAT1, TIMP1, TNFRSF1B, TWIST1, VIM and ZEB1.

One of the advantages of the above method is that it allows the detection of target genes for multiple cellular signaling pathways, and can be performed in a single reaction. This allows the very quick (e.g. within 2-3 hours) detection of expression levels in target genes and subsequent determination of cellular signaling pathway activities. This is very advantageous, for example in critical care, where cellular signaling pathway analysis may be used for quick diagnosis of a patient. In such cases it is essential that the method can be performed fast and without specialized equipment or personnel, as is the case with the disclosed method. The present method allows the detection of the expression levels of target genes in a sample for multiple cellular signaling pathways in a single reaction.

A further advantage of the method as disclosed herein is that it allows reliable determination of expression levels even in samples in which this is typically difficult to do so such as Formalin-Fixed Paraffin-Embedded (FFPE) tissue.

The present method requires a simple qPCR device to run, preferably a thermal cycler with fluorescence readout such as, but not limited to, the Idylla platform. For example a premade plate or container can be used comprising the required reagents and primers and probes, on which the sample is deposited. The PCR protocol does not need to be amended depending on the pathway(s) that are to be analyzed as it is standardized. During amplification cycling, probe intensity can be measured and pathway activity can be determined following conclusion of the protocol, based on the determined expression levels of the target genes. This final step uses a mathematical method to relate the input numerical values representing the expression levels of three or more target genes to a pathway activity. This step is almost instantaneously and can either be performed locally e.g. on a computer or a phone or on a remote server.

Although the method describes target genes for each cellular signaling pathway, it is envisioned that due to the selection criteria for the primers and probes, the method can be applied to alternative target genes for the mentioned cellular signaling pathways that are not listed here. Alternatively, the expression levels of the target genes for cellular signaling pathways not listed herein may be determined using primers and probes constructed using the above criteria combined with the above method.

PCR reactions are typically performed in a 96 well format, however it will be obvious to the skilled artisan that the method is not limited to this format. It is envisioned that the method may also be performed for example, but not limited to, in a single reaction tube (e.g. when multiplexing), "PCR strips" such as 8 well strips or 12 well strips, 384 well plates or 1536 well plates, or any other format, depending on the available volume of sample and the amount of target genes that need to be analyzed.

The method of the invention determines the expression level of six or more genes in a sample, as it is envisioned that preferably for each pathway the expression level of at least three target genes is analyzed, combined with the expression level of at least three reference genes. It is therefore understood that for each additional pathway that is to be analyzed (i.e. for which the activity is to be determined) the expression levels of an additional three or more target genes should be determined in the method. Therefore in order to determine the activity of a single cellular signaling pathway, the expression levels of at least six target genes should be determined, in order to determine the activity of two cellular signaling pathways, the expression levels of at least nine target genes should be determined, in order to determine the activity of three cellular signaling pathways, the expression levels of at least twelve target genes should be determined, etcetera. The method of the invention may be used to determine the activity of one or more, e.g. one, two, three, four, five, six, seven, eight, nine, ten, eleven, twelve, thirteen or more activities of cellular signaling pathways.

For each cellular signaling pathway individually, the expression levels of three or more target genes are to be determined in the method of the invention, e.g. the expression levels of three, four, five, six, seven, eight, nine, ten, eleven, twelve, thirteen or more target genes. Similarly, the expression levels of three or more reference genes are to be determined in the method of the invention, e.g. the expression levels of three, four, five, six, seven, eight, nine, ten, eleven, twelve, thirteen or more reference genes.

When used herein, "simultaneously determining the expression levels" should be interpreted as in a single PCR run, meaning the genes may be amplified and detected in individual wells or containers in the PCR device (e.g. in the separate wells of a 96 well plate, or in separate reaction tubes) or they may also be pooled and amplified in a single reaction (multiplexed), or partially pooled.

The term "sample" as used herein refers to any medium containing nucleotides, preferably RNA, such as but not limited to a medium containing cells, tissue, body fluids, culture medium, or any medium derive from these after further processing steps such as lysis, fixation or isolation of nucleotides, preferably RNA.

It is understood that in order to amplify RNA using PCR technology, first a reverse transcriptase step must be performed. In such a step a reverse transcriptase enzyme is used to generate a complementary DNA form an RNA template. The reverse transcriptase enzyme typically employs a primer sequence which is reverse complementary to a part of the RNA. Generally oligo dT primers may be used for such reaction, or target specific primers. For the method disclosed herein, preferably the reverse primers are used both for the reverse transcriptase reaction and the amplification reaction.

Amplification using DNA primers (polymerase chain reaction) is a technology well known to the skilled person. PCR methods rely on thermal cycling. Thermal cycling exposes reactants to repeated cycles of heating and cooling to permit different temperature dependent reactions, such as DNA melting and enzyme driven DNA replication. PCR employs two main reagents, the forward and reverse primers, which are short single strand DNA fragments known as oligonucleotides that are a complementary sequence to the target DNA region, and a DNA polymerase.

In method disclosed herein the primers and probes amplify and detect of the expression levels of three or more of the reference genes selected from: ACTB, ALAS1, B2M, EEF1A1 POLR2A, PUM1, RPLPO, TBP, TPT1 and TUBA1B, and wherein the primers and probes further amplify and detect the expression levels of three or more target genes for one or more cellular signaling pathways selected from the group consisting of: AR, ER, PI3K-FOXO, MAPK-AP1, HH, Notch, TGFbeta, WNT, PR, NFkB, JAK-STAT1/2 and JAK-STAT3. Therefore the method may include primers and probes for the amplification and detection of three or more target genes for one, two, three, four, five, six seven, eight, nine, ten, eleven, twelve or more cellular signaling pathways. In a preferred embodiment the one or more, e.g. one, two, three, four, five, six, seven, eight, nine, ten, eleven, twelve or more, cellular signaling pathways comprise one or more, e.g. one, two, three, four, five, six or seven, cellular signaling pathways selected from the group consisting of the AR, ER, PI3K-FOXO, MAPK-AP1, HH, Notch and TGFbeta cellular signaling pathways. In a more preferred embodiment the one or more, e.g. one, two, three, four, five, six seven, eight, nine, ten, eleven, twelve or more, cellular signaling pathways comprise one or more, e.g. one, two, three or four, cellular signaling pathways selected from the group consisting of the AR, ER, PI3K-FOXO and MAPK-AP1 cellular signaling pathways, and may optionally further comprise one or more, e.g. one two or three cellular signaling pathway selected from the group consisting of HH, Notch and TGFbeta.

Suitable probes types for qPCR are known to the skilled person. For example, the probes may be fluorophore based, such as TaqMan probes. Fluorophore based probes generally work by including a quencher. TaqMan probes consist of a fluorophore covalently attached to the 5'-end of the oligonucleotide probe and a quencher at the 3'-end. The principle relies on the 5'-3' exonuclease activity of Taq polymerase to cleave a dual-labeled probe during hybridization to the complementary target sequence and fluorophore-based detection. When bound to the probe the fluorophore is quenched, but once cleaved from the probe the distance with the quencher is increased resulting in fluorescence of the fluorophore when excited at the right wavelength.

In a preferred embodiment of the method of the first aspect of the invention, the primers and probes are able to amplify and detect the respective genes under the following reaction conditions:

| | |
|---|---|
| 50 mM | monovalent salt; |
| 400 nM | forward primer |
| 400 nM | reverse primer |
| 3.0 mM | divalent salt, preferably the divalent salt being Mg²⁺; |
| 100 nM | probe; and |
| 0.8 mM | dNTP. |

Therefore, the primers and probes are preferably able to detect the expression level of a single target gen in a medium comprising 50 mM monovalent salt, 3.0 mM divalent salt, preferably the divalent salt being Mg²⁺ and 0.8 mM dNTP, when the primers are present in a concentration of 400 nM for each forward and reverse primer, and wherein the probe is present in a concentration of 100 nM. It is understood that the primers and probes may work outside these parameters, but that the parameters for designing the primers and probe combinations are designed are such that they are suitable for these reaction conditions.

Preferably the divalent salt is MgSO₄ or MgCl₂.
In further preferred embodiment of the method of the first aspect of the invention, the primers and probes are able to amplify and detect the respective genes under the following reaction conditions:
a RT reaction at 50° Celsius for 30 minutes in order to synthesize cDNA, followed by a 5 minute denaturation step at 95° Celsius, followed by 45 cycli of a 15 second denaturation step at 95° Celsius and a 30 second Elongation step at 60°Celsius.

In an embodiment of the method of the first aspect of the invention, the method is used to determine one or more cellular signaling pathway activity or activities. Preferably the one or more cellular signaling Pathway activities are selected from the group consisting of AR, ER, PI3K-FOXO, MAPK-AP1, HH, Notch, TGFbeta, WNT, PR, NFkB, JAK-STAT1/2 and JAK-STAT3. An especially advantageous aspect of the invention is the ability to determine multiple pathway activities in a sample in a single reaction, therefore preferably two or more, e.g. three, four, five, six or seven or more cellular signaling pathway activities are determined using the method of the invention. Particularly preferred is using the method to determining four or more cellular signaling pathway activities, wherein the four or more cellular signaling pathway activities comprise the ER, AR, PI3K-FOXO and AP1-MAPK cellular signaling pathways. Further particularly preferred is using the method to determining seven or more cellular signaling pathway activities, wherein the seven or more cellular signaling pathway activities comprise the ER, AR, PI3K-FOXO, AP1-MAPK, Notch, HH and TGFbeta cellular signaling pathways. It is considered that there are multiple practical applications where the determination of multiple cellular signaling pathway activities may be extremely useful. Although determining pathway activity has been described in the literature by alternative means, known methods are generally not very quantitative and/or easy and/or fast to perform. It is therefore postulated that the presently described solution wherein in a single qPCR reaction multiple cellular signaling pathways activities can be inferred offers extremely useful applications in research and diagnostics, as often multiple cellular signaling pathways are relevant for e.g. making a clinical decision based on pathway activities in a patient sample. The invention provides an easy to perform assay where a quantitative assessment of the activities of e.g. 7 cellular signaling pathways can be determined on a difficult to process sample such as FFPE (Formalin-fixed paraffin embedded) tissue in a matter of 2-3 hours in a single reaction.

When used herein, the terms AP1-MAPK, MAPK-AP1 and MAPK are used interchangeably and refer to the MAPK signaling pathway controlled by the API transcription factor complex. When used herein the terms PI3K-FOXO, FOXO-PI3K and PI3K are used interchangeably and refer to the PI3K signaling pathway, which activity may be determined by taking the inverse of the determined by FOXO pathway activity. When used herein, the terms HH and Hedgehog are used interchangeably and refer to the cellular signaling pathway.

In a second aspect the invention relates to an assembly of primers and probe for determining the activity of the ER cellular signaling pathway, wherein the assembly of primers and probes comprises at least three sets of primers or probes for determining the expression level of three or more target genes of the ER cellular signaling pathway, wherein said three or more sets of primers and probes are selected from Table 1 below, wherein each primer and/or probe individually is identical to the corresponding sequence in Table 1 or differs at 1, 2 or 3 positions, wherein said 1, 2 or 3 differences are selected from a single base substitution, a single base deletion or a single base addition.

Table 1 to 8 disclose different sets of primers and probes for the different target genes of the referred cellular signaling pathways. The lists are sorted per gene, each gene may have multiple sets of primers and probes, each set of primers and probes consisting of a forward primer, a reverse primer and a probe. Each set is indicated as an "assay", therefore when used herein, a set of primers and probe refers to a forward primer, a reverse primer and a probe with the same assay name as indicated in one of tables 1 to 8. The primers and probes are indicated with their respective SEQ ID NO and name (assay), the name being generated according to the following format: [GENE NAME]_[NUMBER], wherein primers and probe with the same name (thus same gene name and number) are part of a single set.

Therefore, the invention further relates to an assembly of primers and probes for determining the activity of the ER cellular signaling pathway, wherein the set of primers and probes comprises at least three sets of primers or probes for determining the expression level of three or more target genes of the ER cellular signaling pathway, wherein said sets of primers and probes are selected from Table 1 wherein the forward primer, the reverse primer and the probe have the same assay name; and
wherein each primer and/or probe individually has the listed nucleotide sequence identified by the corresponding SEQ ID NO or has a sequence that differs at 1, 2 or 3 positions, wherein said 1, 2 or 3 differences are individually selected from a single base substitution, a single base deletion or a single base addition.

Preferably the assembly further comprises three or more sets of primers and probes for determining the expression levels of three or more reference genes, wherein said sets of primers and probes are selected from Table 8 wherein the forward primer, the reverse primer and the probe have the same assay name; and
wherein each primer and/or probe individually has the listed nucleotide sequence identified by the corresponding SEQ ID NO or has a sequence that differs at 1, 2 or 3 positions, wherein said 1, 2 or 3 differences are individually selected from a single base substitution, a single base deletion or a single base addition.

It is recognized that the PCR reaction is tolerant to a few, e.g. 1, 2 or 3, mismatches in the primer or probe sequences and still allows amplification and detection of the target gene expression level. Therefore, up to 3 mismatches may be allowed in the sequences of the primers and probes each. A mismatch or difference at a position of a sequence when used herein refers to a single base substitution, a single base deletion or a single base insertion with respect to the reference sequence (e.g. the respective SEQ ID NO). It is recognized that the PCR reaction is general more tolerant to a substitution as compared to a deletion or insertion in the primers or probes, therefore preferably the mismatch or difference at a position of a sequence is a single base substitution, although a deletion or insertion ay be desirable to account for genetic variation in the target DNA of the sample.

It is understood that avoiding mismatches is preferable for optimal amplification and detection of the target gene. Therefore preferably each primer and/or probe individually is identical to the corresponding SEQ ID NO or differs at 1 or 2 positions, more preferably each primer and/or probe individually is identical to the corresponding SEQ ID NO or differs at 1 position, most preferably each primer and/or probe individually is identical to the corresponding SEQ ID NO.

When used herein, a set of primers and probe refers to set consisting of a forward primer and a reverse primer for amplifying a genomic sequence and a probe for detecting the genomic sequence, where the genomic sequence is a target gene suited for determining the activity of a cellular signaling pathway. The forward and reverse primer and corresponding probe belonging to a single "set" are defined in the "assays" listed in Tables 1 to 8, and thus the primers and probe have an identical assay name when belonging to the same "set".

When used herein, an assembly of primers and probes refers to multiple (e.g. at least two) sets of primers and probes as defined herein.

In a third aspect the invention further relates to an assembly of primers and probes for determining the activity of the AR cellular signaling pathway, wherein the set of primers and probes comprises at least three sets of primers or probes for determining the expression level of three or more target genes of the AR cellular signaling pathway, wherein said sets of primers and probes are selected from Table 2 wherein the forward primer, the reverse primer and the probe have the same assay name; and
wherein each primer and/or probe individually has the listed nucleotide sequence identified by the corresponding SEQ ID NO or has a sequence that differs at 1, 2 or 3 positions, wherein said 1, 2 or 3 differences are individually selected from a single base substitution, a single base deletion or a single base addition.

Preferably the assembly further comprises three or more sets of primers and probes for determining the expression levels of three or more reference genes, wherein said sets of primers and probes are selected from Table 8 wherein the forward primer, the reverse primer and the probe have the same assay name; and
wherein each primer and/or probe individually has the listed nucleotide sequence identified by the corresponding SEQ ID NO or has a sequence that differs at 1, 2 or 3 positions, wherein said 1, 2 or 3 differences are individually selected from a single base substitution, a single base deletion or a single base addition.

It is recognized that the PCR reaction is tolerant to a few, e.g. 1, 2 or 3, mismatches in the primer or probe sequences and still allows amplification and detection of the target gene expression level. Therefore, up to 3 mismatches may be allowed in the sequences of the primers and probes each. A mismatch or difference at a position of a sequence when used herein refers to a single base substitution, a single base deletion or a single base insertion with respect to the reference sequence (e.g. the respective SEQ ID NO). It is recognized that the PCR reaction is general more tolerant to a substitution as compared to a deletion or insertion in the primers or probes, therefore preferably the mismatch or difference at a position of a sequence is a single base substitution, although a deletion or insertion ay be desirable to account for genetic variation in the target DNA of the sample.

It is understood that avoiding mismatches is preferable for optimal amplification and detection of the target gene. Therefore preferably each primer and/or probe individually is identical to the corresponding SEQ ID NO or differs at 1 or 2 positions, more preferably each primer and/or probe individually is identical to the corresponding SEQ ID NO or differs at 1 position, most preferably each primer and/or probe individually is identical to the corresponding SEQ ID NO.

In a fourth aspect the invention further relates to an assembly of primers and probes for determining the activity of the PI3K-FOXO cellular signaling pathway, wherein the set of primers and probes comprises at least three sets of primers or probes for determining the expression level of three or more target genes of the PI3K-FOXO cellular signaling pathway, wherein said sets of primers and probes are selected from Table 3 wherein the forward primer, the reverse primer and the probe have the same assay name; and
wherein each primer and/or probe individually has the listed nucleotide sequence identified by the corresponding SEQ ID NO or has a sequence that differs at 1, 2 or 3 positions, wherein said 1, 2 or 3 differences are individually selected from a single base substitution, a single base deletion or a single base addition.

Preferably the assembly further comprises three or more sets of primers and probes for determining the expression levels of three or more reference genes, wherein said sets of primers and probes are selected from Table 8 wherein the forward primer, the reverse primer and the probe have the same assay name; and
wherein each primer and/or probe individually has the listed nucleotide sequence identified by the corresponding SEQ ID NO or has a sequence that differs at 1, 2 or 3 positions, wherein said 1, 2 or 3 differences are individually selected from a single base substitution, a single base deletion or a single base addition.

It is recognized that the PCR reaction is tolerant to a few, e.g. 1, 2 or 3, mismatches in the primer or probe sequences and still allows amplification and detection of the target gene expression level. Therefore, up to 3 mismatches may be allowed in the sequences of the primers and probes each. A mismatch or difference at a position of a sequence when used herein refers to a single base substitution, a single base deletion or a single base insertion with respect to the reference sequence (e.g. the respective SEQ ID NO). It is recognized that the PCR reaction is general more tolerant to a substitution as compared to a deletion or insertion in the primers or probes, therefore preferably the mismatch or difference at a position of a sequence is a single base substitution, although a deletion or insertion ay be desirable to account for genetic variation in the target DNA of the sample.

It is understood that avoiding mismatches is preferable for optimal amplification and detection of the target gene. Therefore preferably each primer and/or probe individually is identical to the corresponding SEQ ID NO or differs at 1 or 2 positions, more preferably each primer and/or probe individually is identical to the corresponding SEQ ID NO or differs at 1 position, most preferably each primer and/or probe individually is identical to the corresponding SEQ ID NO.

In a fifth aspect the invention further relates to an assembly of primers and probes for determining the activity of the AP1-MAPK cellular signaling pathway, wherein the set of primers and probes comprises at least three sets of primers or probes for determining the expression level of three or more target genes of the AP1-MAPK cellular signaling pathway, wherein said sets of primers and probes are selected from Table 4 wherein the forward primer, the reverse primer and the probe have the same assay name; and
wherein each primer and/or probe individually has the listed nucleotide sequence identified by the corresponding SEQ ID NO or has a sequence that differs at 1, 2 or 3 positions, wherein said 1, 2 or 3 differences are individually selected from a single base substitution, a single base deletion or a single base addition.

Preferably the assembly further comprises three or more sets of primers and probes for determining the expression levels of three or more reference genes, wherein said sets of primers and probes are selected from Table 8 wherein the forward primer, the reverse primer and the probe have the same assay name; and
wherein each primer and/or probe individually has the listed nucleotide sequence identified by the corresponding SEQ ID NO or has a sequence that differs at 1, 2 or 3 positions, wherein said 1, 2 or 3 differences are individually selected from a single base substitution, a single base deletion or a single base addition.

It is recognized that the PCR reaction is tolerant to a few, e.g. 1, 2 or 3, mismatches in the primer or probe sequences and still allows amplification and detection of the target gene expression level. Therefore, up to 3 mismatches may be allowed in the sequences of the primers and probes each. A mismatch or difference at a position of a sequence when used herein refers to a single base substitution, a single base deletion or a single base insertion with respect to the reference sequence (e.g. the respective SEQ ID NO). It is recognized that the PCR reaction is general more tolerant to a substitution as compared to a deletion or insertion in the primers or probes, therefore preferably the mismatch or difference at a position of a sequence is a single base substitution, although a deletion or insertion ay be desirable to account for genetic variation in the target DNA of the sample.

It is understood that avoiding mismatches is preferable for optimal amplification and detection of the target gene. Therefore preferably each primer and/or probe individually is identical to the corresponding SEQ ID NO or differs at 1 or 2 positions, more preferably each primer and/or probe individually is identical to the corresponding SEQ ID NO or differs at 1 position, most preferably each primer and/or probe individually is identical to the corresponding SEQ ID NO.

In a sixth aspect the invention further relates to an assembly of primers and probes for determining the activity of the Notch cellular signaling pathway, wherein the set of primers and probes comprises at least three sets of primers or probes for determining the expression level of three or more target genes of the Notch cellular signaling pathway, wherein said sets of primers and probes are selected from Table 5 wherein the forward primer, the reverse primer and the probe have the same assay name; and
wherein each primer and/or probe individually has the listed nucleotide sequence identified by the corresponding SEQ ID NO or has a sequence that differs at 1, 2 or 3 positions, wherein said 1, 2 or 3 differences are individually selected from a single base substitution, a single base deletion or a single base addition.

Preferably the assembly further comprises three or more sets of primers and probes for determining the expression levels of three or more reference genes, wherein said sets of primers and probes are selected from Table 8 wherein the forward primer, the reverse primer and the probe have the same assay name; and
wherein each primer and/or probe individually has the listed nucleotide sequence identified by the corresponding SEQ ID NO or has a sequence that differs at 1, 2 or 3 positions, wherein said 1, 2 or 3 differences are individually selected from a single base substitution, a single base deletion or a single base addition.

It is recognized that the PCR reaction is tolerant to a few, e.g. 1, 2 or 3, mismatches in the primer or probe sequences and still allows amplification and detection of the target gene expression level. Therefore, up to 3 mismatches may be allowed in the sequences of the primers and probes each. A mismatch or difference at a position of a sequence when used herein refers to a single base substitution, a single base deletion or a single base insertion with respect to the reference sequence (e.g. the respective SEQ ID NO). It is recognized that the PCR reaction is general more tolerant to a substitution as compared to a deletion or insertion in the primers or probes, therefore preferably the mismatch or difference at a position of a sequence is a single base substitution, although a deletion or insertion ay be desirable to account for genetic variation in the target DNA of the sample.

It is understood that avoiding mismatches is preferable for optimal amplification and detection of the target gene. Therefore preferably each primer and/or probe individually is identical to the corresponding SEQ ID NO or differs at 1 or 2 positions, more preferably each primer and/or probe individually is identical to the corresponding SEQ ID NO or differs at 1 position, most preferably each primer and/or probe individually is identical to the corresponding SEQ ID NO.

In a seventh aspect the invention further relates to an assembly of primers and probes for determining the activity of the HH cellular signaling pathway, wherein the set of primers and probes comprises at least three sets of primers or probes for determining the expression level of three or more target genes of the HH cellular signaling pathway, wherein said sets of primers and probes are selected from Table 6 wherein the forward primer, the reverse primer and the probe have the same assay name; and
wherein each primer and/or probe individually has the listed nucleotide sequence identified by the corresponding SEQ ID NO or has a sequence that differs at 1, 2 or 3 positions, wherein said 1, 2 or 3 differences are individually selected from a single base substitution, a single base deletion or a single base addition.

Preferably the assembly further comprises three or more sets of primers and probes for determining the expression levels of three or more reference genes, wherein said sets of primers and probes are selected from Table 8 wherein the forward primer, the reverse primer and the probe have the same assay name; and
wherein each primer and/or probe individually has the listed nucleotide sequence identified by the corresponding SEQ ID NO or has a sequence that differs at 1, 2 or 3 positions, wherein said 1, 2 or 3 differences are individually selected from a single base substitution, a single base deletion or a single base addition.

It is recognized that the PCR reaction is tolerant to a few, e.g. 1, 2 or 3, mismatches in the primer or probe sequences and still allows amplification and detection of the target gene expression level. Therefore, up to 3 mismatches may be allowed in the sequences of the primers and probes each. A mismatch or difference at a position of a sequence when used herein refers to a single base substitution, a single base deletion or a single base insertion with respect to the reference sequence (e.g. the respective SEQ ID NO). It is recognized that the PCR reaction is general more tolerant to a substitution as compared to a deletion or insertion in the primers or probes, therefore preferably the mismatch or difference at a position of a sequence is a single base substitution, although a deletion or insertion ay be desirable to account for genetic variation in the target DNA of the sample.

It is understood that avoiding mismatches is preferable for optimal amplification and detection of the target gene. Therefore preferably each primer and/or probe individually is identical to the corresponding SEQ ID NO or differs at 1 or 2 positions, more preferably each primer and/or probe individually is identical to the corresponding SEQ ID NO or differs at 1 position, most preferably each primer and/or probe individually is identical to the corresponding SEQ ID NO.

In a eight aspect the invention further relates to an assembly of primers and probes for determining the activity of the TGFbeta cellular signaling pathway, wherein the set of primers and probes comprises at least three sets of primers or probes for determining the expression level of three or more target genes of the TGFbeta cellular signaling pathway, wherein said sets of primers and probes are selected from Table 7 wherein the forward primer, the reverse primer and the probe have the same assay name; and
wherein each primer and/or probe individually has the listed nucleotide sequence identified by the corresponding SEQ ID NO or has a sequence that differs at 1, 2 or 3 positions, wherein said 1, 2 or 3 differences are individually selected from a single base substitution, a single base deletion or a single base addition.

Preferably the assembly further comprises three or more sets of primers and probes for determining the expression levels of three or more reference genes, wherein said sets of primers and probes are selected from Table 8 wherein the forward primer, the reverse primer and the probe have the same assay name; and
wherein each primer and/or probe individually has the listed nucleotide sequence identified by the corresponding SEQ ID NO or has a sequence that differs at 1, 2 or 3 positions, wherein said 1, 2 or 3 differences are individually selected from a single base substitution, a single base deletion or a single base addition.

It is recognized that the PCR reaction is tolerant to a few, e.g. 1, 2 or 3, mismatches in the primer or probe sequences and still allows amplification and detection of the target gene expression level. Therefore, up to 3 mismatches may be allowed in the sequences of the primers and probes each. A mismatch or difference at a position of a sequence when used herein refers to a single base substitution, a single base deletion or a single base insertion with respect to the reference sequence (e.g. the respective SEQ ID NO). It is recognized that the PCR reaction is general more tolerant to a substitution as compared to a deletion or insertion in the primers or probes, therefore preferably the mismatch or difference at a position of a sequence is a single base substitution, although a deletion or insertion ay be desirable to account for genetic variation in the target DNA of the sample.

It is understood that avoiding mismatches is preferable for optimal amplification and detection of the target gene. Therefore preferably each primer and/or probe individually is identical to the corresponding SEQ ID NO or differs at 1 or 2 positions, more preferably each primer and/or probe individually is identical to the corresponding SEQ ID NO or differs at 1 position, most preferably each primer and/or probe individually is identical to the corresponding SEQ ID NO.

In an embodiment of the assembly according the second to the eight aspect of the invention, all of the primers and probes in the three or more sets of primers and probes in the assembly are identical to the corresponding sequence according to Tables 1 to 7 or the sequences represented by the indicated SEQ ID Nos.

In a ninth aspect the invention relates to a kit of parts for determining the expression levels for a plurality of genes, the kit comprising primers and probes for the amplification and detection of the expression levels of the plurality of genes, wherein the kit comprises primers and probes are as defined in the first aspect of the invention or an assembly of primers and probes as defined in any one of the second to the eight aspect of the invention, wherein the kit further comprises primers and probes for the amplification and detection of three or more of the reference genes selected from ACTB, ALAS1, B2M, EEF1A1 POLR2A, PUM1, RPLP0, TBP, TPT1 and TUBA1B.

Preferably the kit is suitable for determining the activity of a cellular signaling pathway. Therefore the kit preferably comprises at least three sets of primers which are suitable for the amplification and detection of the expression levels of at least three target genes of a cellular signaling pathway as described in the first to the eight aspect of the invention. Preferably the cellular signaling pathway is selected from the group consisting of ER, AR, PI3K-FOXO, MAPK-AP1, WNT, HH, PR, TGFbeta, NFkB, STAT1/2, STAT3, and Notch. More preferably the kit comprises three or more sets of primers and probes, e.g. three, four, five, six, seven, eight, nine, ten, eleven or twelve or more, as described herein each for the detection of one or more, e.g. one, two, three, four, five, six, seven, eight, nine, ten, eleven or twelve, pathway activities selected from the group consisting of ER, AR, PI3K-FOXO, MAPK-AP1, WNT, HH, PR, TGFbeta, NFkB, STAT1/2, STAT3, and Notch, more preferably selected from the group consisting of ER, AR, PI3K-FOXO, MAPK-AP1, Notch, HH and TGFbeta. Preferably said one or more, e.g. one, two, three, four, five, six, seven, eight, nine, ten, eleven or twelve, pathway activities selected from the group consisting of ER, AR, PI3K-FOXO, MAPK-AP1, WNT, HH, PR, TGFbeta, NFkB, STAT1/2, STAT3, and Notch comprise one or more, e.g. one, two, three, four, five, six or seven, pathways selected from the group consisting of ER, AR, PI3K-FOXO, MAPK-AP1, Notch, HH and TGFbeta. Preferably said one or more, e.g. one, two, three, four, five, six, seven, eight, nine, ten, eleven or twelve, pathway activities selected from the group consisting of ER, AR, PI3K-FOXO, MAPK-AP1, WNT, HH, PR, TGFbeta, NFkB, STAT1/2, STAT3, and Notch comprise one or more, e.g. one, two, three or four, pathways selected from the group consisting of ER, AR, PI3K-FOXO and MAPK-AP1.

Optionally the primers and probes for the amplification and detection of the reference genes are selected from Table 8 below, although it is understood that other sets of primers and probes may be used, provided the primers and probes are suitable at the reaction conditions described herein above.

The kit may optionally further comprise one or more of a polymerase enzyme, a reverse transcriptase enzyme, a suitable buffer and a container for performing the PCR reaction. Suitable containers may be reaction tubes, PCR trips such as 8 well or 12 well strips or multiwell plates, also known as microwell plates, microtiter plates or microplates, such as 6, 12, 24, 48, 96, 384, or 1536 well plates, or any other container which can be used in a thermal cycler, preferably a thermal cycler with fluorescence readout capability.

In an tenth aspect the invention relates to the use of the primers and probes as defined in the first aspect or the assembly of primers and probes as defined in the second to the eight aspect or the kit as defined in ninth aspect for determining the cellular signaling pathway activity for one or more cellular signaling pathways selected from the group consisting of: ER, AR, PI3K-FOXO, MAPK-AP1, WNT, HH, PR, TGFbeta, NFkB, STAT1/2, STAT3, and Notch.

In a eleventh aspect the invention relates to the use of a set of three or more primers and probes to determine the expression levels of three or more target genes of a cellular signaling pathway, wherein the primers and probe combinations are as defined in the second to the ninth apsect, and wherein the target genes are as defined in the first aspect.

In a twelfth aspect the invention relates to a method for designing primers and probes for the detection of the expression levels of target genes of a cellular signaling pathway suitable for determining the activity of said cellular signaling pathway, the method comprising:
- designing for a target gene of a cellular signaling pathway a forward primer and a reverse primer such that:
- the forward and reverse primer have a GC content between 35% and 69%, preferably between 35% and 65%;
- the forward and reverse primer have a melting temperature between 50 and 71 degrees Celsius, preferably between 58 and 64 degrees Celsius;
- the forward and reverse primer have a length between 16 and 25 nucleotides, preferably between 17 and 24 nucleotides;
wherein the amplification product, when using the forward and reverse primers in a PCR amplification reaction, has a size between 60 and 240 base pairs, preferably between 65 and 150 base pairs, and preferably wherein the amplicon product is intron spanning;
- designing the probe such that:
- the probe used for detection of an amplification product comprises a binding part which is complementary to a part of the amplification product, the binding part further having the following characteristics:
- the binding part of the probe has a GC content between 35% and 69%, preferably between 40% and 60%;
- the binding part of the probe has a melting temperature between 56 and 72 degrees Celsius, preferably between 64 and 72 degrees Celsius;
- the binding part of the probe has a length between 17 and 31 nucleotides, preferably between 18 and 30 nucleotides;
- the binding part of the probe does not have a G at the 5' part.
Primers designed according t this method are useful as they can be used in an assay for determining one or more cellular signaling pathway activities. Preferably the primers and probes are able to detect the expression level of a target gene under the following reaction conditions:
50 mM monovalent salt;
400 nM forward primer
400 nM reverse primer
3.0 mM divalent salt, preferably the divalent salt being Mg²⁺;
100 nM probe; and
0.8 mM dNTP

Preferably the divalent salt is MgSO₄ or MgCl₂.

Preferably the primers and probes are able to amplify and detect the respective genes under the following reaction conditions:
a RT reaction at 50° Celsius for 30 minutes in order to synthesize cDNA, followed by a 5 minute denaturation step at 95° Celsius, followed by 44 cycli of a 15 second denaturation step at 95° Celsius and a 30 second Elongation step at 60°Celsius.

This application describes several preferred embodiments. Modifications and alterations may occur to others upon reading and understanding the preceding detailed description. It is intended that the application is construed as including all such modifications and alterations insofar as they come within the scope of the appended claims or the equivalents thereof.

Other variations to the disclosed embodiments can be understood and effected by those skilled in the art in practicing the claimed invention, from a study of the drawings, the disclosure, and the appended claims.

It shall be understood that the methods of the first aspect, the computer implemented invention of the second aspect, the apparatus of the third aspect, the non-transitory storage medium of fourth aspect, the computer program of the fifth aspect, the kits of the sixth aspect have similar and/or identical preferred embodiments, in particular, as defined in the dependent claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

It shall be understood that a preferred embodiment of the present invention can also be any combination of the dependent claims or above embodiments with the respective independent claim.

These and other aspects of the invention will be apparent from and elucidated with reference to the embodiments described hereinafter.

### BRIEF DESCRIPTION OF THE DRAWINGS

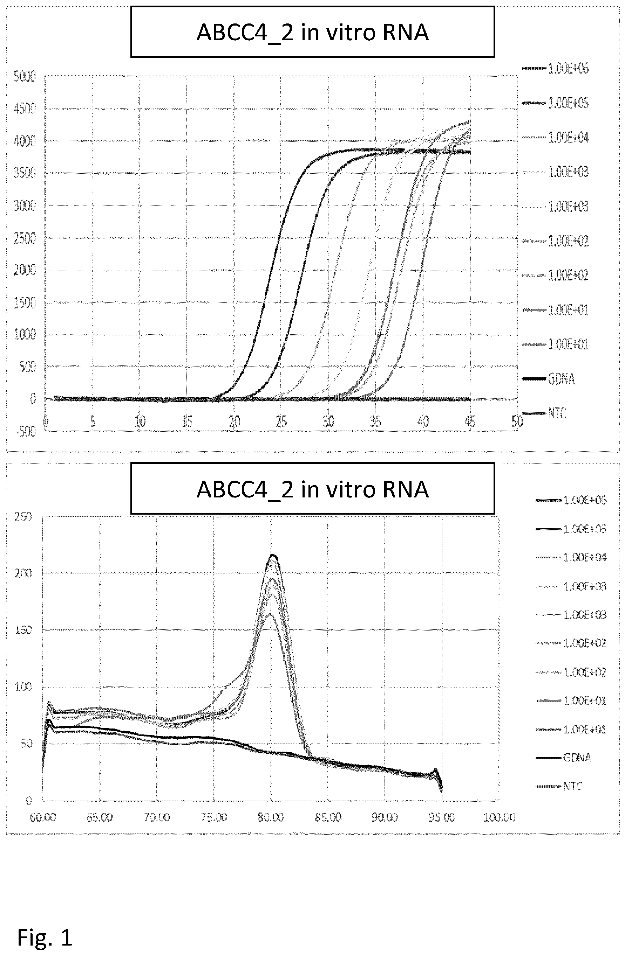

Fig. 1 depicts probe fluorescence of the ABCC4_2 assay (top panel) using different concentrations of target RNA. The vertical axis depicts relative fluorescence units (RFU), the horizontal axis depicts the cycle number of the qPCR protocol. The bottom panel depicts the melting curves for the same conditions. The vertical axis depicts the change of relative fluorescence units over time (-d(RFU)/dT), the horizontal axis depicts the temperature in degrees Celsius.

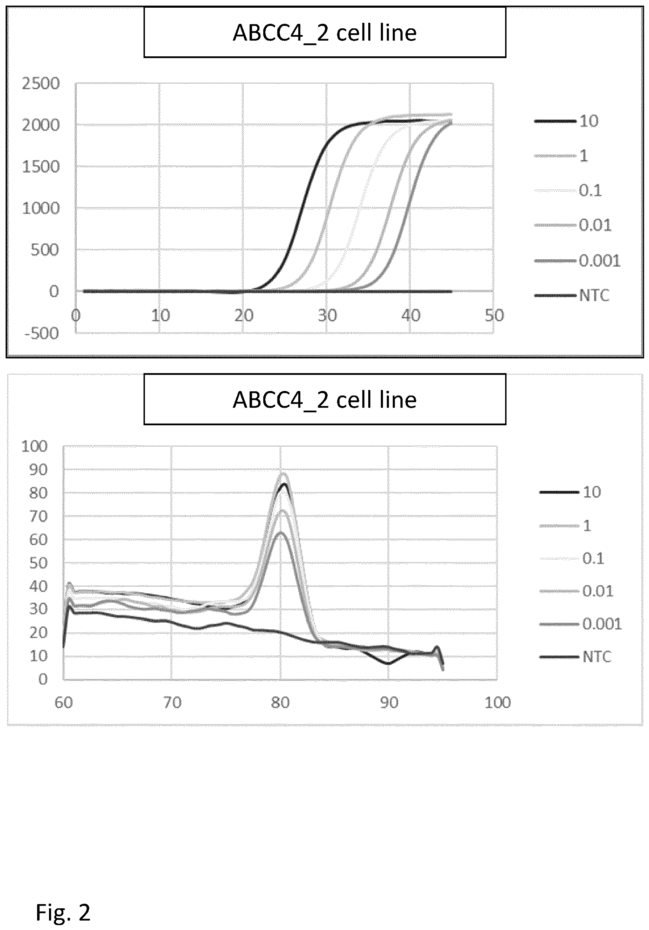

Fig. 2 depicts probe fluorescence of the ABCC4_2 assay (top panel) using different amounts of starting material (cell line derived RNA). The vertical axis depicts relative fluorescence units (RFU), the horizontal axis depicts the cycle number of the qPCR protocol. The bottom panel depicts the melting curves for the same conditions. The vertical axis depicts the change of relative fluorescence units over time (-d(RFU)/dT), the horizontal axis depicts the temperature in degrees Celsius.

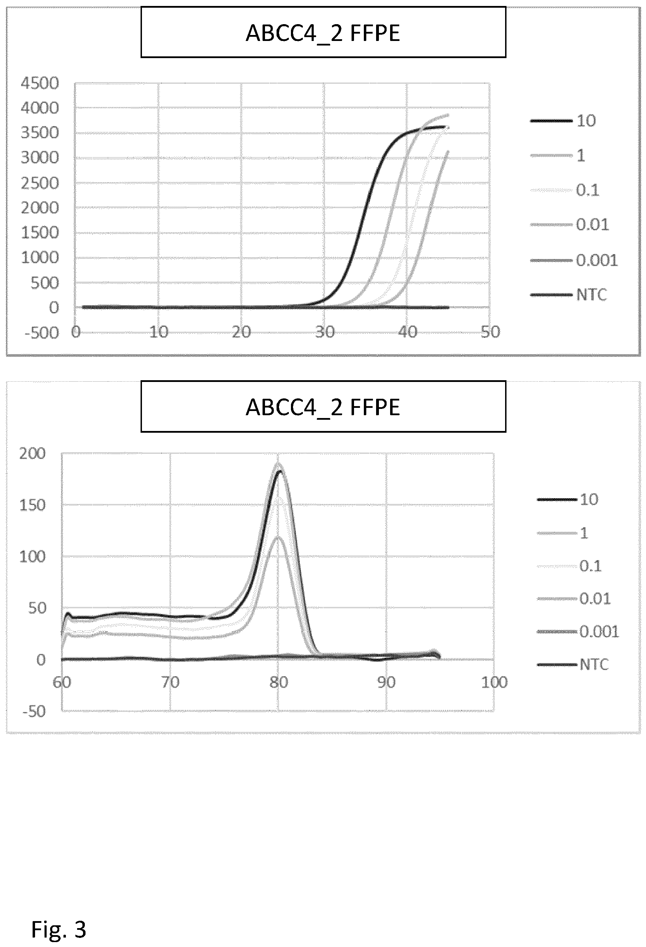

Fig. 3 depicts probe fluorescence of the ABCC4_2 assay (top panel) using different amounts of starting material (PPFE sample derived RNA). The vertical axis depicts relative fluorescence units (RFU), the horizontal axis depicts the cycle number of the qPCR protocol. The bottom panel depicts the melting curves for the same conditions. The vertical axis depicts the change of relative fluorescence units over time (-d(RFU)/dT), the horizontal axis depicts the temperature in degrees Celsius.

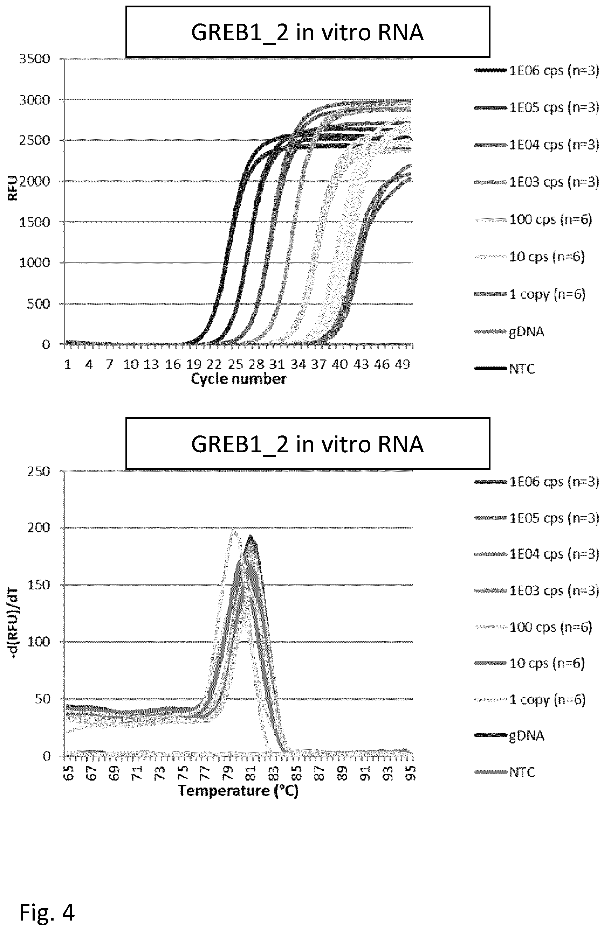

Fig. 4 depicts probe fluorescence of the GREB1_2 assay (top panel) using different concentrations of target RNA. The vertical axis depicts relative fluorescence units (RFU), the horizontal axis depicts the cycle number of the qPCR protocol. The bottom panel depicts the melting curves for the same conditions. The vertical axis depicts the change of relative fluorescence units over time (-d(RFU)/dT), the horizontal axis depicts the temperature in degrees Celsius.

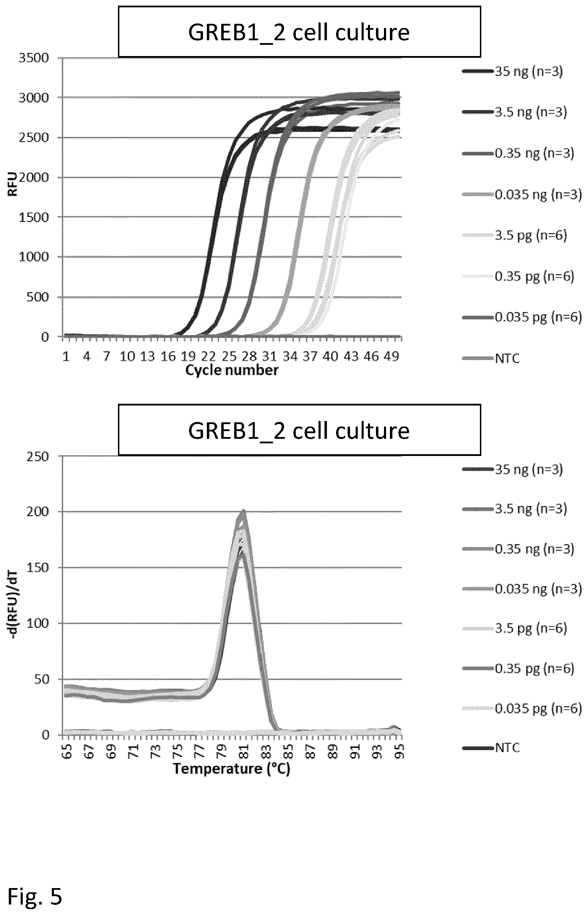

Fig. 5 depicts probe fluorescence of the GREB1_2 assay (top panel) using different amounts of starting material (cell line derived RNA). The vertical axis depicts relative fluorescence units (RFU), the horizontal axis depicts the cycle number of the qPCR protocol. The bottom panel depicts the melting curves for the same conditions. The vertical axis depicts the change of relative fluorescence units over time (-d(RFU)/dT), the horizontal axis depicts the temperature in degrees Celsius.

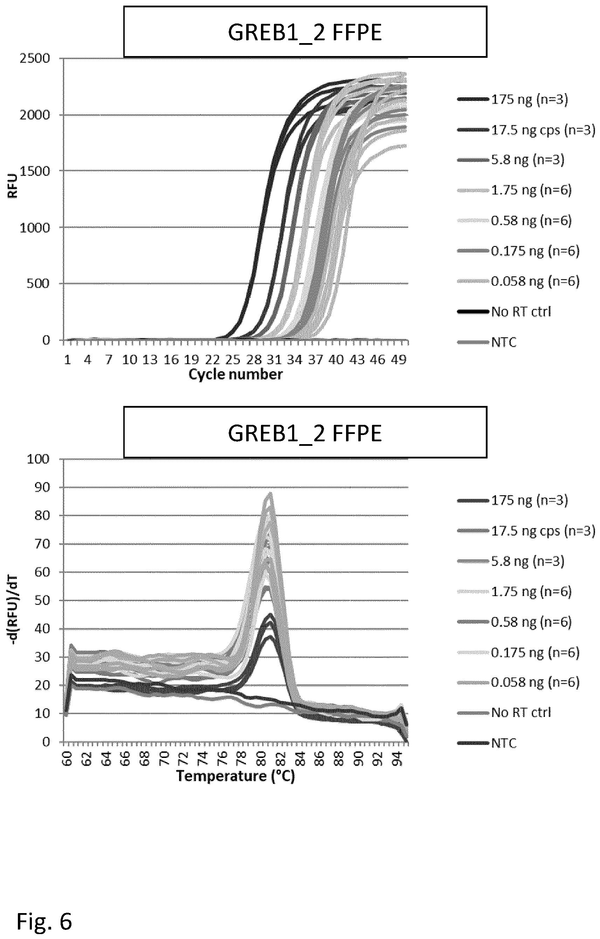

Fig. 6 depicts probe fluorescence of the GREB1_2 assay (top panel) using different amounts of starting material (PPFE sample derived RNA). The vertical axis depicts relative fluorescence units (RFU), the horizontal axis depicts the cycle number of the qPCR protocol. The bottom panel depicts the melting curves for the same conditions. The vertical axis depicts the change of relative fluorescence units over time (-d(RFU)/dT), the horizontal axis depicts the temperature in degrees Celsius.

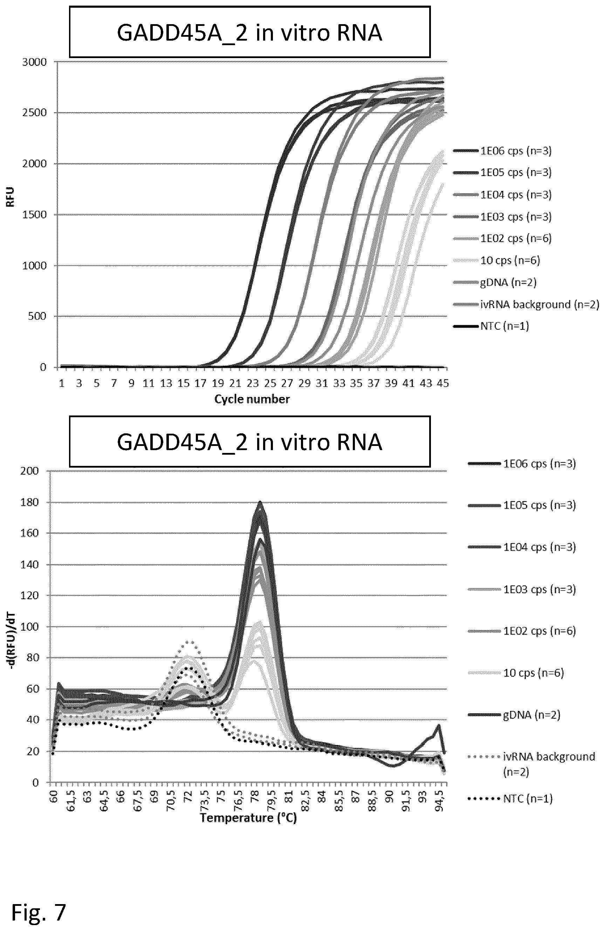

Fig. 7 depicts probe fluorescence of the GADD45A_2 assay (top panel) using different concentrations of target RNA. The vertical axis depicts relative fluorescence units (RFU), the horizontal axis depicts the cycle number of the qPCR protocol. The bottom panel depicts the melting curves for the same conditions. The vertical axis depicts the change of relative fluorescence units over time (-d(RFU)/dT), the horizontal axis depicts the temperature in degrees Celsius.

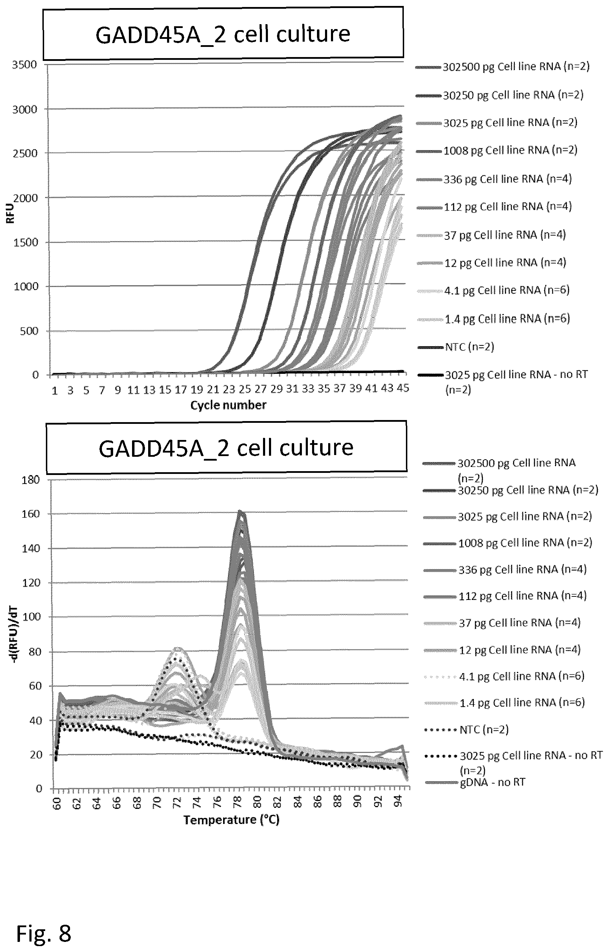

Fig. 8 depicts probe fluorescence of the GADD45A_2 assay (top panel) using different amounts of starting material (cell line derived RNA). The vertical axis depicts relative fluorescence units (RFU), the horizontal axis depicts the cycle number of the qPCR protocol. The bottom panel depicts the melting curves for the same conditions. The vertical axis depicts the change of relative fluorescence units over time (-d(RFU)/dT), the horizontal axis depicts the temperature in degrees Celsius.

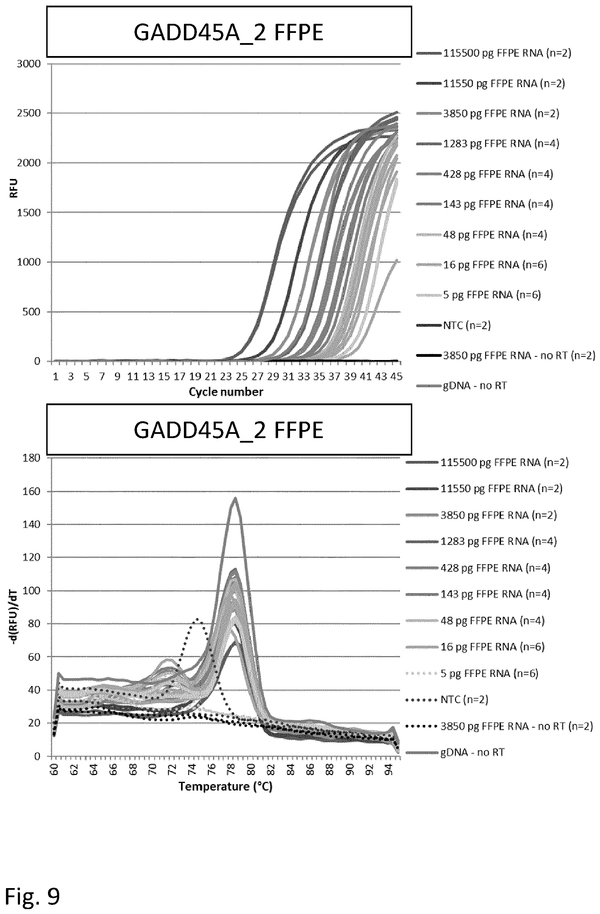

Fig. 9 depicts probe fluorescence of the GADD45A_2 assay (top panel) using different amounts of starting material (PPFE sample derived RNA). The vertical axis depicts relative fluorescence units (RFU), the horizontal axis depicts the cycle number of the qPCR protocol. The bottom panel depicts the melting curves for the same conditions. The vertical axis depicts the change of relative fluorescence units over time (-d(RFU)/dT), the horizontal axis depicts the temperature in degrees Celsius.

### EXAMPLES

### Example 1 design of forward and reverse primers and probes

Primers and probes for target genes of the following cellular signaling pathways were designed as described herein:
ER, AR, PI3K-FOXO, AP1-MAPK, Notch, HH and TGFbeta.

The designed primers and probes are listed below in Tables 1 to 7. Further, the same principle was used to design primers and probes to detect the expression levels of several reference genes, which are listed below in Table 8. All primers and probes were validated using the following conditions:

### medium conditions

50 mM monovalent salt;
400 nM forward primer
400 nM reverse primer
3.0 mM MgCl₂;
100 nM probe; and
0.8 mM dNTP

### Reaction reaction conditions:

a RT reaction at 50° Celsius for 30 minutes (using the reverse primer) in order to synthesize cDNA, followed by a 5 minute denaturation step at 95° Celsius, followed by 45 cycli of a 15 second denaturation step at 95° Celsius and a 30 second Elongation step at 60°Celsius.

All sets of primers and probes were found to amplify and detect the expression levels of the desired target genes at the indicated reaction conditions.

### Example 2 - validation of primers and probes

All primers and probes were validated under the following conditions:
A RT reaction at 50° Celsius for 30 minutes (using the reverse primer) in order to synthesize cDNA, followed by a 5 minute denaturation step at 95° Celsius, followed by 45 cycli of a 15 second denaturation step at 95° Celsius and a 30 second Elongation step at 60°Celsius, the reaction was performed on a qPCR device with fluorescence readout. Melt curves were determined in the range of 60°C to 95°C.

### Example 3 - Representative examples of primer/probe pairs

Some exemplary data sets for validated genes are provided below, although it is noted that all genes described below were validated.

Additional information is provided for the following assays: ABCC4 2, GREB1_2 and GADD45A 2, which comprise primers and probes for the amplification and detection of the respective genes ABCC4, GREB1 and GADD45A. Assay ABCC4_2 results in an amplification product of 82 nucleotides spanning an 11594 nucleotide intron. Assay GREB1_2 results in an amplification product of 105 nucleotides spanning an 9696 nucleotide intron. Assay GADD45A_2 results in an amplification product of 82 nucleotides spanning an 1037 nucleotide intron.

Some general information on the assays is listed in Table 9 below.

In order to validate the assays, they are tested on a Bio-Rad CFX96 Touch Real-Time PCR Detection System. The assays were tested on in vitro RNA, cell culture isolate RNA and RNA isolated from a formalin fixed paraffin embedded (FFPE) sample, to test their suitability. For all tests the probes were tested on decreasing amounts of material (RNA) in order to test their efficiency.

Real-time probe fluorescence curves were generated for all tests performed, as well temperature melt curves over a range of 60 to 95 degrees Celsius. These data are depicting in figures 1 to 9 for the three exemplary genes ABCC4, GREB1 and GADD45A. In each figure, the top panel depicts the probe fluoresce intensity and the bottom panel the melt curves.

Most probes and conditions resulted in a probe efficiency of over 90%, indicating the probes are suitable for quantitative measurements of target gene expression levels.

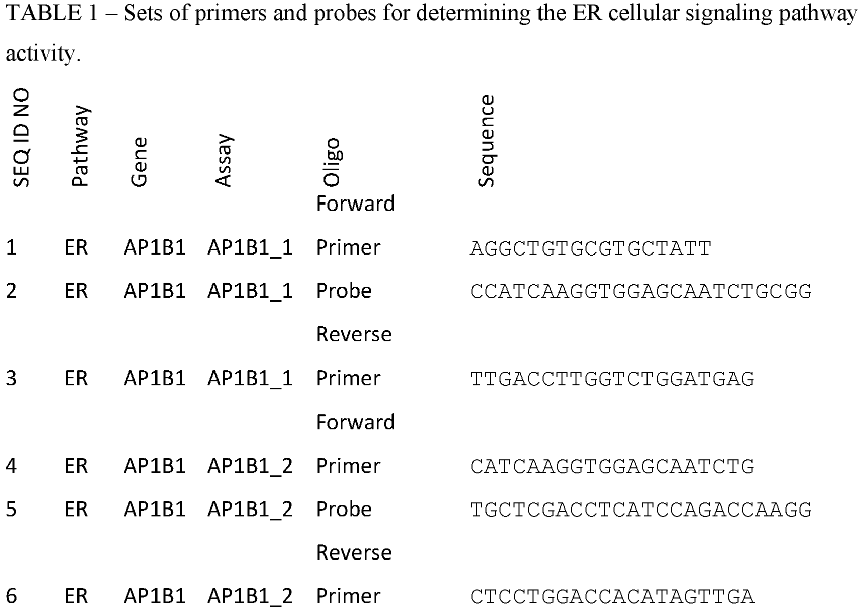

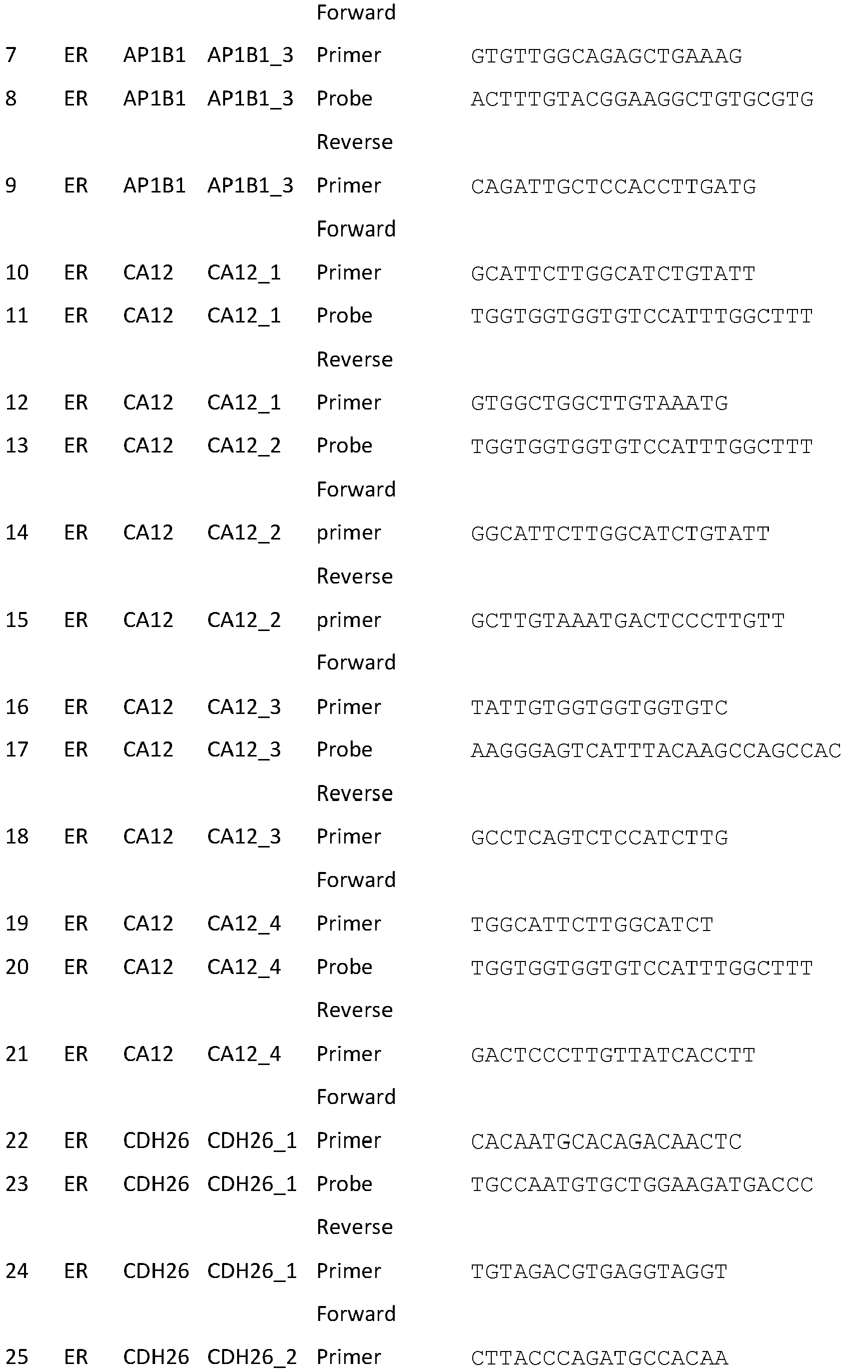

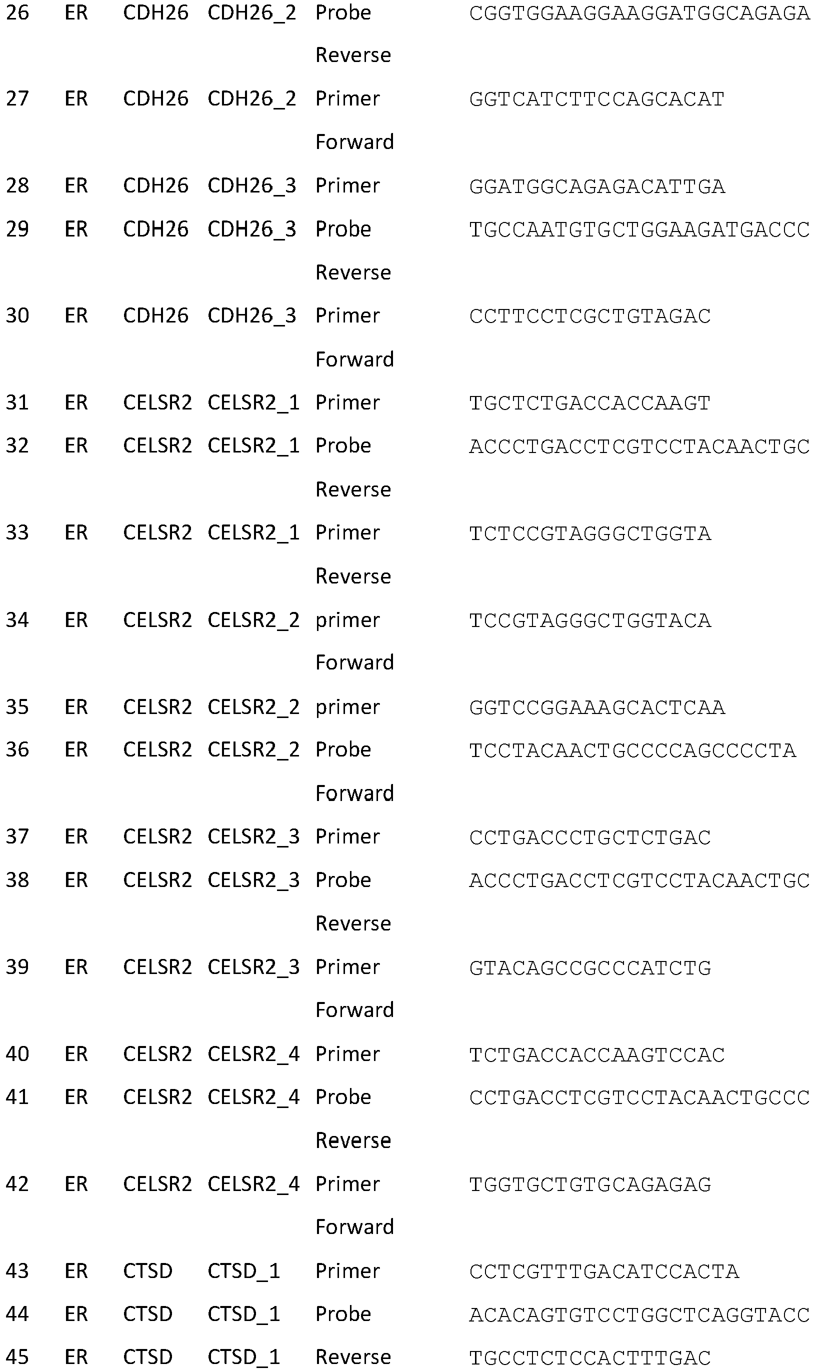

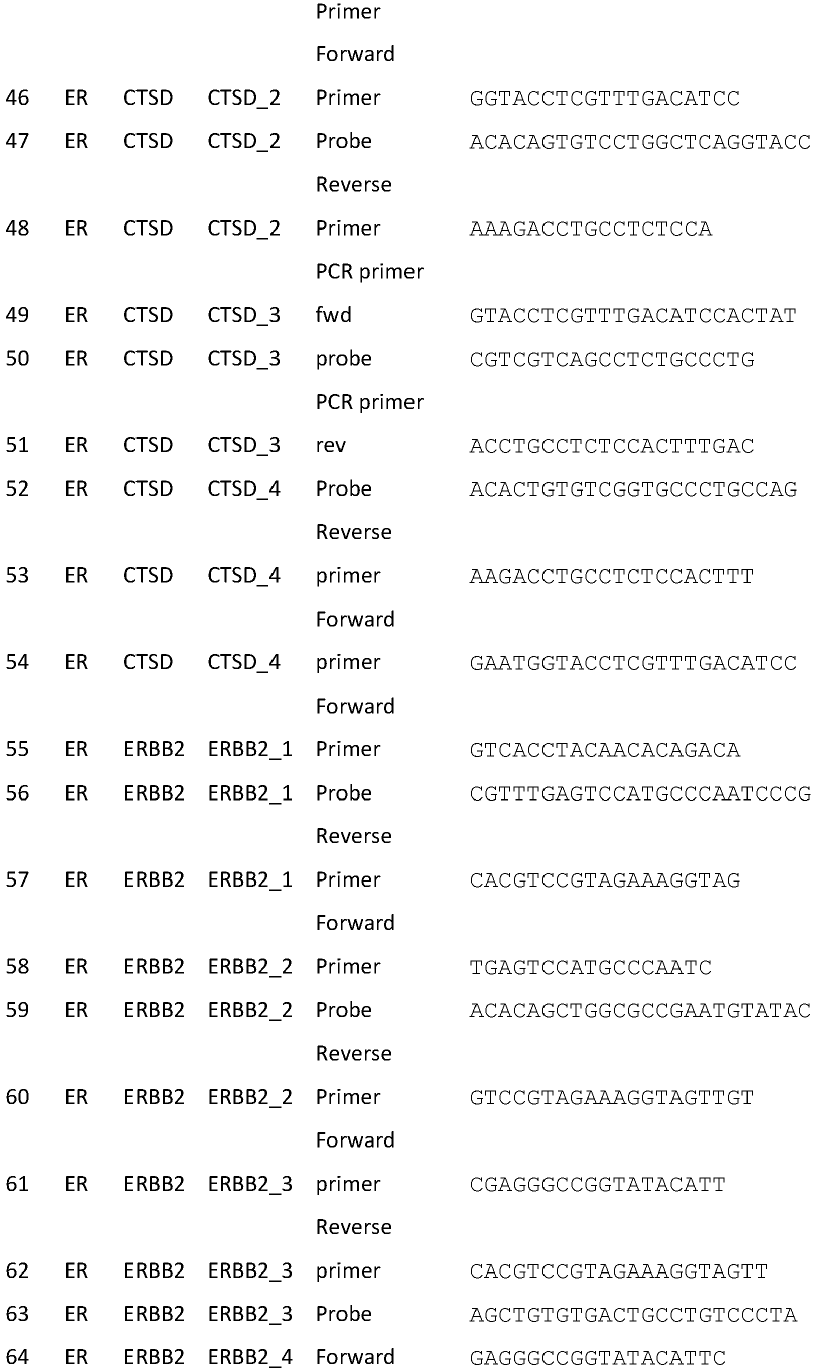

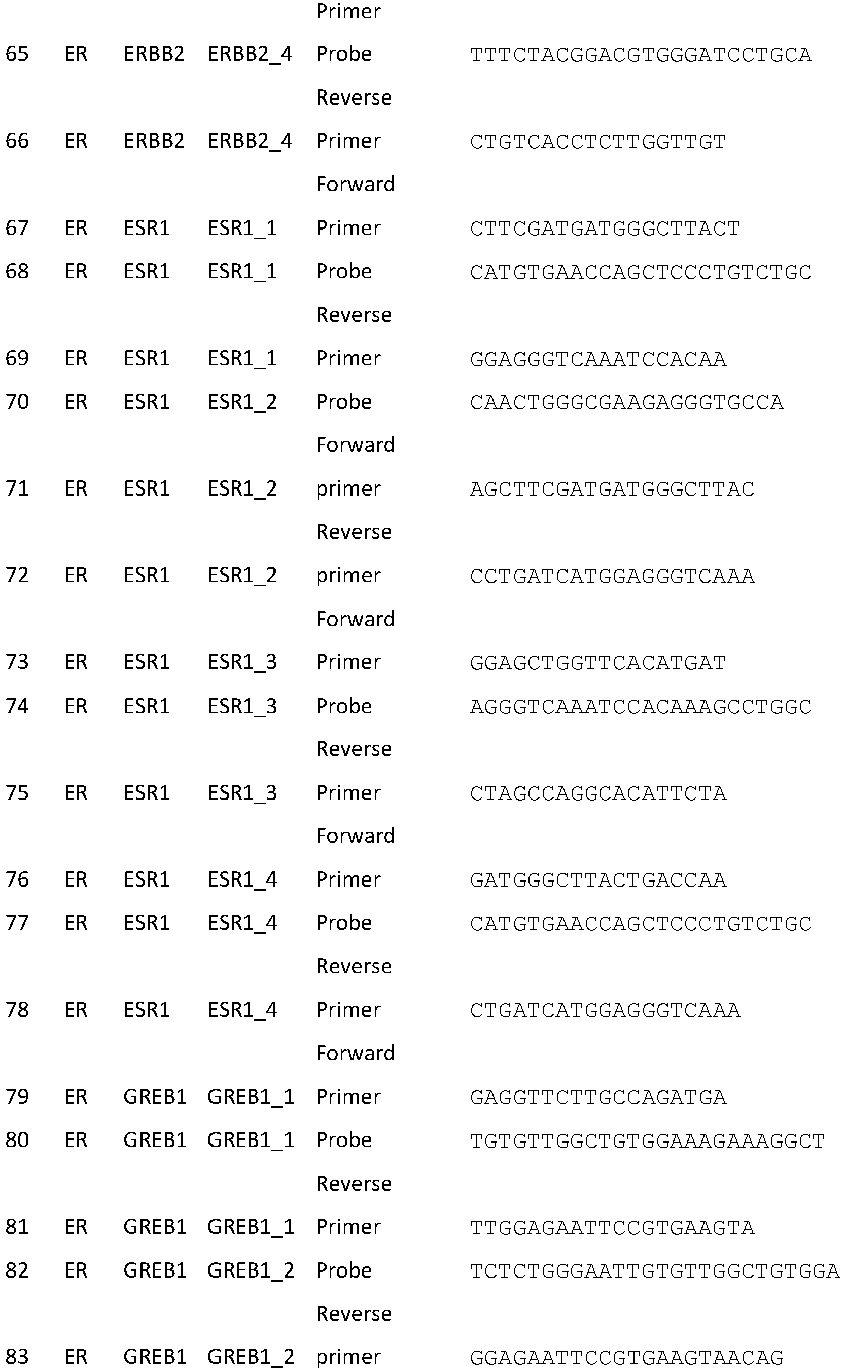

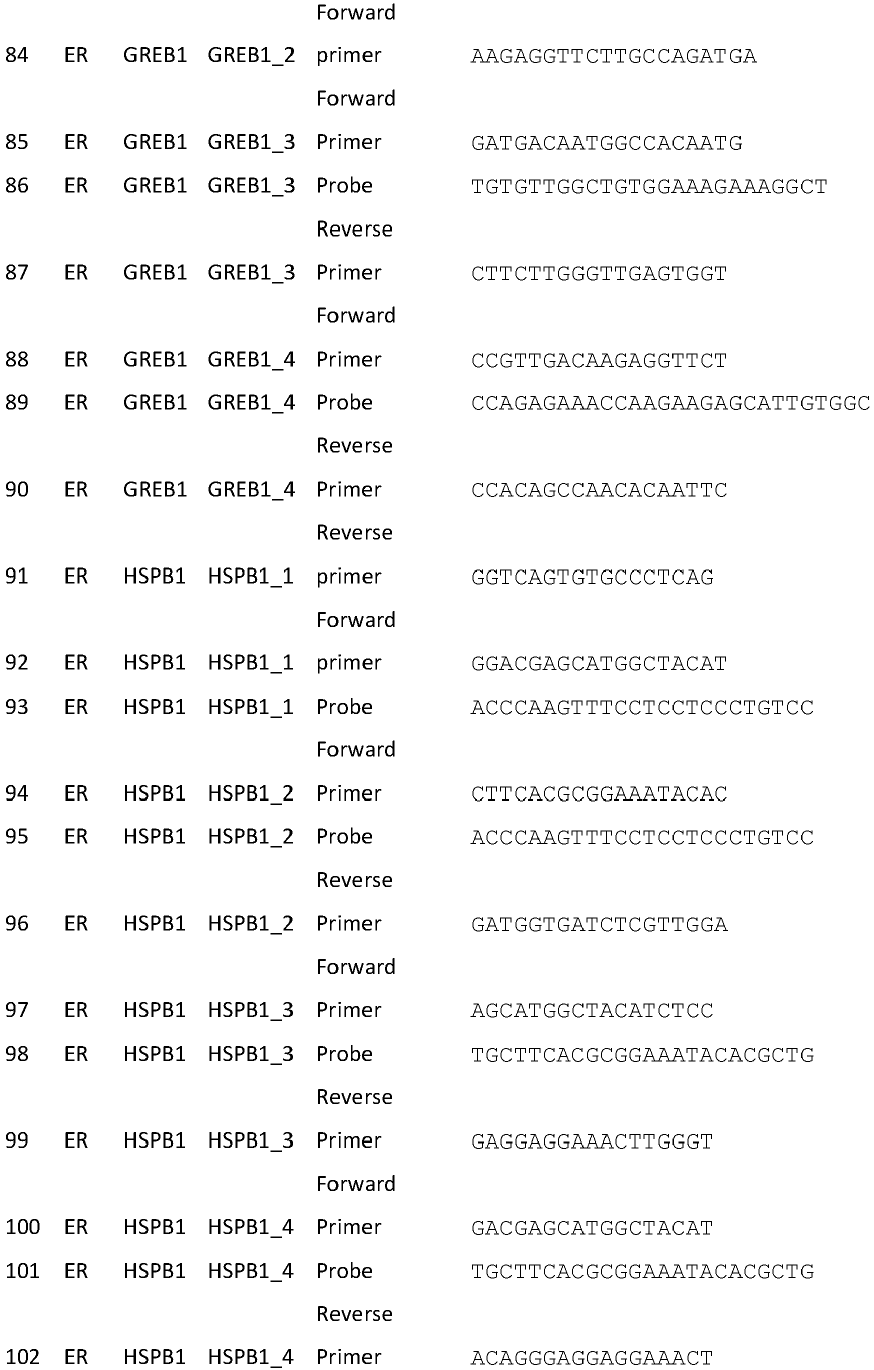

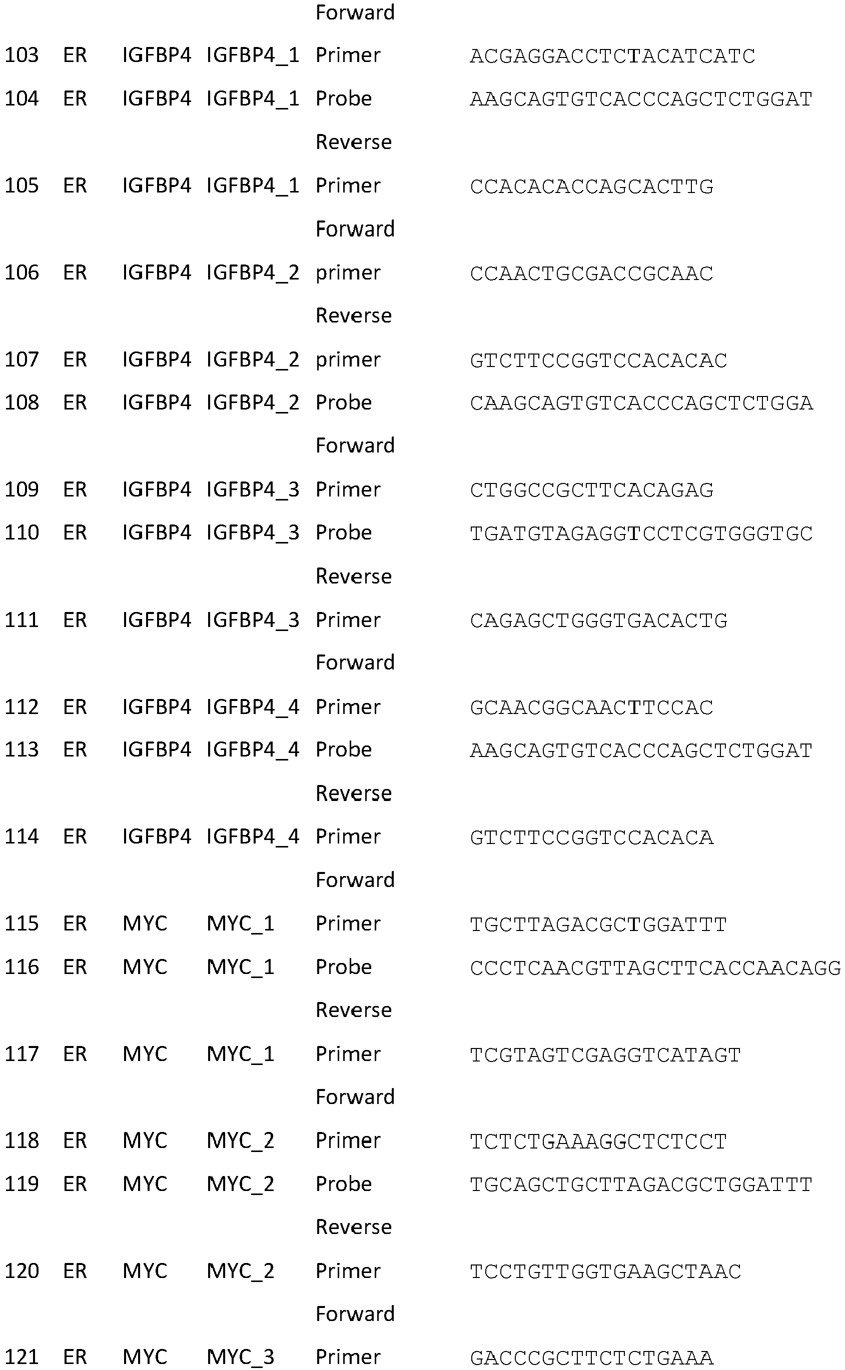

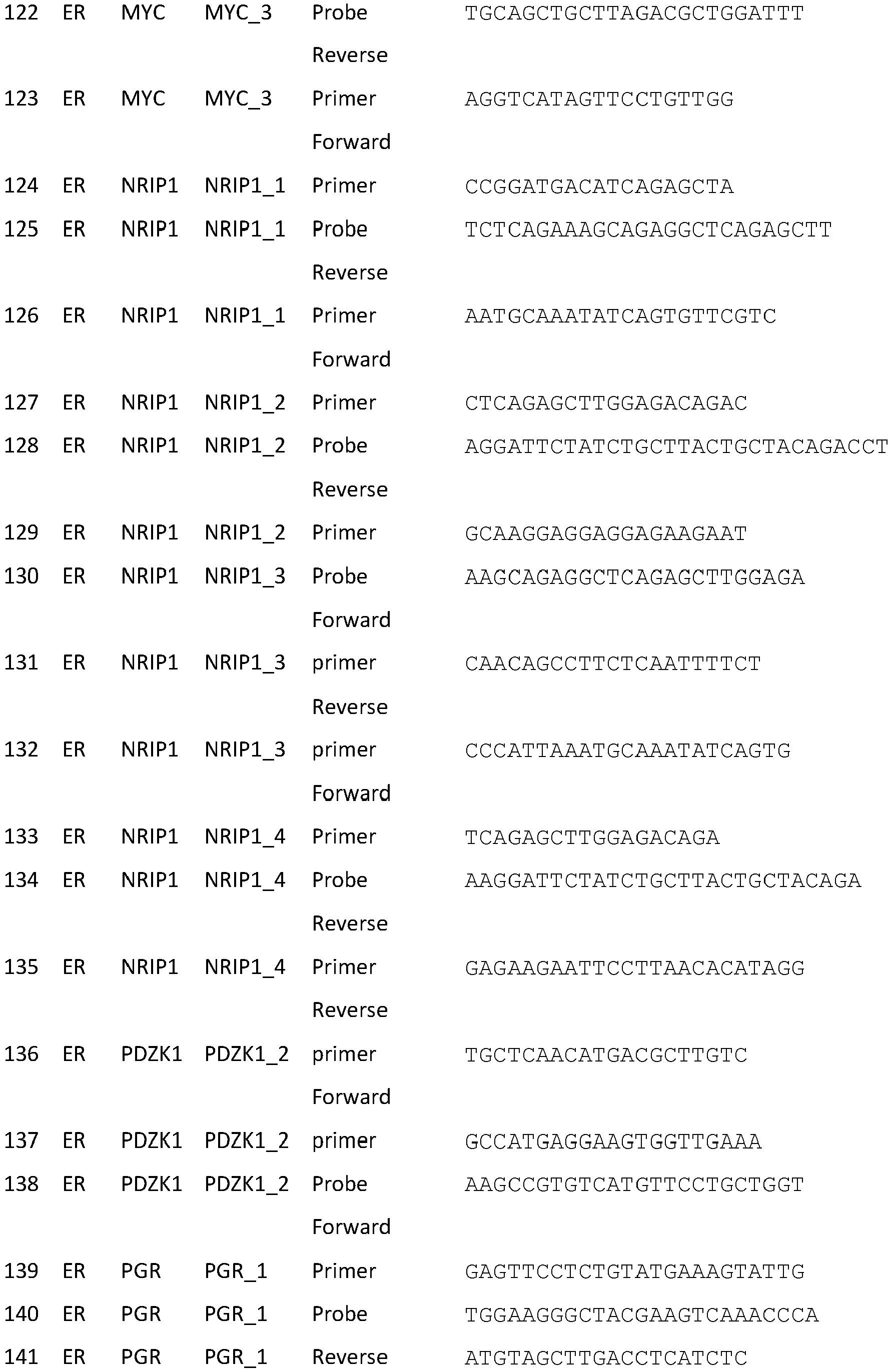

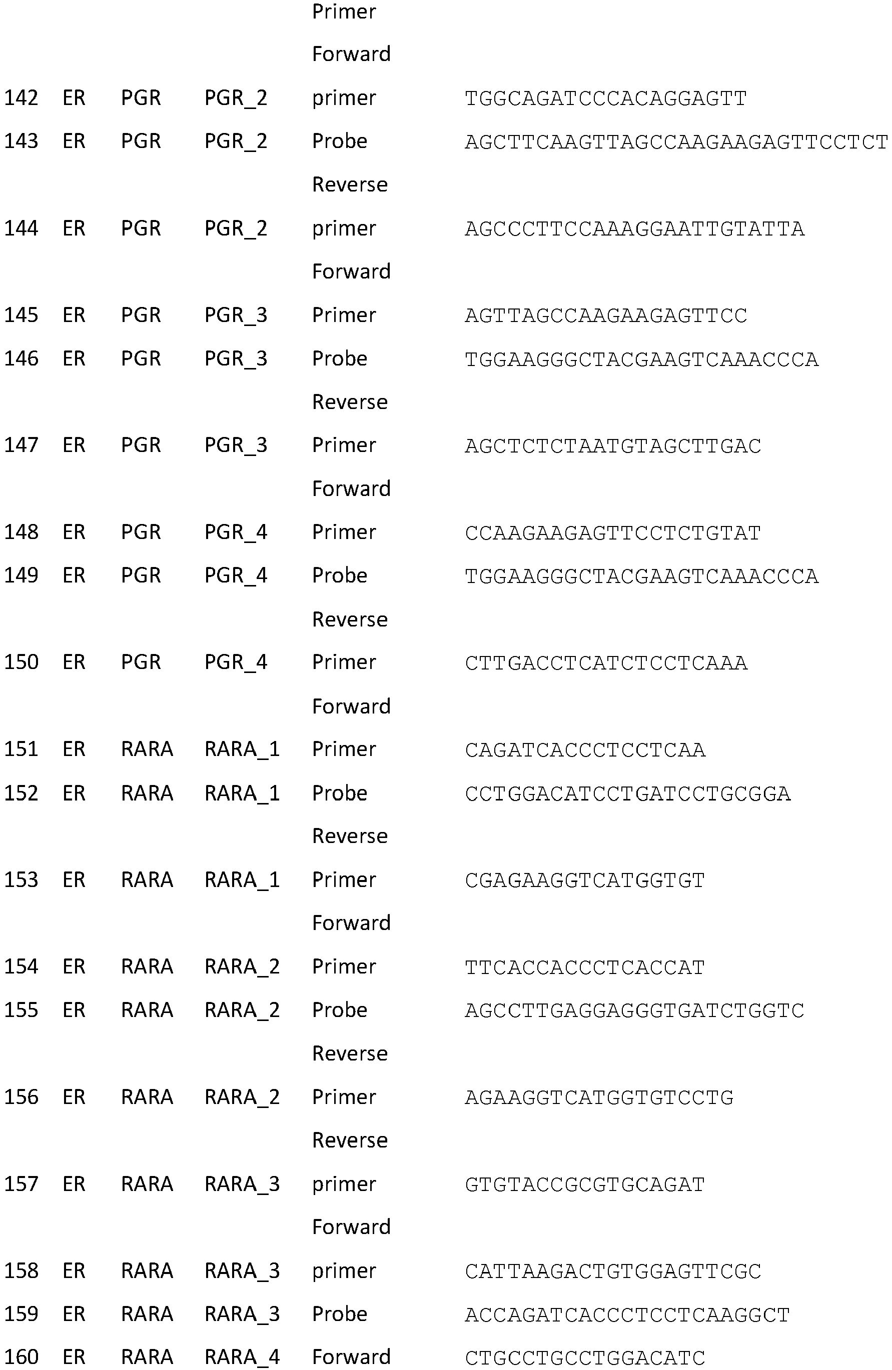

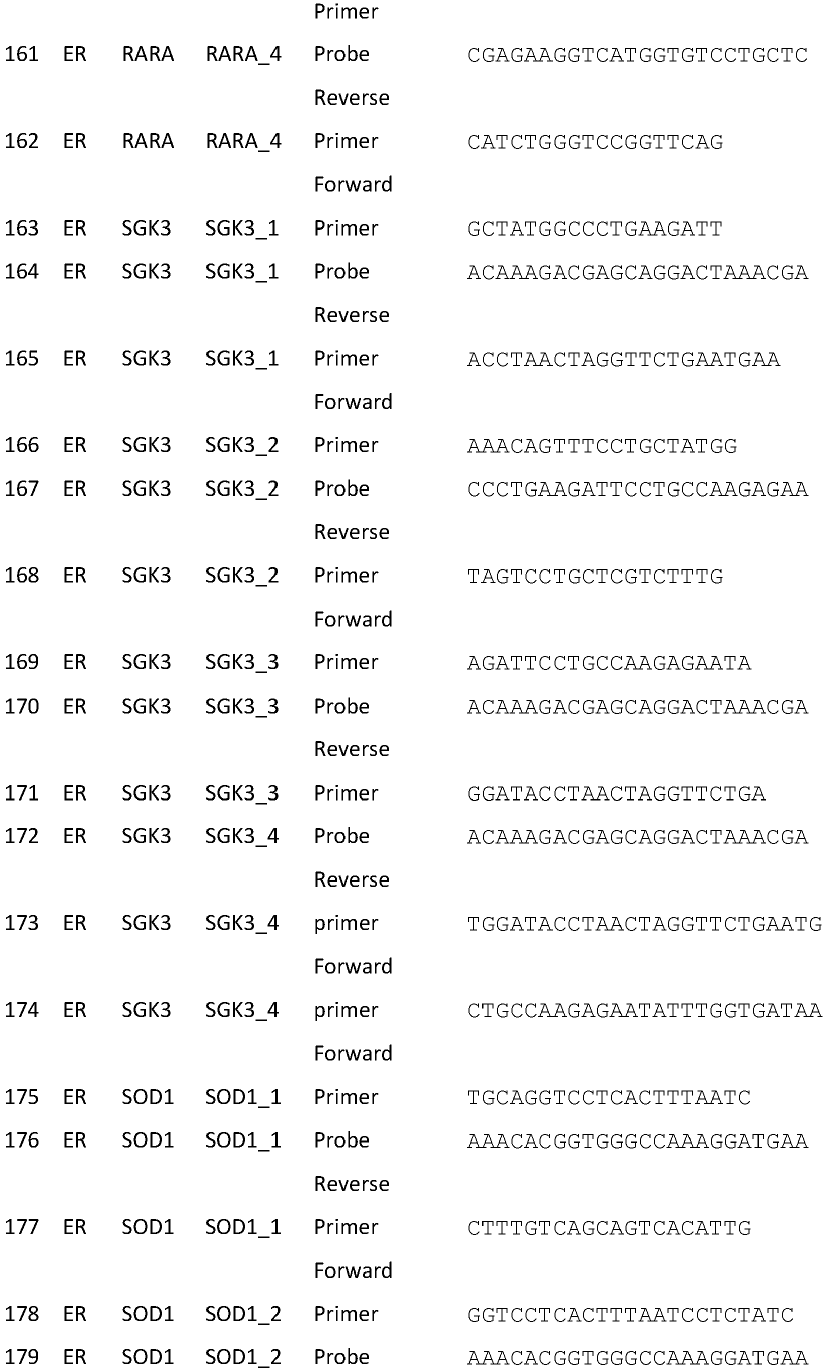

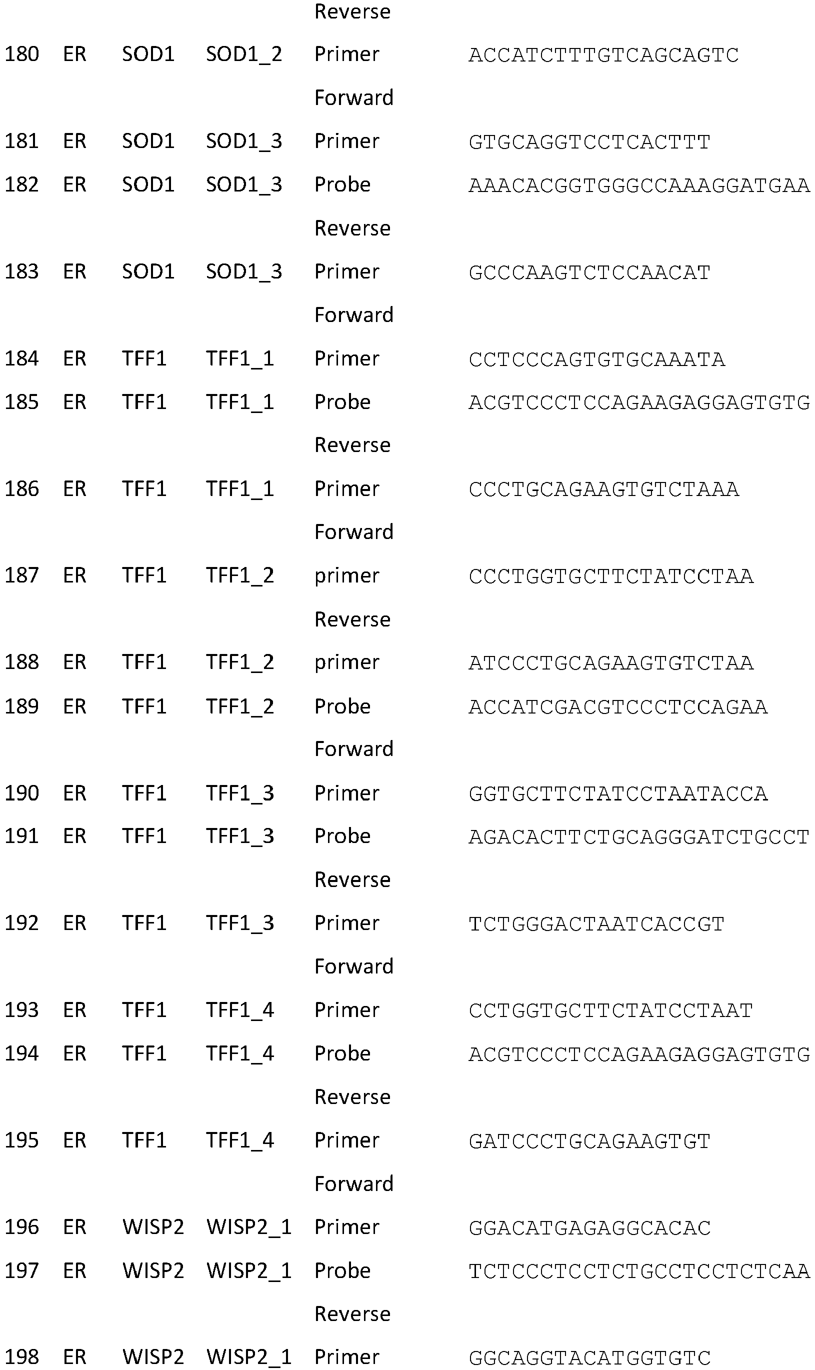

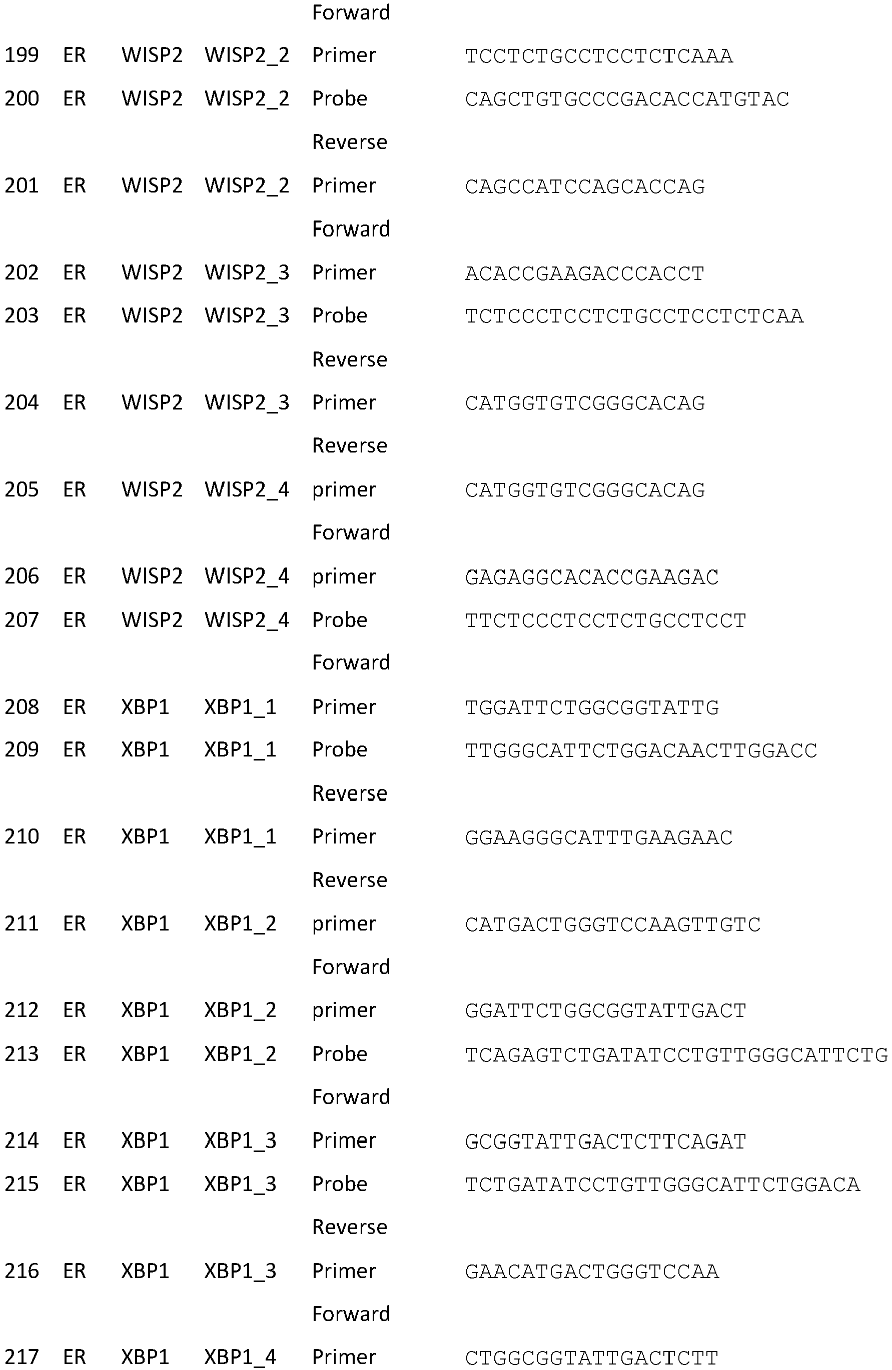

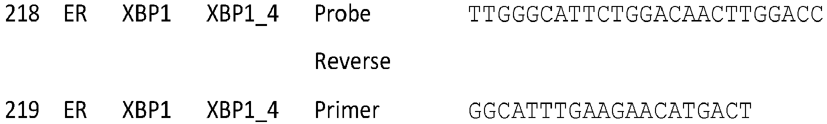

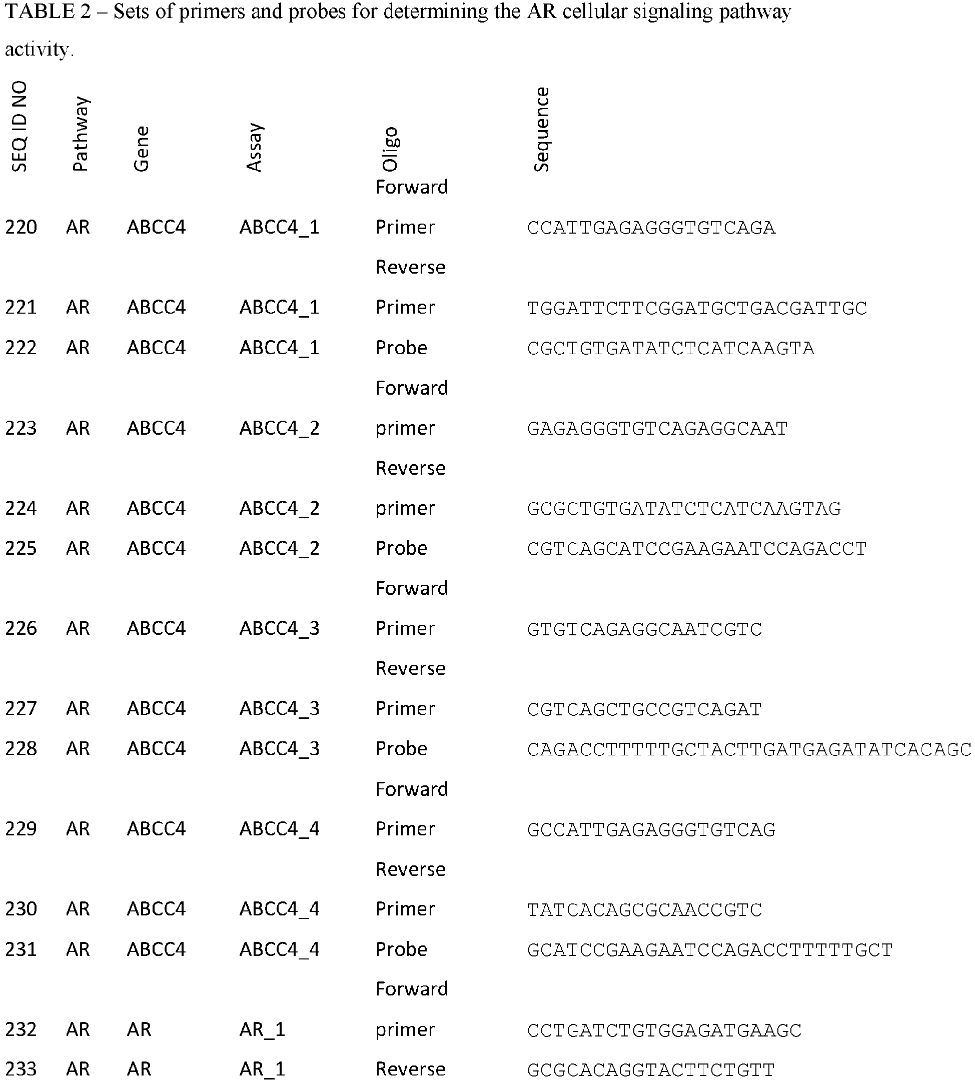

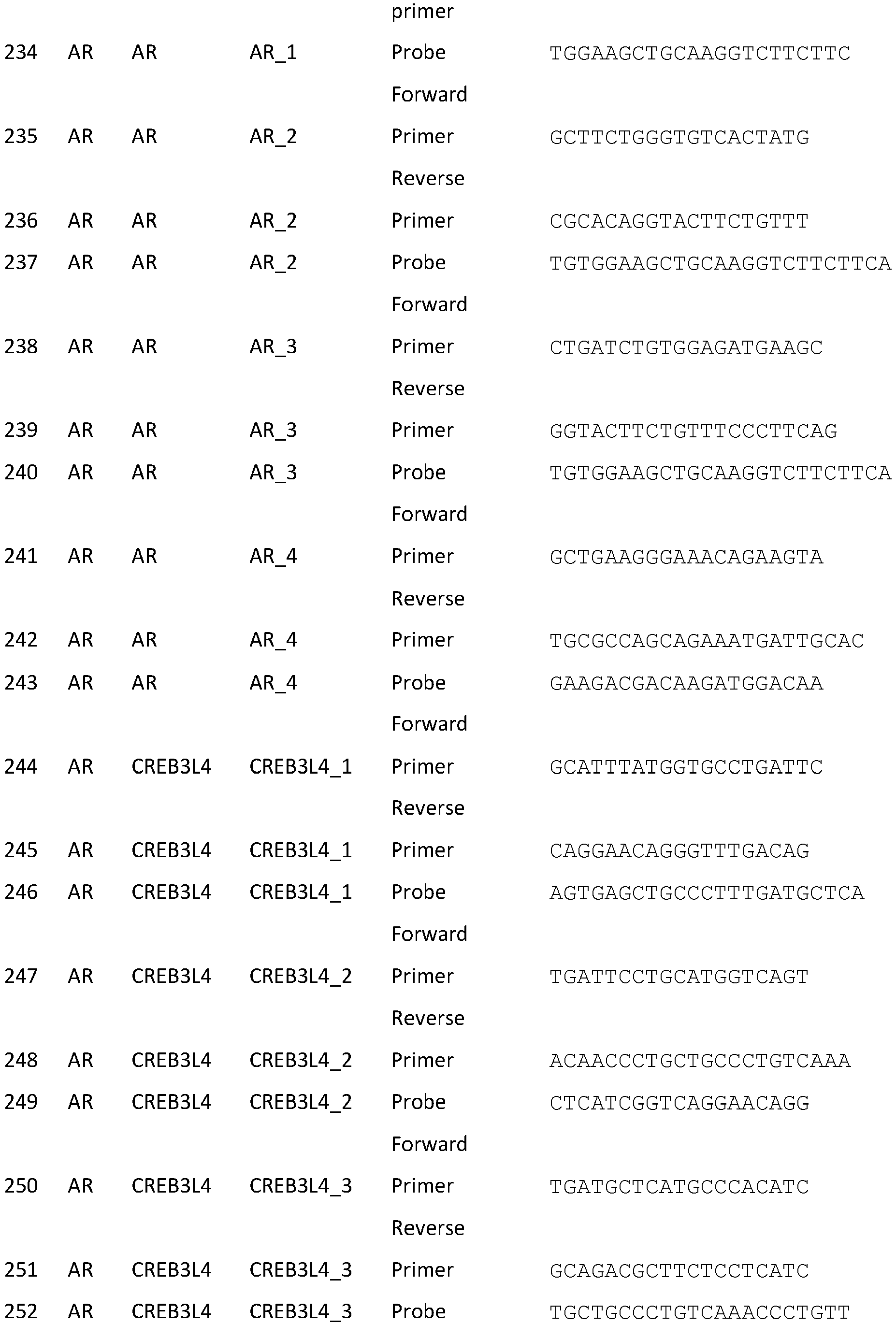

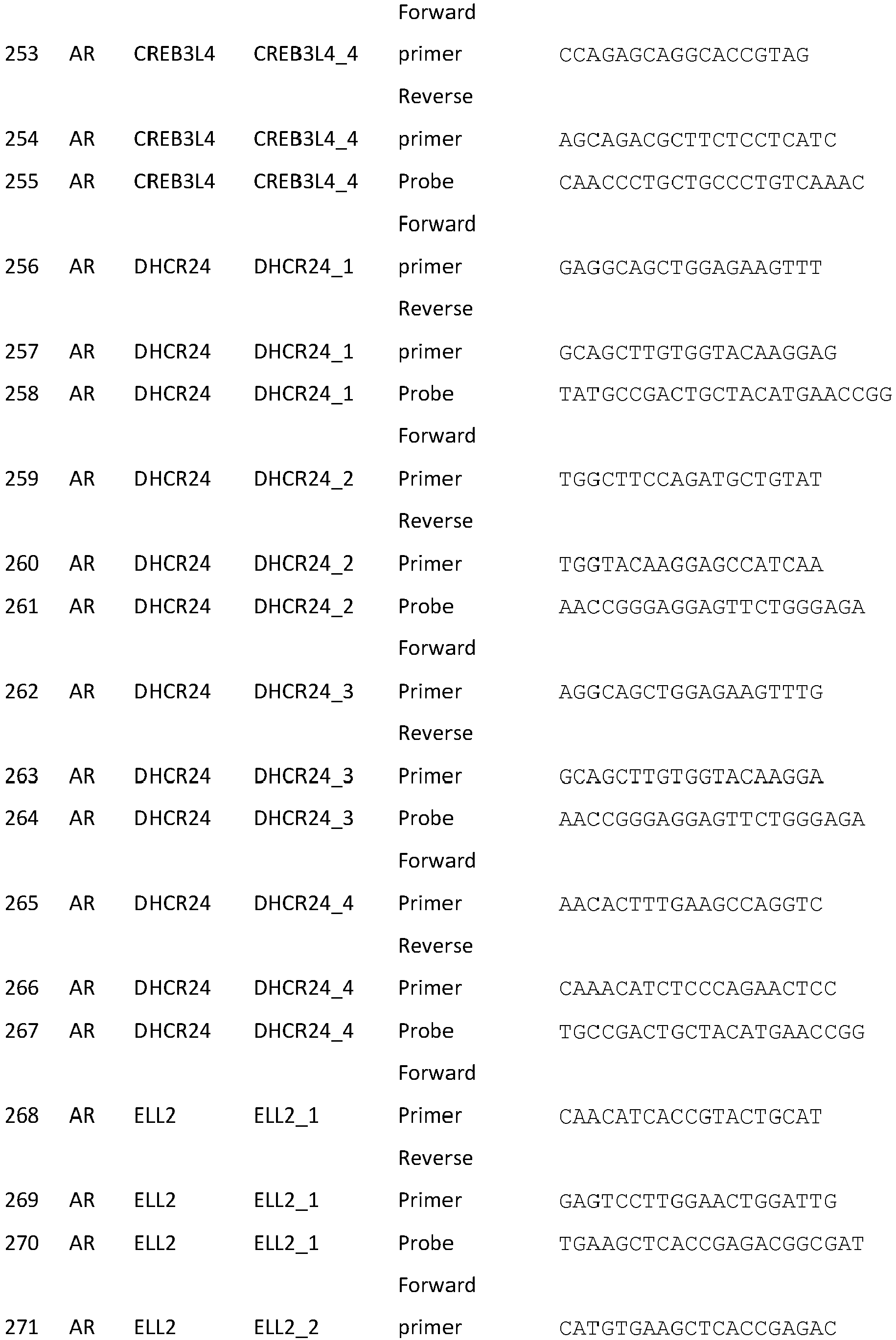

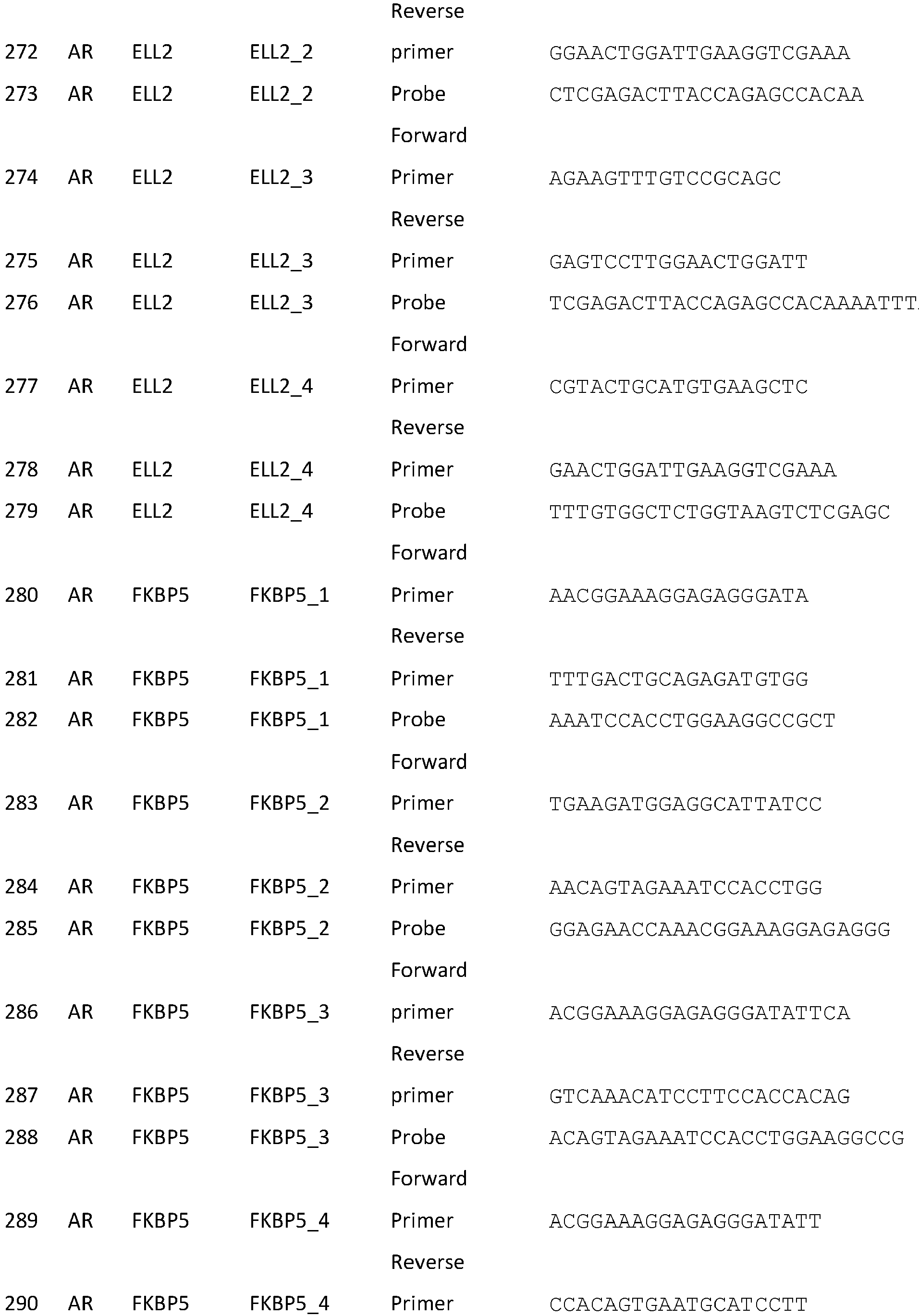

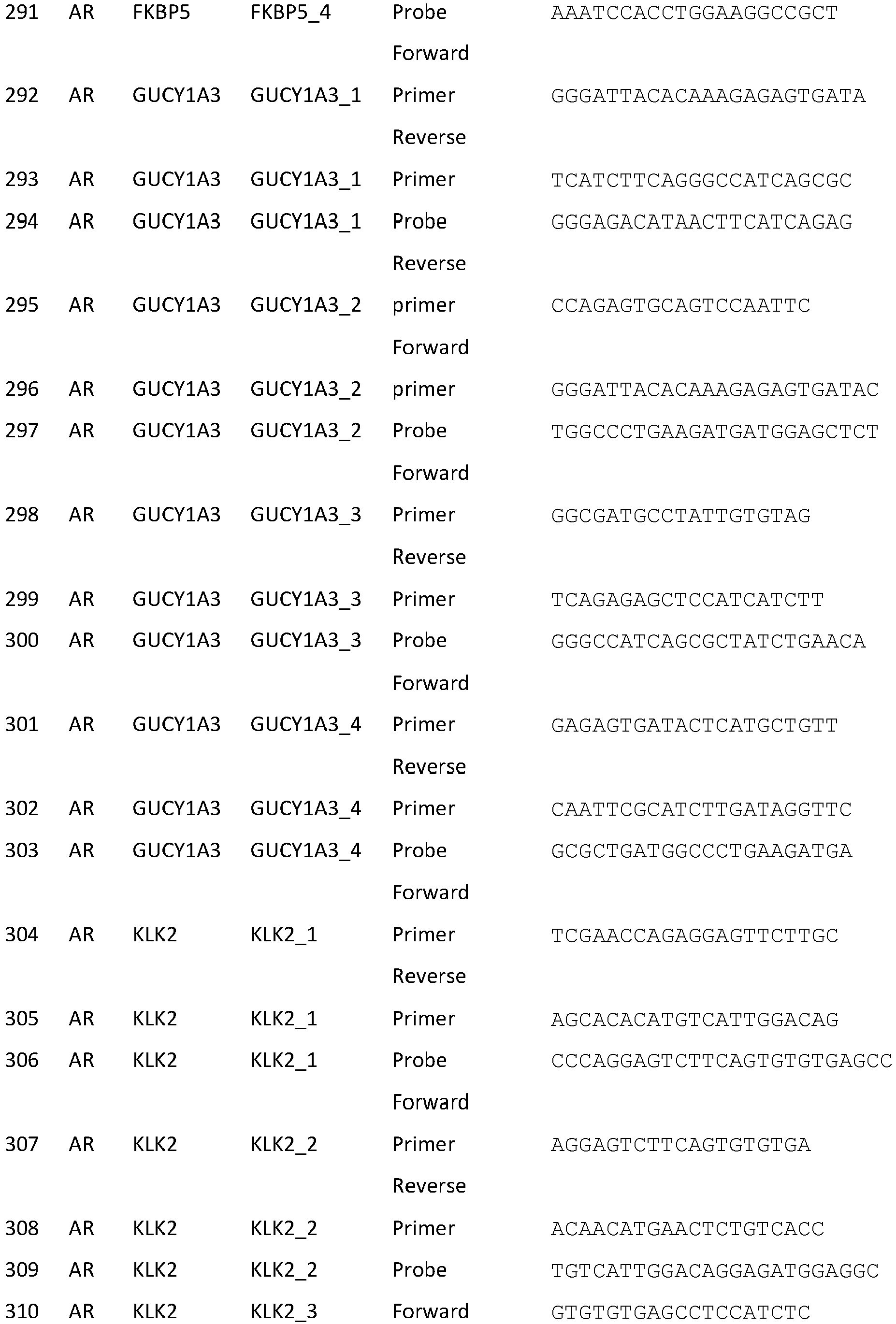

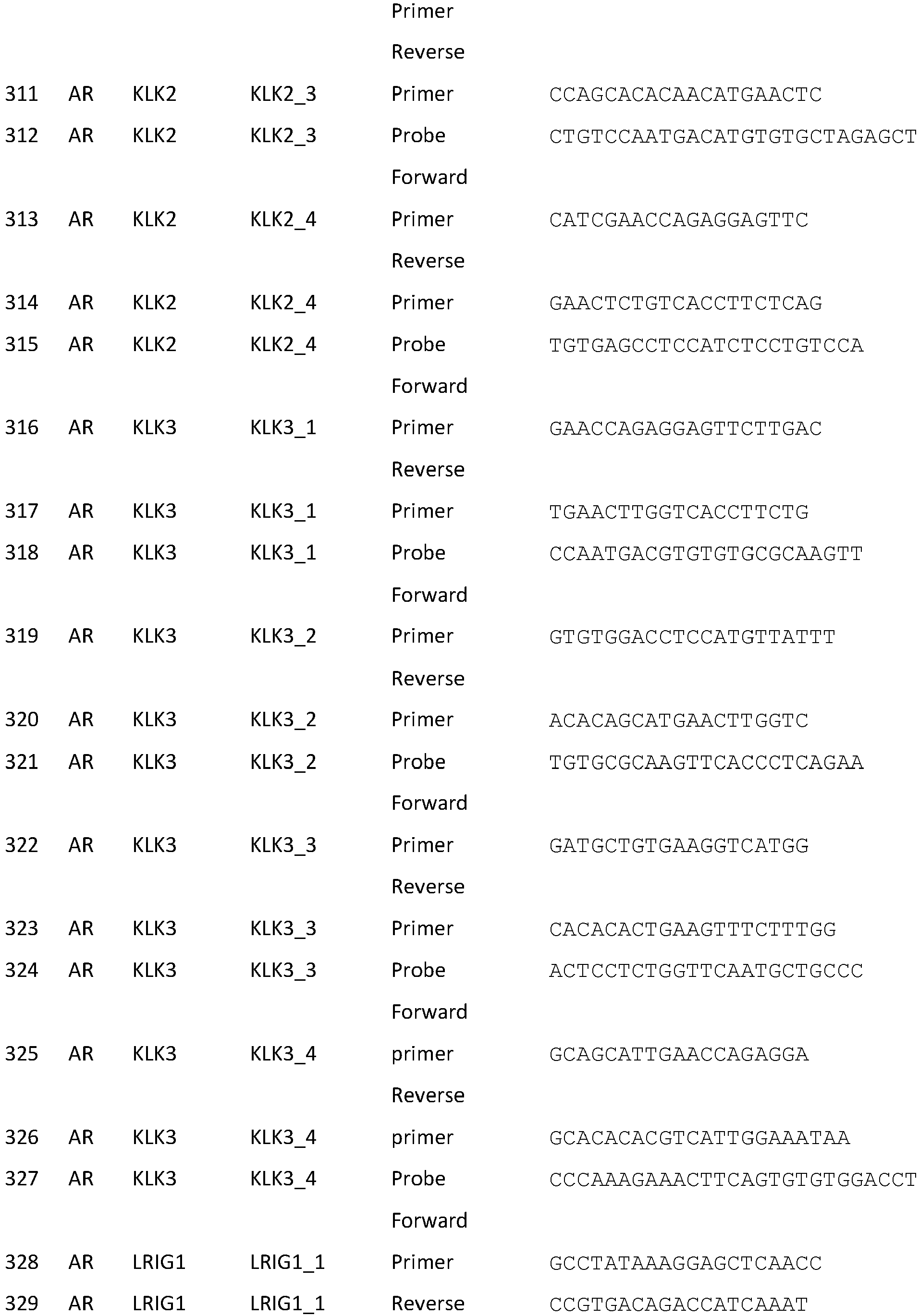

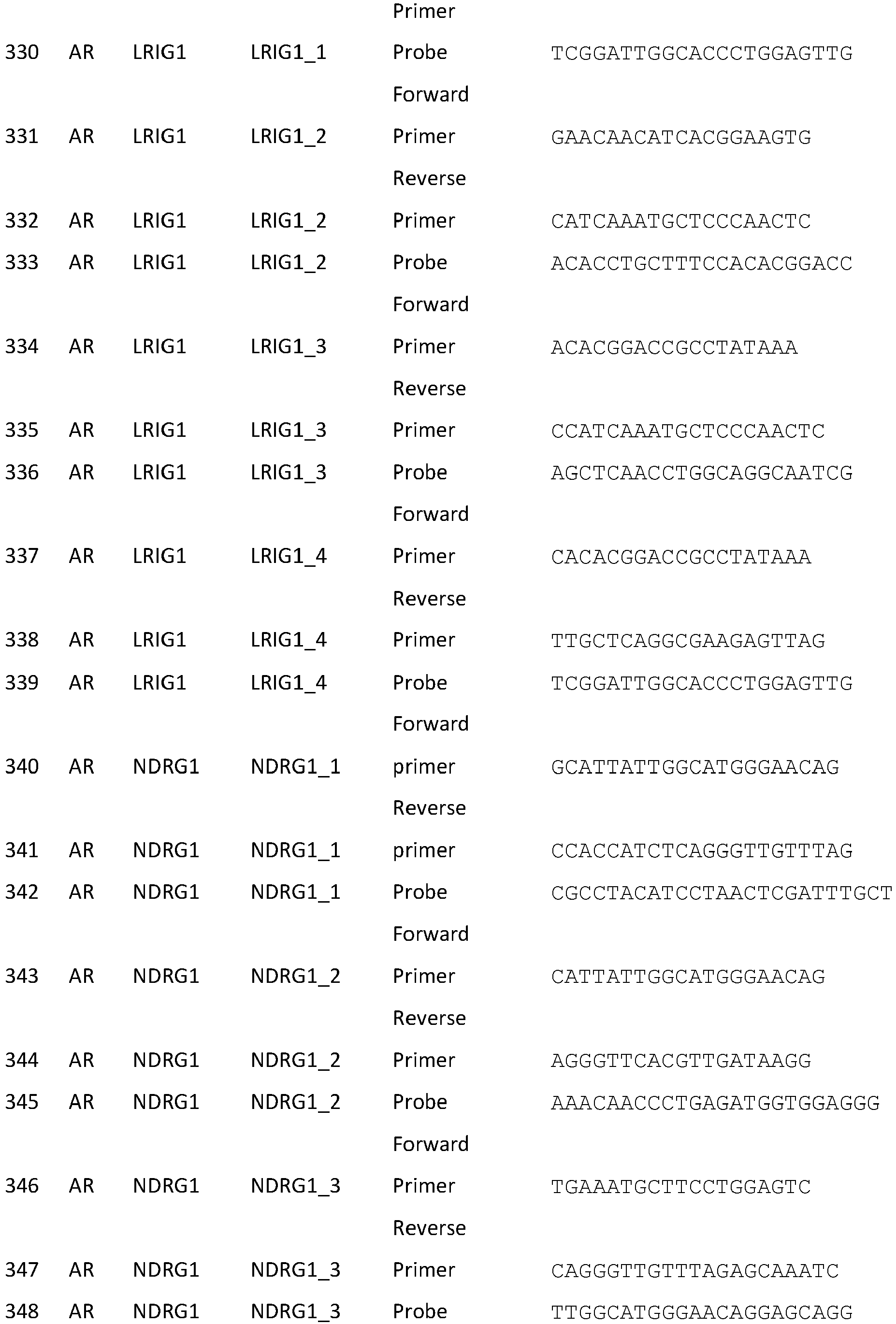

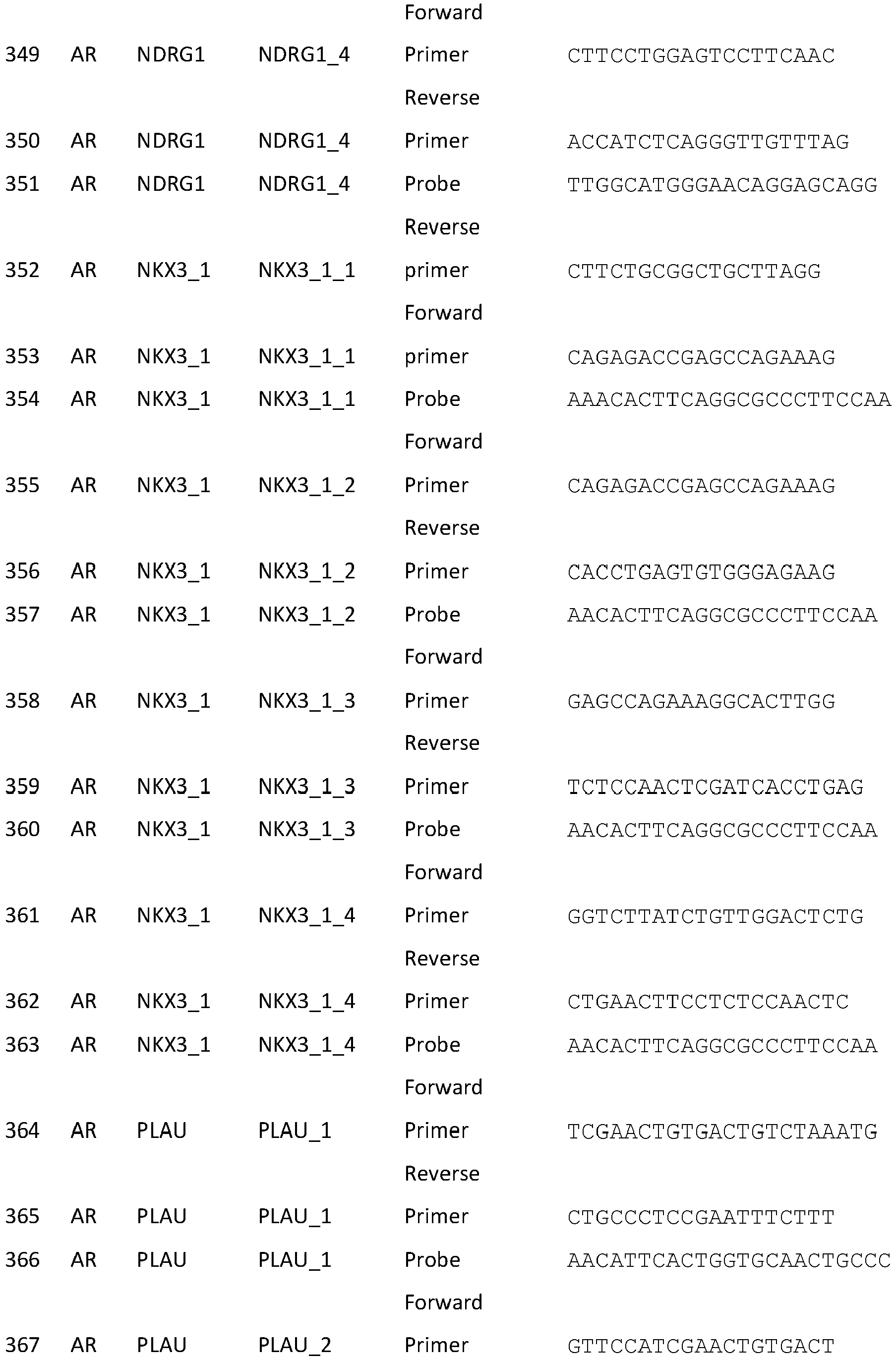

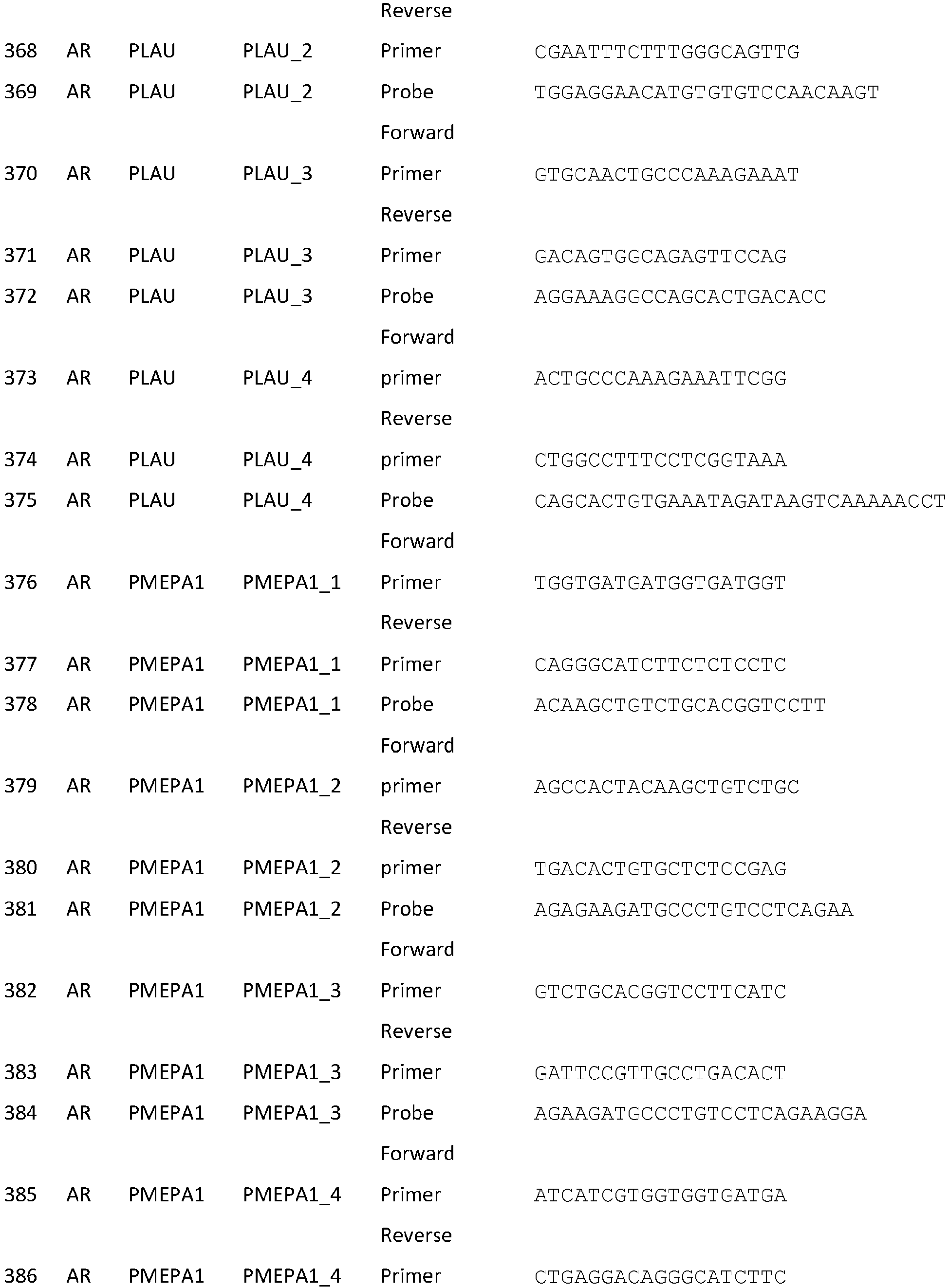

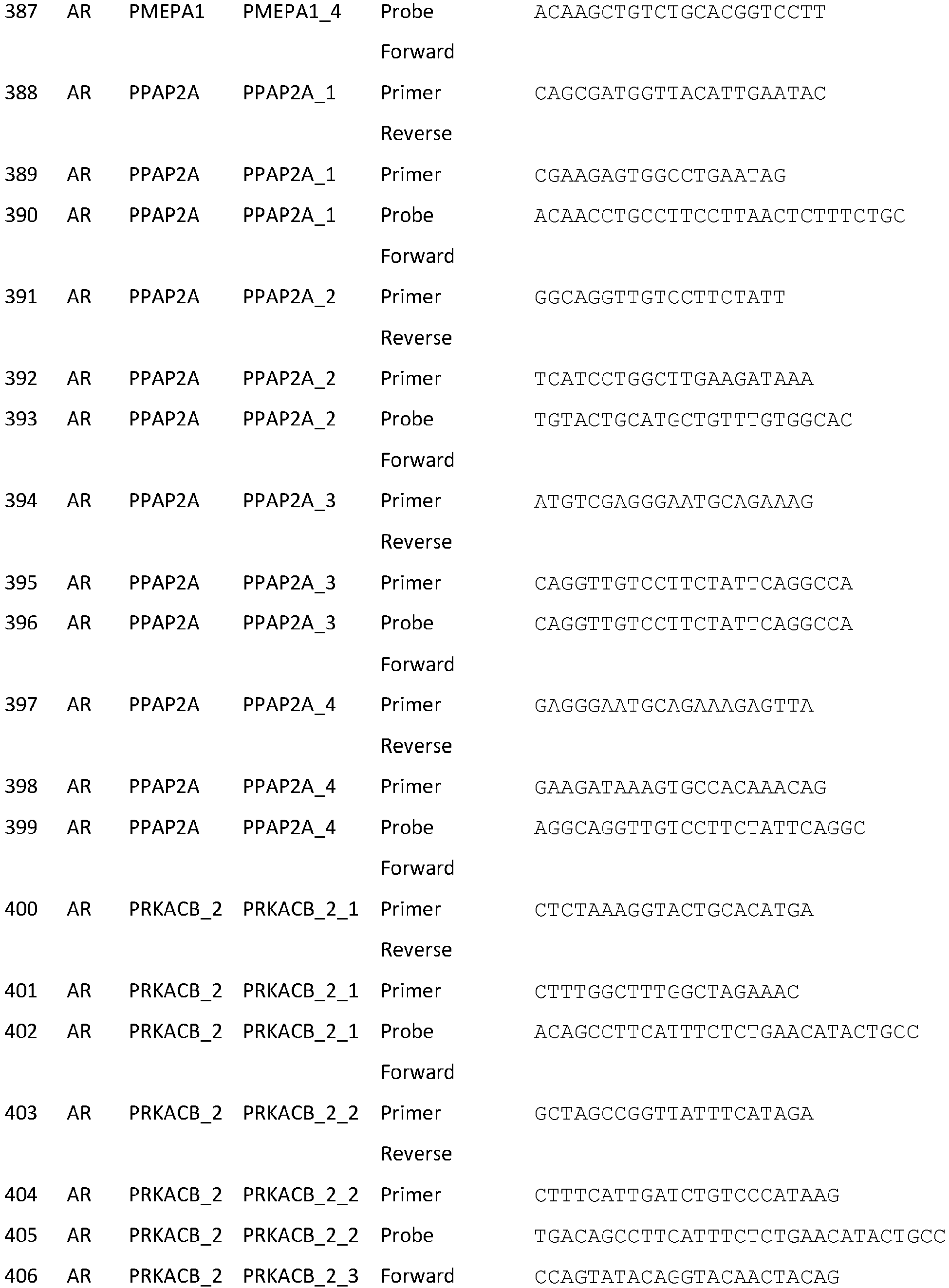

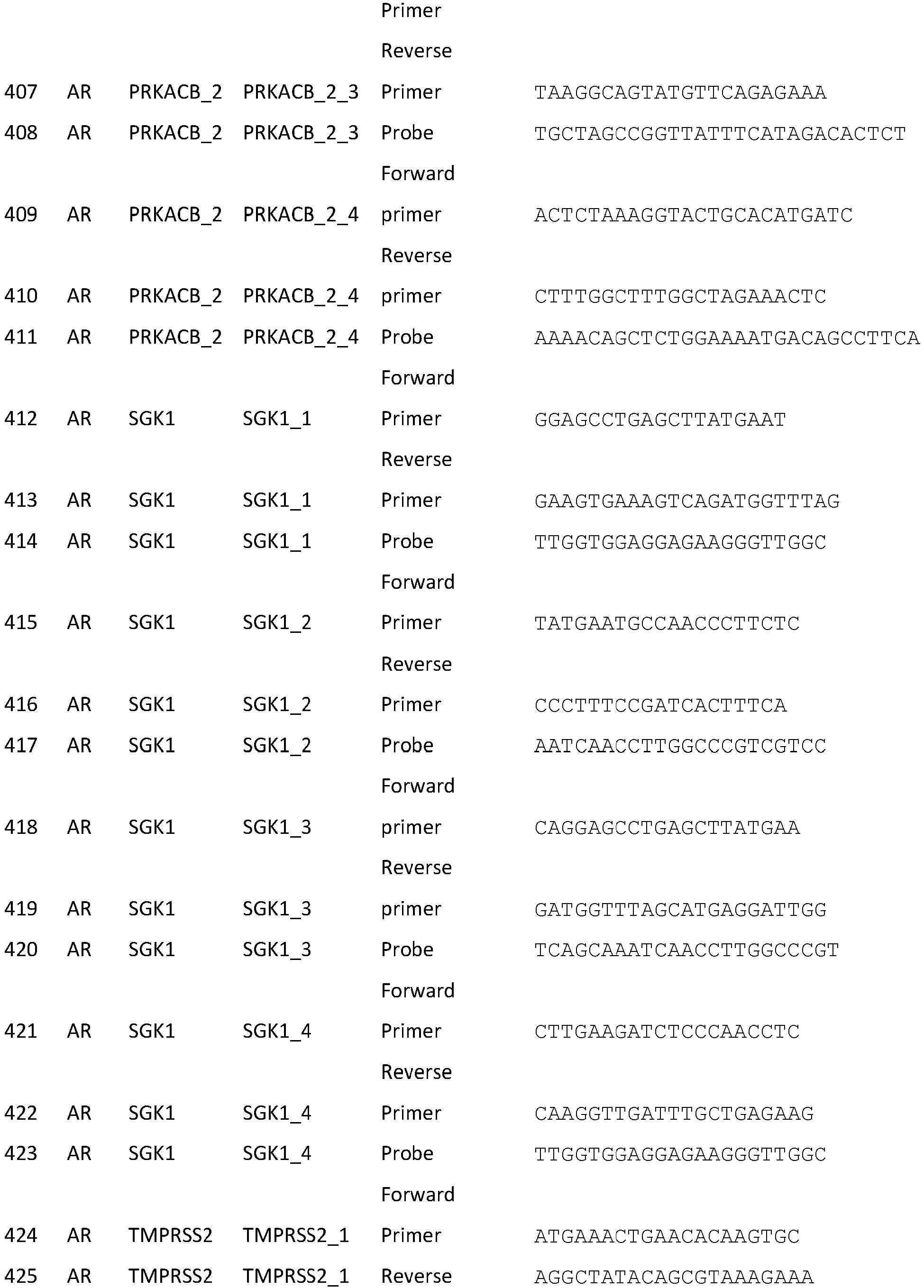

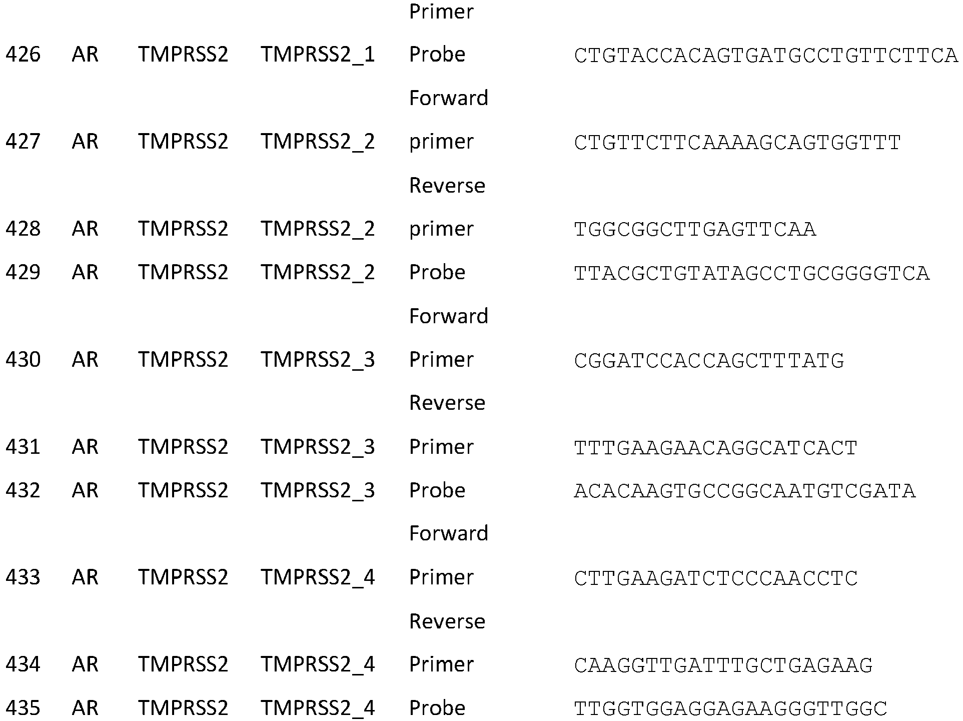

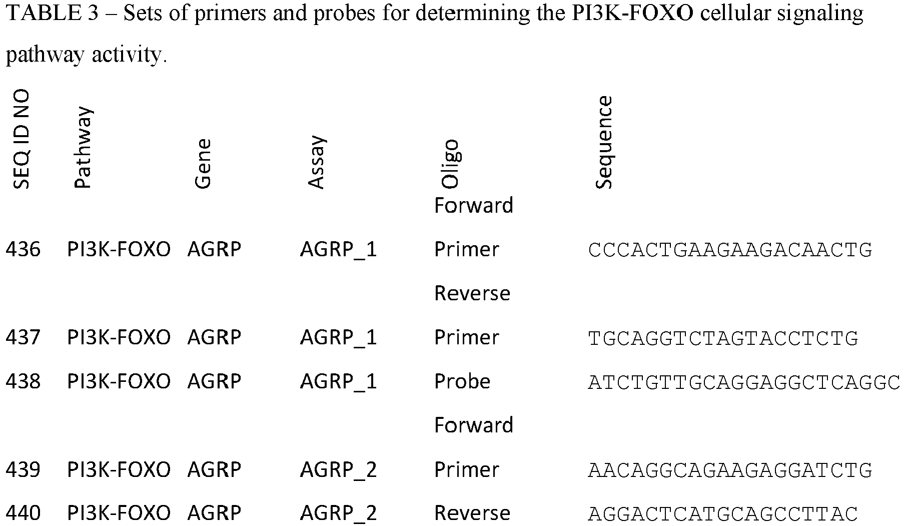

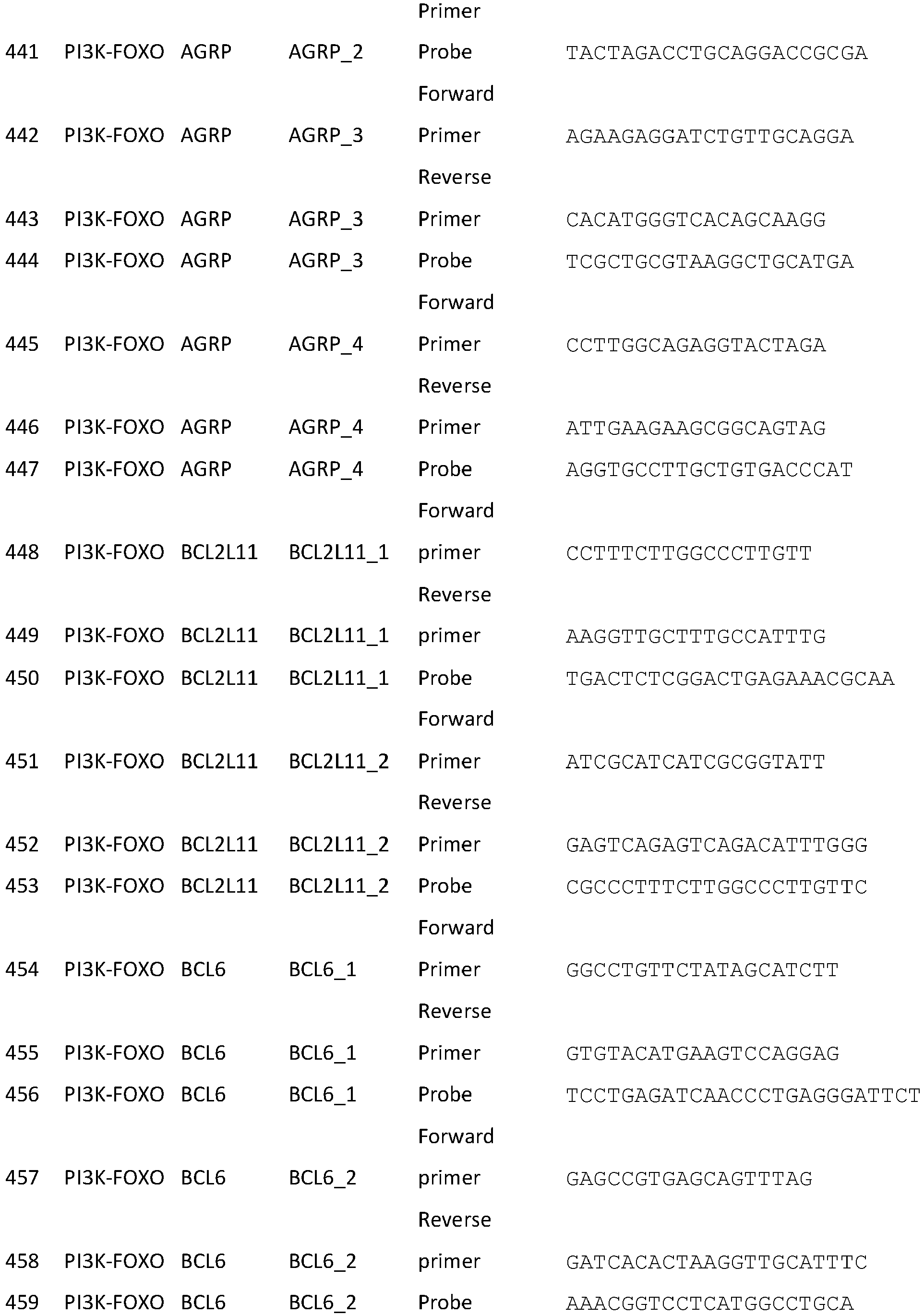

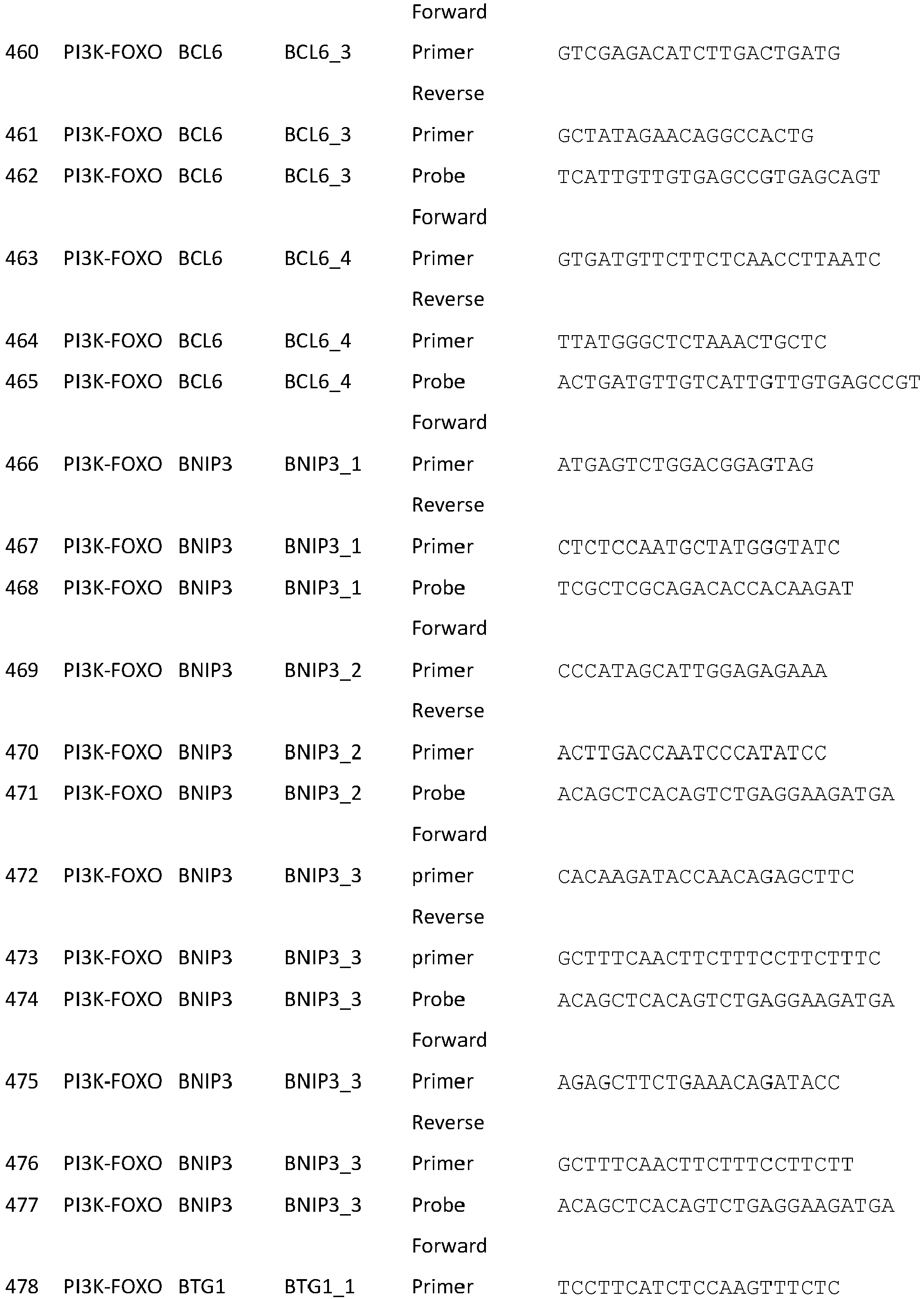

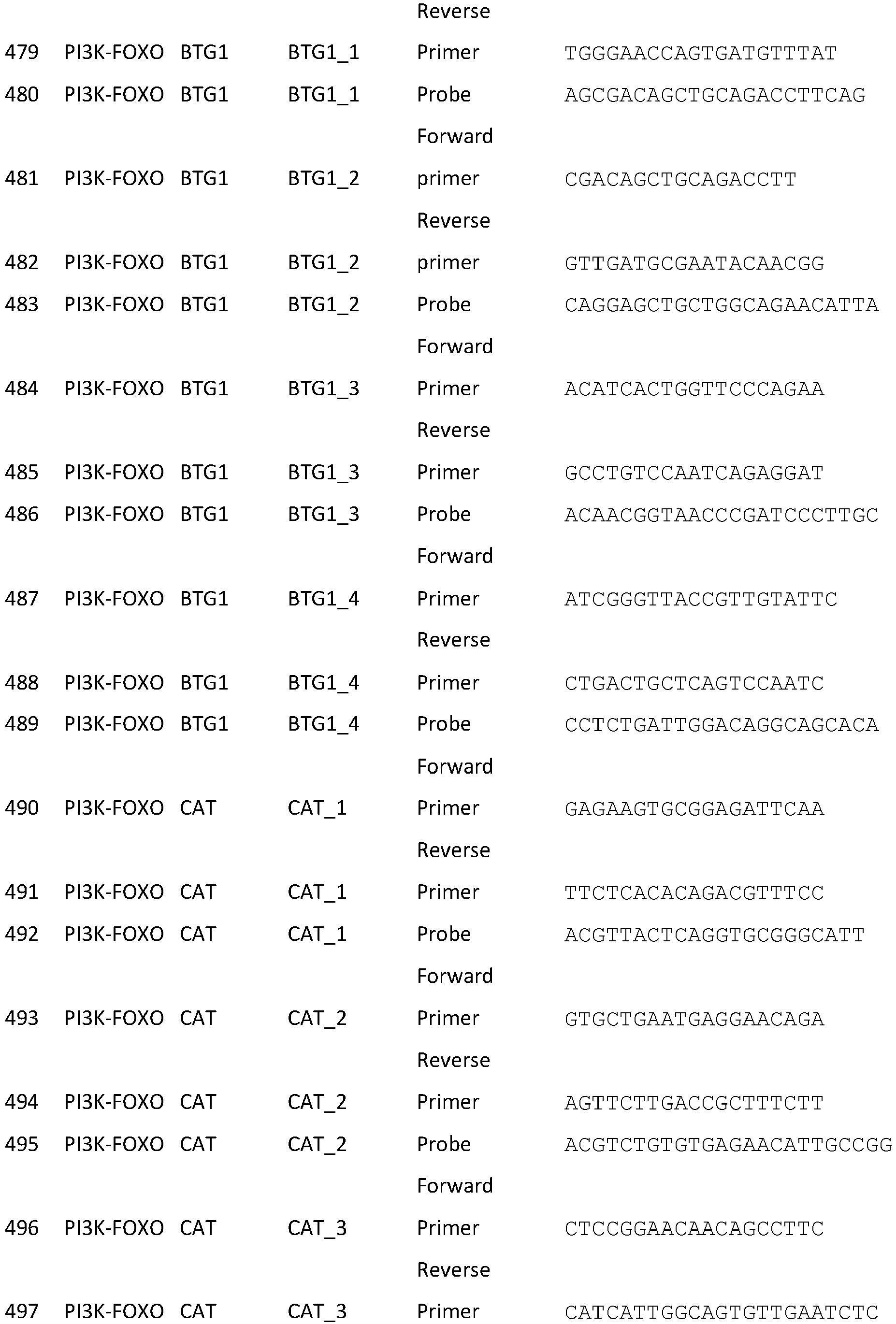

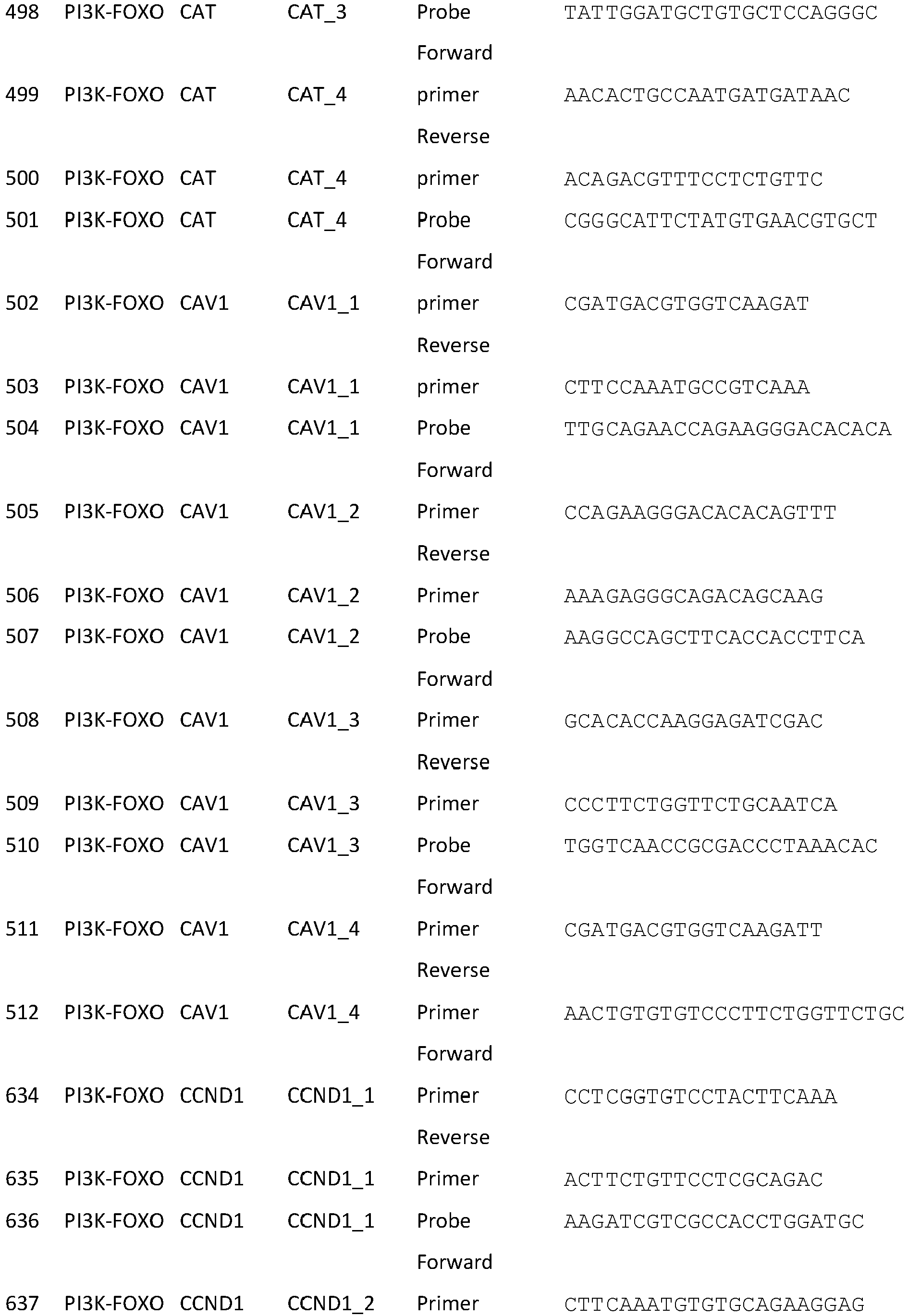

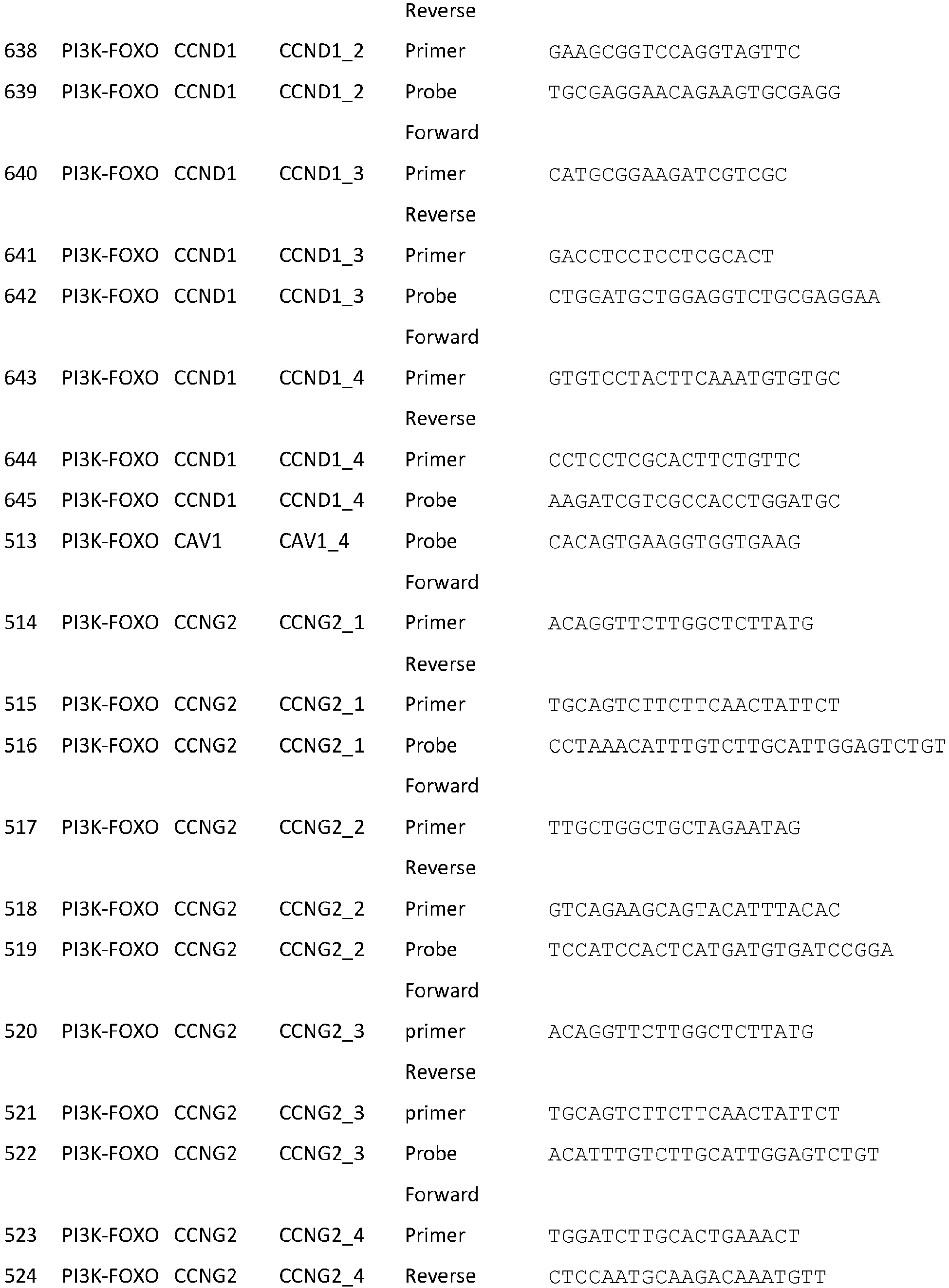

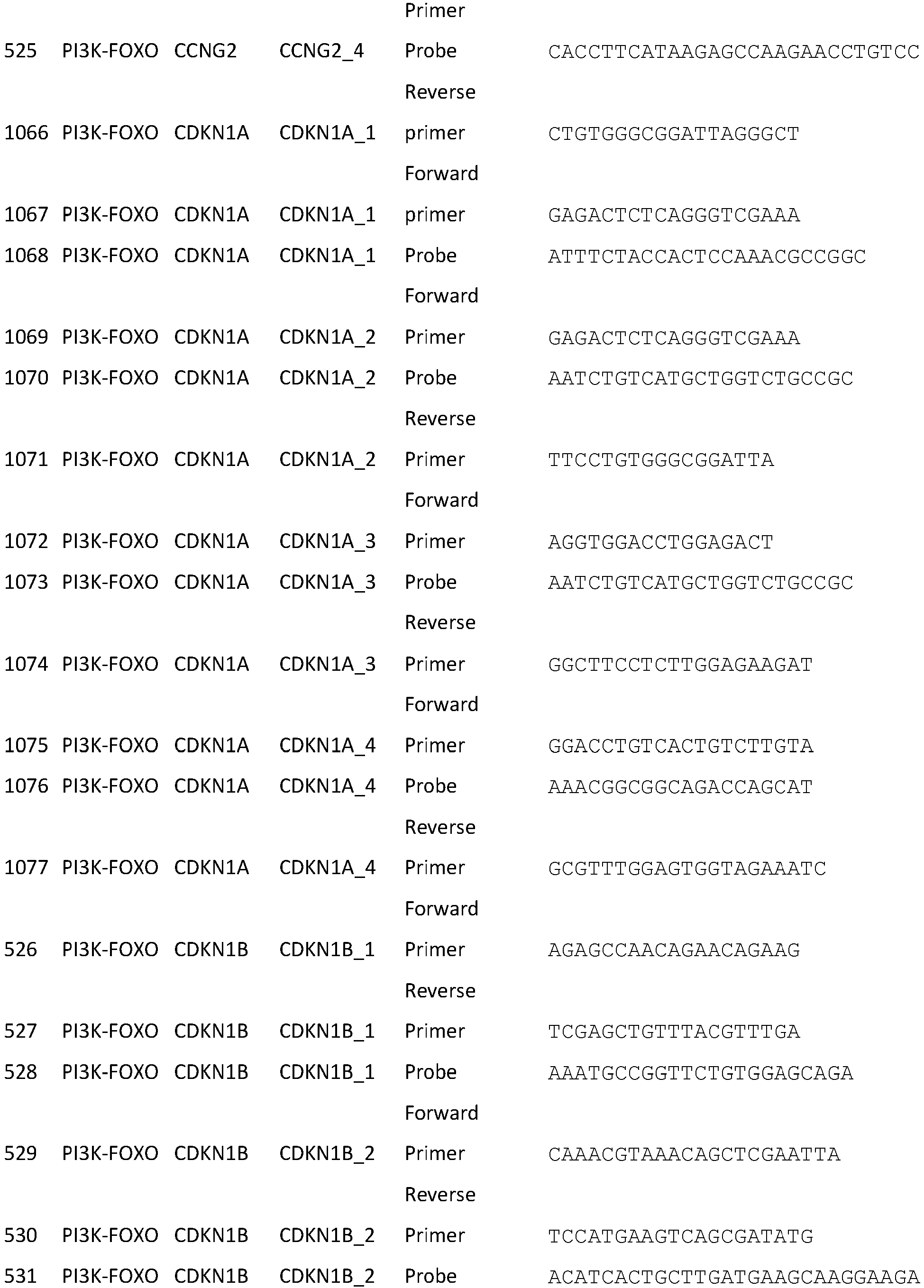

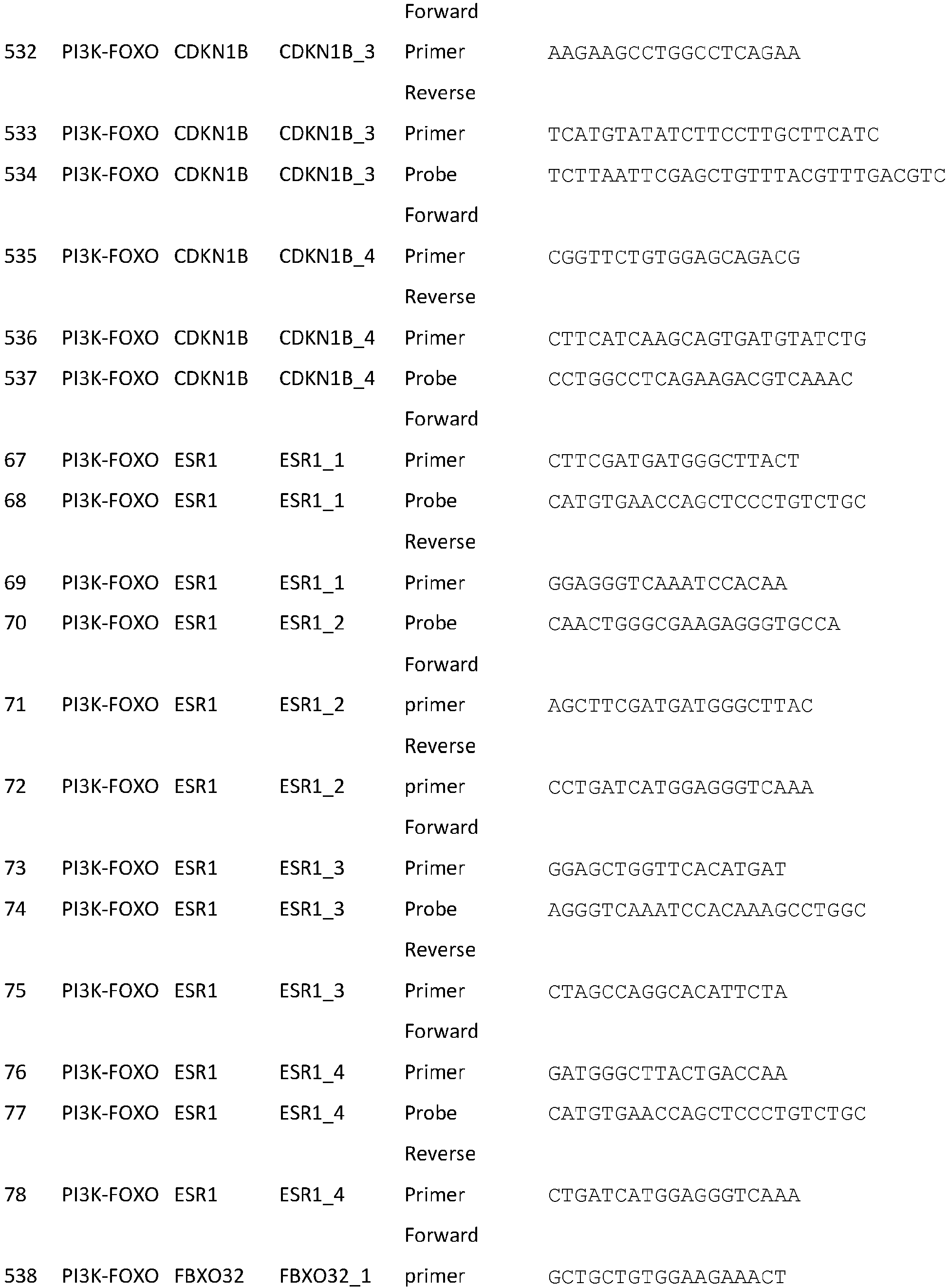

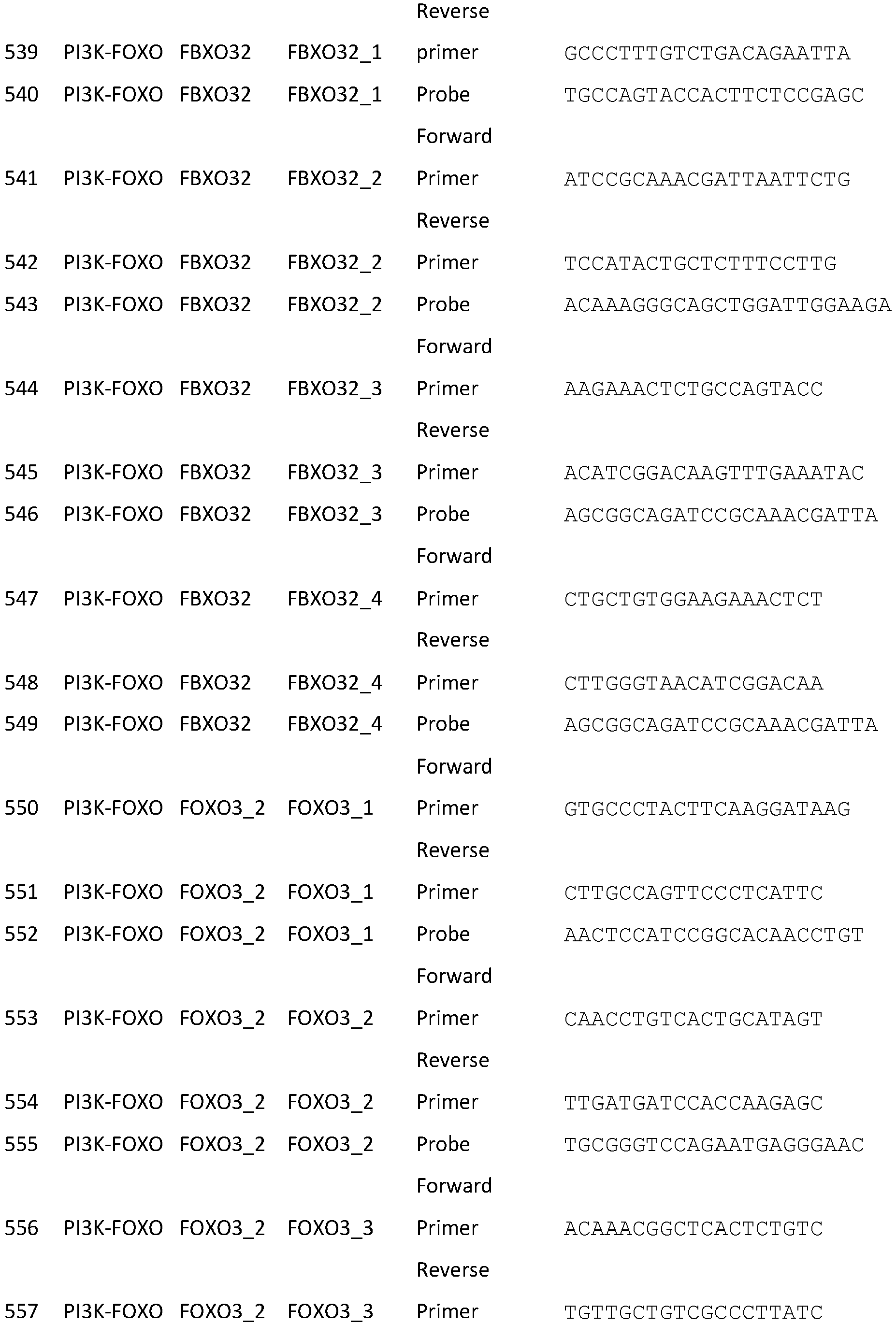

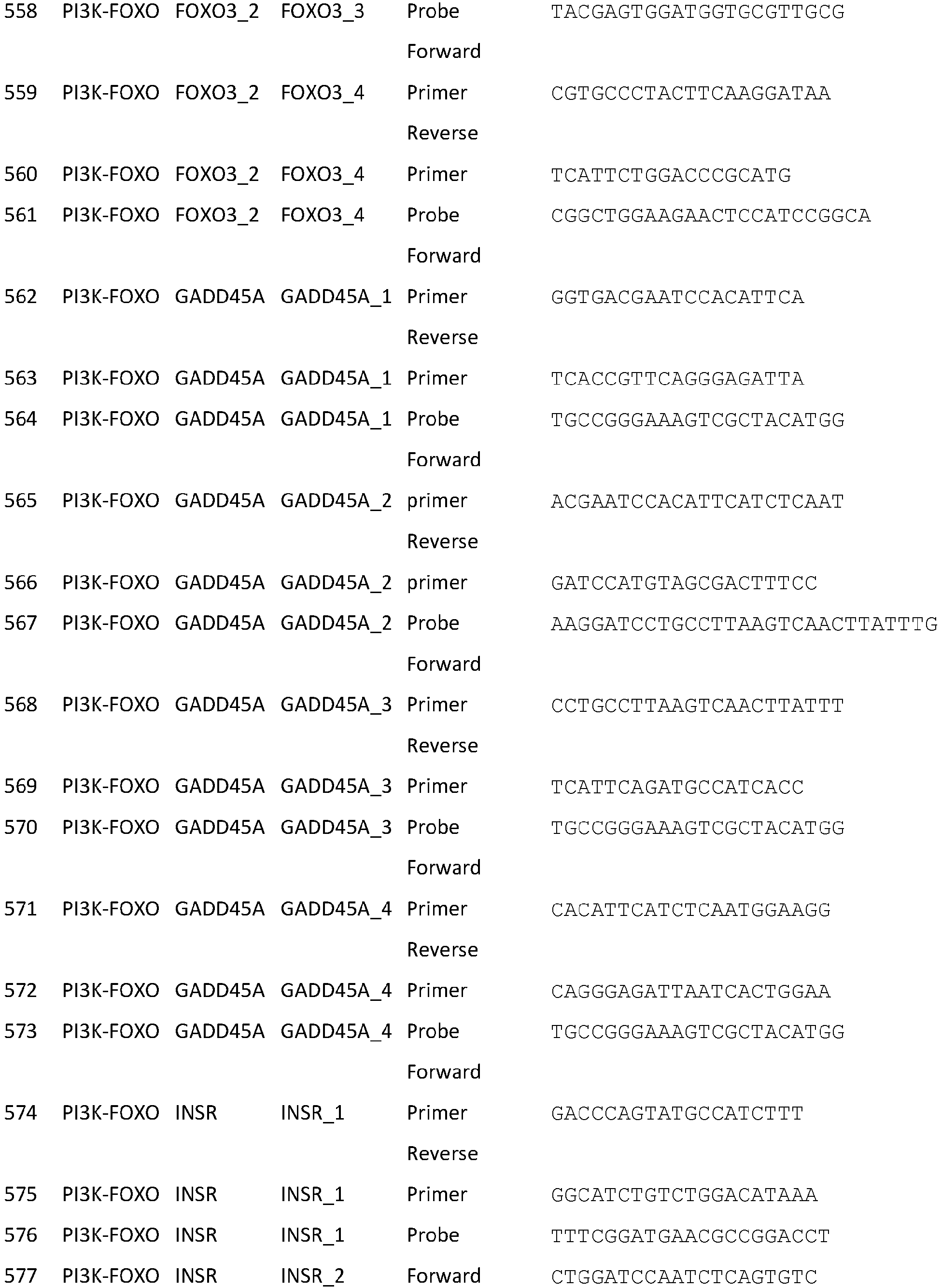

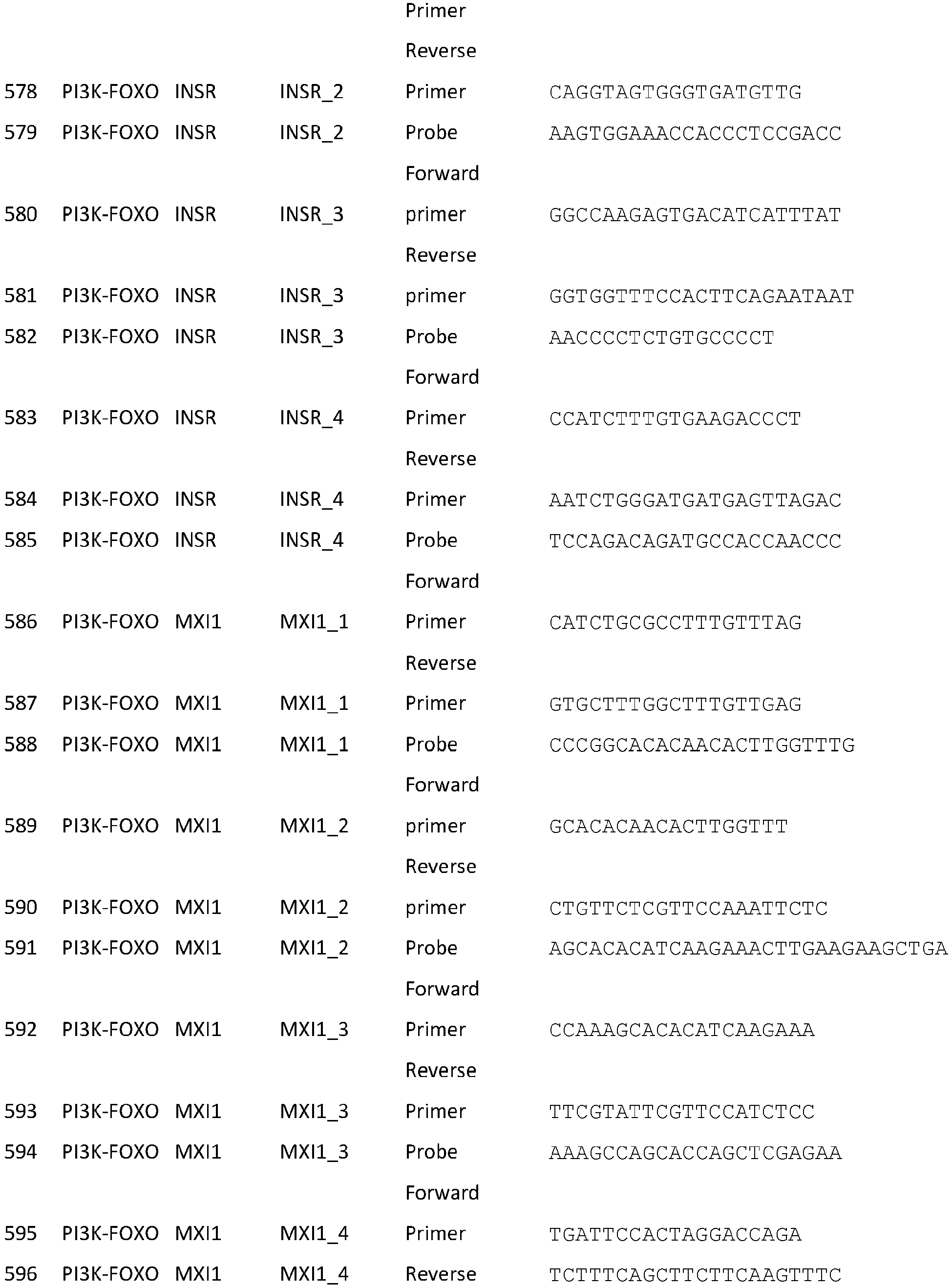

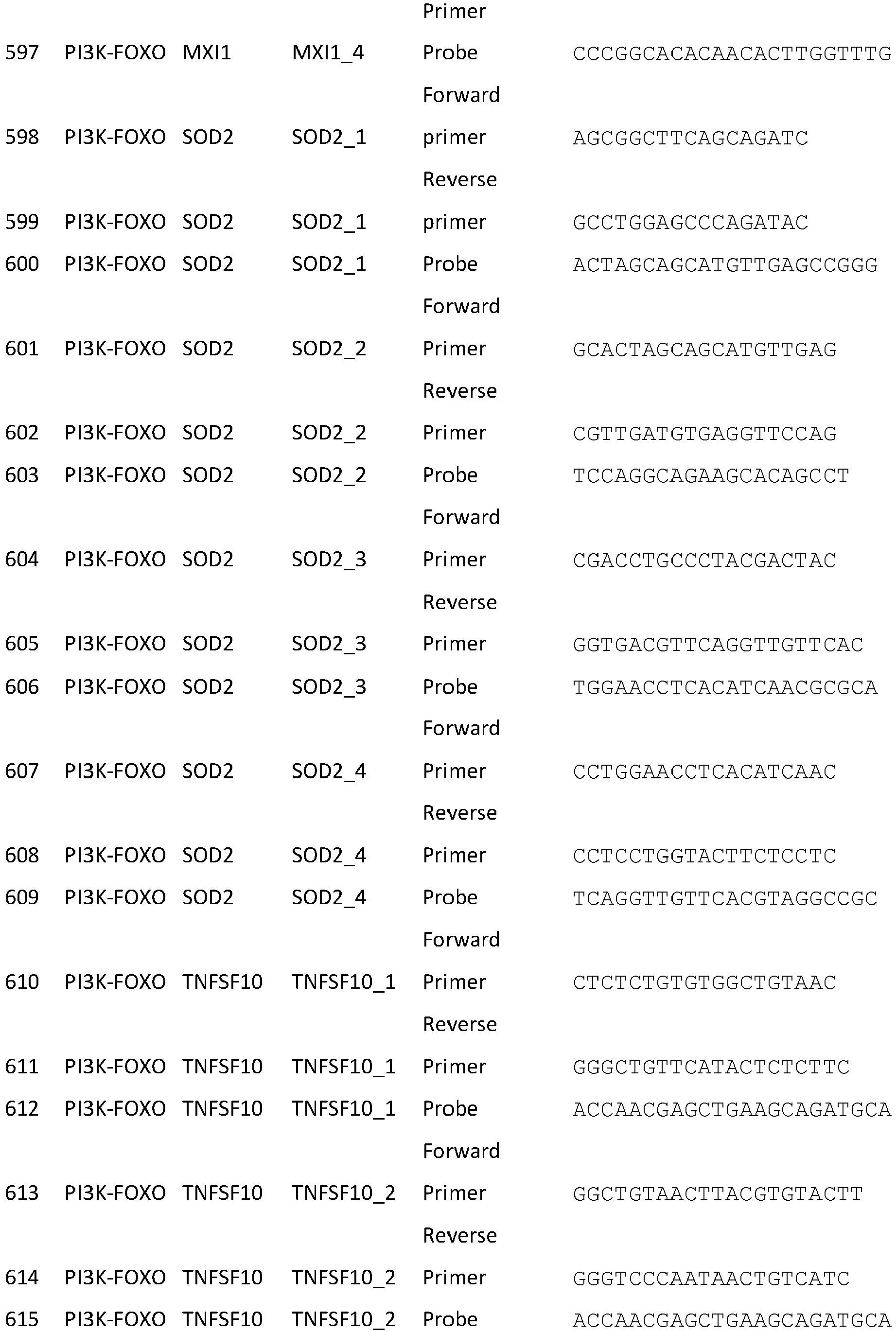

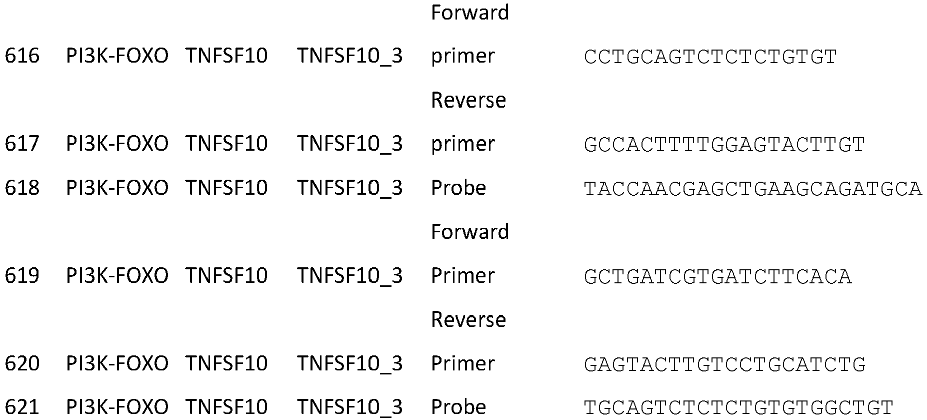

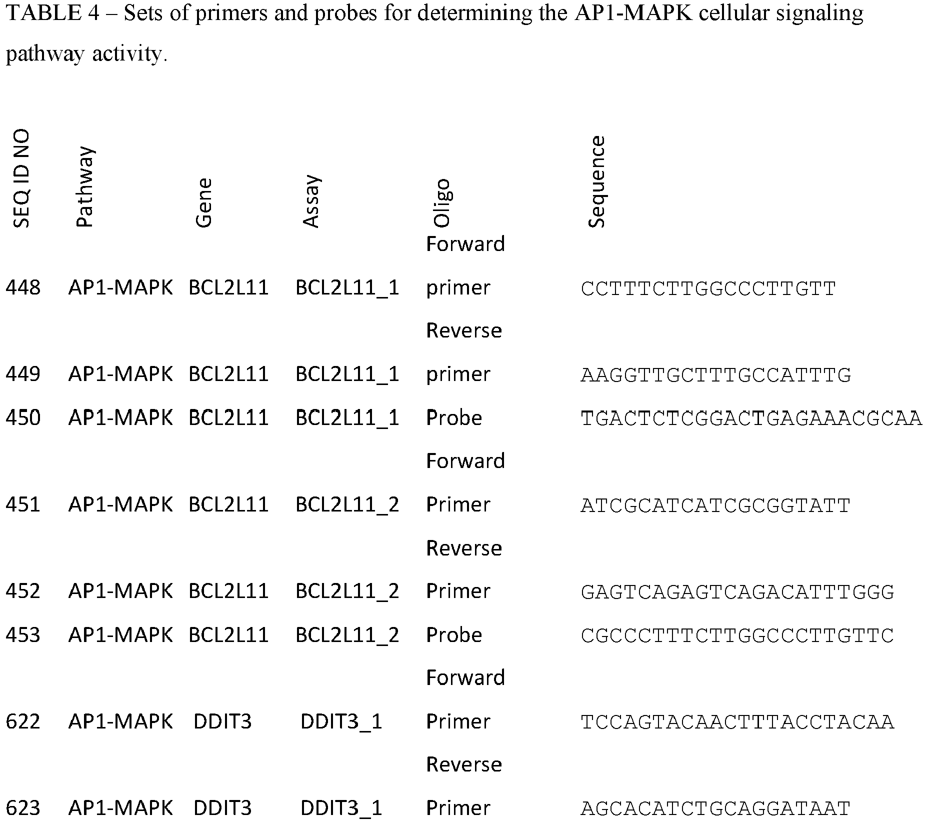

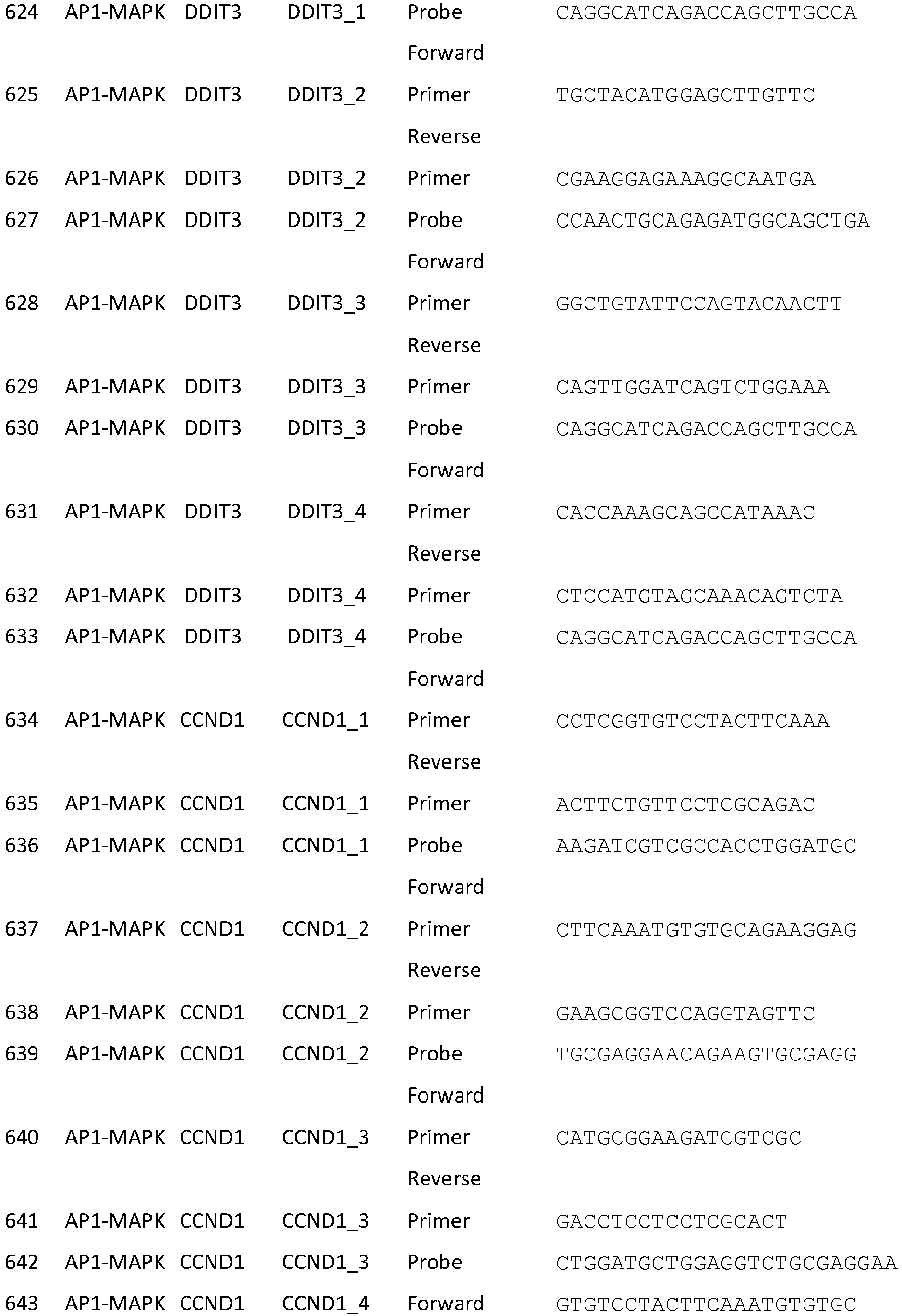

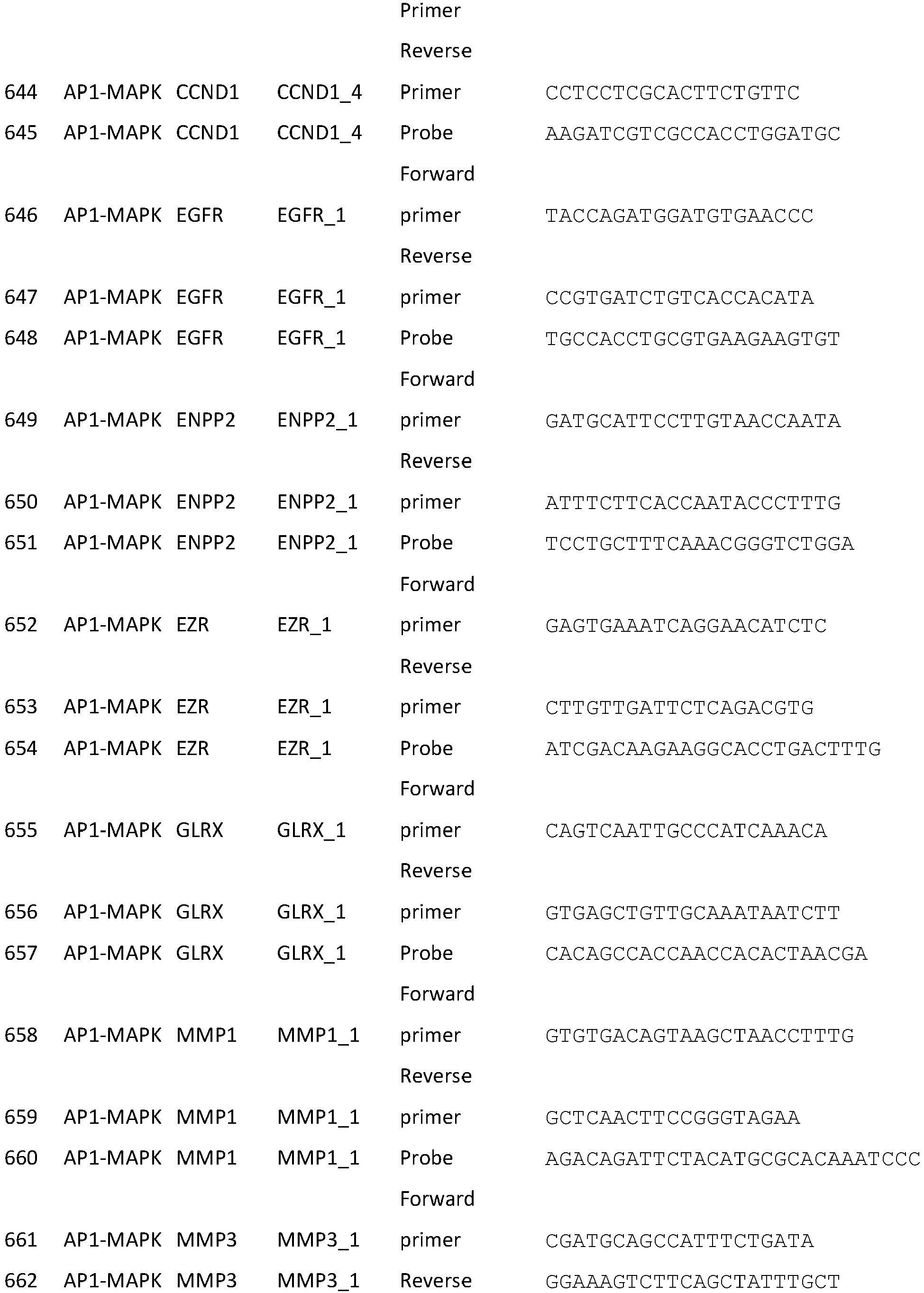

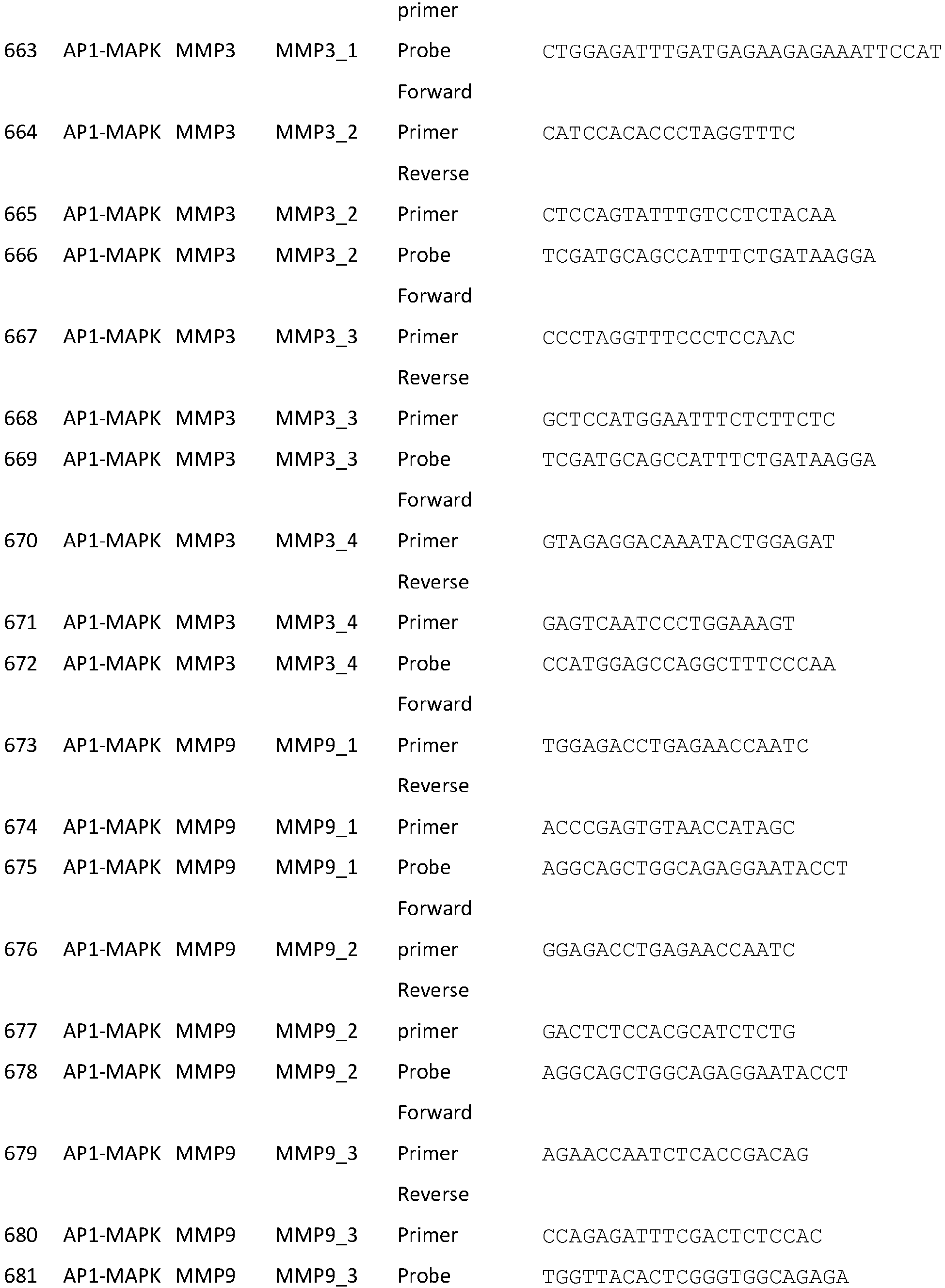

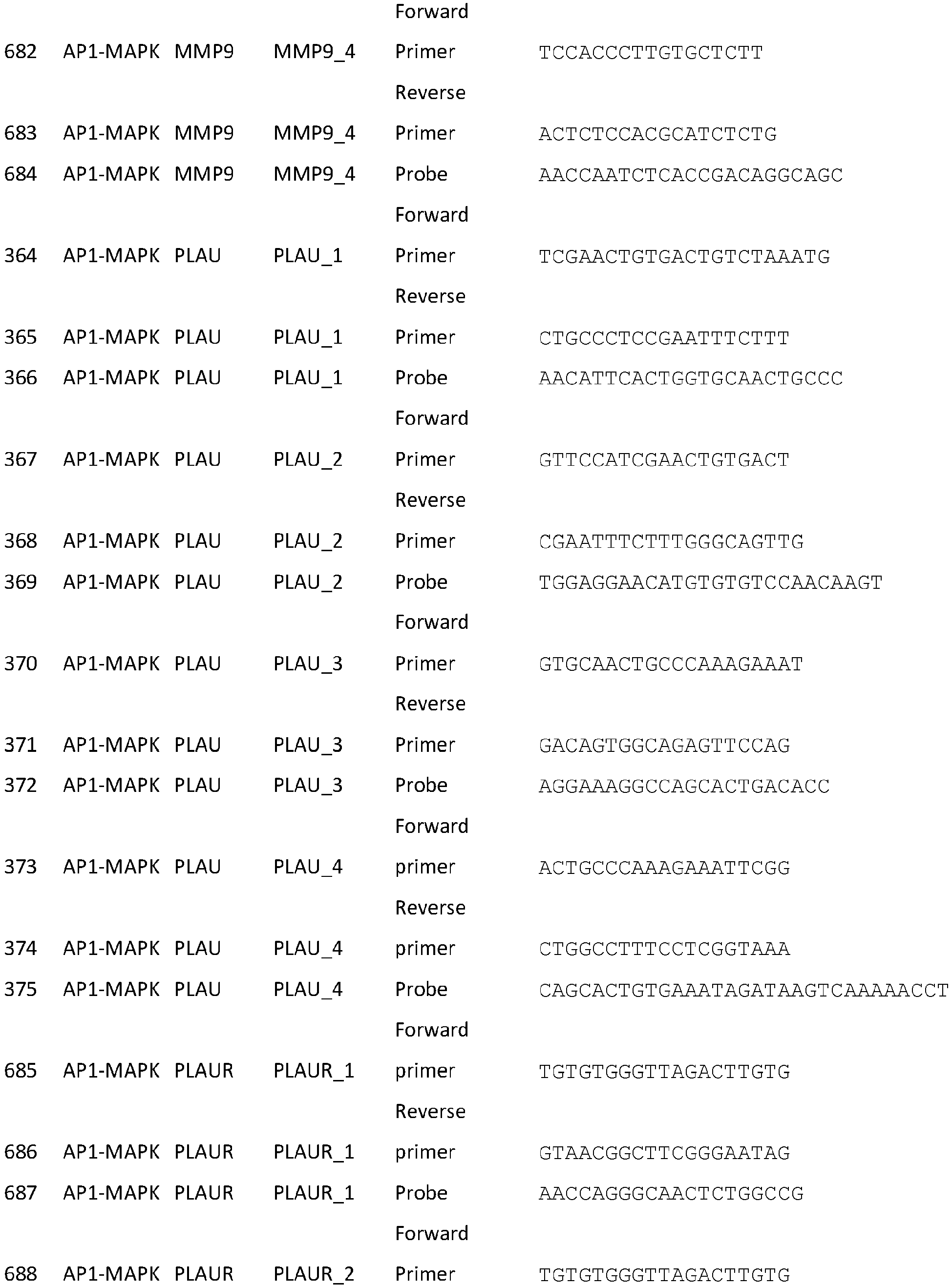

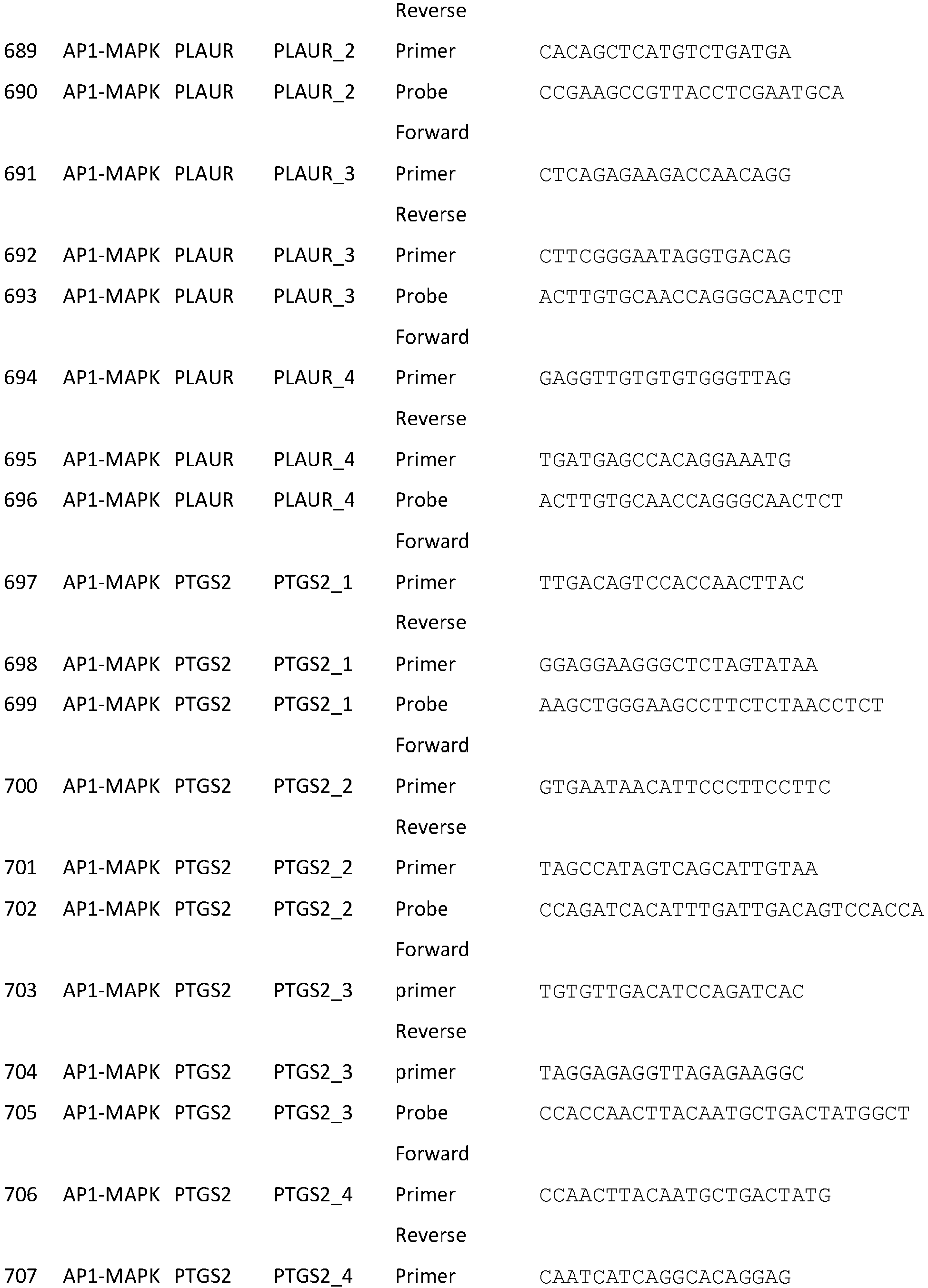

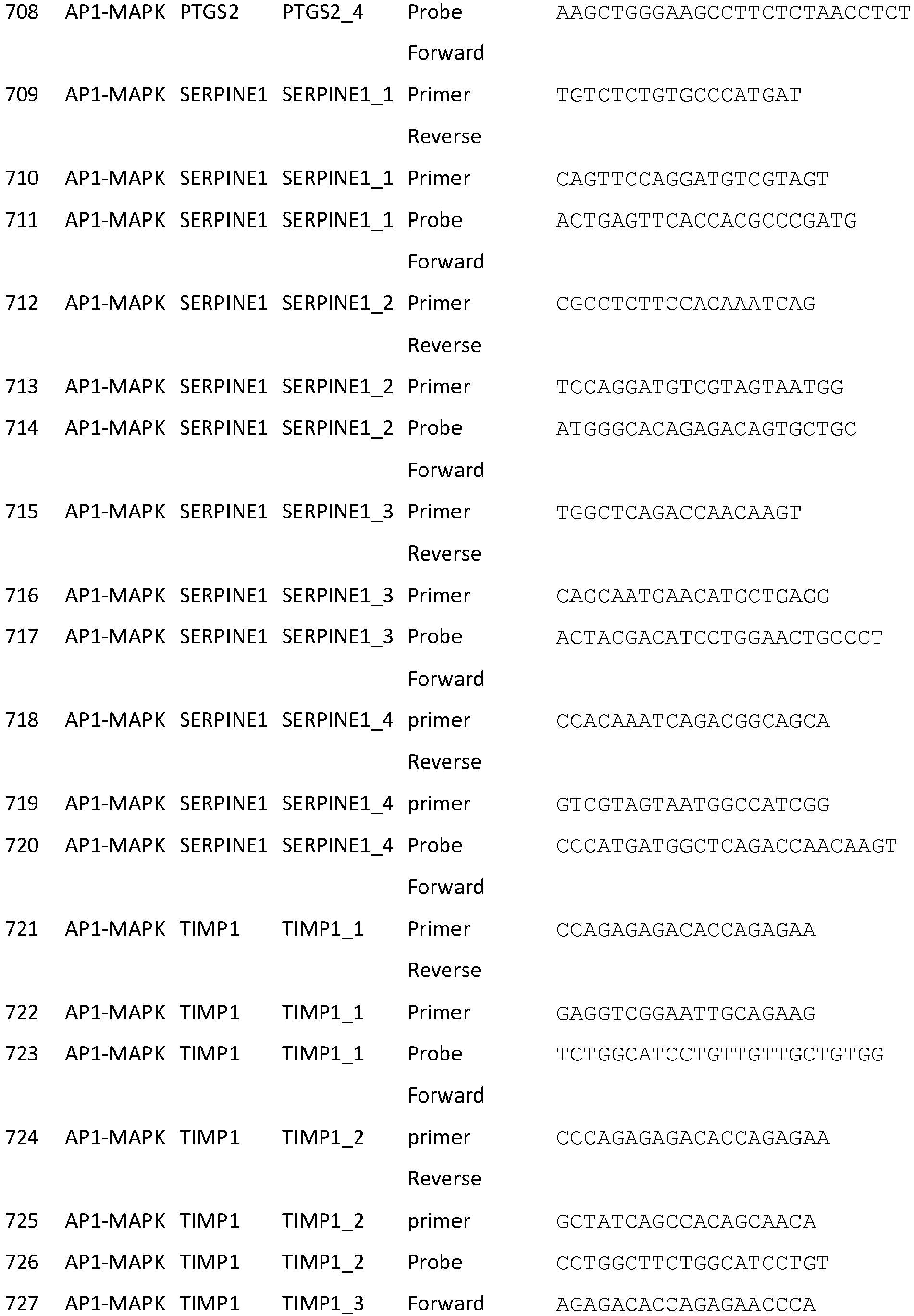

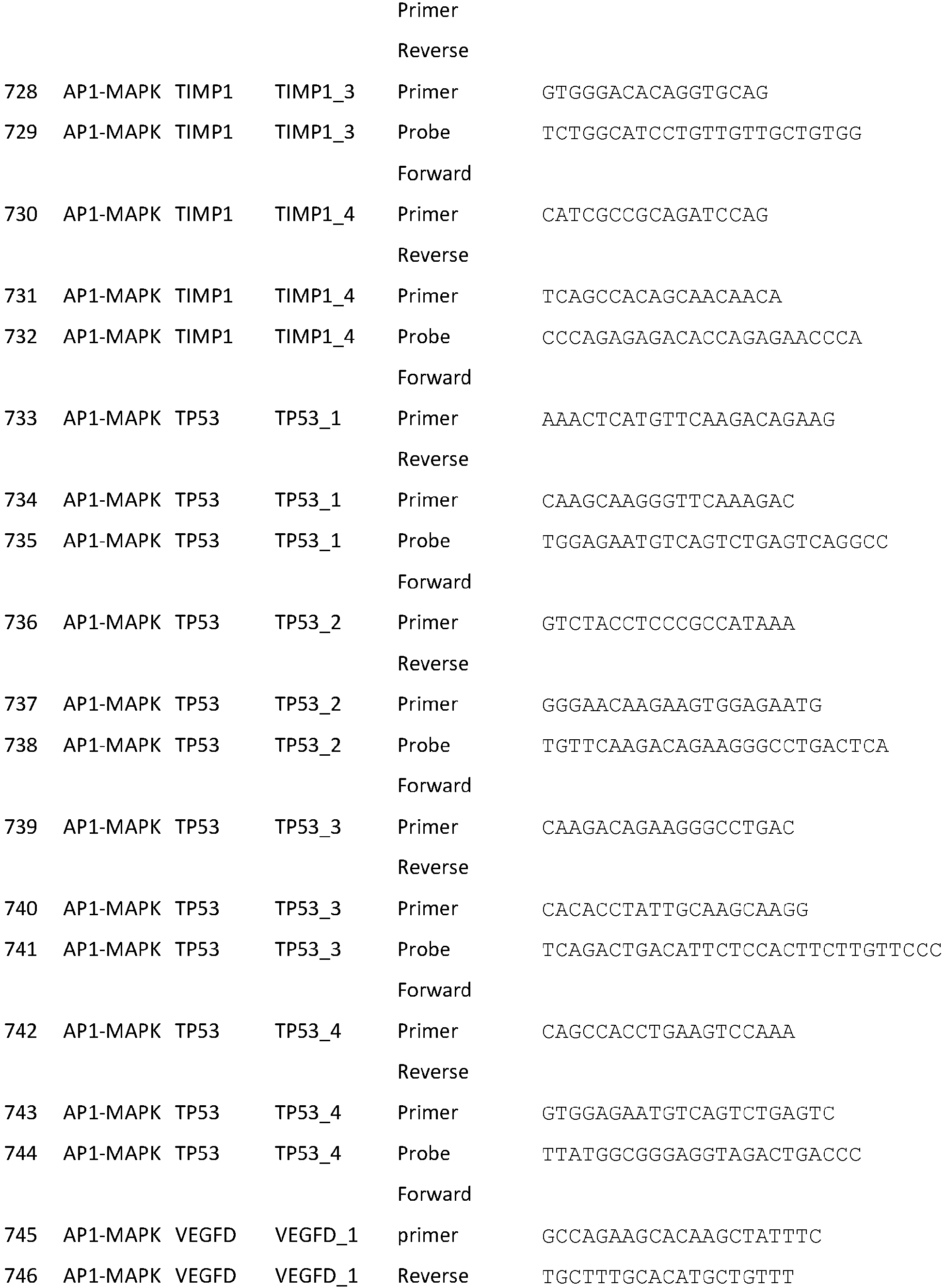

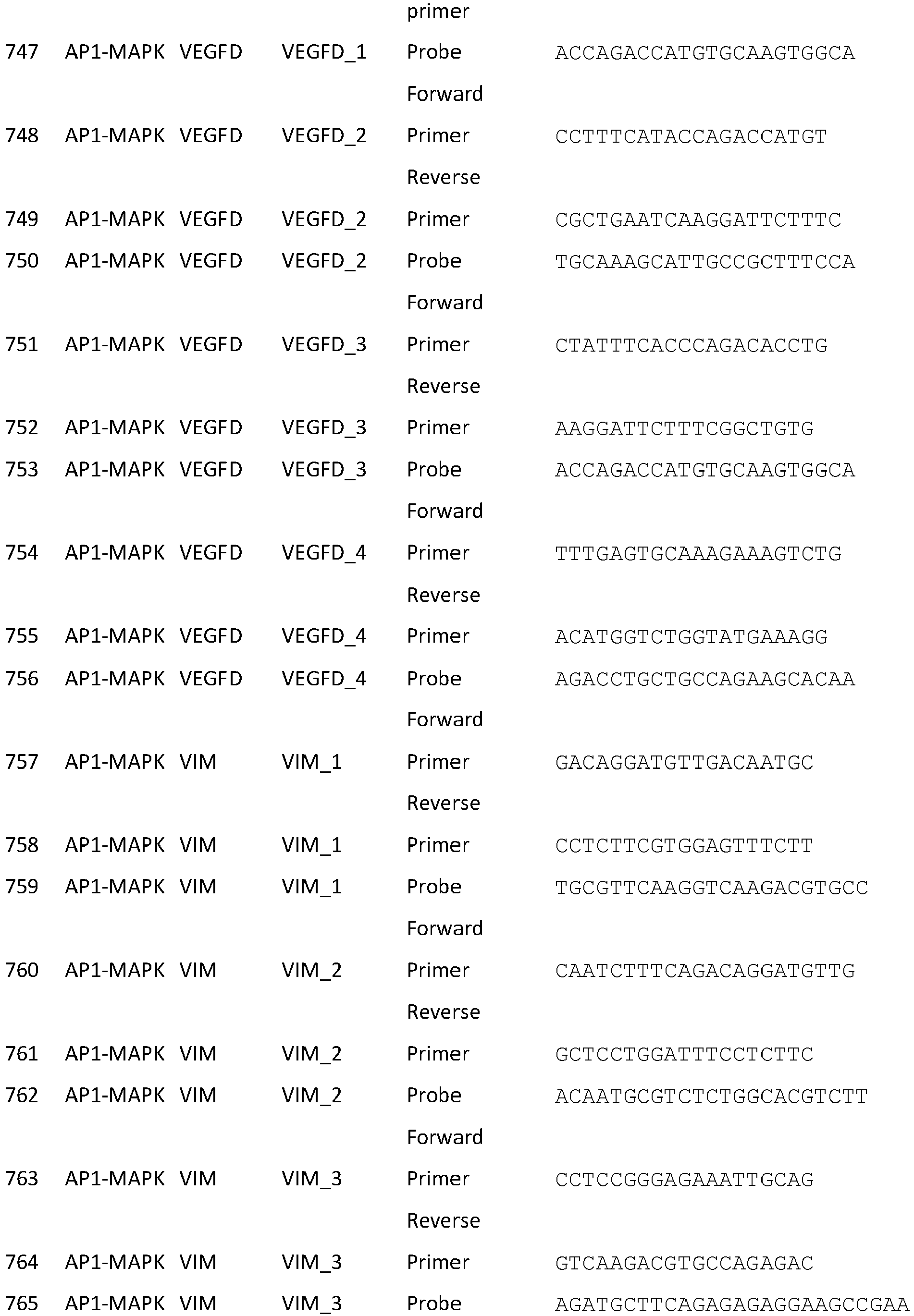

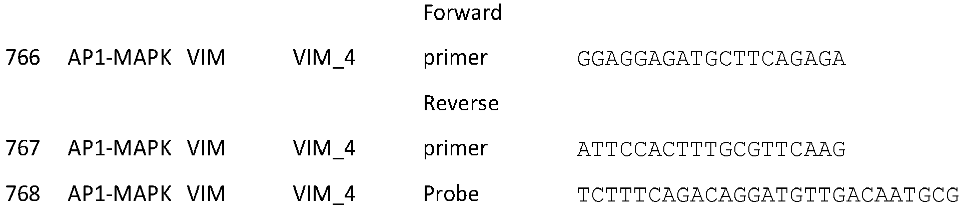

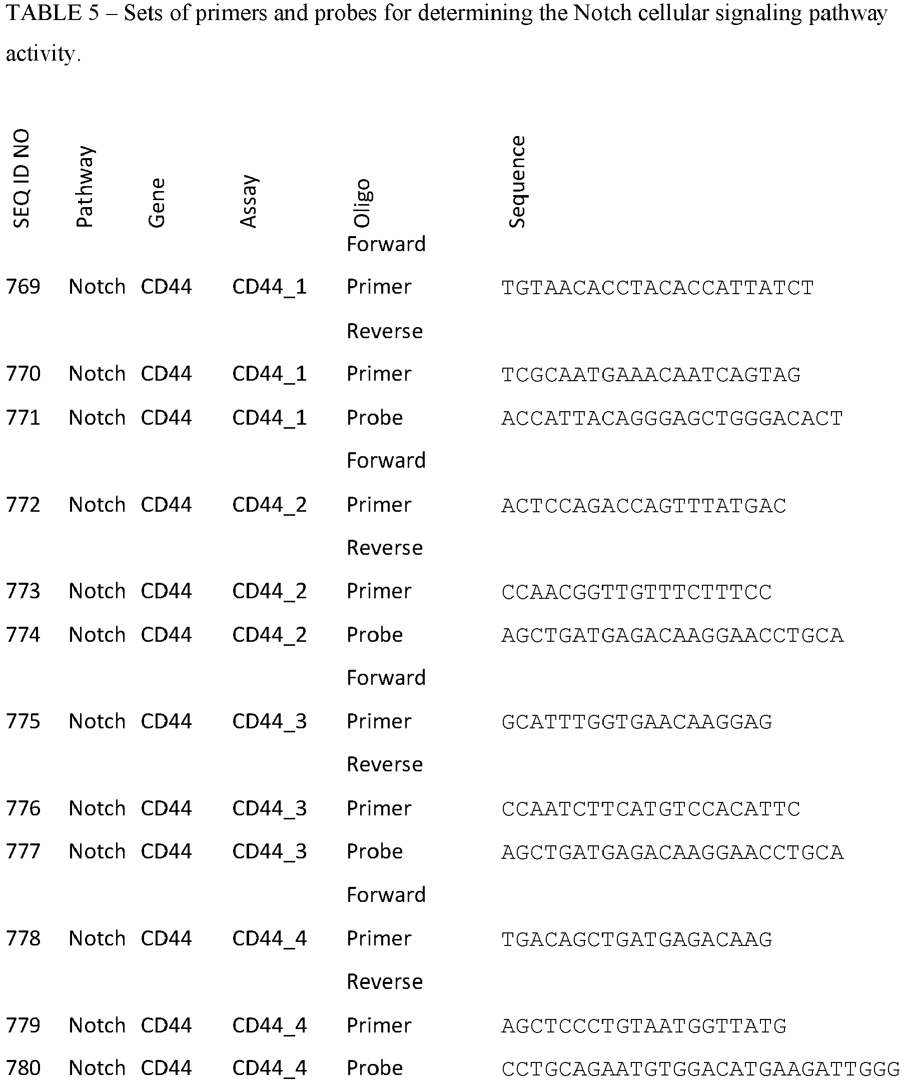

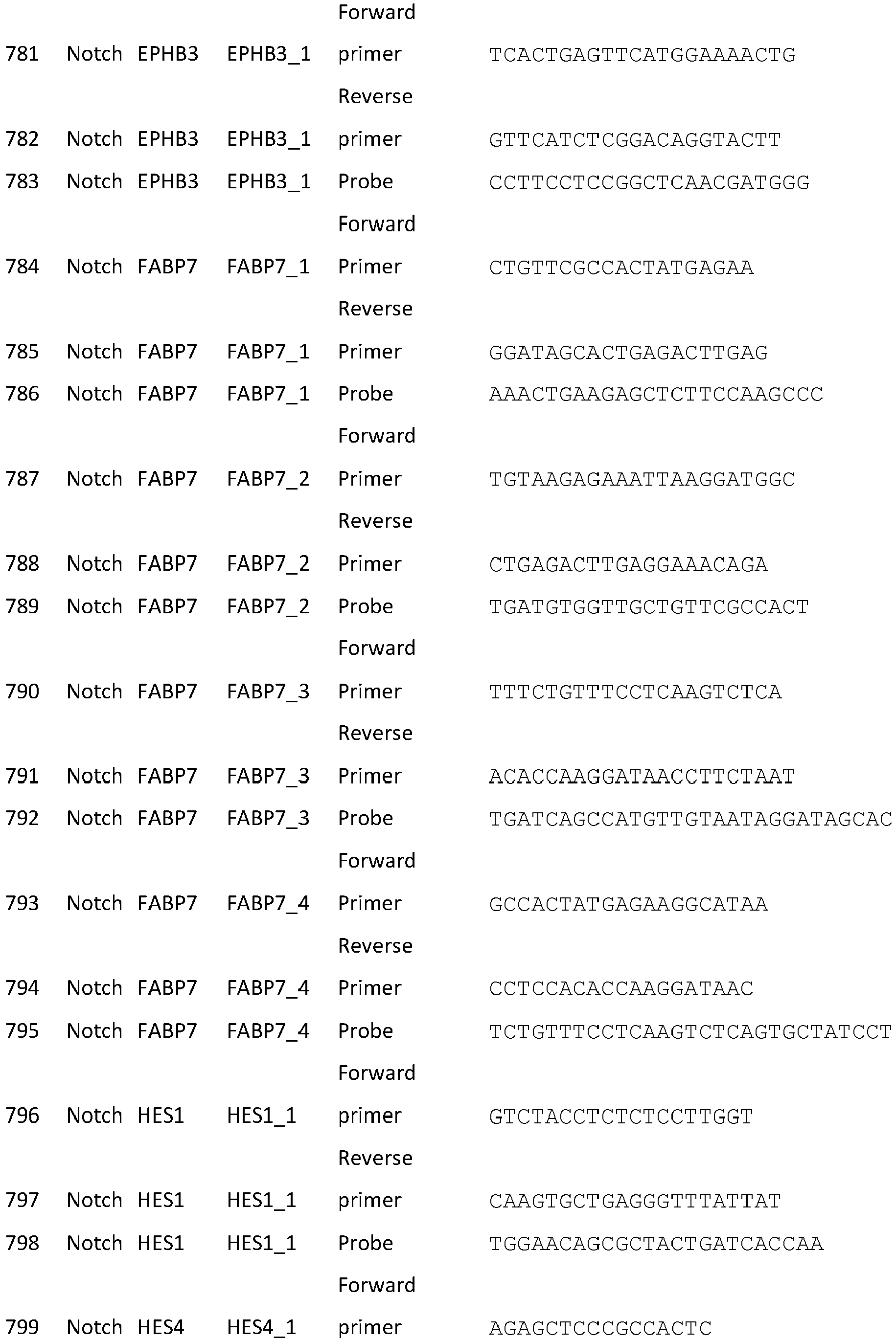

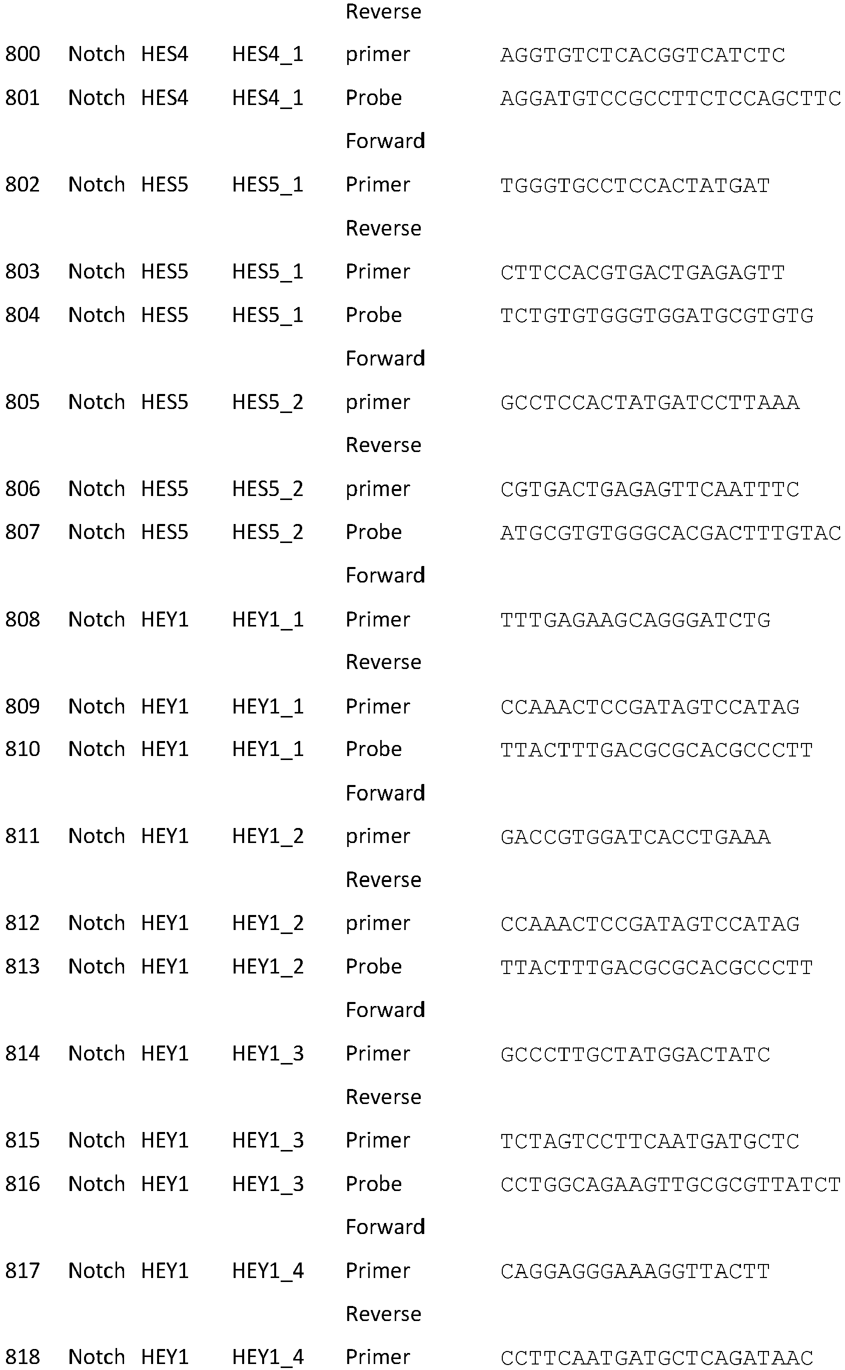

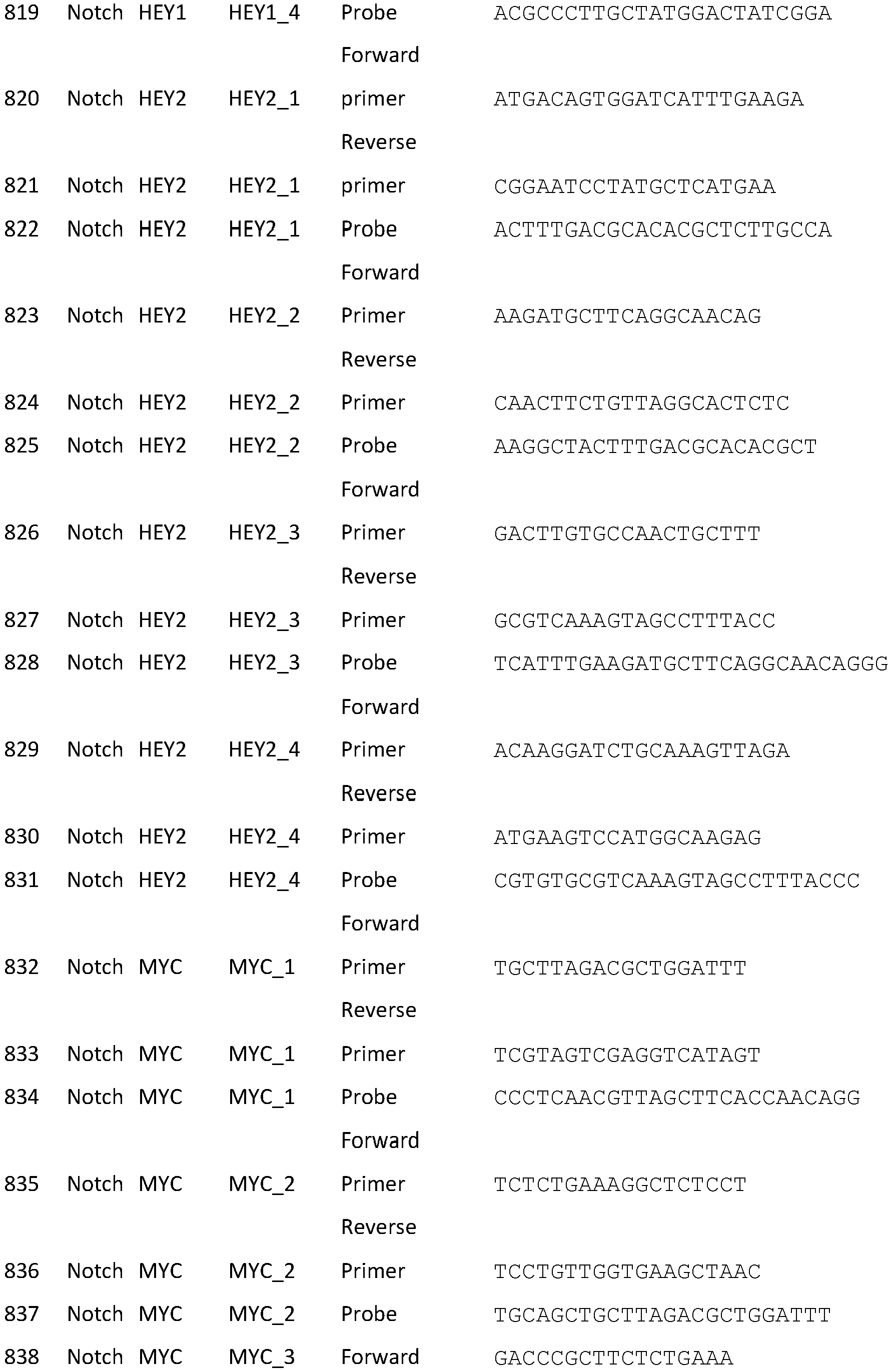

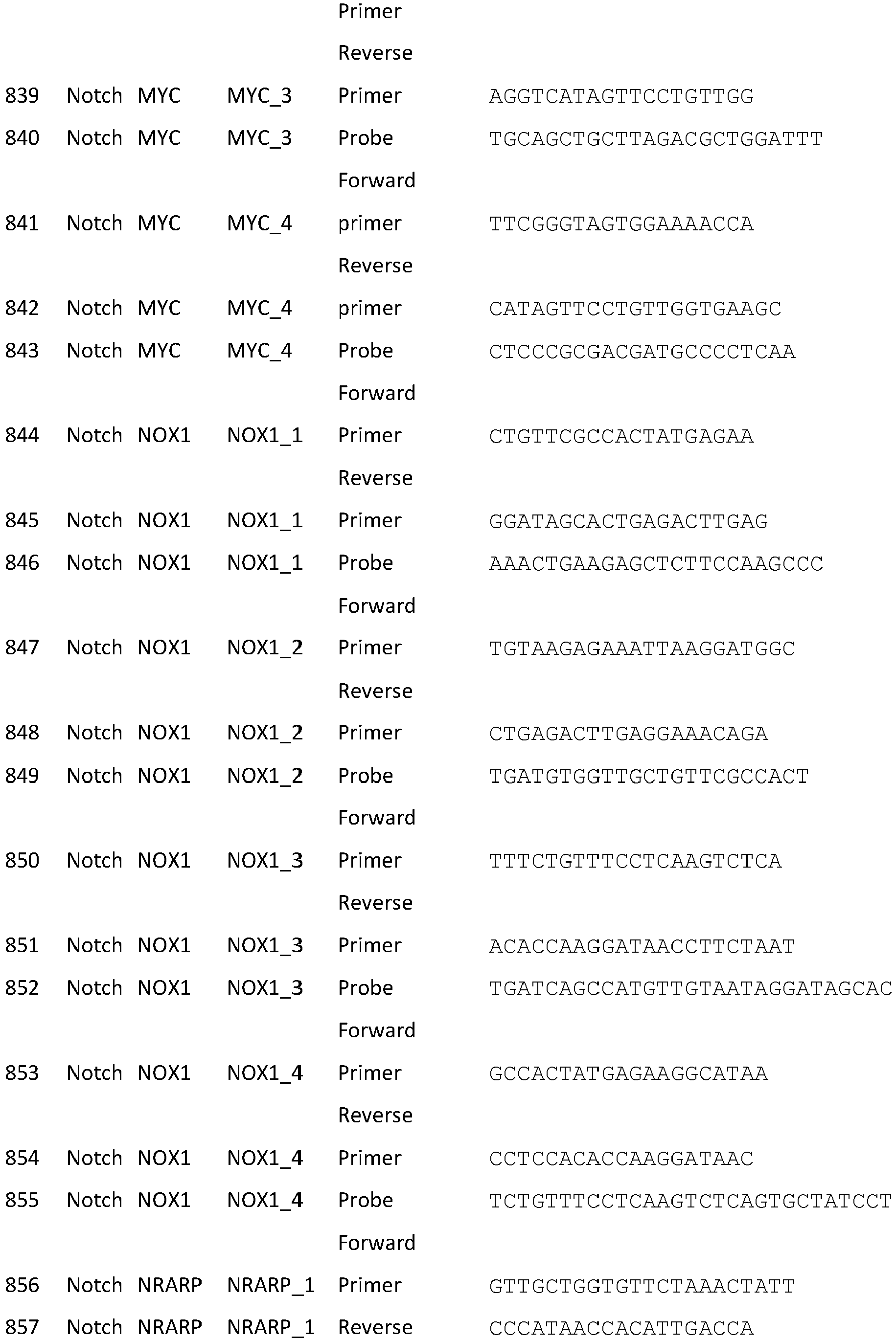

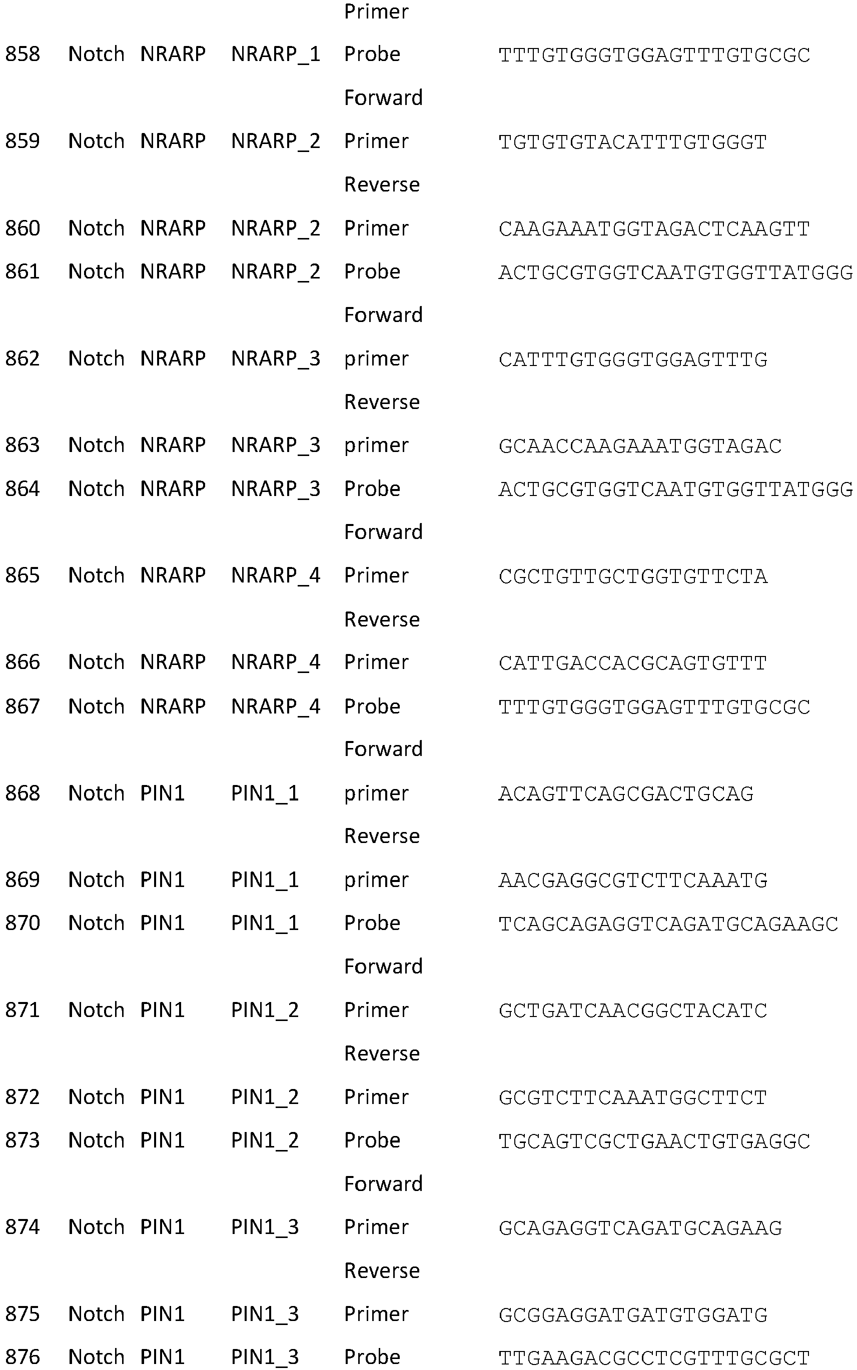

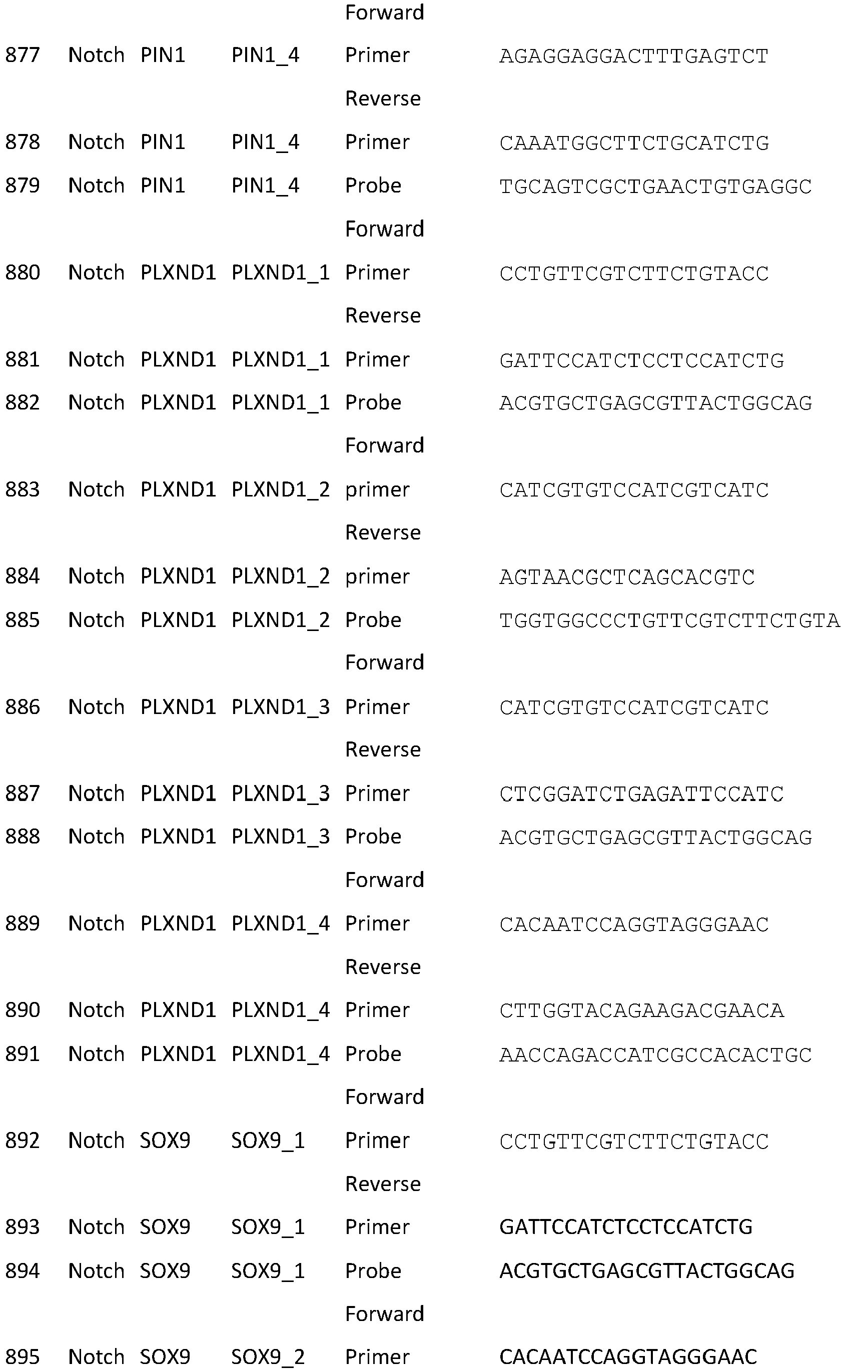

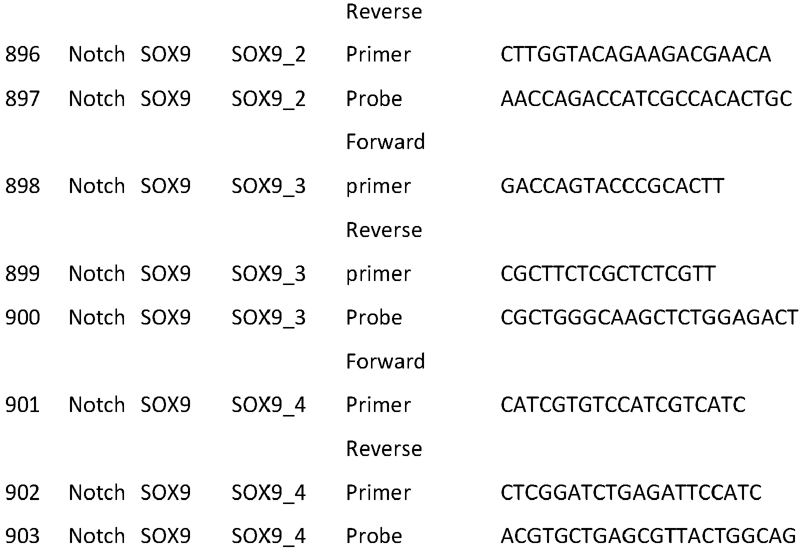

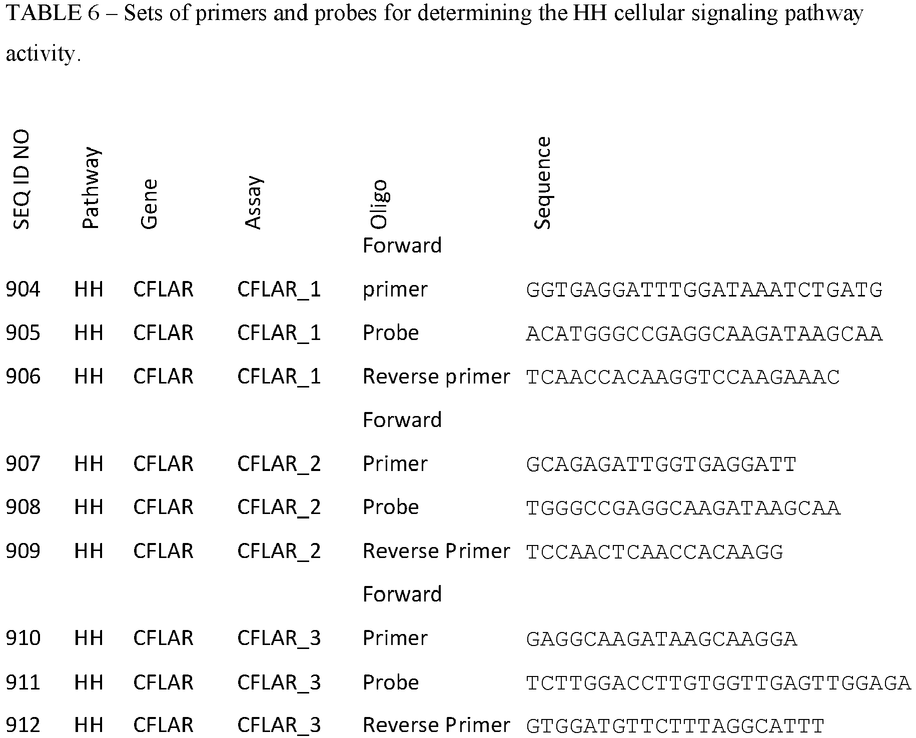

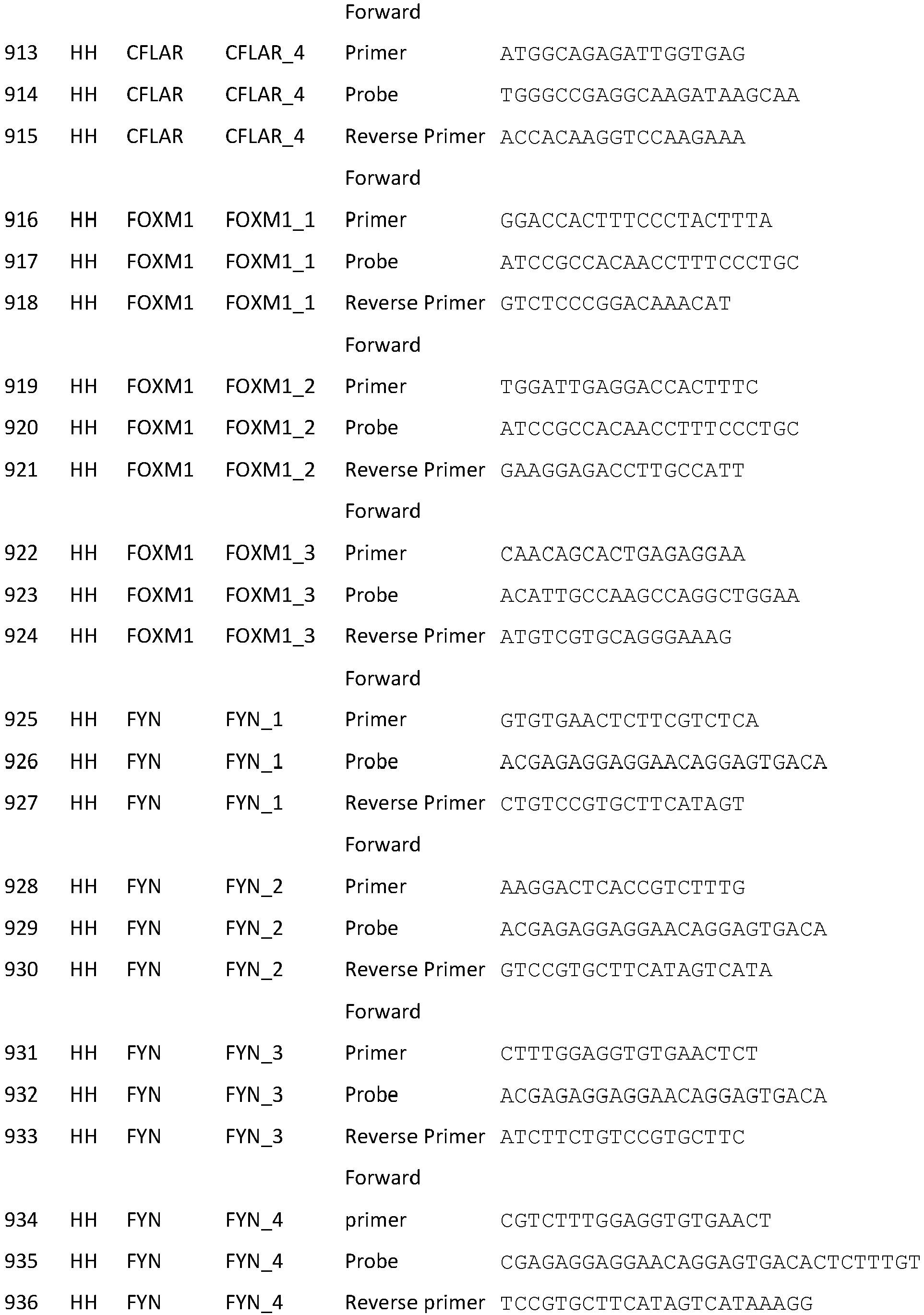

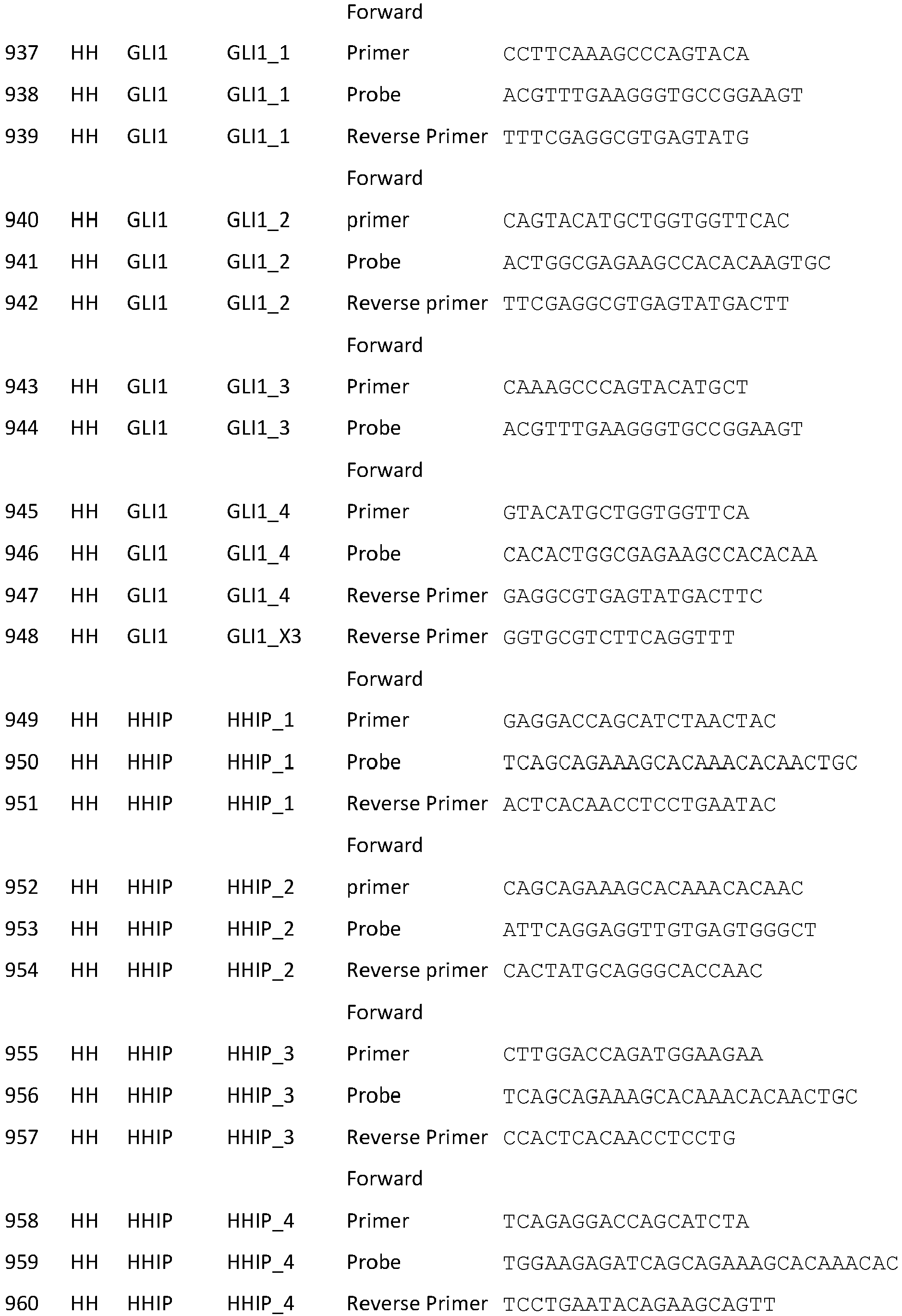

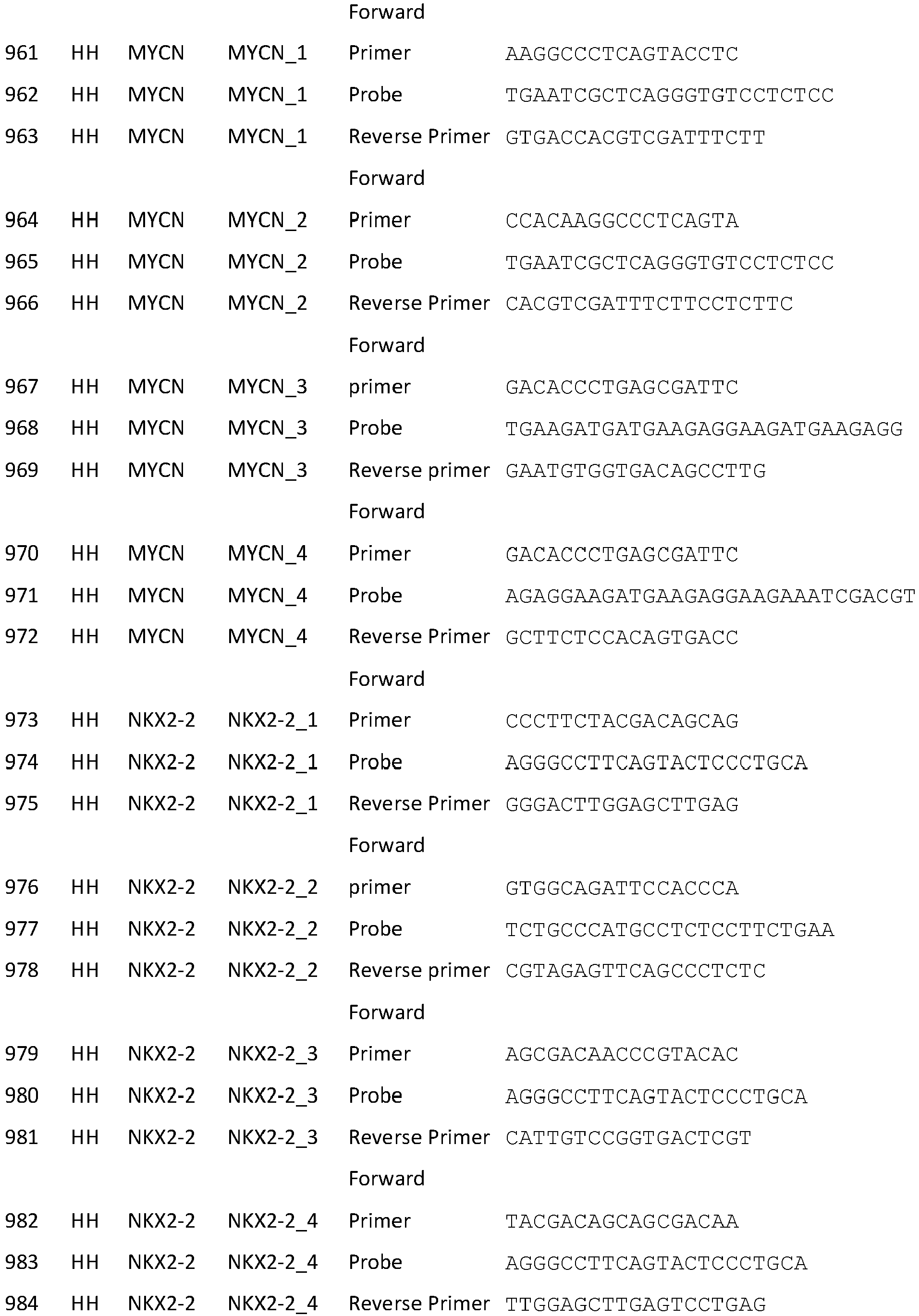

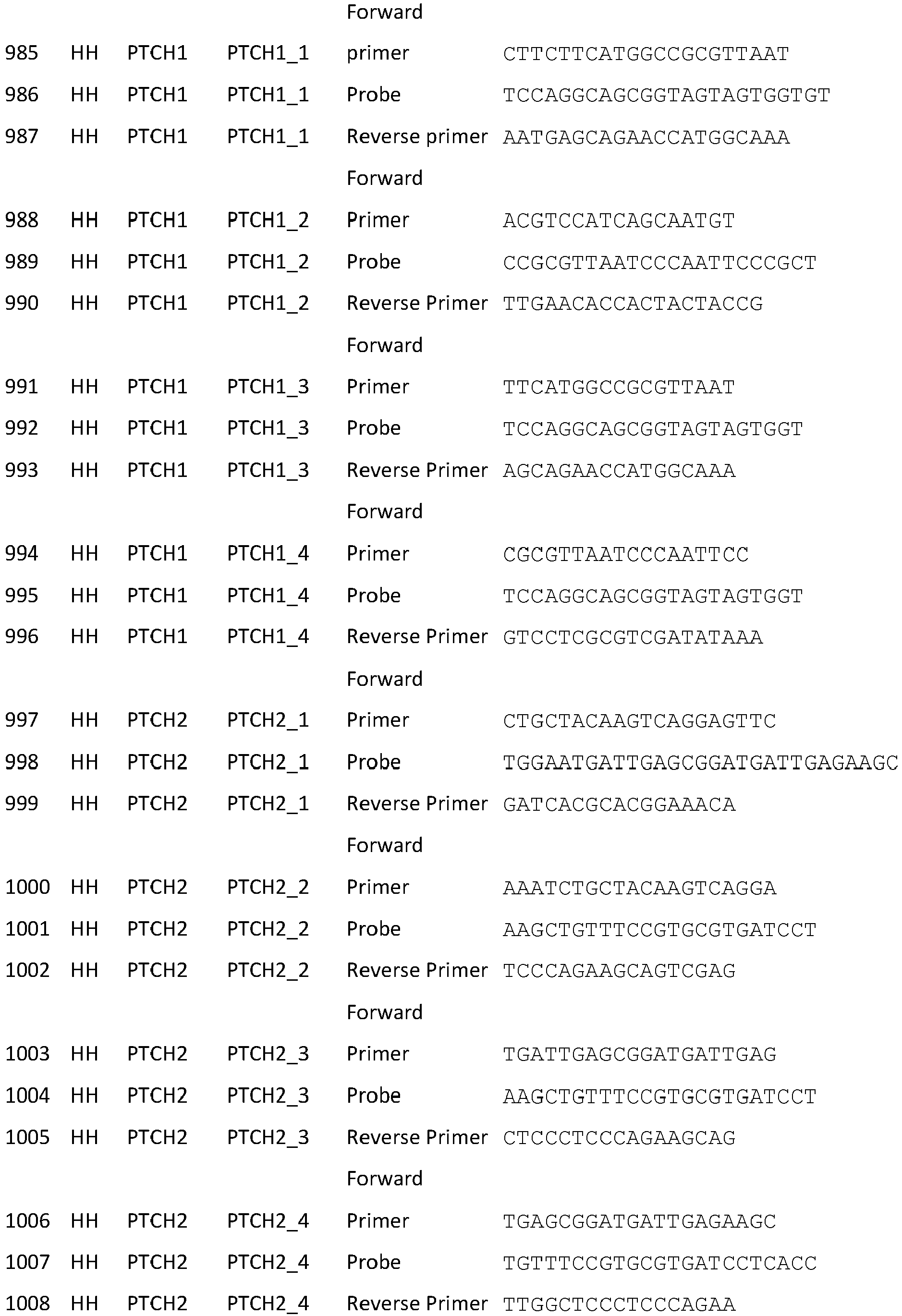

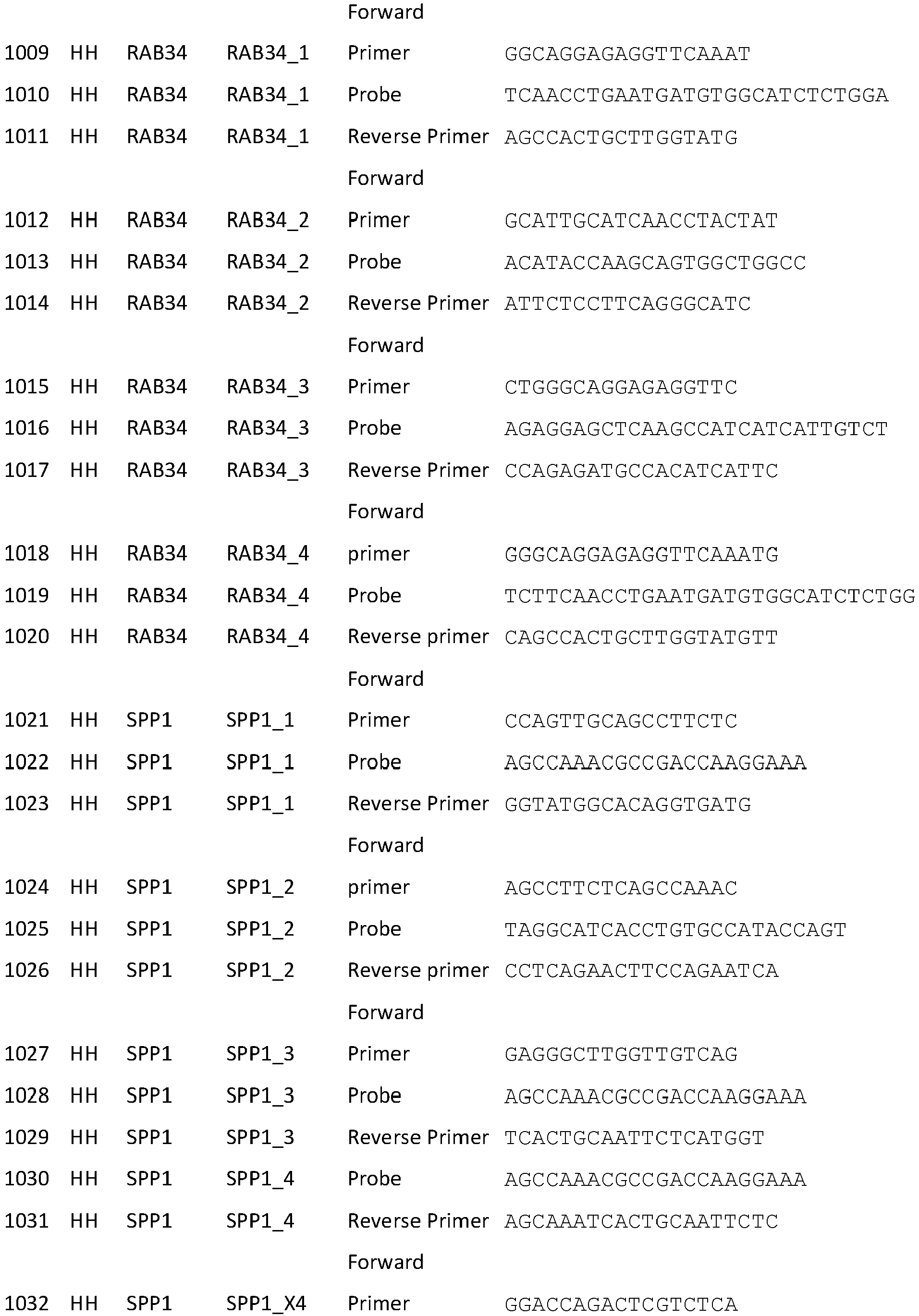

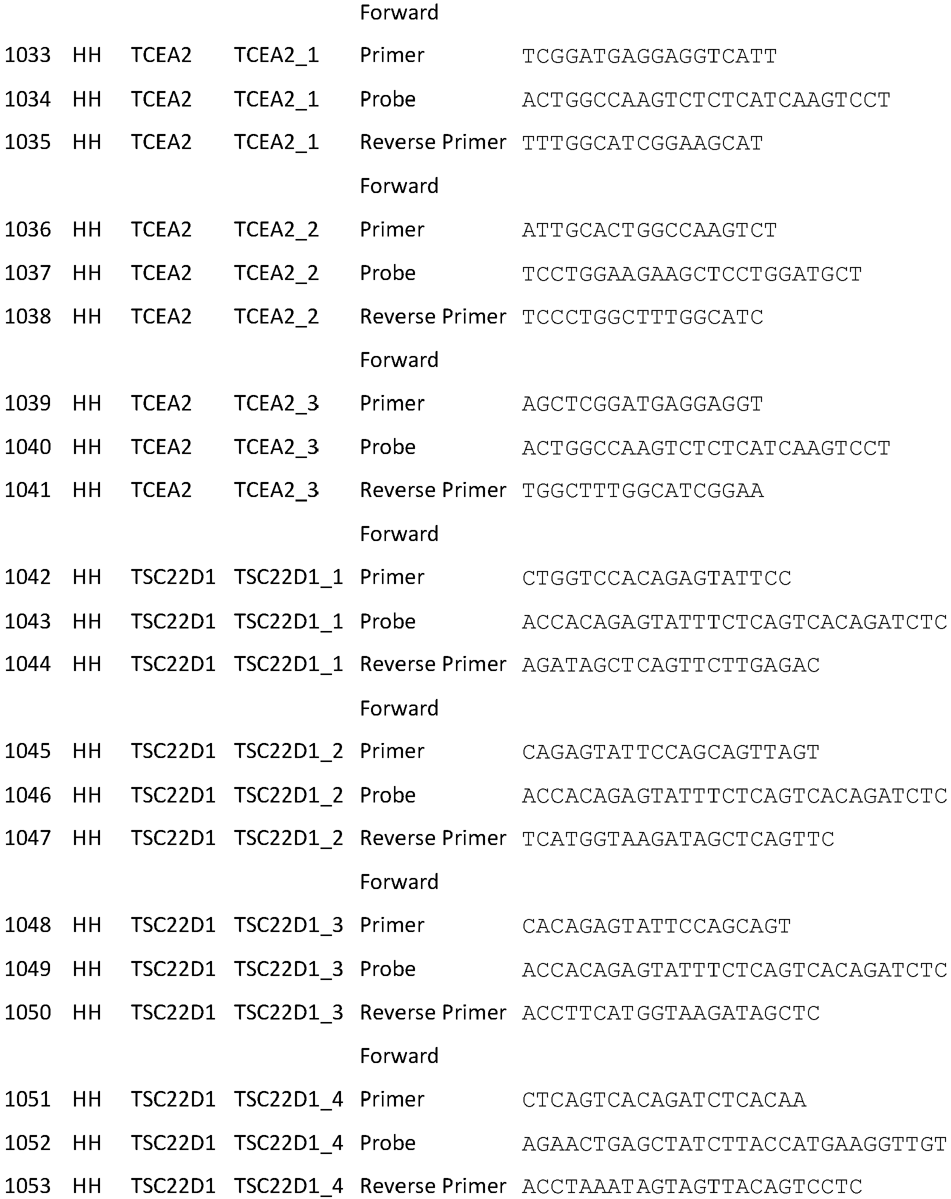

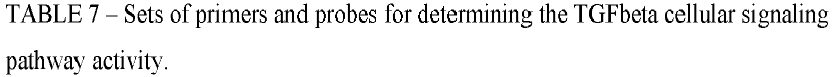

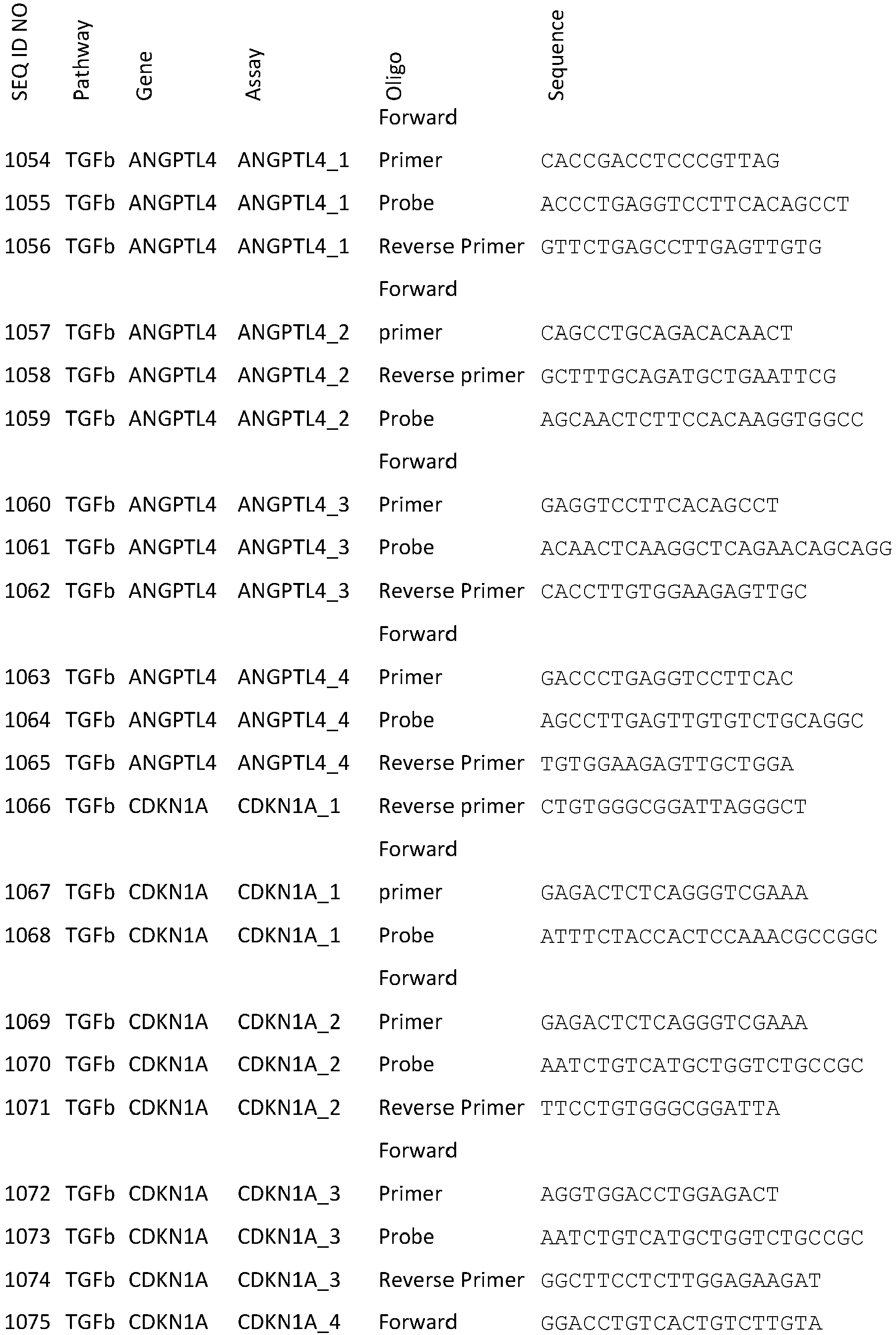

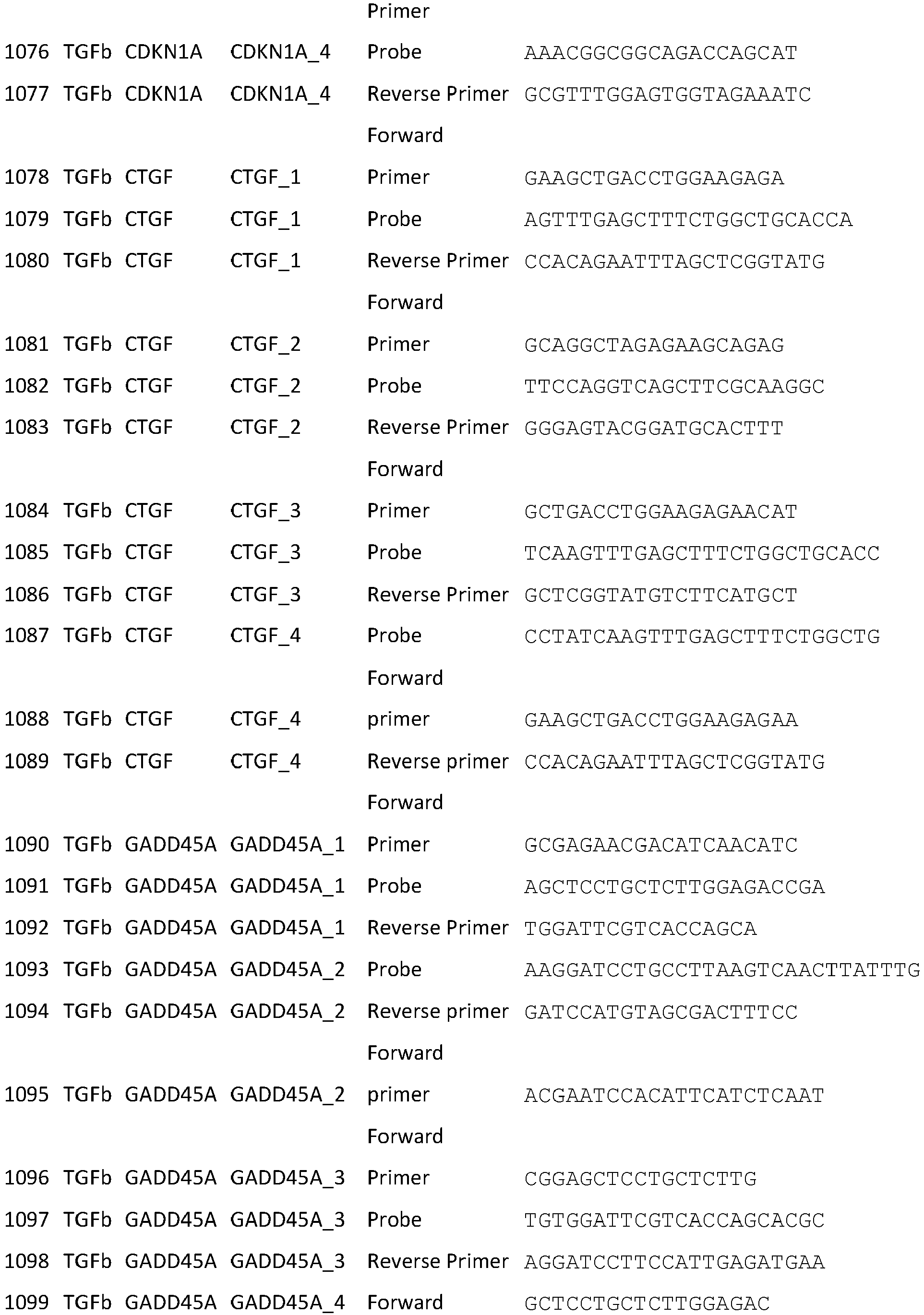

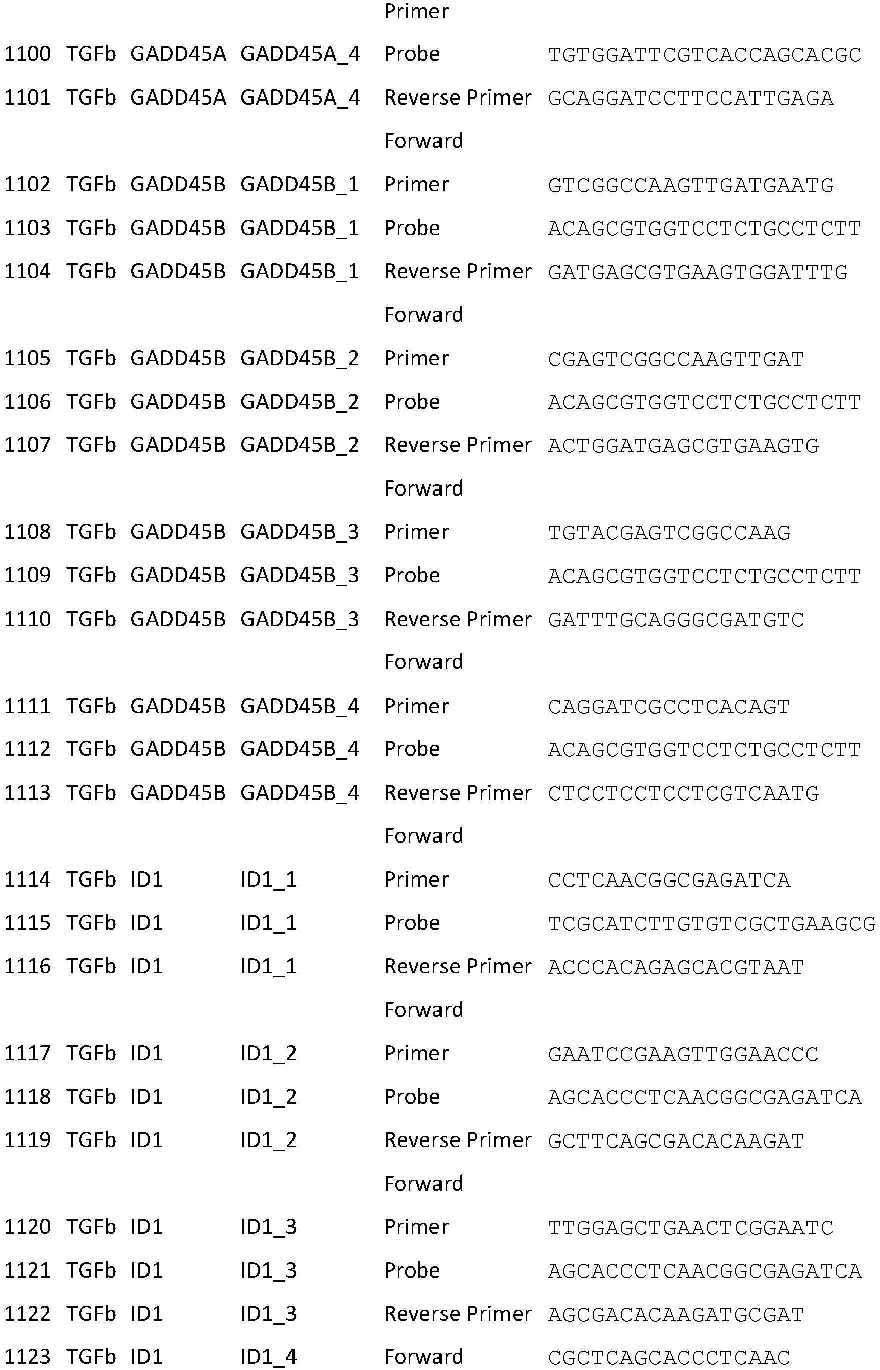

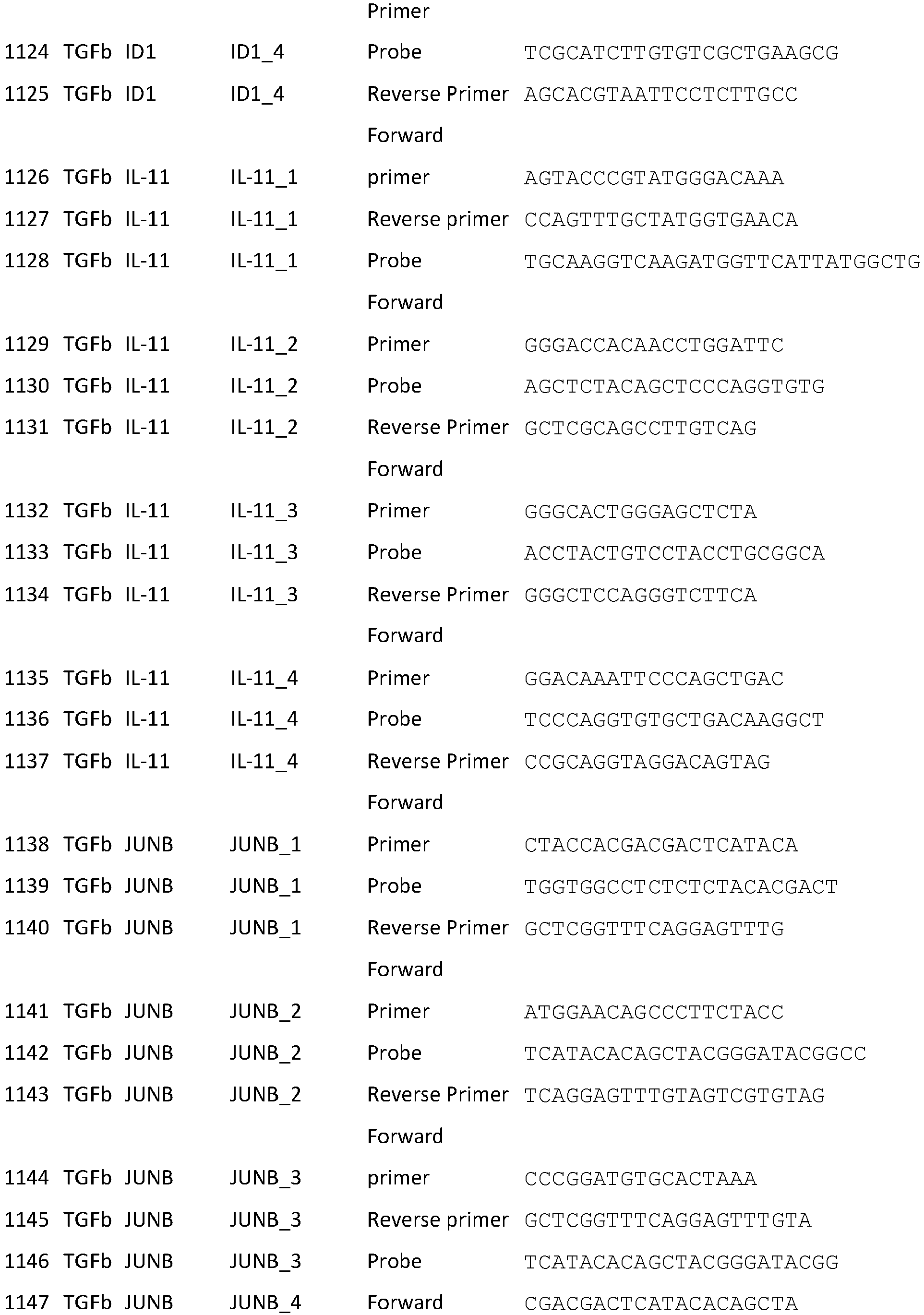

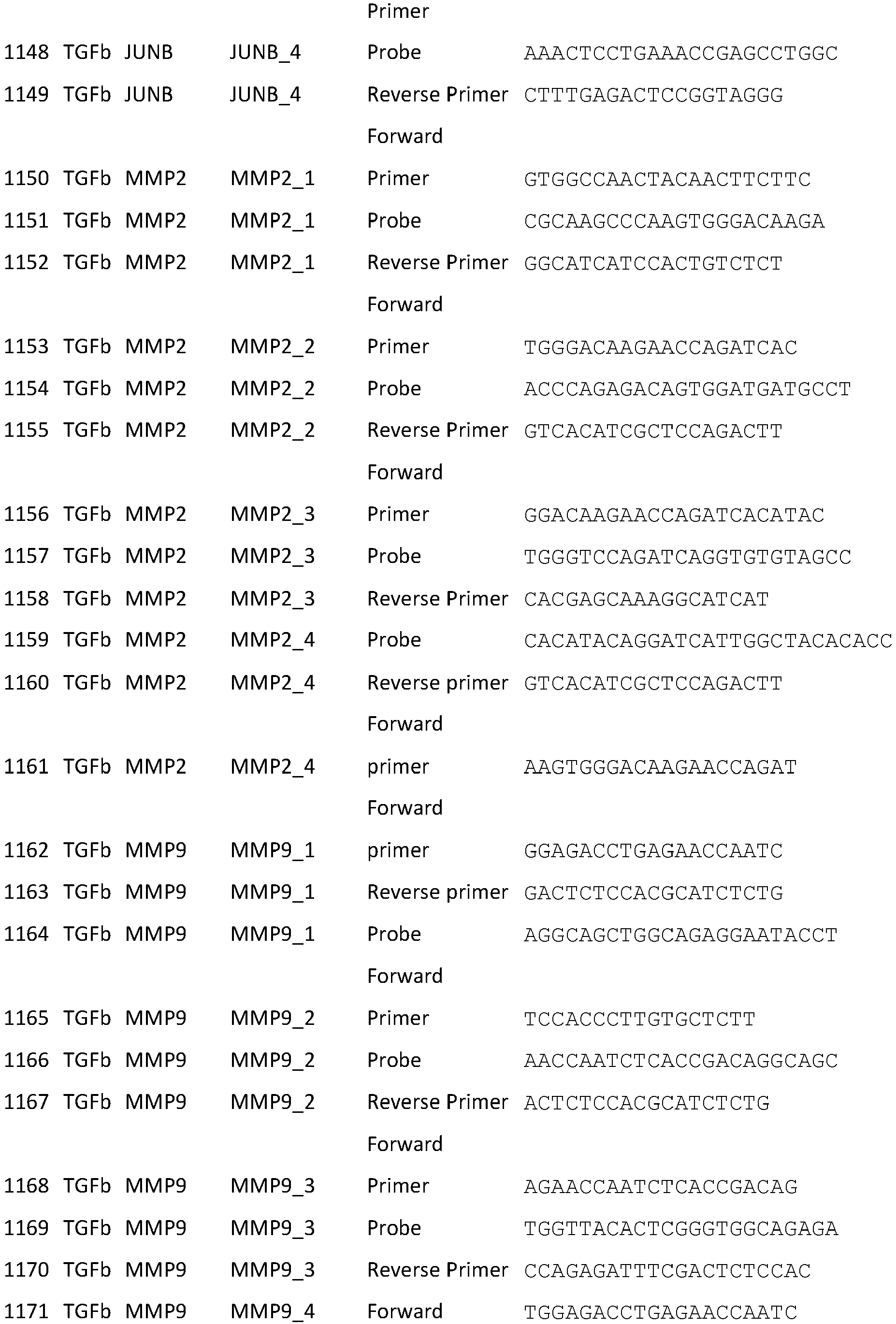

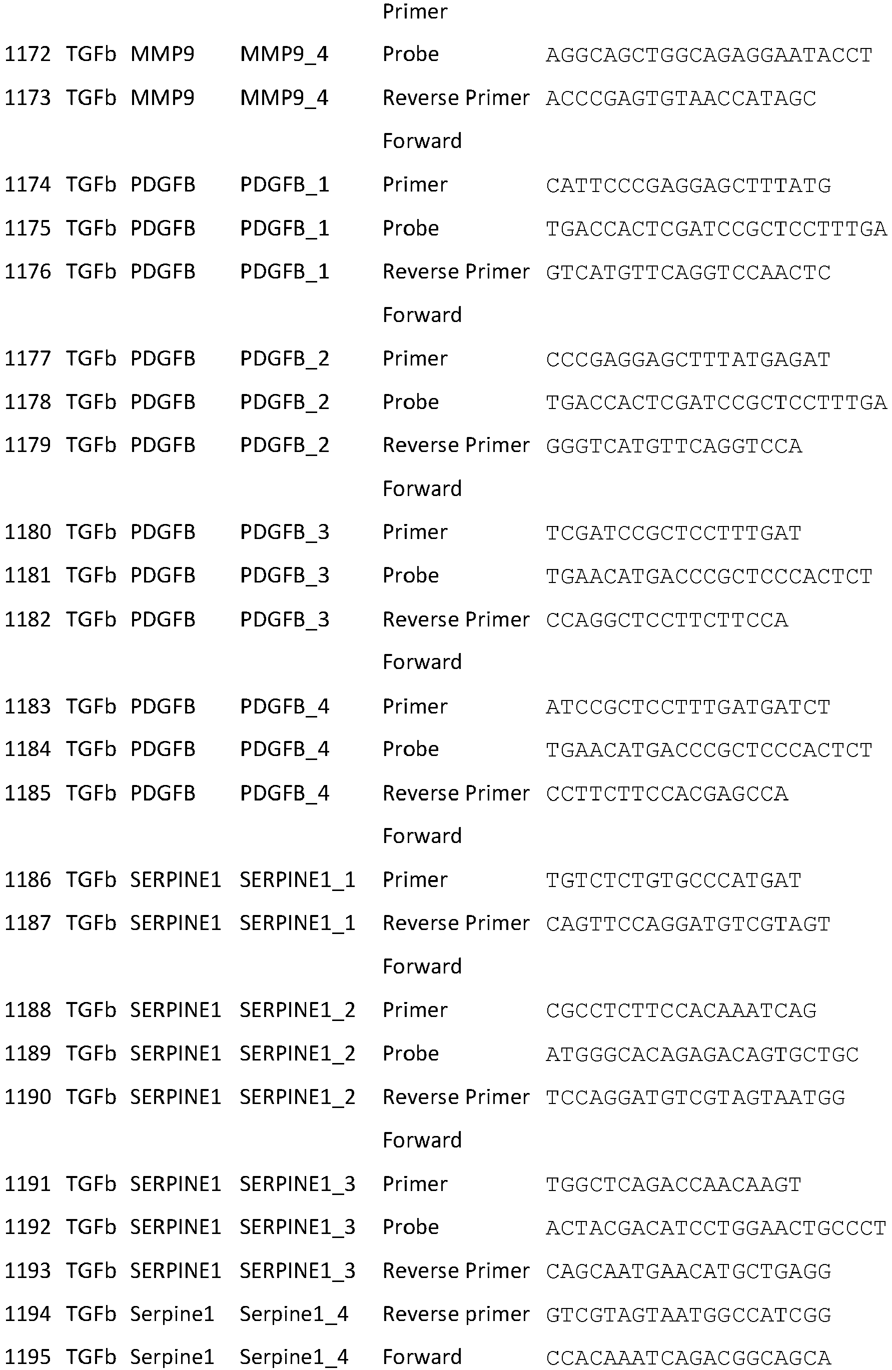

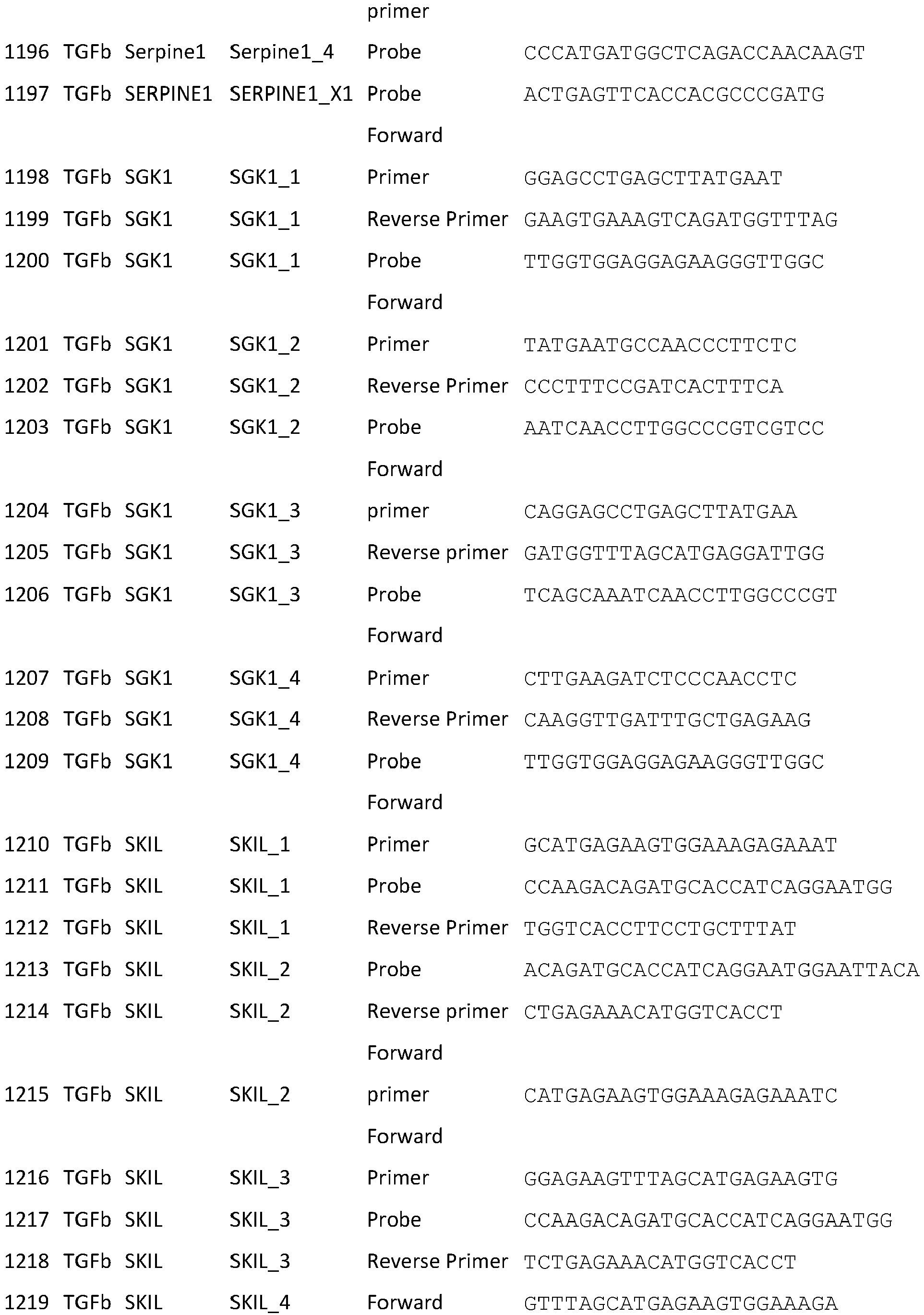

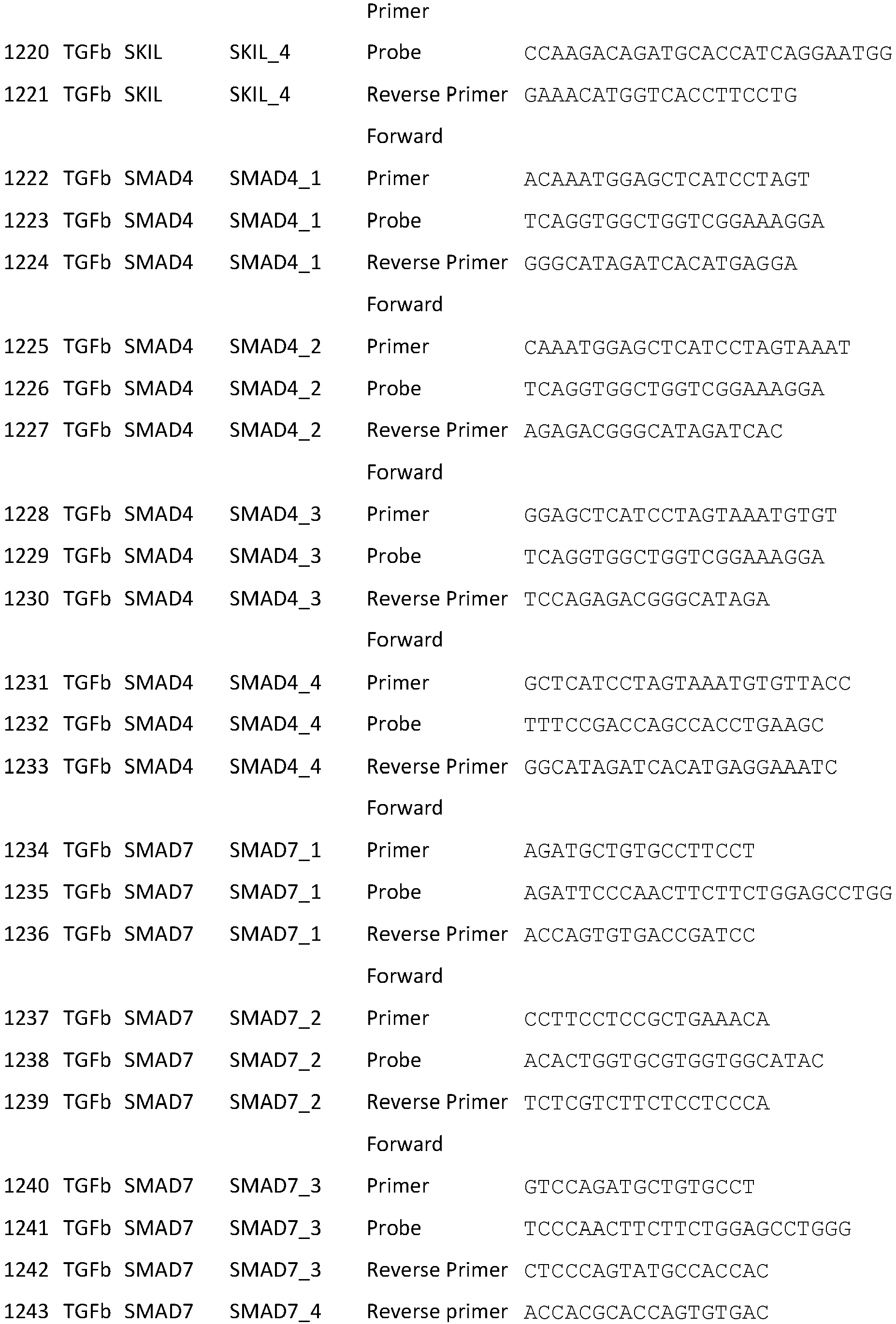

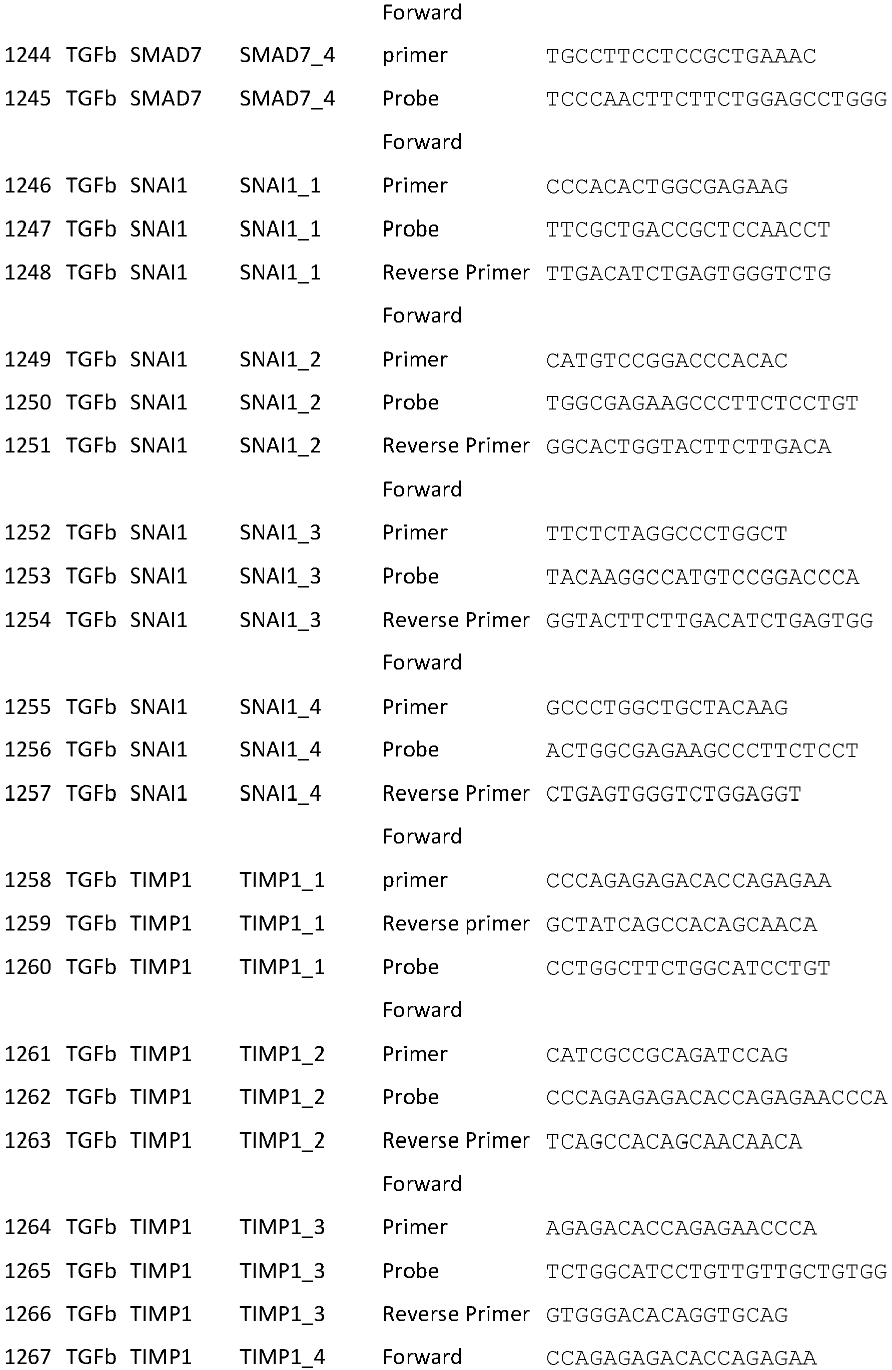

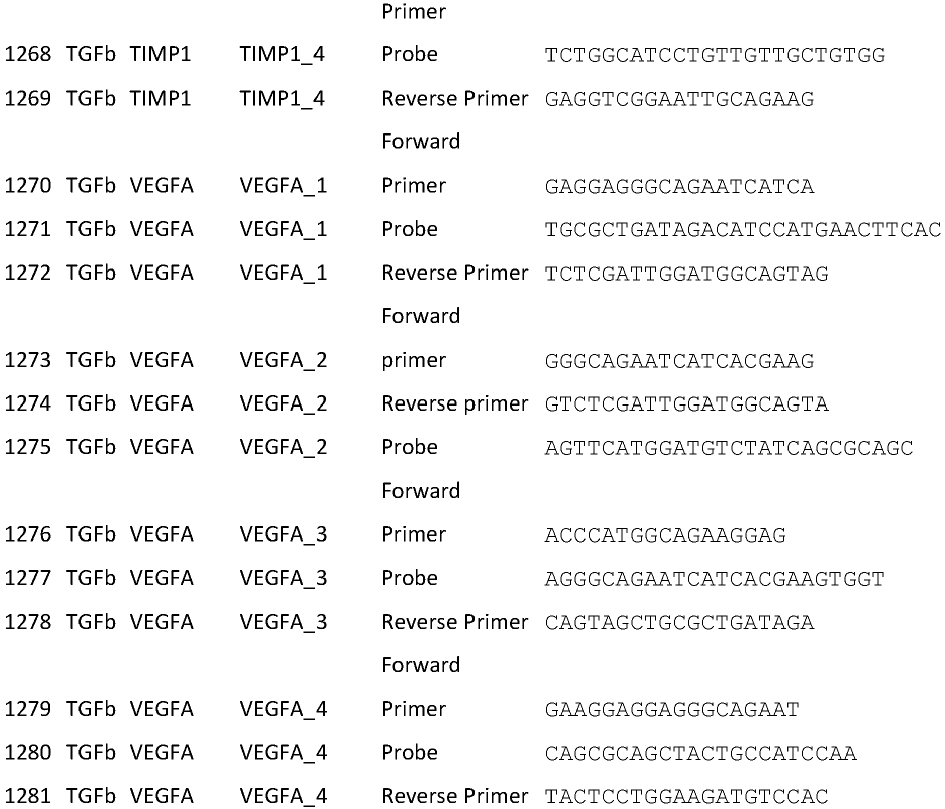

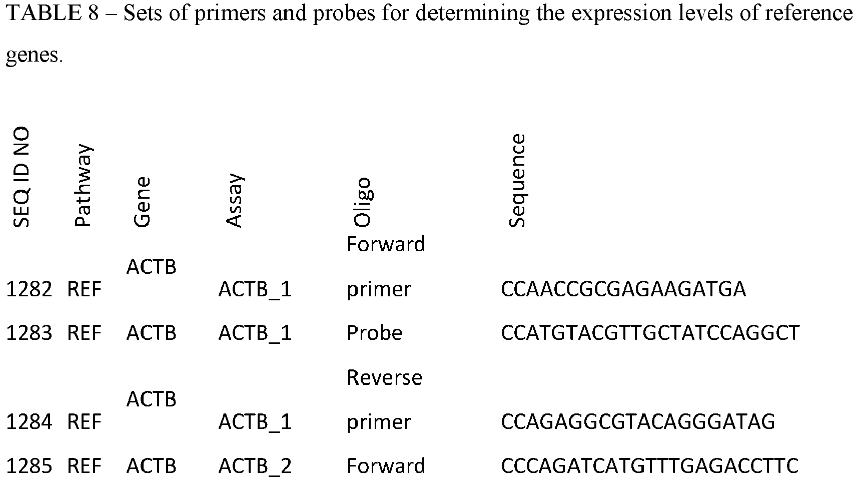

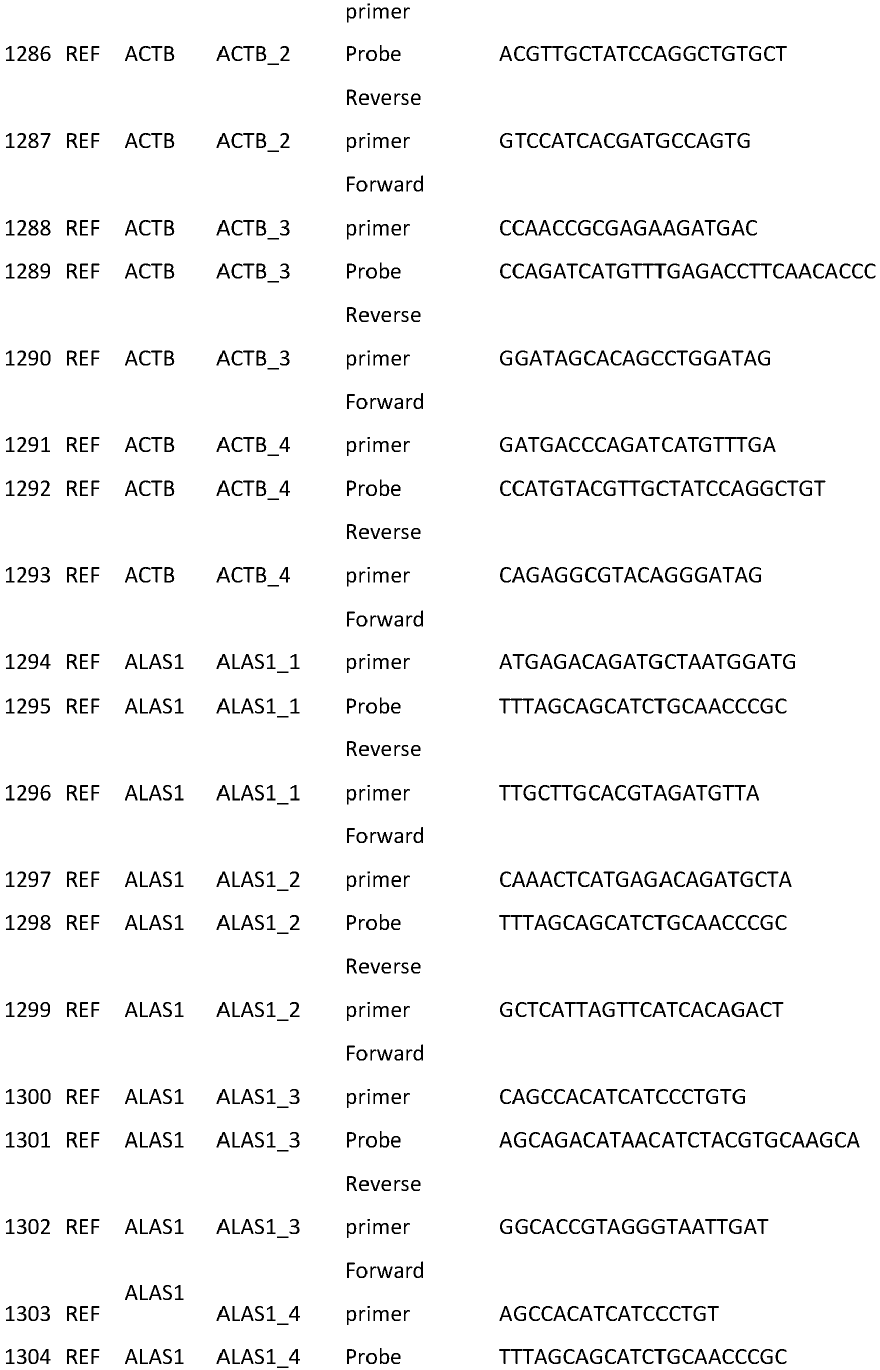

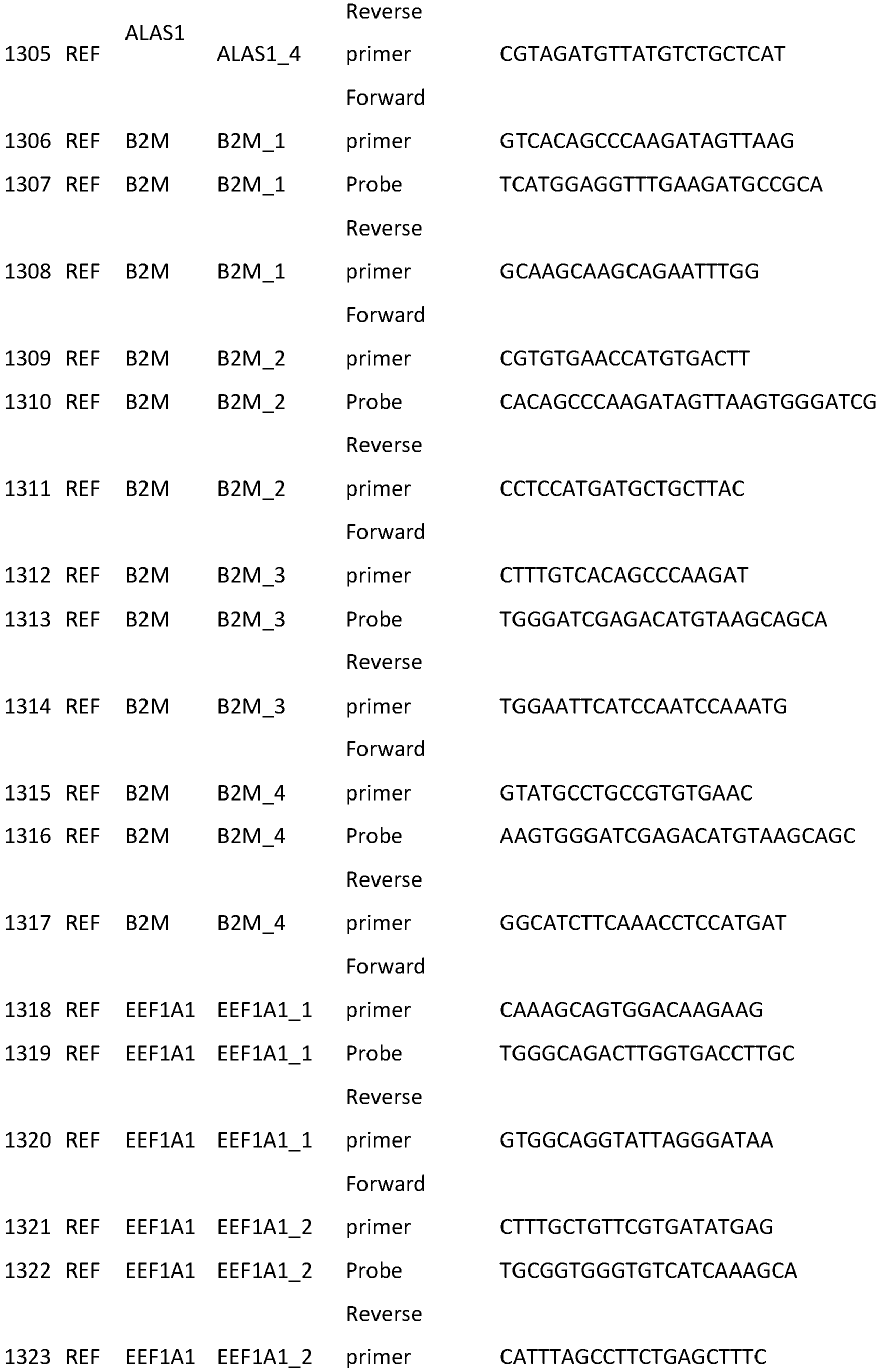

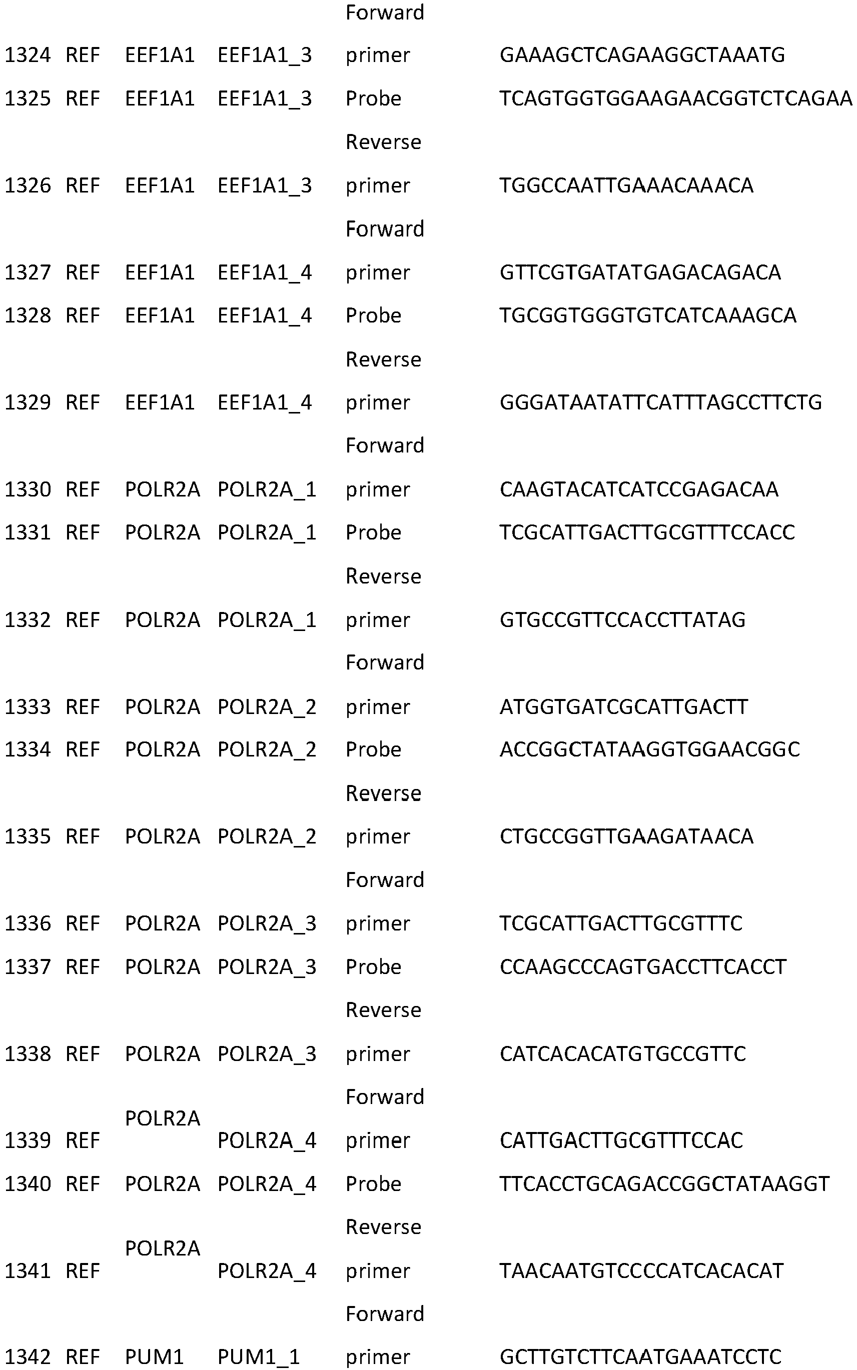

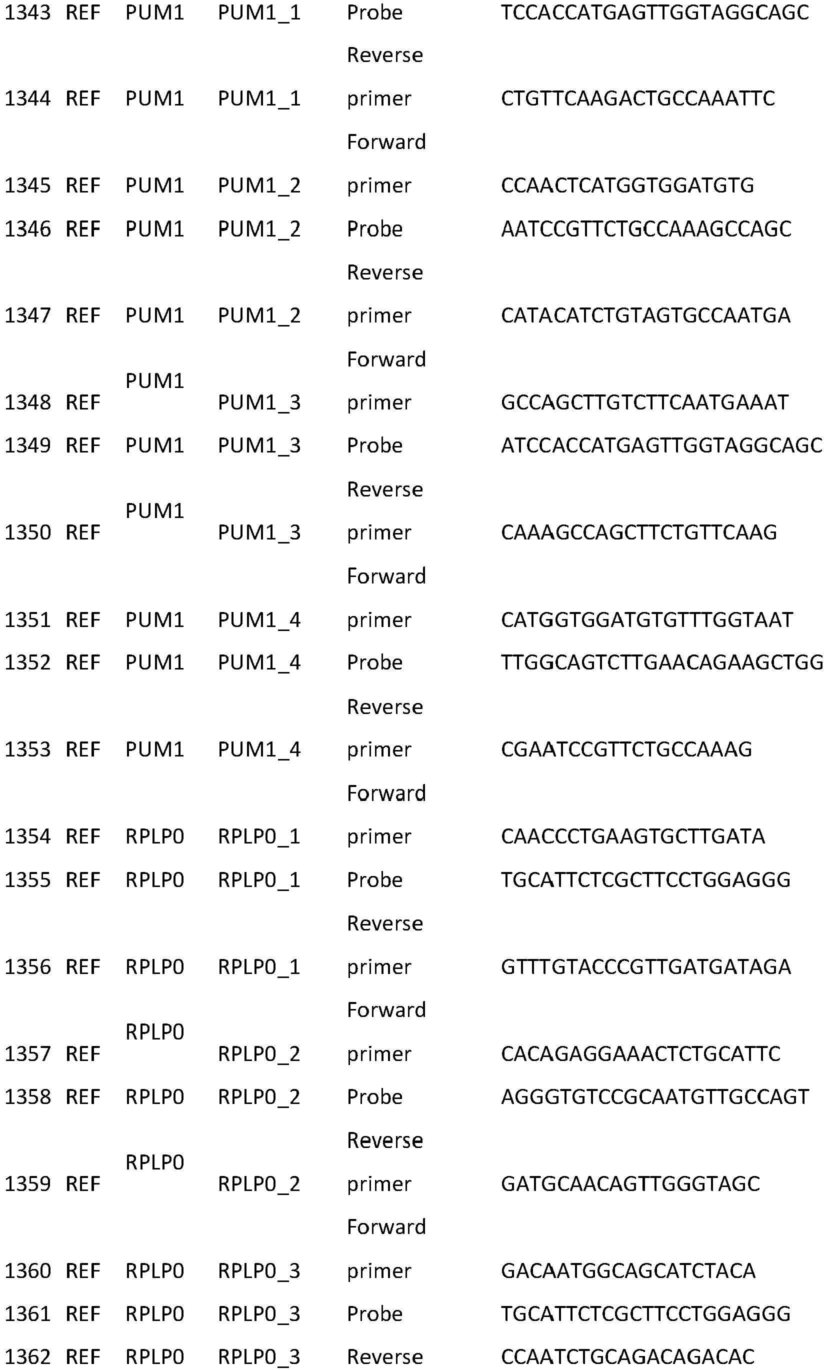

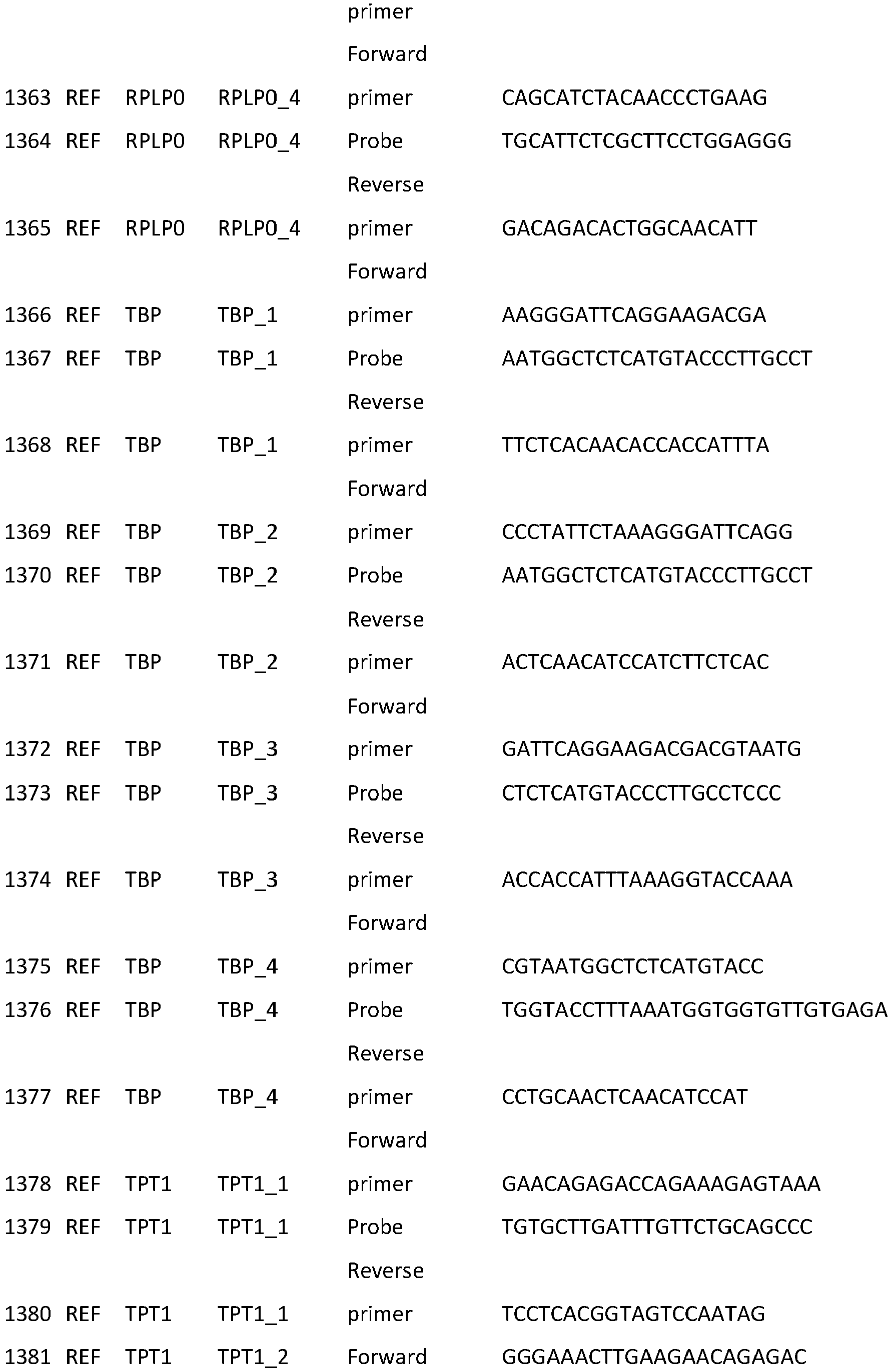

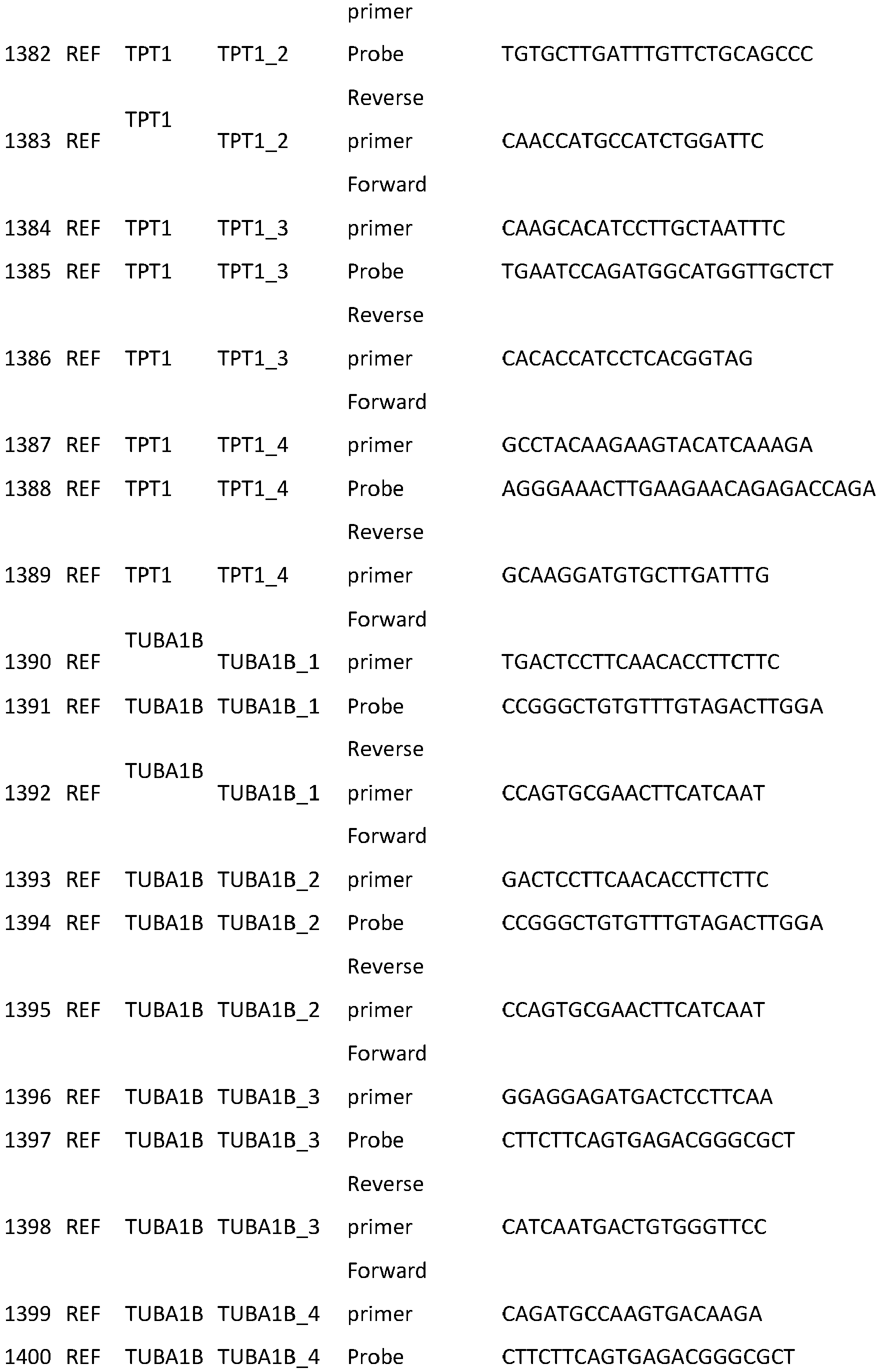

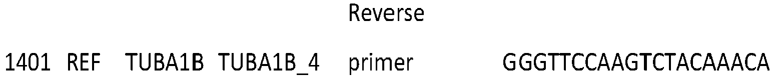

**TABLE 9 - Validation of selected representative primers and probes**

| **SEQ ID NO:** | **Assay** | **Oligo** | **Strand** | **GC content** | **Length** | **Tm*** |
|---|---|---|---|---|---|---|
| 223 | ABCC4_2 | Forward primer | Sense | 55,0% | 20 | 64,4 |
| 224 | ABCC4_2 | Reverse Primer | Anti-sense | 45,8% | 24 | 63,6 |
| 225 | ABCC4_2 | Probe | Sense | 53,8% | 26 | 69,8 |
| 84 | GREB1_2 | Forward primer | Sense | 45% | 20 | 61 |
| 83 | GREB1_2 | Reverse Primer | Anti-Sense | 46% | 22 | 69 |
| 82 | GREB1_2 | Probe | Sense | 50% | 26 | 61 |
| 565 | GADD45A_2 | Forward primer | Sense | 36% | 22 | 52,2 |
| 566 | GADD45A_2 | Reverse Primer | Anti-Sense | 50% | 20 | 53 |
| 567 | GADD45A_2 | Probe | Sense | 38% | 29 | 57,1 |

## Claims

1. A method for simultaneously determining the expression level of six or more genes in a sample, the method comprising simultaneously amplifying six or more gene products using a polymerase chain reaction to generate a plurality of amplification products, followed by the detection of the plurality of amplification products using a plurality of probes,
wherein the polymerase chain reaction uses, for each amplification product, a forward and a reverse primer which have the following characteristics:
- the forward and reverse primer have a GC content between 35% and 69%, preferably between 35% and 65%;
- the forward and reverse primer have a melting temperature between 50 and 71 degrees Celsius, preferably between 58 and 64 degrees Celsius;
- the forward and reverse primer have a length between 16 and 25 nucleotides, preferably between 17 and 24 nucleotides;
wherein the amplification products have a size between 60 and 240 base pairs, preferably between 65 and 150 base pairs, and preferably wherein the amplicon product is intron spanning,
wherein each of the probes used for detection of an amplification product comprises a binding part which is complementary to a part of the amplification product, the binding part further having the following characteristics:
- the binding part of the probe has a GC content between 35% and 69%, preferably between 40% and 60%;
- the binding part of the probe has a melting temperature between 56 and 72 degrees Celsius, preferably between 64 and 72 degrees Celsius;
- the binding part of the probe has a length between 17 and 31 nucleotides, preferably between 18 and 30 nucleotides;
- the binding part of the probe does not have a G at the 5' part,
wherein the expression levels are suitable for use in a method for determining one or more cellular signaling pathway activities selected from the group consisting of:
WNT, HH, AR, ER, PR, PR, TGFbeta, NFkB, STAT1/2, STAT3, PI3K-FOXO, Notch, MAPK-AP1, and
wherein the primers and probes amplify and detect of the expression levels of three or more of the reference genes selected from: ACTB, ALAS1, B2M, EEF1A1 POLR2A, PUM1, RPLPO, TBP, TPT1 and TUBA1B, and
wherein the primers and probes further amplify and detect the expression levels of three or more target genes for one or more cellular signaling pathways selected from the group consisting of: AR, ER, PI3K-FOXO, MAPK-AP1, HH, Notch, TGFbeta, WNT, PR, NFkB, JAK-STAT1/2, JAK-STAT3,
wherein the three or more target genes for the ER cellular signaling pathway are selected from the group consisting of: AP1B1, CA12, CDH26, CELSR2, CTSD, ERBB2, ESR1, GREB1, HSPB1, IGFBP4, MYC, NRIP1, PDZK1, PGR, RARA, SGK3, SOD1, TFF1, WISP2, and XBP1;
the three or more target genes for the AR cellular signaling pathway are selected from the group consisting of: ABCC4, AR, CREB3L4, DHCR24, ELL2, FKBP5, GUCY1A3, KLK2, KLK3, LRIG1, NDRG1, NKX3.1 (also known as NKX3_1), PLAU, PMEPA1, PPAP2A, PRKACB 2, SGK1, and TMPRSS2;
the three or more target genes for the PI3K-FOXO cellular signaling pathway are selected from the group consisting of: AGRP, BCL2L11, BCL6, BNIP3, BTG1, CAT, CAV1, CCND1, CCNG2, CDKN1A, CDKN1B, ESR1,FBXO32, FOXO3, GADD45A, INSR, MXI1, SOD2, TNFSF10;
wherein the three or more target genes for the MAPK-AP1 cellular signaling pathway are selected from the group consisting of: BCL2L11, CCND1, DDIT3, EGFR, ENPP2, EZR, GLRX, MMP1, MMP3, MMP9, PLAU, PLAUR, PTGS2, SERPINE1, TIMP1, TP53, VEGFD, and VIM;
wherein the three or more target genes for the Notch cellular signaling pathway are selected from the group consisting of: CD44, EPHB3, FABP7, HES1, HES4, HES5, HEY1, HEY2, MYC, NOX1, NRARP, PIN1, PLXND1, and SOX9;
wherein the three or more target genes for the HH cellular signaling pathway are selected from the group consisting of: CFLAR, FOXM1, FYN, GLI1, HHIP, MYCN, NKX2-2, PTCH1, PTCH2, RAB34, SPP1, TCEA2, and TSC22D1;
wherein the three or more target genes for the TGFbeta cellular signaling pathway are selected from the group consisting of: ANGPTL4, CDKN1A, CTGF, GADD45A, GADD45B, ID1, IL11, JUNB, MMP2, MMP9, PDGFB, SERPINE1, SGK1, SKIL, SMAD4, SMAD7, SNAI1, TIMP1, and VEGFA;
wherein the three or more target genes for the WNT cellular signaling pathway are selected from the group consisting of: CEMIP, AXIN2, CD44, RNF43, MYC, TBX3, TDGF1, SOX9, ASCL2, CXCL8, SP5, ZNRF3, EPHB2, LGR5, EPHB3, KLF6, CCND1, DEFA6, and FZD7;
wherein the three or more target genes for the PR cellular signaling pathway are selected from the group consisting of: AGRP, BCL2L11, BCL6, BNIP3, BTG1, CAT, CAV1, CCND1, CCND2, CCNG2, CDKN1A, CDKN1B, ESR1, FASLG, FBXO32, GADD45A, INSR, MXI1, NOS3, PCK1, POMC, PPARGC1A, PRDX3, RBL2, SOD2 and TNFSF10;
wherein the three or more target genes for the NFkB cellular signaling pathway are selected from the group consisting of: BCL2L1, BIRC3, CCL2, CCL3, CCL4, CCL5, CCL20, CCL22, CX3CL1, CXCL1, CXCL2, CXCL3, ICAM1, IL1B, IL6, IL8, IRF1, MMP9, NFKB2, NFKBIA, NFKBIE, PTGS2, SELE, STAT5A, TNF, TNFAIP2, TNIP1 , TRAF1 , and VCAM1;
wherein the three or more target genes for the JAK-STAT1/2 cellular signaling pathway are selected from the group consisting of: APOL1, BID, CXCL9, GBP1, GNAZ, IFI6, IFIT2, IFITM1, IRF1, IRF7, IRF8, IRF9, ISG15, LGALS1, NCF4, NFAM1, OAS1, PDCD1, RAB36, RBX1, RFPL3, SAMM50, SMARCB1, SSTR3, ST13, STAT1, TAP1, TRMT1, UFD1L, USP18, and ZNRF3;
wherein the three or more target genes for the JAK-STAT3 cellular signaling pathway are selected from the group consisting of: AKT1, BCL2, BCL2L1, BIRC5, CCND1, CD274, CDKNIA, CRP, FGF2, FOS, FSCN1, FSCN2, FSCN3, HIFIA, HSP90AA1, HSP90AB1, HSP90B1, HSPA1A, HSPA1B, ICAM1, IFNG, IL10, JunB, MCL1, MMP1, MMP3, MMP9, MUC1, MYC, NOS2, POU2F1, PTGS2, SAA1, STAT1, TIMP1, TNFRSF1B, TWIST1, VIM and ZEB1.

2. Method according to claim 1, wherein the primers and probes are able to amplify and detect the respective genes under the following reaction conditions:
| | |
|---|---|
| 50 mM | monovalent salt; |
| 400 nM | forward primer |
| 400 nM | reverse primer |
| 3.0 mM | divalent salt, preferably the divalent salt being Mg²⁺; |
| | |
|---|---|
| 100 nM | probe; and |
| 0.8 mM | dNTP. |

3. Method according to any one of the previous claims, wherein the method is used to determine one or more cellular signaling pathway activity or activities.

4. Assembly of primers and probes for determining the activity of the ER cellular signaling pathway, wherein the assembly of primers and probes comprises at least three sets of primers or probes for determining the expression level of three or more target genes of the ER cellular signaling pathway, wherein said three or more sets of primers and probes are selected from Table 1 of the description,
wherein each primer and/or probe individually has the listed nucleotide sequence identified by the corresponding SEQ ID NO or has a sequence that differs at 1, 2 or 3 positions, wherein said 1, 2 or 3 differences are individually selected from a single base substitution, a single base deletion or a single base addition.

5. Assembly of primers and probe for determining the activity of the AR cellular signaling pathway, wherein the assembly of primers and probes comprises at least three sets of primers or probes for determining the expression level of three or more target genes of the AR cellular signaling pathway, wherein said three or more sets of primers and probes are selected from Table 2 of the description,
wherein each primer and/or probe individually has the listed nucleotide sequence identified by the corresponding SEQ ID NO or has a sequence that differs at 1, 2 or 3 positions, wherein said 1, 2 or 3 differences are individually selected from a single base substitution, a single base deletion or a single base addition.

6. Assembly of primers and probe for determining the activity of the PI3K-FOXO cellular signaling pathway, wherein the assembly of primers and probes comprises at least three sets of primers or probes for determining the expression level of three or more target genes of the PI3K-FOXO cellular signaling pathway, wherein said three or more sets of primers and probes are selected from Table 3 of the description,
wherein each primer and/or probe individually has the listed nucleotide sequence identified by the corresponding SEQ ID NO or has a sequence that differs at 1, 2 or 3 positions, wherein said 1, 2 or 3 differences are individually selected from a single base substitution, a single base deletion or a single base addition.

7. Assembly of primers and probe for determining the activity of the MAPK-AP1 cellular signaling pathway, wherein the assembly of primers and probes comprises at least three sets of primers or probes for determining the expression level of three or more target genes of the MAPK-AP1 cellular signaling pathway, wherein said three or more sets of primers and probes are selected from Table 4 of the description,
wherein each primer and/or probe individually has the listed nucleotide sequence identified by the corresponding SEQ ID NO or has a sequence that differs at 1, 2 or 3 positions, wherein said 1, 2 or 3 differences are individually selected from a single base substitution, a single base deletion or a single base addition.

8. Assembly of primers and probe for determining the activity of the Notch cellular signaling pathway, wherein the assembly of primers and probes comprises at least three sets of primers or probes for determining the expression level of three or more target genes of the Notch cellular signaling pathway, wherein said three or more sets of primers and probes are selected from Table 5 of the description,
wherein each primer and/or probe individually has the listed nucleotide sequence identified by the corresponding SEQ ID NO or has a sequence that differs at 1, 2 or 3 positions, wherein said 1, 2 or 3 differences are individually selected from a single base substitution, a single base deletion or a single base addition.

9. Assembly of primers and probe for determining the activity of the Hedgehog (HH) cellular signaling pathway, wherein the assembly of primers and probes comprises at least three sets of primers or probes for determining the expression level of three or more target genes of the HH cellular signaling pathway, wherein said three or more sets of primers and probes are selected from Table 6 of the description,
wherein each primer and/or probe individually has the listed nucleotide sequence identified by the corresponding SEQ ID NO or has a sequence that differs at 1, 2 or 3 positions, wherein said 1, 2 or 3 differences are individually selected from a single base substitution, a single base deletion or a single base addition.

10. Assembly of primers and probe for determining the activity of the TGFbeta cellular signaling pathway, wherein the assembly of primers and probes comprises at least three sets of primers or probes for determining the expression level of three or more target genes of the TGFbeta cellular signaling pathway, wherein said three or more sets of primers and probes are selected from Table 4 of the description,
wherein each primer and/or probe individually has the listed nucleotide sequence identified by the corresponding SEQ ID NO or has a sequence that differs at 1, 2 or 3 positions, wherein said 1, 2 or 3 differences are individually selected from a single base substitution, a single base deletion or a single base addition.

11. Assembly of primers and probes according to any one of claims 4 to 10, wherein all of the primers and probes in the three or more sets of primers and probes in the assembly are identical to the corresponding sequence according to Tables 1 to 7.

12. A kit of parts for determining the expression levels for a plurality of genes, the kit comprising primers and probes for the amplification and detection of the expression levels of the plurality of genes, wherein the kit comprises primers and probes are as defined in any one of claims 1 to 3 or an assembly of primers and probes as defined in any one of claims 4 to 11, wherein the kit further comprises primers and probes for the amplification and detection of three or more of the reference genes selected from ACTB, ALAS1, B2M, EEF1A1 POLR2A, PUM1, RPLP0, TBP, TPT1 and TUBA1B.

13. Use of the primers and probes as defined in any one of claims 1 to 11 or the kit as defined in claim 12 for determining the cellular signaling pathway activity for one or more cellular signaling pathways selected from the group consisting of: HH, AR, ER, TGFbeta, PI3K-FOXO, Notch and MAPK-AP1.

14. Use of a set of three or more primers and probes to determine the expression levels of three or more target genes of a cellular signaling pathway, wherein the set of primers and probe combinations are as defined in any one of claims 1 to 11, and wherein the target genes are as defined in claim 1.

15. A method for designing primers and probes for the detection of the expression levels of target genes of a cellular signaling pathway suitable for determining the activity of said cellular signaling pathway, the method comprising:
- designing for a target gene of a cellular signaling pathway a forward primer and a reverse primer such that:
- the forward and reverse primer have a GC content between 35% and 69%, preferably between 35% and 65%;
- the forward and reverse primer have a melting temperature between 50 and 71 degrees Celsius, preferably between 58 and 64 degrees Celsius;
- the forward and reverse primer have a length between 16 and 25 nucleotides, preferably between 17 and 24 nucleotides;
wherein the amplification product, when using the forward and reverse primers in a PCR amplification reaction, has a size between 60 and 240 base pairs, preferably between 65 and 150 base pairs, and preferably wherein the amplicon product is intron spanning;
- designing the probe such that:
- the probe used for detection of an amplification product comprises a binding part which is complementary to a part of the amplification product, the binding part further having the following characteristics:
- the binding part of the probe has a GC content between 35% and 69%, preferably between 40% and 60%;
- the binding part of the probe has a melting temperature between 56 and 72 degrees Celsius, preferably between 64 and 72 degrees Celsius;
- the binding part of the probe has a length between 17 and 31 nucleotides, preferably between 18 and 30 nucleotides;
- the binding part of the probe does not have a G at the 5' part.


SEQUENCE LISTING

<110>  Koninklijke Philips N.V.

<120>  PCR Kit for cellular signaling pathway analysis

<130>  2020PF00289

<160>  1401

<170>  PatentIn version 3.5

<210>  1
<211>  17
<212>  DNA
<213>  Artificial Sequence

<220>
<223>  oligonucleotide

<400>  1
aggctgtgcg tgctatt                                                    17


<210>  2
<211>  24
<212>  DNA
<213>  Artificial Sequence

<220>
<223>  oligonucleotide

<400>  2
ccatcaaggt ggagcaatct gcgg                                            24


<210>  3
<211>  20
<212>  DNA
<213>  Artificial Sequence

<220>
<223>  oligonucleotide

<400>  3
ttgaccttgg tctggatgag                                                 20


<210>  4
<211>  20
<212>  DNA
<213>  Artificial Sequence

<220>
<223>  oligonucleotide

<400>  4
catcaaggtg gagcaatctg                                                 20


<210>  5
<211>  24
<212>  DNA

<213>  Artificial Sequence

<220>
<223>  oligonucleotide

<400>  5
tgctcgacct catccagacc aagg                                              24


<210>  6
<211>  20
<212>  DNA
<213>  Artificial Sequence

<220>
<223>  oligonucleotide

<400>  6
ctcctggacc acatagttga                                                   20


<210>  7
<211>  19
<212>  DNA
<213>  Artificial Sequence

<220>
<223>  oligonucleotide

<400>  7
gtgttggcag agctgaaag                                                    19


<210>  8
<211>  24
<212>  DNA
<213>  Artificial Sequence

<220>
<223>  oligonucleotide

<400>  8
actttgtacg gaaggctgtg cgtg                                              24


<210>  9
<211>  20
<212>  DNA
<213>  Artificial Sequence

<220>
<223>  oligonucleotide

<400>  9
cagattgctc caccttgatg                                                   20


<210>  10
<211>  20
<212>  DNA
<213>  Artificial Sequence

<220>

<223>  oligonucleotide

<400>  10
gcattcttgg catctgtatt                                                    20


<210>  11
<211>  24
<212>  DNA
<213>  Artificial Sequence

<220>
<223>  oligonucleotide

<400>  11
tggtggtggt gtccatttgg cttt                                               24


<210>  12
<211>  18
<212>  DNA
<213>  Artificial Sequence

<220>
<223>  oligonucleotide

<400>  12
gtggctggct tgtaaatg                                                      18


<210>  13
<211>  24
<212>  DNA
<213>  Artificial Sequence

<220>
<223>  oligonucleotide

<400>  13
tggtggtggt gtccatttgg cttt                                               24


<210>  14
<211>  21
<212>  DNA
<213>  Artificial Sequence

<220>
<223>  oligonucleotide

<400>  14
ggcattcttg gcatctgtat t                                                  21


<210>  15
<211>  22
<212>  DNA
<213>  Artificial Sequence

<220>
<223>  oligonucleotide

<400>  15

gcttgtaaat gactcccttg tt                                                22

<210>  16
<211>  18
<212>  DNA
<213>  Artificial Sequence

<220>
<223>  oligonucleotide

<400>  16
tattgtggtg gtggtgtc                                                    18

<210>  17
<211>  26
<212>  DNA
<213>  Artificial Sequence

<220>
<223>  oligonucleotide

<400>  17
aagggagtca tttacaagcc agccac                                           26

<210>  18
<211>  18
<212>  DNA
<213>  Artificial Sequence

<220>
<223>  oligonucleotide

<400>  18
gcctcagtct ccatcttg                                                    18

<210>  19
<211>  17
<212>  DNA
<213>  Artificial Sequence

<220>
<223>  oligonucleotide

<400>  19
tggcattctt ggcatct                                                     17

<210>  20
<211>  24
<212>  DNA
<213>  Artificial Sequence

<220>
<223>  oligonucleotide

<400>  20
tggtggtggt gtccatttgg cttt                                             24

<210>  21
<211>  20
<212>  DNA
<213>  Artificial Sequence

<220>
<223>  oligonucleotide

<400>  21
gactcccttg ttatcacctt                                                    20


<210>  22
<211>  19
<212>  DNA
<213>  Artificial Sequence

<220>
<223>  oligonucleotide

<400>  22
cacaatgcac agacaactc                                                     19


<210>  23
<211>  24
<212>  DNA
<213>  Artificial Sequence

<220>
<223>  oligonucleotide

<400>  23
tgccaatgtg ctggaagatg accc                                               24


<210>  24
<211>  18
<212>  DNA
<213>  Artificial Sequence

<220>
<223>  oligonucleotide

<400>  24
tgtagacgtg aggtaggt                                                      18


<210>  25
<211>  18
<212>  DNA
<213>  Artificial Sequence

<220>
<223>  oligonucleotide

<400>  25
cttacccaga tgccacaa                                                      18


<210>  26
<211>  24
<212>  DNA

<213>  Artificial Sequence

<220>
<223>  oligonucleotide

<400>  26
cggtggaagg aaggatggca gaga                                              24


<210>  27
<211>  18
<212>  DNA
<213>  Artificial Sequence

<220>
<223>  oligonucleotide

<400>  27
ggtcatcttc cagcacat                                                     18


<210>  28
<211>  18
<212>  DNA
<213>  Artificial Sequence

<220>
<223>  oligonucleotide

<400>  28
ggatggcaga gacattga                                                     18


<210>  29
<211>  24
<212>  DNA
<213>  Artificial Sequence

<220>
<223>  oligonucleotide

<400>  29
tgccaatgtg ctggaagatg accc                                              24


<210>  30
<211>  17
<212>  DNA
<213>  Artificial Sequence

<220>
<223>  oligonucleotide

<400>  30
ccttcctcgc tgtagac                                                      17


<210>  31
<211>  17
<212>  DNA
<213>  Artificial Sequence

<220>

<223>  oligonucleotide

<400>  31
tgctctgacc accaagt                                                    17


<210>  32
<211>  24
<212>  DNA
<213>  Artificial Sequence

<220>
<223>  oligonucleotide

<400>  32
accctgacct cgtcctacaa ctgc                                            24


<210>  33
<211>  17
<212>  DNA
<213>  Artificial Sequence

<220>
<223>  oligonucleotide

<400>  33
tctccgtagg gctggta                                                    17


<210>  34
<211>  17
<212>  DNA
<213>  Artificial Sequence

<220>
<223>  oligonucleotide

<400>  34
tccgtagggc tggtaca                                                    17


<210>  35
<211>  18
<212>  DNA
<213>  Artificial Sequence

<220>
<223>  oligonucleotide

<400>  35
ggtccggaaa gcactcaa                                                   18


<210>  36
<211>  23
<212>  DNA
<213>  Artificial Sequence

<220>
<223>  oligonucleotide

<400>  36

tcctacaact gccccagccc cta                                              23


<210>  37
<211>  17
<212>  DNA
<213>  Artificial Sequence

<220>
<223>  oligonucleotide

<400>  37
cctgaccctg ctctgac                                                    17


<210>  38
<211>  24
<212>  DNA
<213>  Artificial Sequence

<220>
<223>  oligonucleotide

<400>  38
accctgacct cgtcctacaa ctgc                                            24


<210>  39
<211>  17
<212>  DNA
<213>  Artificial Sequence

<220>
<223>  oligonucleotide

<400>  39
gtacagccgc ccatctg                                                    17


<210>  40
<211>  18
<212>  DNA
<213>  Artificial Sequence

<220>
<223>  oligonucleotide

<400>  40
tctgaccacc aagtccac                                                   18


<210>  41
<211>  24
<212>  DNA
<213>  Artificial Sequence

<220>
<223>  oligonucleotide

<400>  41
cctgacctcg tcctacaact gccc                                            24

<210>  42
<211>  17
<212>  DNA
<213>  Artificial Sequence

<220>
<223>  oligonucleotide

<400>  42
tggtgctgtg cagagag                                                      17


<210>  43
<211>  19
<212>  DNA
<213>  Artificial Sequence

<220>
<223>  oligonucleotide

<400>  43
cctcgtttga catccacta                                                    19


<210>  44
<211>  24
<212>  DNA
<213>  Artificial Sequence

<220>
<223>  oligonucleotide

<400>  44
acacagtgtc ctggctcagg tacc                                              24


<210>  45
<211>  17
<212>  DNA
<213>  Artificial Sequence

<220>
<223>  oligonucleotide

<400>  45
tgcctctcca ctttgac                                                      17


<210>  46
<211>  19
<212>  DNA
<213>  Artificial Sequence

<220>
<223>  oligonucleotide

<400>  46
ggtacctcgt ttgacatcc                                                    19


<210>  47
<211>  24
<212>  DNA

<213>  Artificial Sequence

<220>
<223>  oligonucleotide

<400>  47
acacagtgtc ctggctcagg tacc                                              24


<210>  48
<211>  17
<212>  DNA
<213>  Artificial Sequence

<220>
<223>  oligonucleotide

<400>  48
aaagacctgc ctctcca                                                      17


<210>  49
<211>  23
<212>  DNA
<213>  Artificial Sequence

<220>
<223>  oligonucleotide

<400>  49
gtacctcgtt tgacatccac tat                                               23


<210>  50
<211>  20
<212>  DNA
<213>  Artificial Sequence

<220>
<223>  oligonucleotide

<400>  50
cgtcgtcagc ctctgccctg                                                   20


<210>  51
<211>  20
<212>  DNA
<213>  Artificial Sequence

<220>
<223>  oligonucleotide

<400>  51
acctgcctct ccactttgac                                                   20


<210>  52
<211>  23
<212>  DNA
<213>  Artificial Sequence

<220>

<223>  oligonucleotide

<400>  52
acactgtgtc ggtgccctgc cag                                               23


<210>  53
<211>  20
<212>  DNA
<213>  Artificial Sequence

<220>
<223>  oligonucleotide

<400>  53
aagacctgcc tctccacttt                                                   20


<210>  54
<211>  23
<212>  DNA
<213>  Artificial Sequence

<220>
<223>  oligonucleotide

<400>  54
gaatggtacc tcgtttgaca tcc                                               23


<210>  55
<211>  19
<212>  DNA
<213>  Artificial Sequence

<220>
<223>  oligonucleotide

<400>  55
gtcacctaca acacagaca                                                    19


<210>  56
<211>  24
<212>  DNA
<213>  Artificial Sequence

<220>
<223>  oligonucleotide

<400>  56
cgtttgagtc catgcccaat cccg                                              24


<210>  57
<211>  19
<212>  DNA
<213>  Artificial Sequence

<220>
<223>  oligonucleotide

<400>  57

cacgtccgta gaaaggtag                                                    19

<210>  58
<211>  17
<212>  DNA
<213>  Artificial Sequence

<220>
<223>  oligonucleotide

<400>  58
tgagtccatg cccaatc                                                      17

<210>  59
<211>  24
<212>  DNA
<213>  Artificial Sequence

<220>
<223>  oligonucleotide

<400>  59
acacagctgg cgccgaatgt atac                                              24

<210>  60
<211>  20
<212>  DNA
<213>  Artificial Sequence

<220>
<223>  oligonucleotide

<400>  60
gtccgtagaa aggtagttgt                                                   20

<210>  61
<211>  18
<212>  DNA
<213>  Artificial Sequence

<220>
<223>  oligonucleotide

<400>  61
cgagggccgg tatacatt                                                     18

<210>  62
<211>  21
<212>  DNA
<213>  Artificial Sequence

<220>
<223>  oligonucleotide

<400>  62
cacgtccgta gaaaggtagt t                                                 21

<210>  63
<211>  23
<212>  DNA
<213>  Artificial Sequence

<220>
<223>  oligonucleotide

<400>  63
agctgtgtga ctgcctgtcc cta                                              23


<210>  64
<211>  18
<212>  DNA
<213>  Artificial Sequence

<220>
<223>  oligonucleotide

<400>  64
gagggccggt atacattc                                                    18


<210>  65
<211>  24
<212>  DNA
<213>  Artificial Sequence

<220>
<223>  oligonucleotide

<400>  65
tttctacgga cgtgggatcc tgca                                             24


<210>  66
<211>  18
<212>  DNA
<213>  Artificial Sequence

<220>
<223>  oligonucleotide

<400>  66
ctgtcacctc ttggttgt                                                    18


<210>  67
<211>  19
<212>  DNA
<213>  Artificial Sequence

<220>
<223>  oligonucleotide

<400>  67
cttcgatgat gggcttact                                                   19


<210>  68
<211>  24
<212>  DNA

<213>  Artificial Sequence

<220>
<223>  oligonucleotide

<400>  68
catgtgaacc agctccctgt ctgc                                              24


<210>  69
<211>  18
<212>  DNA
<213>  Artificial Sequence

<220>
<223>  oligonucleotide

<400>  69
ggagggtcaa atccacaa                                                     18


<210>  70
<211>  22
<212>  DNA
<213>  Artificial Sequence

<220>
<223>  oligonucleotide

<400>  70
caactgggcg aagagggtgc ca                                                22


<210>  71
<211>  20
<212>  DNA
<213>  Artificial Sequence

<220>
<223>  oligonucleotide

<400>  71
agcttcgatg atgggcttac                                                   20


<210>  72
<211>  20
<212>  DNA
<213>  Artificial Sequence

<220>
<223>  oligonucleotide

<400>  72
cctgatcatg gagggtcaaa                                                   20


<210>  73
<211>  18
<212>  DNA
<213>  Artificial Sequence

<220>

<223>  oligonucleotide

<400>  73
ggagctggtt cacatgat                                                    18


<210>  74
<211>  24
<212>  DNA
<213>  Artificial Sequence

<220>
<223>  oligonucleotide

<400>  74
agggtcaaat ccacaaagcc tggc                                             24


<210>  75
<211>  18
<212>  DNA
<213>  Artificial Sequence

<220>
<223>  oligonucleotide

<400>  75
ctagccaggc acattcta                                                    18


<210>  76
<211>  18
<212>  DNA
<213>  Artificial Sequence

<220>
<223>  oligonucleotide

<400>  76
gatgggctta ctgaccaa                                                    18


<210>  77
<211>  24
<212>  DNA
<213>  Artificial Sequence

<220>
<223>  oligonucleotide

<400>  77
catgtgaacc agctccctgt ctgc                                             24


<210>  78
<211>  19
<212>  DNA
<213>  Artificial Sequence

<220>
<223>  oligonucleotide

<400>  78

ctgatcatgg agggtcaaa                                                    19


<210>  79
<211>  18
<212>  DNA
<213>  Artificial Sequence

<220>
<223>  oligonucleotide

<400>  79
gaggttcttg ccagatga                                                     18


<210>  80
<211>  25
<212>  DNA
<213>  Artificial Sequence

<220>
<223>  oligonucleotide

<400>  80
tgtgttggct gtggaaagaa aggct                                             25


<210>  81
<211>  20
<212>  DNA
<213>  Artificial Sequence

<220>
<223>  oligonucleotide

<400>  81
ttggagaatt ccgtgaagta                                                   20


<210>  82
<211>  26
<212>  DNA
<213>  Artificial Sequence

<220>
<223>  oligonucleotide

<400>  82
tctctgggaa ttgtgttggc tgtgga                                            26


<210>  83
<211>  22
<212>  DNA
<213>  Artificial Sequence

<220>
<223>  oligonucleotide

<400>  83
ggagaattcc gtgaagtaac ag                                                22

<210>  84
<211>  20
<212>  DNA
<213>  Artificial Sequence

<220>
<223>  oligonucleotide

<400>  84
aagaggttct tgccagatga                                                    20


<210>  85
<211>  19
<212>  DNA
<213>  Artificial Sequence

<220>
<223>  oligonucleotide

<400>  85
gatgacaatg gccacaatg                                                     19


<210>  86
<211>  25
<212>  DNA
<213>  Artificial Sequence

<220>
<223>  oligonucleotide

<400>  86
tgtgttggct gtggaaagaa aggct                                              25


<210>  87
<211>  18
<212>  DNA
<213>  Artificial Sequence

<220>
<223>  oligonucleotide

<400>  87
cttcttgggt tgagtggt                                                      18


<210>  88
<211>  18
<212>  DNA
<213>  Artificial Sequence

<220>
<223>  oligonucleotide

<400>  88
ccgttgacaa gaggttct                                                      18


<210>  89
<211>  28
<212>  DNA

<213>  Artificial Sequence

<220>
<223>  oligonucleotide

<400>  89
ccagagaaac caagaagagc attgtggc                                          28


<210>  90
<211>  18
<212>  DNA
<213>  Artificial Sequence

<220>
<223>  oligonucleotide

<400>  90
ccacagccaa cacaattc                                                     18


<210>  91
<211>  17
<212>  DNA
<213>  Artificial Sequence

<220>
<223>  oligonucleotide

<400>  91
ggtcagtgtg ccctcag                                                      17


<210>  92
<211>  18
<212>  DNA
<213>  Artificial Sequence

<220>
<223>  oligonucleotide

<400>  92
ggacgagcat ggctacat                                                     18


<210>  93
<211>  24
<212>  DNA
<213>  Artificial Sequence

<220>
<223>  oligonucleotide

<400>  93
acccaagttt cctcctccct gtcc                                              24


<210>  94
<211>  18
<212>  DNA
<213>  Artificial Sequence

<220>

<223>  oligonucleotide

<400>  94
cttcacgcgg aaatacac                                                    18


<210>  95
<211>  24
<212>  DNA
<213>  Artificial Sequence

<220>
<223>  oligonucleotide

<400>  95
acccaagttt cctcctccct gtcc                                             24


<210>  96
<211>  18
<212>  DNA
<213>  Artificial Sequence

<220>
<223>  oligonucleotide

<400>  96
gatggtgatc tcgttgga                                                    18


<210>  97
<211>  17
<212>  DNA
<213>  Artificial Sequence

<220>
<223>  oligonucleotide

<400>  97
agcatggcta catctcc                                                     17


<210>  98
<211>  24
<212>  DNA
<213>  Artificial Sequence

<220>
<223>  oligonucleotide

<400>  98
tgcttcacgc ggaaatacac gctg                                             24


<210>  99
<211>  17
<212>  DNA
<213>  Artificial Sequence

<220>
<223>  oligonucleotide

<400>  99

gaggaggaaa cttgggt                                                    17

<210>  100
<211>  17
<212>  DNA
<213>  Artificial Sequence

<220>
<223>  oligonucleotide

<400>  100
gacgagcatg gctacat                                                    17

<210>  101
<211>  24
<212>  DNA
<213>  Artificial Sequence

<220>
<223>  oligonucleotide

<400>  101
tgcttcacgc ggaaatacac gctg                                            24

<210>  102
<211>  17
<212>  DNA
<213>  Artificial Sequence

<220>
<223>  oligonucleotide

<400>  102
acagggagga ggaaact                                                    17

<210>  103
<211>  20
<212>  DNA
<213>  Artificial Sequence

<220>
<223>  oligonucleotide

<400>  103
acgaggacct ctacatcatc                                                 20

<210>  104
<211>  24
<212>  DNA
<213>  Artificial Sequence

<220>
<223>  oligonucleotide

<400>  104
aagcagtgtc acccagctct ggat                                            24

<210>  105
<211>  17
<212>  DNA
<213>  Artificial Sequence

<220>
<223>  oligonucleotide

<400>  105
ccacacacca gcacttg                                                      17


<210>  106
<211>  17
<212>  DNA
<213>  Artificial Sequence

<220>
<223>  oligonucleotide

<400>  106
ccaactgcga ccgcaac                                                      17


<210>  107
<211>  18
<212>  DNA
<213>  Artificial Sequence

<220>
<223>  oligonucleotide

<400>  107
gtcttccggt ccacacac                                                     18


<210>  108
<211>  24
<212>  DNA
<213>  Artificial Sequence

<220>
<223>  oligonucleotide

<400>  108
caagcagtgt cacccagctc tgga                                              24


<210>  109
<211>  17
<212>  DNA
<213>  Artificial Sequence

<220>
<223>  oligonucleotide

<400>  109
ctggccgctt cacagag                                                      17


<210>  110
<211>  24
<212>  DNA

<213>  Artificial Sequence

<220>
<223>  oligonucleotide

<400>  110
tgatgtagag gtcctcgtgg gtgc                                              24


<210>  111
<211>  18
<212>  DNA
<213>  Artificial Sequence

<220>
<223>  oligonucleotide

<400>  111
cagagctggg tgacactg                                                     18


<210>  112
<211>  17
<212>  DNA
<213>  Artificial Sequence

<220>
<223>  oligonucleotide

<400>  112
gcaacggcaa cttccac                                                      17


<210>  113
<211>  24
<212>  DNA
<213>  Artificial Sequence

<220>
<223>  oligonucleotide

<400>  113
aagcagtgtc acccagctct ggat                                              24


<210>  114
<211>  17
<212>  DNA
<213>  Artificial Sequence

<220>
<223>  oligonucleotide

<400>  114
gtcttccggt ccacaca                                                      17


<210>  115
<211>  18
<212>  DNA
<213>  Artificial Sequence

<220>

<223>  oligonucleotide

<400>  115
tgcttagacg ctggattt                                                    18


<210>  116
<211>  26
<212>  DNA
<213>  Artificial Sequence

<220>
<223>  oligonucleotide

<400>  116
ccctcaacgt tagcttcacc aacagg                                           26


<210>  117
<211>  19
<212>  DNA
<213>  Artificial Sequence

<220>
<223>  oligonucleotide

<400>  117
tcgtagtcga ggtcatagt                                                   19


<210>  118
<211>  18
<212>  DNA
<213>  Artificial Sequence

<220>
<223>  oligonucleotide

<400>  118
tctctgaaag gctctcct                                                    18


<210>  119
<211>  24
<212>  DNA
<213>  Artificial Sequence

<220>
<223>  oligonucleotide

<400>  119
tgcagctgct tagacgctgg attt                                             24


<210>  120
<211>  19
<212>  DNA
<213>  Artificial Sequence

<220>
<223>  oligonucleotide

<400>  120

tcctgttggt gaagctaac                                                    19

<210>  121
<211>  17
<212>  DNA
<213>  Artificial Sequence

<220>
<223>  oligonucleotide

<400>  121
gacccgcttc tctgaaa                                                      17

<210>  122
<211>  24
<212>  DNA
<213>  Artificial Sequence

<220>
<223>  oligonucleotide

<400>  122
tgcagctgct tagacgctgg attt                                              24

<210>  123
<211>  19
<212>  DNA
<213>  Artificial Sequence

<220>
<223>  oligonucleotide

<400>  123
aggtcatagt tcctgttgg                                                    19

<210>  124
<211>  19
<212>  DNA
<213>  Artificial Sequence

<220>
<223>  oligonucleotide

<400>  124
ccggatgaca tcagagcta                                                    19

<210>  125
<211>  26
<212>  DNA
<213>  Artificial Sequence

<220>
<223>  oligonucleotide

<400>  125
tctcagaaag cagaggctca gagctt                                            26

<210>  126
<211>  22
<212>  DNA
<213>  Artificial Sequence

<220>
<223>  oligonucleotide

<400>  126
aatgcaaata tcagtgttcg tc                                                22


<210>  127
<211>  20
<212>  DNA
<213>  Artificial Sequence

<220>
<223>  oligonucleotide

<400>  127
ctcagagctt ggagacagac                                                   20


<210>  128
<211>  30
<212>  DNA
<213>  Artificial Sequence

<220>
<223>  oligonucleotide

<400>  128
aggattctat ctgcttactg ctacagacct                                        30


<210>  129
<211>  20
<212>  DNA
<213>  Artificial Sequence

<220>
<223>  oligonucleotide

<400>  129
gcaaggagga ggagaagaat                                                   20


<210>  130
<211>  24
<212>  DNA
<213>  Artificial Sequence

<220>
<223>  oligonucleotide

<400>  130
aagcagaggc tcagagcttg gaga                                              24


<210>  131
<211>  21
<212>  DNA

<213>  Artificial Sequence

<220>
<223>  oligonucleotide

<400>  131
caacagcctt ctcaattttc t                                                21


<210>  132
<211>  23
<212>  DNA
<213>  Artificial Sequence

<220>
<223>  oligonucleotide

<400>  132
cccattaaat gcaaatatca gtg                                              23


<210>  133
<211>  18
<212>  DNA
<213>  Artificial Sequence

<220>
<223>  oligonucleotide

<400>  133
tcagagcttg gagacaga                                                    18


<210>  134
<211>  28
<212>  DNA
<213>  Artificial Sequence

<220>
<223>  oligonucleotide

<400>  134
aaggattcta tctgcttact gctacaga                                         28


<210>  135
<211>  24
<212>  DNA
<213>  Artificial Sequence

<220>
<223>  oligonucleotide

<400>  135
gagaagaatt ccttaacaca tagg                                             24


<210>  136
<211>  20
<212>  DNA
<213>  Artificial Sequence

<220>

<223>  oligonucleotide

<400>  136
tgctcaacat gacgcttgtc                                                    20


<210>  137
<211>  21
<212>  DNA
<213>  Artificial Sequence

<220>
<223>  oligonucleotide

<400>  137
gccatgagga agtggttgaa a                                                  21


<210>  138
<211>  24
<212>  DNA
<213>  Artificial Sequence

<220>
<223>  oligonucleotide

<400>  138
aagccgtgtc atgttcctgc tggt                                               24


<210>  139
<211>  24
<212>  DNA
<213>  Artificial Sequence

<220>
<223>  oligonucleotide

<400>  139
gagttcctct gtatgaaagt attg                                               24


<210>  140
<211>  25
<212>  DNA
<213>  Artificial Sequence

<220>
<223>  oligonucleotide

<400>  140
tggaagggct acgaagtcaa accca                                              25


<210>  141
<211>  20
<212>  DNA
<213>  Artificial Sequence

<220>
<223>  oligonucleotide

<400>  141

atgtagcttg acctcatctc                                                    20

<210>  142
<211>  20
<212>  DNA
<213>  Artificial Sequence

<220>
<223>  oligonucleotide

<400>  142
tggcagatcc cacaggagtt                                                    20

<210>  143
<211>  30
<212>  DNA
<213>  Artificial Sequence

<220>
<223>  oligonucleotide

<400>  143
agcttcaagt tagccaagaa gagttcctct                                         30

<210>  144
<211>  24
<212>  DNA
<213>  Artificial Sequence

<220>
<223>  oligonucleotide

<400>  144
agcccttcca aaggaattgt atta                                               24

<210>  145
<211>  20
<212>  DNA
<213>  Artificial Sequence

<220>
<223>  oligonucleotide

<400>  145
agttagccaa gaagagttcc                                                    20

<210>  146
<211>  25
<212>  DNA
<213>  Artificial Sequence

<220>
<223>  oligonucleotide

<400>  146
tggaagggct acgaagtcaa accca                                              25

<210>  147
<211>  21
<212>  DNA
<213>  Artificial Sequence

<220>
<223>  oligonucleotide

<400>  147
agctctctaa tgtagcttga c                                                21


<210>  148
<211>  21
<212>  DNA
<213>  Artificial Sequence

<220>
<223>  oligonucleotide

<400>  148
ccaagaagag ttcctctgta t                                                21


<210>  149
<211>  25
<212>  DNA
<213>  Artificial Sequence

<220>
<223>  oligonucleotide

<400>  149
tggaagggct acgaagtcaa accca                                            25


<210>  150
<211>  20
<212>  DNA
<213>  Artificial Sequence

<220>
<223>  oligonucleotide

<400>  150
cttgacctca tctcctcaaa                                                  20


<210>  151
<211>  17
<212>  DNA
<213>  Artificial Sequence

<220>
<223>  oligonucleotide

<400>  151
cagatcaccc tcctcaa                                                     17


<210>  152
<211>  23
<212>  DNA

<213>  Artificial Sequence

<220>
<223>  oligonucleotide

<400>  152
cctggacatc ctgatcctgc gga                                              23


<210>  153
<211>  17
<212>  DNA
<213>  Artificial Sequence

<220>
<223>  oligonucleotide

<400>  153
cgagaaggtc atggtgt                                                     17


<210>  154
<211>  17
<212>  DNA
<213>  Artificial Sequence

<220>
<223>  oligonucleotide

<400>  154
ttcaccaccc tcaccat                                                     17


<210>  155
<211>  24
<212>  DNA
<213>  Artificial Sequence

<220>
<223>  oligonucleotide

<400>  155
agccttgagg agggtgatct ggtc                                             24


<210>  156
<211>  19
<212>  DNA
<213>  Artificial Sequence

<220>
<223>  oligonucleotide

<400>  156
agaaggtcat ggtgtcctg                                                   19


<210>  157
<211>  17
<212>  DNA
<213>  Artificial Sequence

<220>

<223>  oligonucleotide

<400>  157
gtgtaccgcg tgcagat                                                    17


<210>  158
<211>  21
<212>  DNA
<213>  Artificial Sequence

<220>
<223>  oligonucleotide

<400>  158
cattaagact gtggagttcg c                                               21


<210>  159
<211>  23
<212>  DNA
<213>  Artificial Sequence

<220>
<223>  oligonucleotide

<400>  159
accagatcac cctcctcaag gct                                             23


<210>  160
<211>  17
<212>  DNA
<213>  Artificial Sequence

<220>
<223>  oligonucleotide

<400>  160
ctgcctgcct ggacatc                                                    17


<210>  161
<211>  24
<212>  DNA
<213>  Artificial Sequence

<220>
<223>  oligonucleotide

<400>  161
cgagaaggtc atggtgtcct gctc                                            24


<210>  162
<211>  18
<212>  DNA
<213>  Artificial Sequence

<220>
<223>  oligonucleotide

<400>  162

catctgggtc cggttcag                                                    18

<210>  163
<211>  18
<212>  DNA
<213>  Artificial Sequence

<220>
<223>  oligonucleotide

<400>  163
gctatggccc tgaagatt                                                    18

<210>  164
<211>  24
<212>  DNA
<213>  Artificial Sequence

<220>
<223>  oligonucleotide

<400>  164
acaaagacga gcaggactaa acga                                             24

<210>  165
<211>  22
<212>  DNA
<213>  Artificial Sequence

<220>
<223>  oligonucleotide

<400>  165
acctaactag gttctgaatg aa                                               22

<210>  166
<211>  19
<212>  DNA
<213>  Artificial Sequence

<220>
<223>  oligonucleotide

<400>  166
aaacagtttc ctgctatgg                                                   19

<210>  167
<211>  24
<212>  DNA
<213>  Artificial Sequence

<220>
<223>  oligonucleotide

<400>  167
ccctgaagat tcctgccaag agaa                                             24

<210>  168
<211>  18
<212>  DNA
<213>  Artificial Sequence

<220>
<223>  oligonucleotide

<400>  168
tagtcctgct cgtctttg                                                    18


<210>  169
<211>  20
<212>  DNA
<213>  Artificial Sequence

<220>
<223>  oligonucleotide

<400>  169
agattcctgc caagagaata                                                  20


<210>  170
<211>  24
<212>  DNA
<213>  Artificial Sequence

<220>
<223>  oligonucleotide

<400>  170
acaaagacga gcaggactaa acga                                             24


<210>  171
<211>  21
<212>  DNA
<213>  Artificial Sequence

<220>
<223>  oligonucleotide

<400>  171
ggatacctaa ctaggttctg a                                                21


<210>  172
<211>  24
<212>  DNA
<213>  Artificial Sequence

<220>
<223>  oligonucleotide

<400>  172
acaaagacga gcaggactaa acga                                             24


<210>  173
<211>  25
<212>  DNA

<213>  Artificial Sequence

<220>
<223>  oligonucleotide

<400>  173
tggataccta actaggttct gaatg                                          25


<210>  174
<211>  25
<212>  DNA
<213>  Artificial Sequence

<220>
<223>  oligonucleotide

<400>  174
ctgccaagag aatatttggt gataa                                          25


<210>  175
<211>  20
<212>  DNA
<213>  Artificial Sequence

<220>
<223>  oligonucleotide

<400>  175
tgcaggtcct cactttaatc                                                20


<210>  176
<211>  24
<212>  DNA
<213>  Artificial Sequence

<220>
<223>  oligonucleotide

<400>  176
aaacacggtg ggccaaagga tgaa                                           24


<210>  177
<211>  20
<212>  DNA
<213>  Artificial Sequence

<220>
<223>  oligonucleotide

<400>  177
ctttgtcagc agtcacattg                                                20


<210>  178
<211>  23
<212>  DNA
<213>  Artificial Sequence

<220>

<223>  oligonucleotide

<400>  178
ggtcctcact ttaatcctct atc                                               23


<210>  179
<211>  24
<212>  DNA
<213>  Artificial Sequence

<220>
<223>  oligonucleotide

<400>  179
aaacacggtg ggccaaagga tgaa                                              24


<210>  180
<211>  19
<212>  DNA
<213>  Artificial Sequence

<220>
<223>  oligonucleotide

<400>  180
accatctttg tcagcagtc                                                    19


<210>  181
<211>  17
<212>  DNA
<213>  Artificial Sequence

<220>
<223>  oligonucleotide

<400>  181
gtgcaggtcc tcacttt                                                      17


<210>  182
<211>  24
<212>  DNA
<213>  Artificial Sequence

<220>
<223>  oligonucleotide

<400>  182
aaacacggtg ggccaaagga tgaa                                              24


<210>  183
<211>  17
<212>  DNA
<213>  Artificial Sequence

<220>
<223>  oligonucleotide

<400>  183

gcccaagtct ccaacat                                                    17

<210>  184
<211>  18
<212>  DNA
<213>  Artificial Sequence

<220>
<223>  oligonucleotide

<400>  184
cctcccagtg tgcaaata                                                   18

<210>  185
<211>  24
<212>  DNA
<213>  Artificial Sequence

<220>
<223>  oligonucleotide

<400>  185
acgtccctcc agaagaggag tgtg                                            24

<210>  186
<211>  19
<212>  DNA
<213>  Artificial Sequence

<220>
<223>  oligonucleotide

<400>  186
ccctgcagaa gtgtctaaa                                                  19

<210>  187
<211>  20
<212>  DNA
<213>  Artificial Sequence

<220>
<223>  oligonucleotide

<400>  187
ccctggtgct tctatcctaa                                                 20

<210>  188
<211>  20
<212>  DNA
<213>  Artificial Sequence

<220>
<223>  oligonucleotide

<400>  188
atccctgcag aagtgtctaa                                                 20

<210>  189
<211>  21
<212>  DNA
<213>  Artificial Sequence

<220>
<223>  oligonucleotide

<400>  189
accatcgacg tccctccaga a                                                21


<210>  190
<211>  21
<212>  DNA
<213>  Artificial Sequence

<220>
<223>  oligonucleotide

<400>  190
ggtgcttcta tcctaatacc a                                                21


<210>  191
<211>  24
<212>  DNA
<213>  Artificial Sequence

<220>
<223>  oligonucleotide

<400>  191
agacacttct gcagggatct gcct                                             24


<210>  192
<211>  18
<212>  DNA
<213>  Artificial Sequence

<220>
<223>  oligonucleotide

<400>  192
tctgggacta atcaccgt                                                    18


<210>  193
<211>  20
<212>  DNA
<213>  Artificial Sequence

<220>
<223>  oligonucleotide

<400>  193
cctggtgctt ctatcctaat                                                  20


<210>  194
<211>  24
<212>  DNA

<213>  Artificial Sequence

<220>
<223>  oligonucleotide

<400>  194
acgtccctcc agaagaggag tgtg                                              24


<210>  195
<211>  17
<212>  DNA
<213>  Artificial Sequence

<220>
<223>  oligonucleotide

<400>  195
gatccctgca gaagtgt                                                      17


<210>  196
<211>  17
<212>  DNA
<213>  Artificial Sequence

<220>
<223>  oligonucleotide

<400>  196
ggacatgaga ggcacac                                                      17


<210>  197
<211>  24
<212>  DNA
<213>  Artificial Sequence

<220>
<223>  oligonucleotide

<400>  197
tctccctcct ctgcctcctc tcaa                                              24


<210>  198
<211>  17
<212>  DNA
<213>  Artificial Sequence

<220>
<223>  oligonucleotide

<400>  198
ggcaggtaca tggtgtc                                                      17


<210>  199
<211>  19
<212>  DNA
<213>  Artificial Sequence

<220>

<223>  oligonucleotide

<400>  199
tcctctgcct cctctcaaa                                                    19


<210>  200
<211>  23
<212>  DNA
<213>  Artificial Sequence

<220>
<223>  oligonucleotide

<400>  200
cagctgtgcc cgacaccatg tac                                               23


<210>  201
<211>  17
<212>  DNA
<213>  Artificial Sequence

<220>
<223>  oligonucleotide

<400>  201
cagccatcca gcaccag                                                      17


<210>  202
<211>  17
<212>  DNA
<213>  Artificial Sequence

<220>
<223>  oligonucleotide

<400>  202
acaccgaaga cccacct                                                      17


<210>  203
<211>  24
<212>  DNA
<213>  Artificial Sequence

<220>
<223>  oligonucleotide

<400>  203
tctccctcct ctgcctcctc tcaa                                              24


<210>  204
<211>  17
<212>  DNA
<213>  Artificial Sequence

<220>
<223>  oligonucleotide

<400>  204

catggtgtcg ggcacag 17

<210> 205
<211> 17
<212> DNA
<213> Artificial Sequence

<220>
<223> oligonucleotide

<400> 205
catggtgtcg ggcacag 17

<210> 206
<211> 18
<212> DNA
<213> Artificial Sequence

<220>
<223> oligonucleotide

<400> 206
gagaggcaca ccgaagac 18

<210> 207
<211> 20
<212> DNA
<213> Artificial Sequence

<220>
<223> oligonucleotide

<400> 207
ttctccctcc tctgcctcct 20

<210> 208
<211> 18
<212> DNA
<213> Artificial Sequence

<220>
<223> oligonucleotide

<400> 208
tggattctgg cggtattg 18

<210> 209
<211> 25
<212> DNA
<213> Artificial Sequence

<220>
<223> oligonucleotide

<400> 209
ttgggcattc tggacaactt ggacc 25

<210>  210
<211>  19
<212>  DNA
<213>  Artificial Sequence

<220>
<223>  oligonucleotide

<400>  210
ggaagggcat ttgaagaac                                                    19


<210>  211
<211>  21
<212>  DNA
<213>  Artificial Sequence

<220>
<223>  oligonucleotide

<400>  211
catgactggg tccaagttgt c                                                 21


<210>  212
<211>  20
<212>  DNA
<213>  Artificial Sequence

<220>
<223>  oligonucleotide

<400>  212
ggattctggc ggtattgact                                                   20


<210>  213
<211>  30
<212>  DNA
<213>  Artificial Sequence

<220>
<223>  oligonucleotide

<400>  213
tcagagtctg atatcctgtt gggcattctg                                        30


<210>  214
<211>  20
<212>  DNA
<213>  Artificial Sequence

<220>
<223>  oligonucleotide

<400>  214
gcggtattga ctcttcagat                                                   20


<210>  215
<211>  28
<212>  DNA

<213>  Artificial Sequence

<220>
<223>  oligonucleotide

<400>  215
tctgatatcc tgttgggcat tctggaca                                          28


<210>  216
<211>  18
<212>  DNA
<213>  Artificial Sequence

<220>
<223>  oligonucleotide

<400>  216
gaacatgact gggtccaa                                                     18


<210>  217
<211>  18
<212>  DNA
<213>  Artificial Sequence

<220>
<223>  oligonucleotide

<400>  217
ctggcggtat tgactctt                                                     18


<210>  218
<211>  25
<212>  DNA
<213>  Artificial Sequence

<220>
<223>  oligonucleotide

<400>  218
ttgggcattc tggacaactt ggacc                                             25


<210>  219
<211>  20
<212>  DNA
<213>  Artificial Sequence

<220>
<223>  oligonucleotide

<400>  219
ggcatttgaa gaacatgact                                                   20


<210>  220
<211>  19
<212>  DNA
<213>  Artificial Sequence

<220>

<223>  oligonucleotide

<400>  220
ccattgagag ggtgtcaga                                                    19


<210>  221
<211>  26
<212>  DNA
<213>  Artificial Sequence

<220>
<223>  oligonucleotide

<400>  221
tggattcttc ggatgctgac gattgc                                            26


<210>  222
<211>  22
<212>  DNA
<213>  Artificial Sequence

<220>
<223>  oligonucleotide

<400>  222
cgctgtgata tctcatcaag ta                                                22


<210>  223
<211>  20
<212>  DNA
<213>  Artificial Sequence

<220>
<223>  oligonucleotide

<400>  223
gagagggtgt cagaggcaat                                                   20


<210>  224
<211>  24
<212>  DNA
<213>  Artificial Sequence

<220>
<223>  oligonucleotide

<400>  224
gcgctgtgat atctcatcaa gtag                                              24


<210>  225
<211>  26
<212>  DNA
<213>  Artificial Sequence

<220>
<223>  oligonucleotide

<400>  225

cgtcagcatc cgaagaatcc agacct                                          26


<210>  226
<211>  18
<212>  DNA
<213>  Artificial Sequence

<220>
<223>  oligonucleotide

<400>  226
gtgtcagagg caatcgtc                                                    18


<210>  227
<211>  18
<212>  DNA
<213>  Artificial Sequence

<220>
<223>  oligonucleotide

<400>  227
cgtcagctgc cgtcagat                                                    18


<210>  228
<211>  34
<212>  DNA
<213>  Artificial Sequence

<220>
<223>  oligonucleotide

<400>  228
cagacctttt tgctacttga tgagatatca cagc                                  34


<210>  229
<211>  19
<212>  DNA
<213>  Artificial Sequence

<220>
<223>  oligonucleotide

<400>  229
gccattgaga gggtgtcag                                                   19


<210>  230
<211>  18
<212>  DNA
<213>  Artificial Sequence

<220>
<223>  oligonucleotide

<400>  230
tatcacagcg caaccgtc                                                    18

<210>  231
<211>  28
<212>  DNA
<213>  Artificial Sequence

<220>
<223>  oligonucleotide

<400>  231
gcatccgaag aatccagacc tttttgct                                          28


<210>  232
<211>  21
<212>  DNA
<213>  Artificial Sequence

<220>
<223>  oligonucleotide

<400>  232
cctgatctgt ggagatgaag c                                                 21


<210>  233
<211>  19
<212>  DNA
<213>  Artificial Sequence

<220>
<223>  oligonucleotide

<400>  233
gcgcacaggt acttctgtt                                                    19


<210>  234
<211>  22
<212>  DNA
<213>  Artificial Sequence

<220>
<223>  oligonucleotide

<400>  234
tggaagctgc aaggtcttct tc                                                22


<210>  235
<211>  19
<212>  DNA
<213>  Artificial Sequence

<220>
<223>  oligonucleotide

<400>  235
gcttctgggt gtcactatg                                                    19


<210>  236
<211>  19
<212>  DNA

<213>  Artificial Sequence

<220>
<223>  oligonucleotide

<400>  236
cgcacaggta cttctgttt                                                    19


<210>  237
<211>  25
<212>  DNA
<213>  Artificial Sequence

<220>
<223>  oligonucleotide

<400>  237
tgtggaagct gcaaggtctt cttca                                             25


<210>  238
<211>  20
<212>  DNA
<213>  Artificial Sequence

<220>
<223>  oligonucleotide

<400>  238
ctgatctgtg gagatgaagc                                                   20


<210>  239
<211>  21
<212>  DNA
<213>  Artificial Sequence

<220>
<223>  oligonucleotide

<400>  239
ggtacttctg tttcccttca g                                                 21


<210>  240
<211>  25
<212>  DNA
<213>  Artificial Sequence

<220>
<223>  oligonucleotide

<400>  240
tgtggaagct gcaaggtctt cttca                                             25


<210>  241
<211>  20
<212>  DNA
<213>  Artificial Sequence

<220>

<223>  oligonucleotide

<400>  241
gctgaaggga aacagaagta                                                   20


<210>  242
<211>  23
<212>  DNA
<213>  Artificial Sequence

<220>
<223>  oligonucleotide

<400>  242
tgcgccagca gaaatgattg cac                                               23


<210>  243
<211>  20
<212>  DNA
<213>  Artificial Sequence

<220>
<223>  oligonucleotide

<400>  243
gaagacgaca agatggacaa                                                   20


<210>  244
<211>  20
<212>  DNA
<213>  Artificial Sequence

<220>
<223>  oligonucleotide

<400>  244
gcatttatgg tgcctgattc                                                   20


<210>  245
<211>  19
<212>  DNA
<213>  Artificial Sequence

<220>
<223>  oligonucleotide

<400>  245
caggaacagg gtttgacag                                                    19


<210>  246
<211>  23
<212>  DNA
<213>  Artificial Sequence

<220>
<223>  oligonucleotide

<400>  246

agtgagctgc cctttgatgc tca                                              23

<210>  247
<211>  19
<212>  DNA
<213>  Artificial Sequence

<220>
<223>  oligonucleotide

<400>  247
tgattcctgc atggtcagt                                                    19

<210>  248
<211>  22
<212>  DNA
<213>  Artificial Sequence

<220>
<223>  oligonucleotide

<400>  248
acaaccctgc tgccctgtca aa                                                22

<210>  249
<211>  19
<212>  DNA
<213>  Artificial Sequence

<220>
<223>  oligonucleotide

<400>  249
ctcatcggtc aggaacagg                                                    19

<210>  250
<211>  19
<212>  DNA
<213>  Artificial Sequence

<220>
<223>  oligonucleotide

<400>  250
tgatgctcat gcccacatc                                                    19

<210>  251
<211>  19
<212>  DNA
<213>  Artificial Sequence

<220>
<223>  oligonucleotide

<400>  251
gcagacgctt ctcctcatc                                                    19

<210>  252
<211>  22
<212>  DNA
<213>  Artificial Sequence

<220>
<223>  oligonucleotide

<400>  252
tgctgccctg tcaaaccctg tt                                                22


<210>  253
<211>  18
<212>  DNA
<213>  Artificial Sequence

<220>
<223>  oligonucleotide

<400>  253
ccagagcagg caccgtag                                                     18


<210>  254
<211>  20
<212>  DNA
<213>  Artificial Sequence

<220>
<223>  oligonucleotide

<400>  254
agcagacgct tctcctcatc                                                   20


<210>  255
<211>  22
<212>  DNA
<213>  Artificial Sequence

<220>
<223>  oligonucleotide

<400>  255
caaccctgct gccctgtcaa ac                                                22


<210>  256
<211>  19
<212>  DNA
<213>  Artificial Sequence

<220>
<223>  oligonucleotide

<400>  256
gaggcagctg gagaagttt                                                    19


<210>  257
<211>  20
<212>  DNA

<213>  Artificial Sequence

<220>
<223>  oligonucleotide

<400>  257
gcagcttgtg gtacaaggag                                                   20


<210>  258
<211>  24
<212>  DNA
<213>  Artificial Sequence

<220>
<223>  oligonucleotide

<400>  258
tatgccgact gctacatgaa ccgg                                              24


<210>  259
<211>  19
<212>  DNA
<213>  Artificial Sequence

<220>
<223>  oligonucleotide

<400>  259
tggcttccag atgctgtat                                                    19


<210>  260
<211>  19
<212>  DNA
<213>  Artificial Sequence

<220>
<223>  oligonucleotide

<400>  260
tggtacaagg agccatcaa                                                    19


<210>  261
<211>  22
<212>  DNA
<213>  Artificial Sequence

<220>
<223>  oligonucleotide

<400>  261
aaccgggagg agttctggga ga                                                22


<210>  262
<211>  19
<212>  DNA
<213>  Artificial Sequence

<220>

<223>  oligonucleotide

<400>  262
aggcagctgg agaagtttg                                                    19


<210>  263
<211>  19
<212>  DNA
<213>  Artificial Sequence

<220>
<223>  oligonucleotide

<400>  263
gcagcttgtg gtacaagga                                                    19


<210>  264
<211>  22
<212>  DNA
<213>  Artificial Sequence

<220>
<223>  oligonucleotide

<400>  264
aaccgggagg agttctggga ga                                                22


<210>  265
<211>  19
<212>  DNA
<213>  Artificial Sequence

<220>
<223>  oligonucleotide

<400>  265
aacactttga agccaggtc                                                    19


<210>  266
<211>  20
<212>  DNA
<213>  Artificial Sequence

<220>
<223>  oligonucleotide

<400>  266
caaacatctc ccagaactcc                                                   20


<210>  267
<211>  22
<212>  DNA
<213>  Artificial Sequence

<220>
<223>  oligonucleotide

<400>  267

tgccgactgc tacatgaacc gg                                              22


<210>  268
<211>  19
<212>  DNA
<213>  Artificial Sequence

<220>
<223>  oligonucleotide

<400>  268
caacatcacc gtactgcat                                                  19


<210>  269
<211>  20
<212>  DNA
<213>  Artificial Sequence

<220>
<223>  oligonucleotide

<400>  269
gagtccttgg aactggattg                                                 20


<210>  270
<211>  22
<212>  DNA
<213>  Artificial Sequence

<220>
<223>  oligonucleotide

<400>  270
tgaagctcac cgagacggcg at                                              22


<210>  271
<211>  20
<212>  DNA
<213>  Artificial Sequence

<220>
<223>  oligonucleotide

<400>  271
catgtgaagc tcaccgagac                                                 20


<210>  272
<211>  22
<212>  DNA
<213>  Artificial Sequence

<220>
<223>  oligonucleotide

<400>  272
ggaactggat tgaaggtcga aa                                              22

<210>  273
<211>  23
<212>  DNA
<213>  Artificial Sequence

<220>
<223>  oligonucleotide

<400>  273
ctcgagactt accagagcca caa                                              23


<210>  274
<211>  17
<212>  DNA
<213>  Artificial Sequence

<220>
<223>  oligonucleotide

<400>  274
agaagtttgt ccgcagc                                                     17


<210>  275
<211>  19
<212>  DNA
<213>  Artificial Sequence

<220>
<223>  oligonucleotide

<400>  275
gagtccttgg aactggatt                                                   19


<210>  276
<211>  32
<212>  DNA
<213>  Artificial Sequence

<220>
<223>  oligonucleotide

<400>  276
tcgagactta ccagagccac aaaatttaat tc                                    32


<210>  277
<211>  19
<212>  DNA
<213>  Artificial Sequence

<220>
<223>  oligonucleotide

<400>  277
cgtactgcat gtgaagctc                                                   19


<210>  278
<211>  21
<212>  DNA

<213>  Artificial Sequence

<220>
<223>  oligonucleotide

<400>  278
gaactggatt gaaggtcgaa a                                                21


<210>  279
<211>  25
<212>  DNA
<213>  Artificial Sequence

<220>
<223>  oligonucleotide

<400>  279
tttgtggctc tggtaagtct cgagc                                            25


<210>  280
<211>  19
<212>  DNA
<213>  Artificial Sequence

<220>
<223>  oligonucleotide

<400>  280
aacggaaagg agagggata                                                   19


<210>  281
<211>  19
<212>  DNA
<213>  Artificial Sequence

<220>
<223>  oligonucleotide

<400>  281
tttgactgca gagatgtgg                                                   19


<210>  282
<211>  21
<212>  DNA
<213>  Artificial Sequence

<220>
<223>  oligonucleotide

<400>  282
aaatccacct ggaaggccgc t                                                21


<210>  283
<211>  20
<212>  DNA
<213>  Artificial Sequence

<220>

<223>  oligonucleotide

<400>  283
tgaagatgga ggcattatcc                                                   20


<210>  284
<211>  20
<212>  DNA
<213>  Artificial Sequence

<220>
<223>  oligonucleotide

<400>  284
aacagtagaa atccacctgg                                                   20


<210>  285
<211>  25
<212>  DNA
<213>  Artificial Sequence

<220>
<223>  oligonucleotide

<400>  285
ggagaaccaa acggaaagga gaggg                                             25


<210>  286
<211>  22
<212>  DNA
<213>  Artificial Sequence

<220>
<223>  oligonucleotide

<400>  286
acggaaagga gagggatatt ca                                                22


<210>  287
<211>  22
<212>  DNA
<213>  Artificial Sequence

<220>
<223>  oligonucleotide

<400>  287
gtcaaacatc cttccaccac ag                                                22


<210>  288
<211>  26
<212>  DNA
<213>  Artificial Sequence

<220>
<223>  oligonucleotide

<400>  288

acagtagaaa tccacctgga aggccg 26

<210> 289
<211> 20
<212> DNA
<213> Artificial Sequence

<220>
<223> oligonucleotide

<400> 289
acggaaagga gagggatatt 20

<210> 290
<211> 19
<212> DNA
<213> Artificial Sequence

<220>
<223> oligonucleotide

<400> 290
ccacagtgaa tgcatcctt 19

<210> 291
<211> 21
<212> DNA
<213> Artificial Sequence

<220>
<223> oligonucleotide

<400> 291
aaatccacct ggaaggccgc t 21

<210> 292
<211> 23
<212> DNA
<213> Artificial Sequence

<220>
<223> oligonucleotide

<400> 292
gggattacac aaagagagtg ata 23

<210> 293
<211> 22
<212> DNA
<213> Artificial Sequence

<220>
<223> oligonucleotide

<400> 293
tcatcttcag ggccatcagc gc 22

<210>  294
<211>  22
<212>  DNA
<213>  Artificial Sequence

<220>
<223>  oligonucleotide

<400>  294
gggagacata acttcatcag ag                                                22


<210>  295
<211>  19
<212>  DNA
<213>  Artificial Sequence

<220>
<223>  oligonucleotide

<400>  295
ccagagtgca gtccaattc                                                    19


<210>  296
<211>  24
<212>  DNA
<213>  Artificial Sequence

<220>
<223>  oligonucleotide

<400>  296
gggattacac aaagagagtg atac                                              24


<210>  297
<211>  24
<212>  DNA
<213>  Artificial Sequence

<220>
<223>  oligonucleotide

<400>  297
tggccctgaa gatgatggag ctct                                              24


<210>  298
<211>  19
<212>  DNA
<213>  Artificial Sequence

<220>
<223>  oligonucleotide

<400>  298
ggcgatgcct attgtgtag                                                    19


<210>  299
<211>  20
<212>  DNA

<213>  Artificial Sequence

<220>
<223>  oligonucleotide

<400>  299
tcagagagct ccatcatctt                                                   20


<210>  300
<211>  23
<212>  DNA
<213>  Artificial Sequence

<220>
<223>  oligonucleotide

<400>  300
gggccatcag cgctatctga aca                                               23


<210>  301
<211>  21
<212>  DNA
<213>  Artificial Sequence

<220>
<223>  oligonucleotide

<400>  301
gagagtgata ctcatgctgt t                                                 21


<210>  302
<211>  22
<212>  DNA
<213>  Artificial Sequence

<220>
<223>  oligonucleotide

<400>  302
caattcgcat cttgataggt tc                                                22


<210>  303
<211>  22
<212>  DNA
<213>  Artificial Sequence

<220>
<223>  oligonucleotide

<400>  303
gcgctgatgg ccctgaagat ga                                                22


<210>  304
<211>  21
<212>  DNA
<213>  Artificial Sequence

<220>

<223>  oligonucleotide

<400>  304
tcgaaccaga ggagttcttg c                                                21


<210>  305
<211>  21
<212>  DNA
<213>  Artificial Sequence

<220>
<223>  oligonucleotide

<400>  305
agcacacatg tcattggaca g                                                21


<210>  306
<211>  25
<212>  DNA
<213>  Artificial Sequence

<220>
<223>  oligonucleotide

<400>  306
cccaggagtc ttcagtgtgt gagcc                                            25


<210>  307
<211>  19
<212>  DNA
<213>  Artificial Sequence

<220>
<223>  oligonucleotide

<400>  307
aggagtcttc agtgtgtga                                                   19


<210>  308
<211>  20
<212>  DNA
<213>  Artificial Sequence

<220>
<223>  oligonucleotide

<400>  308
acaacatgaa ctctgtcacc                                                  20


<210>  309
<211>  24
<212>  DNA
<213>  Artificial Sequence

<220>
<223>  oligonucleotide

<400>  309

tgtcattgga caggagatgg aggc                                              24

<210>  310
<211>  19
<212>  DNA
<213>  Artificial Sequence

<220>
<223>  oligonucleotide

<400>  310
gtgtgtgagc ctccatctc                                                   19

<210>  311
<211>  20
<212>  DNA
<213>  Artificial Sequence

<220>
<223>  oligonucleotide

<400>  311
ccagcacaca acatgaactc                                                  20

<210>  312
<211>  27
<212>  DNA
<213>  Artificial Sequence

<220>
<223>  oligonucleotide

<400>  312
ctgtccaatg acatgtgtgc tagagct                                          27

<210>  313
<211>  19
<212>  DNA
<213>  Artificial Sequence

<220>
<223>  oligonucleotide

<400>  313
catcgaacca gaggagttc                                                   19

<210>  314
<211>  20
<212>  DNA
<213>  Artificial Sequence

<220>
<223>  oligonucleotide

<400>  314
gaactctgtc accttctcag                                                  20

<210>  315
<211>  23
<212>  DNA
<213>  Artificial Sequence

<220>
<223>  oligonucleotide

<400>  315
tgtgagcctc catctcctgt cca                                               23


<210>  316
<211>  20
<212>  DNA
<213>  Artificial Sequence

<220>
<223>  oligonucleotide

<400>  316
gaaccagagg agttcttgac                                                   20


<210>  317
<211>  19
<212>  DNA
<213>  Artificial Sequence

<220>
<223>  oligonucleotide

<400>  317
tgaacttggt caccttctg                                                    19


<210>  318
<211>  23
<212>  DNA
<213>  Artificial Sequence

<220>
<223>  oligonucleotide

<400>  318
ccaatgacgt gtgtgcgcaa gtt                                               23


<210>  319
<211>  21
<212>  DNA
<213>  Artificial Sequence

<220>
<223>  oligonucleotide

<400>  319
gtgtggacct ccatgttatt t                                                 21


<210>  320
<211>  19
<212>  DNA

<213>  Artificial Sequence

<220>
<223>  oligonucleotide

<400>  320
acacagcatg aacttggtc                                                    19


<210>  321
<211>  23
<212>  DNA
<213>  Artificial Sequence

<220>
<223>  oligonucleotide

<400>  321
tgtgcgcaag ttcaccctca gaa                                               23


<210>  322
<211>  19
<212>  DNA
<213>  Artificial Sequence

<220>
<223>  oligonucleotide

<400>  322
gatgctgtga aggtcatgg                                                    19


<210>  323
<211>  21
<212>  DNA
<213>  Artificial Sequence

<220>
<223>  oligonucleotide

<400>  323
cacacactga agtttctttg g                                                 21


<210>  324
<211>  23
<212>  DNA
<213>  Artificial Sequence

<220>
<223>  oligonucleotide

<400>  324
actcctctgg ttcaatgctg ccc                                               23


<210>  325
<211>  19
<212>  DNA
<213>  Artificial Sequence

<220>

<223>  oligonucleotide

<400>  325
gcagcattga accagagga                                                    19


<210>  326
<211>  22
<212>  DNA
<213>  Artificial Sequence

<220>
<223>  oligonucleotide

<400>  326
gcacacacgt cattggaaat aa                                                22


<210>  327
<211>  27
<212>  DNA
<213>  Artificial Sequence

<220>
<223>  oligonucleotide

<400>  327
cccaaagaaa cttcagtgtg tggacct                                           27


<210>  328
<211>  20
<212>  DNA
<213>  Artificial Sequence

<220>
<223>  oligonucleotide

<400>  328
gcctataaag gagctcaacc                                                   20


<210>  329
<211>  19
<212>  DNA
<213>  Artificial Sequence

<220>
<223>  oligonucleotide

<400>  329
ccgtgacaga ccatcaaat                                                    19


<210>  330
<211>  22
<212>  DNA
<213>  Artificial Sequence

<220>
<223>  oligonucleotide

<400>  330

tcggattggc accctggagt tg                                                22

<210>  331
<211>  19
<212>  DNA
<213>  Artificial Sequence

<220>
<223>  oligonucleotide

<400>  331
gaacaacatc acggaagtg                                                   19

<210>  332
<211>  19
<212>  DNA
<213>  Artificial Sequence

<220>
<223>  oligonucleotide

<400>  332
catcaaatgc tcccaactc                                                   19

<210>  333
<211>  22
<212>  DNA
<213>  Artificial Sequence

<220>
<223>  oligonucleotide

<400>  333
acacctgctt tccacacgga cc                                                22

<210>  334
<211>  18
<212>  DNA
<213>  Artificial Sequence

<220>
<223>  oligonucleotide

<400>  334
acacggaccg cctataaa                                                    18

<210>  335
<211>  20
<212>  DNA
<213>  Artificial Sequence

<220>
<223>  oligonucleotide

<400>  335
ccatcaaatg ctcccaactc                                                  20

<210>  336
<211>  22
<212>  DNA
<213>  Artificial Sequence

<220>
<223>  oligonucleotide

<400>  336
agctcaacct ggcaggcaat cg                                                22


<210>  337
<211>  19
<212>  DNA
<213>  Artificial Sequence

<220>
<223>  oligonucleotide

<400>  337
cacacggacc gcctataaa                                                    19


<210>  338
<211>  20
<212>  DNA
<213>  Artificial Sequence

<220>
<223>  oligonucleotide

<400>  338
ttgctcaggc gaagagttag                                                   20


<210>  339
<211>  22
<212>  DNA
<213>  Artificial Sequence

<220>
<223>  oligonucleotide

<400>  339
tcggattggc accctggagt tg                                                22


<210>  340
<211>  21
<212>  DNA
<213>  Artificial Sequence

<220>
<223>  oligonucleotide

<400>  340
gcattattgg catgggaaca g                                                 21


<210>  341
<211>  22
<212>  DNA

<213>  Artificial Sequence

<220>
<223>  oligonucleotide

<400>  341
ccaccatctc agggttgttt ag                                                22


<210>  342
<211>  25
<212>  DNA
<213>  Artificial Sequence

<220>
<223>  oligonucleotide

<400>  342
cgcctacatc ctaactcgat ttgct                                             25


<210>  343
<211>  20
<212>  DNA
<213>  Artificial Sequence

<220>
<223>  oligonucleotide

<400>  343
cattattggc atgggaacag                                                   20


<210>  344
<211>  19
<212>  DNA
<213>  Artificial Sequence

<220>
<223>  oligonucleotide

<400>  344
agggttcacg ttgataagg                                                    19


<210>  345
<211>  24
<212>  DNA
<213>  Artificial Sequence

<220>
<223>  oligonucleotide

<400>  345
aaacaaccct gagatggtgg aggg                                              24


<210>  346
<211>  19
<212>  DNA
<213>  Artificial Sequence

<220>

<223>  oligonucleotide

<400>  346
tgaaatgctt cctggagtc                                                    19


<210>  347
<211>  21
<212>  DNA
<213>  Artificial Sequence

<220>
<223>  oligonucleotide

<400>  347
cagggttgtt tagagcaaat c                                                 21


<210>  348
<211>  22
<212>  DNA
<213>  Artificial Sequence

<220>
<223>  oligonucleotide

<400>  348
ttggcatggg aacaggagca gg                                                22


<210>  349
<211>  19
<212>  DNA
<213>  Artificial Sequence

<220>
<223>  oligonucleotide

<400>  349
cttcctggag tccttcaac                                                    19


<210>  350
<211>  20
<212>  DNA
<213>  Artificial Sequence

<220>
<223>  oligonucleotide

<400>  350
accatctcag ggttgtttag                                                   20


<210>  351
<211>  22
<212>  DNA
<213>  Artificial Sequence

<220>
<223>  oligonucleotide

<400>  351

ttggcatggg aacaggagca gg                                                22

<210>  352
<211>  18
<212>  DNA
<213>  Artificial Sequence

<220>
<223>  oligonucleotide

<400>  352
cttctgcggc tgcttagg                                                    18

<210>  353
<211>  19
<212>  DNA
<213>  Artificial Sequence

<220>
<223>  oligonucleotide

<400>  353
cagagaccga gccagaaag                                                   19

<210>  354
<211>  23
<212>  DNA
<213>  Artificial Sequence

<220>
<223>  oligonucleotide

<400>  354
aaacacttca ggcgcccttc caa                                              23

<210>  355
<211>  19
<212>  DNA
<213>  Artificial Sequence

<220>
<223>  oligonucleotide

<400>  355
cagagaccga gccagaaag                                                   19

<210>  356
<211>  19
<212>  DNA
<213>  Artificial Sequence

<220>
<223>  oligonucleotide

<400>  356
cacctgagtg tgggagaag                                                   19

<210>  357
<211>  22
<212>  DNA
<213>  Artificial Sequence

<220>
<223>  oligonucleotide

<400>  357
aacacttcag gcgcccttcc aa                                                22


<210>  358
<211>  19
<212>  DNA
<213>  Artificial Sequence

<220>
<223>  oligonucleotide

<400>  358
gagccagaaa ggcacttgg                                                    19


<210>  359
<211>  21
<212>  DNA
<213>  Artificial Sequence

<220>
<223>  oligonucleotide

<400>  359
tctccaactc gatcacctga g                                                 21


<210>  360
<211>  22
<212>  DNA
<213>  Artificial Sequence

<220>
<223>  oligonucleotide

<400>  360
aacacttcag gcgcccttcc aa                                                22


<210>  361
<211>  21
<212>  DNA
<213>  Artificial Sequence

<220>
<223>  oligonucleotide

<400>  361
ggtcttatct gttggactct g                                                 21


<210>  362
<211>  20
<212>  DNA

<213>  Artificial Sequence

<220>
<223>  oligonucleotide

<400>  362
ctgaacttcc tctccaactc                                                    20


<210>  363
<211>  22
<212>  DNA
<213>  Artificial Sequence

<220>
<223>  oligonucleotide

<400>  363
aacacttcag gcgcccttcc aa                                                 22


<210>  364
<211>  22
<212>  DNA
<213>  Artificial Sequence

<220>
<223>  oligonucleotide

<400>  364
tcgaactgtg actgtctaaa tg                                                 22


<210>  365
<211>  19
<212>  DNA
<213>  Artificial Sequence

<220>
<223>  oligonucleotide

<400>  365
ctgccctccg aatttcttt                                                     19


<210>  366
<211>  23
<212>  DNA
<213>  Artificial Sequence

<220>
<223>  oligonucleotide

<400>  366
aacattcact ggtgcaactg ccc                                                23


<210>  367
<211>  19
<212>  DNA
<213>  Artificial Sequence

<220>

<223>  oligonucleotide

<400>  367
gttccatcga actgtgact                                                    19


<210>  368
<211>  20
<212>  DNA
<213>  Artificial Sequence

<220>
<223>  oligonucleotide

<400>  368
cgaatttctt tgggcagttg                                                   20


<210>  369
<211>  26
<212>  DNA
<213>  Artificial Sequence

<220>
<223>  oligonucleotide

<400>  369
tggaggaaca tgtgtgtcca acaagt                                            26


<210>  370
<211>  20
<212>  DNA
<213>  Artificial Sequence

<220>
<223>  oligonucleotide

<400>  370
gtgcaactgc ccaaagaaat                                                   20


<210>  371
<211>  19
<212>  DNA
<213>  Artificial Sequence

<220>
<223>  oligonucleotide

<400>  371
gacagtggca gagttccag                                                    19


<210>  372
<211>  22
<212>  DNA
<213>  Artificial Sequence

<220>
<223>  oligonucleotide

<400>  372

aggaaaggcc agcactgaca cc                                                22

<210>  373
<211>  19
<212>  DNA
<213>  Artificial Sequence

<220>
<223>  oligonucleotide

<400>  373
actgcccaaa gaaattcgg                                                    19

<210>  374
<211>  19
<212>  DNA
<213>  Artificial Sequence

<220>
<223>  oligonucleotide

<400>  374
ctggcctttc ctcggtaaa                                                    19

<210>  375
<211>  31
<212>  DNA
<213>  Artificial Sequence

<220>
<223>  oligonucleotide

<400>  375
cagcactgtg aaatagataa gtcaaaaacc t                                      31

<210>  376
<211>  19
<212>  DNA
<213>  Artificial Sequence

<220>
<223>  oligonucleotide

<400>  376
tggtgatgat ggtgatggt                                                    19

<210>  377
<211>  19
<212>  DNA
<213>  Artificial Sequence

<220>
<223>  oligonucleotide

<400>  377
cagggcatct tctctcctc                                                    19

<210>  378
<211>  22
<212>  DNA
<213>  Artificial Sequence

<220>
<223>  oligonucleotide

<400>  378
acaagctgtc tgcacggtcc tt                                                22


<210>  379
<211>  20
<212>  DNA
<213>  Artificial Sequence

<220>
<223>  oligonucleotide

<400>  379
agccactaca agctgtctgc                                                   20


<210>  380
<211>  19
<212>  DNA
<213>  Artificial Sequence

<220>
<223>  oligonucleotide

<400>  380
tgacactgtg ctctccgag                                                    19


<210>  381
<211>  24
<212>  DNA
<213>  Artificial Sequence

<220>
<223>  oligonucleotide

<400>  381
agagaagatg ccctgtcctc agaa                                              24


<210>  382
<211>  19
<212>  DNA
<213>  Artificial Sequence

<220>
<223>  oligonucleotide

<400>  382
gtctgcacgg tccttcatc                                                    19


<210>  383
<211>  19
<212>  DNA

<213>  Artificial Sequence

<220>
<223>  oligonucleotide

<400>  383
gattccgttg cctgacact                                                    19


<210>  384
<211>  25
<212>  DNA
<213>  Artificial Sequence

<220>
<223>  oligonucleotide

<400>  384
agaagatgcc ctgtcctcag aagga                                             25


<210>  385
<211>  19
<212>  DNA
<213>  Artificial Sequence

<220>
<223>  oligonucleotide

<400>  385
atcatcgtgg tggtgatga                                                    19


<210>  386
<211>  19
<212>  DNA
<213>  Artificial Sequence

<220>
<223>  oligonucleotide

<400>  386
ctgaggacag ggcatcttc                                                    19


<210>  387
<211>  22
<212>  DNA
<213>  Artificial Sequence

<220>
<223>  oligonucleotide

<400>  387
acaagctgtc tgcacggtcc tt                                                22


<210>  388
<211>  22
<212>  DNA
<213>  Artificial Sequence

<220>

<223>  oligonucleotide

<400>  388
cagcgatggt tacattgaat ac                                                22


<210>  389
<211>  19
<212>  DNA
<213>  Artificial Sequence

<220>
<223>  oligonucleotide

<400>  389
cgaagagtgg cctgaatag                                                    19


<210>  390
<211>  28
<212>  DNA
<213>  Artificial Sequence

<220>
<223>  oligonucleotide

<400>  390
acaacctgcc ttccttaact ctttctgc                                          28


<210>  391
<211>  19
<212>  DNA
<213>  Artificial Sequence

<220>
<223>  oligonucleotide

<400>  391
ggcaggttgt ccttctatt                                                    19


<210>  392
<211>  21
<212>  DNA
<213>  Artificial Sequence

<220>
<223>  oligonucleotide

<400>  392
tcatcctggc ttgaagataa a                                                 21


<210>  393
<211>  24
<212>  DNA
<213>  Artificial Sequence

<220>
<223>  oligonucleotide

<400>  393

tgtactgcat gctgtttgtg gcac                                              24

<210>  394
<211>  21
<212>  DNA
<213>  Artificial Sequence

<220>
<223>  oligonucleotide

<400>  394
atgtcgaggg aatgcagaaa g                                                 21

<210>  395
<211>  24
<212>  DNA
<213>  Artificial Sequence

<220>
<223>  oligonucleotide

<400>  395
caggttgtcc ttctattcag gcca                                              24

<210>  396
<211>  24
<212>  DNA
<213>  Artificial Sequence

<220>
<223>  oligonucleotide

<400>  396
caggttgtcc ttctattcag gcca                                              24

<210>  397
<211>  21
<212>  DNA
<213>  Artificial Sequence

<220>
<223>  oligonucleotide

<400>  397
gagggaatgc agaaagagtt a                                                 21

<210>  398
<211>  22
<212>  DNA
<213>  Artificial Sequence

<220>
<223>  oligonucleotide

<400>  398
gaagataaag tgccacaaac ag                                                22

<210>  399
<211>  25
<212>  DNA
<213>  Artificial Sequence

<220>
<223>  oligonucleotide

<400>  399
aggcaggttg tccttctatt caggc                                              25


<210>  400
<211>  21
<212>  DNA
<213>  Artificial Sequence

<220>
<223>  oligonucleotide

<400>  400
ctctaaaggt actgcacatg a                                                  21


<210>  401
<211>  20
<212>  DNA
<213>  Artificial Sequence

<220>
<223>  oligonucleotide

<400>  401
ctttggcttt ggctagaaac                                                    20


<210>  402
<211>  29
<212>  DNA
<213>  Artificial Sequence

<220>
<223>  oligonucleotide

<400>  402
acagccttca tttctctgaa catactgcc                                          29


<210>  403
<211>  21
<212>  DNA
<213>  Artificial Sequence

<220>
<223>  oligonucleotide

<400>  403
gctagccggt tatttcatag a                                                  21


<210>  404
<211>  23
<212>  DNA

<213>  Artificial Sequence

<220>
<223>  oligonucleotide

<400>  404
ctttcattga tctgtcccat aag                                               23


<210>  405
<211>  31
<212>  DNA
<213>  Artificial Sequence

<220>
<223>  oligonucleotide

<400>  405
tgacagcctt catttctctg aacatactgc c                                      31


<210>  406
<211>  23
<212>  DNA
<213>  Artificial Sequence

<220>
<223>  oligonucleotide

<400>  406
ccagtataca ggtacaacta cag                                               23


<210>  407
<211>  22
<212>  DNA
<213>  Artificial Sequence

<220>
<223>  oligonucleotide

<400>  407
taaggcagta tgttcagaga aa                                                22


<210>  408
<211>  28
<212>  DNA
<213>  Artificial Sequence

<220>
<223>  oligonucleotide

<400>  408
tgctagccgg ttatttcata gacactct                                          28


<210>  409
<211>  24
<212>  DNA
<213>  Artificial Sequence

<220>

<223>  oligonucleotide

<400>  409
actctaaagg tactgcacat gatc                                              24


<210>  410
<211>  22
<212>  DNA
<213>  Artificial Sequence

<220>
<223>  oligonucleotide

<400>  410
ctttggcttt ggctagaaac tc                                                22


<210>  411
<211>  29
<212>  DNA
<213>  Artificial Sequence

<220>
<223>  oligonucleotide

<400>  411
aaaacagctc tggaaaatga cagccttca                                         29


<210>  412
<211>  19
<212>  DNA
<213>  Artificial Sequence

<220>
<223>  oligonucleotide

<400>  412
ggagcctgag cttatgaat                                                    19


<210>  413
<211>  23
<212>  DNA
<213>  Artificial Sequence

<220>
<223>  oligonucleotide

<400>  413
gaagtgaaag tcagatggtt tag                                               23


<210>  414
<211>  22
<212>  DNA
<213>  Artificial Sequence

<220>
<223>  oligonucleotide

<400>  414

ttggtggagg agaagggttg gc                                                22

<210>  415
<211>  20
<212>  DNA
<213>  Artificial Sequence

<220>
<223>  oligonucleotide

<400>  415
tatgaatgcc aacccttctc                                                    20

<210>  416
<211>  19
<212>  DNA
<213>  Artificial Sequence

<220>
<223>  oligonucleotide

<400>  416
ccctttccga tcactttca                                                     19

<210>  417
<211>  22
<212>  DNA
<213>  Artificial Sequence

<220>
<223>  oligonucleotide

<400>  417
aatcaacctt ggcccgtcgt cc                                                 22

<210>  418
<211>  20
<212>  DNA
<213>  Artificial Sequence

<220>
<223>  oligonucleotide

<400>  418
caggagcctg agcttatgaa                                                    20

<210>  419
<211>  22
<212>  DNA
<213>  Artificial Sequence

<220>
<223>  oligonucleotide

<400>  419
gatggtttag catgaggatt gg                                                 22

<210>  420
<211>  23
<212>  DNA
<213>  Artificial Sequence

<220>
<223>  oligonucleotide

<400>  420
tcagcaaatc aaccttggcc cgt                                              23


<210>  421
<211>  20
<212>  DNA
<213>  Artificial Sequence

<220>
<223>  oligonucleotide

<400>  421
cttgaagatc tcccaacctc                                                  20


<210>  422
<211>  21
<212>  DNA
<213>  Artificial Sequence

<220>
<223>  oligonucleotide

<400>  422
caaggttgat ttgctgagaa g                                                21


<210>  423
<211>  22
<212>  DNA
<213>  Artificial Sequence

<220>
<223>  oligonucleotide

<400>  423
ttggtggagg agaagggttg gc                                               22


<210>  424
<211>  20
<212>  DNA
<213>  Artificial Sequence

<220>
<223>  oligonucleotide

<400>  424
atgaaactga acacaagtgc                                                  20


<210>  425
<211>  21
<212>  DNA

<213>  Artificial Sequence

<220>
<223>  oligonucleotide

<400>  425
aggctataca gcgtaaagaa a                                                21


<210>  426
<211>  27
<212>  DNA
<213>  Artificial Sequence

<220>
<223>  oligonucleotide

<400>  426
ctgtaccaca gtgatgcctg ttcttca                                          27


<210>  427
<211>  23
<212>  DNA
<213>  Artificial Sequence

<220>
<223>  oligonucleotide

<400>  427
ctgttcttca aaagcagtgg ttt                                              23


<210>  428
<211>  17
<212>  DNA
<213>  Artificial Sequence

<220>
<223>  oligonucleotide

<400>  428
tggcggcttg agttcaa                                                     17


<210>  429
<211>  25
<212>  DNA
<213>  Artificial Sequence

<220>
<223>  oligonucleotide

<400>  429
ttacgctgta tagcctgcgg ggtca                                            25


<210>  430
<211>  19
<212>  DNA
<213>  Artificial Sequence

<220>

<223>  oligonucleotide

<400>  430
cggatccacc agctttatg                                                    19


<210>  431
<211>  20
<212>  DNA
<213>  Artificial Sequence

<220>
<223>  oligonucleotide

<400>  431
tttgaagaac aggcatcact                                                   20


<210>  432
<211>  24
<212>  DNA
<213>  Artificial Sequence

<220>
<223>  oligonucleotide

<400>  432
acacaagtgc cggcaatgtc gata                                              24


<210>  433
<211>  20
<212>  DNA
<213>  Artificial Sequence

<220>
<223>  oligonucleotide

<400>  433
cttgaagatc tcccaacctc                                                   20


<210>  434
<211>  21
<212>  DNA
<213>  Artificial Sequence

<220>
<223>  oligonucleotide

<400>  434
caaggttgat ttgctgagaa g                                                 21


<210>  435
<211>  22
<212>  DNA
<213>  Artificial Sequence

<220>
<223>  oligonucleotide

<400>  435

ttggtggagg agaagggttg gc                                                22

<210>  436
<211>  20
<212>  DNA
<213>  Artificial Sequence

<220>
<223>  oligonucleotide

<400>  436
cccactgaag aagacaactg                                                  20

<210>  437
<211>  19
<212>  DNA
<213>  Artificial Sequence

<220>
<223>  oligonucleotide

<400>  437
tgcaggtcta gtacctctg                                                   19

<210>  438
<211>  22
<212>  DNA
<213>  Artificial Sequence

<220>
<223>  oligonucleotide

<400>  438
atctgttgca ggaggctcag gc                                               22

<210>  439
<211>  20
<212>  DNA
<213>  Artificial Sequence

<220>
<223>  oligonucleotide

<400>  439
aacaggcaga agaggatctg                                                  20

<210>  440
<211>  19
<212>  DNA
<213>  Artificial Sequence

<220>
<223>  oligonucleotide

<400>  440
aggactcatg cagccttac                                                   19

<210>  441
<211>  22
<212>  DNA
<213>  Artificial Sequence

<220>
<223>  oligonucleotide

<400>  441
tactagacct gcaggaccgc ga                                                22


<210>  442
<211>  21
<212>  DNA
<213>  Artificial Sequence

<220>
<223>  oligonucleotide

<400>  442
agaagaggat ctgttgcagg a                                                 21


<210>  443
<211>  19
<212>  DNA
<213>  Artificial Sequence

<220>
<223>  oligonucleotide

<400>  443
cacatgggtc acagcaagg                                                    19


<210>  444
<211>  21
<212>  DNA
<213>  Artificial Sequence

<220>
<223>  oligonucleotide

<400>  444
tcgctgcgta aggctgcatg a                                                 21


<210>  445
<211>  19
<212>  DNA
<213>  Artificial Sequence

<220>
<223>  oligonucleotide

<400>  445
ccttggcaga ggtactaga                                                    19


<210>  446
<211>  19
<212>  DNA

<213>  Artificial Sequence

<220>
<223>  oligonucleotide

<400>  446
attgaagaag cggcagtag                                                    19


<210>  447
<211>  21
<212>  DNA
<213>  Artificial Sequence

<220>
<223>  oligonucleotide

<400>  447
aggtgccttg ctgtgaccca t                                                 21


<210>  448
<211>  18
<212>  DNA
<213>  Artificial Sequence

<220>
<223>  oligonucleotide

<400>  448
cctttcttgg cccttgtt                                                     18


<210>  449
<211>  19
<212>  DNA
<213>  Artificial Sequence

<220>
<223>  oligonucleotide

<400>  449
aaggttgctt tgccatttg                                                    19


<210>  450
<211>  24
<212>  DNA
<213>  Artificial Sequence

<220>
<223>  oligonucleotide

<400>  450
tgactctcgg actgagaaac gcaa                                              24


<210>  451
<211>  19
<212>  DNA
<213>  Artificial Sequence

<220>

<223>  oligonucleotide

<400>  451
atcgcatcat cgcggtatt                                                    19


<210>  452
<211>  22
<212>  DNA
<213>  Artificial Sequence

<220>
<223>  oligonucleotide

<400>  452
gagtcagagt cagacatttg gg                                                22


<210>  453
<211>  22
<212>  DNA
<213>  Artificial Sequence

<220>
<223>  oligonucleotide

<400>  453
cgccctttct tggcccttgt tc                                                22


<210>  454
<211>  20
<212>  DNA
<213>  Artificial Sequence

<220>
<223>  oligonucleotide

<400>  454
ggcctgttct atagcatctt                                                   20


<210>  455
<211>  20
<212>  DNA
<213>  Artificial Sequence

<220>
<223>  oligonucleotide

<400>  455
gtgtacatga agtccaggag                                                   20


<210>  456
<211>  26
<212>  DNA
<213>  Artificial Sequence

<220>
<223>  oligonucleotide

<400>  456

tcctgagatc aaccctgagg gattct                                          26

<210>  457
<211>  18
<212>  DNA
<213>  Artificial Sequence

<220>
<223>  oligonucleotide

<400>  457
gagccgtgag cagtttag                                                   18

<210>  458
<211>  22
<212>  DNA
<213>  Artificial Sequence

<220>
<223>  oligonucleotide

<400>  458
gatcacacta aggttgcatt tc                                              22

<210>  459
<211>  21
<212>  DNA
<213>  Artificial Sequence

<220>
<223>  oligonucleotide

<400>  459
aaacggtcct catggcctgc a                                               21

<210>  460
<211>  21
<212>  DNA
<213>  Artificial Sequence

<220>
<223>  oligonucleotide

<400>  460
gtcgagacat cttgactgat g                                               21

<210>  461
<211>  19
<212>  DNA
<213>  Artificial Sequence

<220>
<223>  oligonucleotide

<400>  461
gctatagaac aggccactg                                                  19

<210>  462
<211>  24
<212>  DNA
<213>  Artificial Sequence

<220>
<223>  oligonucleotide

<400>  462
tcattgttgt gagccgtgag cagt                                              24


<210>  463
<211>  24
<212>  DNA
<213>  Artificial Sequence

<220>
<223>  oligonucleotide

<400>  463
gtgatgttct tctcaacctt aatc                                              24


<210>  464
<211>  20
<212>  DNA
<213>  Artificial Sequence

<220>
<223>  oligonucleotide

<400>  464
ttatgggctc taaactgctc                                                   20


<210>  465
<211>  27
<212>  DNA
<213>  Artificial Sequence

<220>
<223>  oligonucleotide

<400>  465
actgatgttg tcattgttgt gagccgt                                           27


<210>  466
<211>  19
<212>  DNA
<213>  Artificial Sequence

<220>
<223>  oligonucleotide

<400>  466
atgagtctgg acggagtag                                                    19


<210>  467
<211>  21
<212>  DNA

<213>  Artificial Sequence

<220>
<223>  oligonucleotide

<400>  467
ctctccaatg ctatgggtat c                                                21


<210>  468
<211>  22
<212>  DNA
<213>  Artificial Sequence

<220>
<223>  oligonucleotide

<400>  468
tcgctcgcag acaccacaag at                                               22


<210>  469
<211>  20
<212>  DNA
<213>  Artificial Sequence

<220>
<223>  oligonucleotide

<400>  469
cccatagcat tggagagaaa                                                  20


<210>  470
<211>  20
<212>  DNA
<213>  Artificial Sequence

<220>
<223>  oligonucleotide

<400>  470
acttgaccaa tcccatatcc                                                  20


<210>  471
<211>  25
<212>  DNA
<213>  Artificial Sequence

<220>
<223>  oligonucleotide

<400>  471
acagctcaca gtctgaggaa gatga                                            25


<210>  472
<211>  22
<212>  DNA
<213>  Artificial Sequence

<220>

<223>  oligonucleotide

<400>  472
cacaagatac caacagagct tc                                                22


<210>  473
<211>  24
<212>  DNA
<213>  Artificial Sequence

<220>
<223>  oligonucleotide

<400>  473
gctttcaact tctttccttc tttc                                              24


<210>  474
<211>  25
<212>  DNA
<213>  Artificial Sequence

<220>
<223>  oligonucleotide

<400>  474
acagctcaca gtctgaggaa gatga                                             25


<210>  475
<211>  21
<212>  DNA
<213>  Artificial Sequence

<220>
<223>  oligonucleotide

<400>  475
agagcttctg aaacagatac c                                                 21


<210>  476
<211>  22
<212>  DNA
<213>  Artificial Sequence

<220>
<223>  oligonucleotide

<400>  476
gctttcaact tctttccttc tt                                                22


<210>  477
<211>  25
<212>  DNA
<213>  Artificial Sequence

<220>
<223>  oligonucleotide

<400>  477

acagctcaca gtctgaggaa gatga                                                25

<210>  478
<211>  21
<212>  DNA
<213>  Artificial Sequence

<220>
<223>  oligonucleotide

<400>  478
tccttcatct ccaagtttct c                                                    21

<210>  479
<211>  20
<212>  DNA
<213>  Artificial Sequence

<220>
<223>  oligonucleotide

<400>  479
tgggaaccag tgatgtttat                                                      20

<210>  480
<211>  22
<212>  DNA
<213>  Artificial Sequence

<220>
<223>  oligonucleotide

<400>  480
agcgacagct gcagaccttc ag                                                   22

<210>  481
<211>  17
<212>  DNA
<213>  Artificial Sequence

<220>
<223>  oligonucleotide

<400>  481
cgacagctgc agacctt                                                         17

<210>  482
<211>  19
<212>  DNA
<213>  Artificial Sequence

<220>
<223>  oligonucleotide

<400>  482
gttgatgcga atacaacgg                                                       19

<210>  483
<211>  23
<212>  DNA
<213>  Artificial Sequence

<220>
<223>  oligonucleotide

<400>  483
caggagctgc tggcagaaca tta                                              23


<210>  484
<211>  19
<212>  DNA
<213>  Artificial Sequence

<220>
<223>  oligonucleotide

<400>  484
acatcactgg ttcccagaa                                                   19


<210>  485
<211>  19
<212>  DNA
<213>  Artificial Sequence

<220>
<223>  oligonucleotide

<400>  485
gcctgtccaa tcagaggat                                                   19


<210>  486
<211>  23
<212>  DNA
<213>  Artificial Sequence

<220>
<223>  oligonucleotide

<400>  486
acaacggtaa cccgatccct tgc                                              23


<210>  487
<211>  20
<212>  DNA
<213>  Artificial Sequence

<220>
<223>  oligonucleotide

<400>  487
atcgggttac cgttgtattc                                                  20


<210>  488
<211>  19
<212>  DNA

<213>  Artificial Sequence

<220>
<223>  oligonucleotide

<400>  488
ctgactgctc agtccaatc                                                    19


<210>  489
<211>  23
<212>  DNA
<213>  Artificial Sequence

<220>
<223>  oligonucleotide

<400>  489
cctctgattg gacaggcagc aca                                               23


<210>  490
<211>  19
<212>  DNA
<213>  Artificial Sequence

<220>
<223>  oligonucleotide

<400>  490
gagaagtgcg gagattcaa                                                    19


<210>  491
<211>  19
<212>  DNA
<213>  Artificial Sequence

<220>
<223>  oligonucleotide

<400>  491
ttctcacaca gacgtttcc                                                    19


<210>  492
<211>  22
<212>  DNA
<213>  Artificial Sequence

<220>
<223>  oligonucleotide

<400>  492
acgttactca ggtgcgggca tt                                                22


<210>  493
<211>  19
<212>  DNA
<213>  Artificial Sequence

<220>

<223>  oligonucleotide

<400>  493
gtgctgaatg aggaacaga                                                    19


<210>  494
<211>  19
<212>  DNA
<213>  Artificial Sequence

<220>
<223>  oligonucleotide

<400>  494
agttcttgac cgctttctt                                                    19


<210>  495
<211>  24
<212>  DNA
<213>  Artificial Sequence

<220>
<223>  oligonucleotide

<400>  495
acgtctgtgt gagaacattg ccgg                                              24


<210>  496
<211>  19
<212>  DNA
<213>  Artificial Sequence

<220>
<223>  oligonucleotide

<400>  496
ctccggaaca acagccttc                                                    19


<210>  497
<211>  23
<212>  DNA
<213>  Artificial Sequence

<220>
<223>  oligonucleotide

<400>  497
catcattggc agtgttgaat ctc                                               23


<210>  498
<211>  23
<212>  DNA
<213>  Artificial Sequence

<220>
<223>  oligonucleotide

<400>  498

tattggatgc tgtgctccag ggc                                                23

<210>  499
<211>  21
<212>  DNA
<213>  Artificial Sequence

<220>
<223>  oligonucleotide

<400>  499
aacactgcca atgatgataa c                                                  21

<210>  500
<211>  19
<212>  DNA
<213>  Artificial Sequence

<220>
<223>  oligonucleotide

<400>  500
acagacgttt cctctgttc                                                     19

<210>  501
<211>  23
<212>  DNA
<213>  Artificial Sequence

<220>
<223>  oligonucleotide

<400>  501
cgggcattct atgtgaacgt gct                                                23

<210>  502
<211>  18
<212>  DNA
<213>  Artificial Sequence

<220>
<223>  oligonucleotide

<400>  502
cgatgacgtg gtcaagat                                                      18

<210>  503
<211>  18
<212>  DNA
<213>  Artificial Sequence

<220>
<223>  oligonucleotide

<400>  503
cttccaaatg ccgtcaaa                                                      18

<210>  504
<211>  24
<212>  DNA
<213>  Artificial Sequence

<220>
<223>  oligonucleotide

<400>  504
ttgcagaacc agaagggaca caca                                              24


<210>  505
<211>  20
<212>  DNA
<213>  Artificial Sequence

<220>
<223>  oligonucleotide

<400>  505
ccagaaggga cacacagttt                                                   20


<210>  506
<211>  19
<212>  DNA
<213>  Artificial Sequence

<220>
<223>  oligonucleotide

<400>  506
aaagagggca gacagcaag                                                    19


<210>  507
<211>  22
<212>  DNA
<213>  Artificial Sequence

<220>
<223>  oligonucleotide

<400>  507
aaggccagct tcaccacctt ca                                                22


<210>  508
<211>  19
<212>  DNA
<213>  Artificial Sequence

<220>
<223>  oligonucleotide

<400>  508
gcacaccaag gagatcgac                                                    19


<210>  509
<211>  20
<212>  DNA

<213>  Artificial Sequence

<220>
<223>  oligonucleotide

<400>  509
cccttctggt tctgcaatca                                                    20


<210>  510
<211>  23
<212>  DNA
<213>  Artificial Sequence

<220>
<223>  oligonucleotide

<400>  510
tggtcaaccg cgaccctaaa cac                                                23


<210>  511
<211>  19
<212>  DNA
<213>  Artificial Sequence

<220>
<223>  oligonucleotide

<400>  511
cgatgacgtg gtcaagatt                                                     19


<210>  512
<211>  25
<212>  DNA
<213>  Artificial Sequence

<220>
<223>  oligonucleotide

<400>  512
aactgtgtgt cccttctggt tctgc                                              25


<210>  513
<211>  19
<212>  DNA
<213>  Artificial Sequence

<220>
<223>  oligonucleotide

<400>  513
cacagtgaag gtggtgaag                                                     19


<210>  514
<211>  20
<212>  DNA
<213>  Artificial Sequence

<220>

<223>  oligonucleotide

<400>  514
acaggttctt ggctcttatg                                                    20


<210>  515
<211>  22
<212>  DNA
<213>  Artificial Sequence

<220>
<223>  oligonucleotide

<400>  515
tgcagtcttc ttcaactatt ct                                                 22


<210>  516
<211>  30
<212>  DNA
<213>  Artificial Sequence

<220>
<223>  oligonucleotide

<400>  516
cctaaacatt tgtcttgcat tggagtctgt                                         30


<210>  517
<211>  19
<212>  DNA
<213>  Artificial Sequence

<220>
<223>  oligonucleotide

<400>  517
ttgctggctg ctagaatag                                                     19


<210>  518
<211>  22
<212>  DNA
<213>  Artificial Sequence

<220>
<223>  oligonucleotide

<400>  518
gtcagaagca gtacatttac ac                                                 22


<210>  519
<211>  26
<212>  DNA
<213>  Artificial Sequence

<220>
<223>  oligonucleotide

<400>  519

tccatccact catgatgtga tccgga                                              26

<210>  520
<211>  20
<212>  DNA
<213>  Artificial Sequence

<220>
<223>  oligonucleotide

<400>  520
acaggttctt ggctcttatg                                                    20

<210>  521
<211>  22
<212>  DNA
<213>  Artificial Sequence

<220>
<223>  oligonucleotide

<400>  521
tgcagtcttc ttcaactatt ct                                                 22

<210>  522
<211>  25
<212>  DNA
<213>  Artificial Sequence

<220>
<223>  oligonucleotide

<400>  522
acatttgtct tgcattggag tctgt                                              25

<210>  523
<211>  19
<212>  DNA
<213>  Artificial Sequence

<220>
<223>  oligonucleotide

<400>  523
tggatcttgc actgaaact                                                     19

<210>  524
<211>  21
<212>  DNA
<213>  Artificial Sequence

<220>
<223>  oligonucleotide

<400>  524
ctccaatgca agacaaatgt t                                                  21

<210>  525
<211>  28
<212>  DNA
<213>  Artificial Sequence

<220>
<223>  oligonucleotide

<400>  525
caccttcata agagccaaga acctgtcc                                          28


<210>  526
<211>  19
<212>  DNA
<213>  Artificial Sequence

<220>
<223>  oligonucleotide

<400>  526
agagccaaca gaacagaag                                                    19


<210>  527
<211>  19
<212>  DNA
<213>  Artificial Sequence

<220>
<223>  oligonucleotide

<400>  527
tcgagctgtt tacgtttga                                                    19


<210>  528
<211>  23
<212>  DNA
<213>  Artificial Sequence

<220>
<223>  oligonucleotide

<400>  528
aaatgccggt tctgtggagc aga                                               23


<210>  529
<211>  22
<212>  DNA
<213>  Artificial Sequence

<220>
<223>  oligonucleotide

<400>  529
caaacgtaaa cagctcgaat ta                                                22


<210>  530
<211>  20
<212>  DNA

<213>  Artificial Sequence

<220>
<223>  oligonucleotide

<400>  530
tccatgaagt cagcgatatg                                                    20


<210>  531
<211>  28
<212>  DNA
<213>  Artificial Sequence

<220>
<223>  oligonucleotide

<400>  531
acatcactgc ttgatgaagc aaggaaga                                           28


<210>  532
<211>  19
<212>  DNA
<213>  Artificial Sequence

<220>
<223>  oligonucleotide

<400>  532
aagaagcctg gcctcagaa                                                     19


<210>  533
<211>  25
<212>  DNA
<213>  Artificial Sequence

<220>
<223>  oligonucleotide

<400>  533
tcatgtatat cttccttgct tcatc                                              25


<210>  534
<211>  30
<212>  DNA
<213>  Artificial Sequence

<220>
<223>  oligonucleotide

<400>  534
tcttaattcg agctgtttac gtttgacgtc                                         30


<210>  535
<211>  19
<212>  DNA
<213>  Artificial Sequence

<220>

<223>  oligonucleotide

<400>  535
cggttctgtg gagcagacg                                                    19


<210>  536
<211>  24
<212>  DNA
<213>  Artificial Sequence

<220>
<223>  oligonucleotide

<400>  536
cttcatcaag cagtgatgta tctg                                              24


<210>  537
<211>  23
<212>  DNA
<213>  Artificial Sequence

<220>
<223>  oligonucleotide

<400>  537
cctggcctca gaagacgtca aac                                               23


<210>  538
<211>  18
<212>  DNA
<213>  Artificial Sequence

<220>
<223>  oligonucleotide

<400>  538
gctgctgtgg aagaaact                                                     18


<210>  539
<211>  21
<212>  DNA
<213>  Artificial Sequence

<220>
<223>  oligonucleotide

<400>  539
gccctttgtc tgacagaatt a                                                 21


<210>  540
<211>  22
<212>  DNA
<213>  Artificial Sequence

<220>
<223>  oligonucleotide

<400>  540

tgccagtacc acttctccga gc                                              22

<210>  541
<211>  21
<212>  DNA
<213>  Artificial Sequence

<220>
<223>  oligonucleotide

<400>  541
atccgcaaac gattaattct g                                               21

<210>  542
<211>  20
<212>  DNA
<213>  Artificial Sequence

<220>
<223>  oligonucleotide

<400>  542
tccatactgc tctttccttg                                                 20

<210>  543
<211>  24
<212>  DNA
<213>  Artificial Sequence

<220>
<223>  oligonucleotide

<400>  543
acaaagggca gctggattgg aaga                                            24

<210>  544
<211>  19
<212>  DNA
<213>  Artificial Sequence

<220>
<223>  oligonucleotide

<400>  544
aagaaactct gccagtacc                                                  19

<210>  545
<211>  22
<212>  DNA
<213>  Artificial Sequence

<220>
<223>  oligonucleotide

<400>  545
acatcggaca agtttgaaat ac                                              22

<210>  546
<211>  23
<212>  DNA
<213>  Artificial Sequence

<220>
<223>  oligonucleotide

<400>  546
agcggcagat ccgcaaacga tta                                                23


<210>  547
<211>  19
<212>  DNA
<213>  Artificial Sequence

<220>
<223>  oligonucleotide

<400>  547
ctgctgtgga agaaactct                                                     19


<210>  548
<211>  19
<212>  DNA
<213>  Artificial Sequence

<220>
<223>  oligonucleotide

<400>  548
cttgggtaac atcggacaa                                                     19


<210>  549
<211>  23
<212>  DNA
<213>  Artificial Sequence

<220>
<223>  oligonucleotide

<400>  549
agcggcagat ccgcaaacga tta                                                23


<210>  550
<211>  21
<212>  DNA
<213>  Artificial Sequence

<220>
<223>  oligonucleotide

<400>  550
gtgccctact tcaaggataa g                                                  21


<210>  551
<211>  19
<212>  DNA

<213>  Artificial Sequence

<220>
<223>  oligonucleotide

<400>  551
cttgccagtt ccctcattc                                                    19


<210>  552
<211>  22
<212>  DNA
<213>  Artificial Sequence

<220>
<223>  oligonucleotide

<400>  552
aactccatcc ggcacaacct gt                                                22


<210>  553
<211>  19
<212>  DNA
<213>  Artificial Sequence

<220>
<223>  oligonucleotide

<400>  553
caacctgtca ctgcatagt                                                    19


<210>  554
<211>  19
<212>  DNA
<213>  Artificial Sequence

<220>
<223>  oligonucleotide

<400>  554
ttgatgatcc accaagagc                                                    19


<210>  555
<211>  22
<212>  DNA
<213>  Artificial Sequence

<220>
<223>  oligonucleotide

<400>  555
tgcgggtcca gaatgaggga ac                                                22


<210>  556
<211>  19
<212>  DNA
<213>  Artificial Sequence

<220>

<223>  oligonucleotide

<400>  556
acaaacggct cactctgtc                                                    19


<210>  557
<211>  19
<212>  DNA
<213>  Artificial Sequence

<220>
<223>  oligonucleotide

<400>  557
tgttgctgtc gcccttatc                                                    19


<210>  558
<211>  22
<212>  DNA
<213>  Artificial Sequence

<220>
<223>  oligonucleotide

<400>  558
tacgagtgga tggtgcgttg cg                                                22


<210>  559
<211>  21
<212>  DNA
<213>  Artificial Sequence

<220>
<223>  oligonucleotide

<400>  559
cgtgccctac ttcaaggata a                                                 21


<210>  560
<211>  18
<212>  DNA
<213>  Artificial Sequence

<220>
<223>  oligonucleotide

<400>  560
tcattctgga cccgcatg                                                     18


<210>  561
<211>  24
<212>  DNA
<213>  Artificial Sequence

<220>
<223>  oligonucleotide

<400>  561

cggctggaag aactccatcc ggca                                              24

<210>  562
<211>  19
<212>  DNA
<213>  Artificial Sequence

<220>
<223>  oligonucleotide

<400>  562
ggtgacgaat ccacattca                                                   19

<210>  563
<211>  19
<212>  DNA
<213>  Artificial Sequence

<220>
<223>  oligonucleotide

<400>  563
tcaccgttca gggagatta                                                   19

<210>  564
<211>  22
<212>  DNA
<213>  Artificial Sequence

<220>
<223>  oligonucleotide

<400>  564
tgccgggaaa gtcgctacat gg                                               22

<210>  565
<211>  22
<212>  DNA
<213>  Artificial Sequence

<220>
<223>  oligonucleotide

<400>  565
acgaatccac attcatctca at                                               22

<210>  566
<211>  20
<212>  DNA
<213>  Artificial Sequence

<220>
<223>  oligonucleotide

<400>  566
gatccatgta gcgactttcc                                                  20

<210>  567
<211>  29
<212>  DNA
<213>  Artificial Sequence

<220>
<223>  oligonucleotide

<400>  567
aaggatcctg ccttaagtca acttatttg                                          29


<210>  568
<211>  22
<212>  DNA
<213>  Artificial Sequence

<220>
<223>  oligonucleotide

<400>  568
cctgccttaa gtcaacttat tt                                                 22


<210>  569
<211>  19
<212>  DNA
<213>  Artificial Sequence

<220>
<223>  oligonucleotide

<400>  569
tcattcagat gccatcacc                                                     19


<210>  570
<211>  22
<212>  DNA
<213>  Artificial Sequence

<220>
<223>  oligonucleotide

<400>  570
tgccgggaaa gtcgctacat gg                                                 22


<210>  571
<211>  21
<212>  DNA
<213>  Artificial Sequence

<220>
<223>  oligonucleotide

<400>  571
cacattcatc tcaatggaag g                                                  21


<210>  572
<211>  21
<212>  DNA

<213>  Artificial Sequence

<220>
<223>  oligonucleotide

<400>  572
cagggagatt aatcactgga a                                                21


<210>  573
<211>  22
<212>  DNA
<213>  Artificial Sequence

<220>
<223>  oligonucleotide

<400>  573
tgccgggaaa gtcgctacat gg                                               22


<210>  574
<211>  19
<212>  DNA
<213>  Artificial Sequence

<220>
<223>  oligonucleotide

<400>  574
gacccagtat gccatcttt                                                   19


<210>  575
<211>  20
<212>  DNA
<213>  Artificial Sequence

<220>
<223>  oligonucleotide

<400>  575
ggcatctgtc tggacataaa                                                  20


<210>  576
<211>  21
<212>  DNA
<213>  Artificial Sequence

<220>
<223>  oligonucleotide

<400>  576
tttcggatga acgccggacc t                                                21


<210>  577
<211>  20
<212>  DNA
<213>  Artificial Sequence

<220>

<223>  oligonucleotide

<400>  577
ctggatccaa tctcagtgtc                                                   20


<210>  578
<211>  19
<212>  DNA
<213>  Artificial Sequence

<220>
<223>  oligonucleotide

<400>  578
caggtagtgg gtgatgttg                                                    19


<210>  579
<211>  22
<212>  DNA
<213>  Artificial Sequence

<220>
<223>  oligonucleotide

<400>  579
aagtggaaac caccctccga cc                                                22


<210>  580
<211>  22
<212>  DNA
<213>  Artificial Sequence

<220>
<223>  oligonucleotide

<400>  580
ggccaagagt gacatcattt at                                                22


<210>  581
<211>  23
<212>  DNA
<213>  Artificial Sequence

<220>
<223>  oligonucleotide

<400>  581
ggtggtttcc acttcagaat aat                                               23


<210>  582
<211>  17
<212>  DNA
<213>  Artificial Sequence

<220>
<223>  oligonucleotide

<400>  582
   aacccctctg tgcccct                                                    17


<210>  583
<211>  19
<212>  DNA
<213>  Artificial Sequence

<220>
<223>  oligonucleotide

<400>  583
ccatctttgt gaagaccct                                                   19


<210>  584
<211>  22
<212>  DNA
<213>  Artificial Sequence

<220>
<223>  oligonucleotide

<400>  584
aatctgggat gatgagttag ac                                               22


<210>  585
<211>  22
<212>  DNA
<213>  Artificial Sequence

<220>
<223>  oligonucleotide

<400>  585
tccagacaga tgccaccaac cc                                               22


<210>  586
<211>  19
<212>  DNA
<213>  Artificial Sequence

<220>
<223>  oligonucleotide

<400>  586
catctgcgcc tttgtttag                                                   19


<210>  587
<211>  19
<212>  DNA
<213>  Artificial Sequence

<220>
<223>  oligonucleotide

<400>  587
gtgctttggc tttgttgag                                                   19

<210>  588
<211>  23
<212>  DNA
<213>  Artificial Sequence

<220>
<223>  oligonucleotide

<400>  588
cccggcacac aacacttggt ttg                                                23


<210>  589
<211>  18
<212>  DNA
<213>  Artificial Sequence

<220>
<223>  oligonucleotide

<400>  589
gcacacaaca cttggttt                                                      18


<210>  590
<211>  21
<212>  DNA
<213>  Artificial Sequence

<220>
<223>  oligonucleotide

<400>  590
ctgttctcgt tccaaattct c                                                  21


<210>  591
<211>  30
<212>  DNA
<213>  Artificial Sequence

<220>
<223>  oligonucleotide

<400>  591
agcacacatc aagaaacttg aagaagctga                                         30


<210>  592
<211>  20
<212>  DNA
<213>  Artificial Sequence

<220>
<223>  oligonucleotide

<400>  592
ccaaagcaca catcaagaaa                                                    20


<210>  593
<211>  20
<212>  DNA

<213>  Artificial Sequence

<220>
<223>  oligonucleotide

<400>  593
ttcgtattcg ttccatctcc                                                   20


<210>  594
<211>  22
<212>  DNA
<213>  Artificial Sequence

<220>
<223>  oligonucleotide

<400>  594
aaagccagca ccagctcgag aa                                                22


<210>  595
<211>  19
<212>  DNA
<213>  Artificial Sequence

<220>
<223>  oligonucleotide

<400>  595
tgattccact aggaccaga                                                    19


<210>  596
<211>  22
<212>  DNA
<213>  Artificial Sequence

<220>
<223>  oligonucleotide

<400>  596
tctttcagct tcttcaagtt tc                                                22


<210>  597
<211>  23
<212>  DNA
<213>  Artificial Sequence

<220>
<223>  oligonucleotide

<400>  597
cccggcacac aacacttggt ttg                                               23


<210>  598
<211>  17
<212>  DNA
<213>  Artificial Sequence

<220>

<223>  oligonucleotide

<400>  598
agcggcttca gcagatc                                                      17


<210>  599
<211>  17
<212>  DNA
<213>  Artificial Sequence

<220>
<223>  oligonucleotide

<400>  599
gcctggagcc cagatac                                                      17


<210>  600
<211>  22
<212>  DNA
<213>  Artificial Sequence

<220>
<223>  oligonucleotide

<400>  600
actagcagca tgttgagccg gg                                                22


<210>  601
<211>  19
<212>  DNA
<213>  Artificial Sequence

<220>
<223>  oligonucleotide

<400>  601
gcactagcag catgttgag                                                    19


<210>  602
<211>  19
<212>  DNA
<213>  Artificial Sequence

<220>
<223>  oligonucleotide

<400>  602
cgttgatgtg aggttccag                                                    19


<210>  603
<211>  20
<212>  DNA
<213>  Artificial Sequence

<220>
<223>  oligonucleotide

<400>  603

tccaggcaga agcacagcct                                                    20

<210>  604
<211>  19
<212>  DNA
<213>  Artificial Sequence

<220>
<223>  oligonucleotide

<400>  604
cgacctgccc tacgactac                                                     19

<210>  605
<211>  21
<212>  DNA
<213>  Artificial Sequence

<220>
<223>  oligonucleotide

<400>  605
ggtgacgttc aggttgttca c                                                  21

<210>  606
<211>  22
<212>  DNA
<213>  Artificial Sequence

<220>
<223>  oligonucleotide

<400>  606
tggaacctca catcaacgcg ca                                                 22

<210>  607
<211>  19
<212>  DNA
<213>  Artificial Sequence

<220>
<223>  oligonucleotide

<400>  607
cctggaacct cacatcaac                                                     19

<210>  608
<211>  19
<212>  DNA
<213>  Artificial Sequence

<220>
<223>  oligonucleotide

<400>  608
cctcctggta cttctcctc                                                     19

<210>  609
<211>  22
<212>  DNA
<213>  Artificial Sequence

<220>
<223>  oligonucleotide

<400>  609
tcaggttgtt cacgtaggcc gc                                                22


<210>  610
<211>  19
<212>  DNA
<213>  Artificial Sequence

<220>
<223>  oligonucleotide

<400>  610
ctctctgtgt ggctgtaac                                                    19


<210>  611
<211>  20
<212>  DNA
<213>  Artificial Sequence

<220>
<223>  oligonucleotide

<400>  611
gggctgttca tactctcttc                                                   20


<210>  612
<211>  23
<212>  DNA
<213>  Artificial Sequence

<220>
<223>  oligonucleotide

<400>  612
accaacgagc tgaagcagat gca                                               23


<210>  613
<211>  21
<212>  DNA
<213>  Artificial Sequence

<220>
<223>  oligonucleotide

<400>  613
ggctgtaact tacgtgtact t                                                 21


<210>  614
<211>  20
<212>  DNA

<213>  Artificial Sequence

<220>
<223>  oligonucleotide

<400>  614
gggtcccaat aactgtcatc                                                    20


<210>  615
<211>  23
<212>  DNA
<213>  Artificial Sequence

<220>
<223>  oligonucleotide

<400>  615
accaacgagc tgaagcagat gca                                                23


<210>  616
<211>  18
<212>  DNA
<213>  Artificial Sequence

<220>
<223>  oligonucleotide

<400>  616
cctgcagtct ctctgtgt                                                      18


<210>  617
<211>  20
<212>  DNA
<213>  Artificial Sequence

<220>
<223>  oligonucleotide

<400>  617
gccacttttg gagtacttgt                                                    20


<210>  618
<211>  24
<212>  DNA
<213>  Artificial Sequence

<220>
<223>  oligonucleotide

<400>  618
taccaacgag ctgaagcaga tgca                                               24


<210>  619
<211>  19
<212>  DNA
<213>  Artificial Sequence

<220>

<223>  oligonucleotide

<400>  619
gctgatcgtg atcttcaca                                                    19


<210>  620
<211>  20
<212>  DNA
<213>  Artificial Sequence

<220>
<223>  oligonucleotide

<400>  620
gagtacttgt cctgcatctg                                                   20


<210>  621
<211>  22
<212>  DNA
<213>  Artificial Sequence

<220>
<223>  oligonucleotide

<400>  621
tgcagtctct ctgtgtggct gt                                                22


<210>  622
<211>  22
<212>  DNA
<213>  Artificial Sequence

<220>
<223>  oligonucleotide

<400>  622
tccagtacaa ctttacctac aa                                                22


<210>  623
<211>  19
<212>  DNA
<213>  Artificial Sequence

<220>
<223>  oligonucleotide

<400>  623
agcacatctg caggataat                                                    19


<210>  624
<211>  22
<212>  DNA
<213>  Artificial Sequence

<220>
<223>  oligonucleotide

<400>  624

caggcatcag accagcttgc ca                                                22

<210>  625
<211>  19
<212>  DNA
<213>  Artificial Sequence

<220>
<223>  oligonucleotide

<400>  625
tgctacatgg agcttgttc                                                   19

<210>  626
<211>  19
<212>  DNA
<213>  Artificial Sequence

<220>
<223>  oligonucleotide

<400>  626
cgaaggagaa aggcaatga                                                   19

<210>  627
<211>  23
<212>  DNA
<213>  Artificial Sequence

<220>
<223>  oligonucleotide

<400>  627
ccaactgcag agatggcagc tga                                              23

<210>  628
<211>  21
<212>  DNA
<213>  Artificial Sequence

<220>
<223>  oligonucleotide

<400>  628
ggctgtattc cagtacaact t                                                21

<210>  629
<211>  20
<212>  DNA
<213>  Artificial Sequence

<220>
<223>  oligonucleotide

<400>  629
cagttggatc agtctggaaa                                                  20

<210>  630
<211>  22
<212>  DNA
<213>  Artificial Sequence

<220>
<223>  oligonucleotide

<400>  630
caggcatcag accagcttgc ca                                                22


<210>  631
<211>  19
<212>  DNA
<213>  Artificial Sequence

<220>
<223>  oligonucleotide

<400>  631
caccaaagca gccataaac                                                    19


<210>  632
<211>  21
<212>  DNA
<213>  Artificial Sequence

<220>
<223>  oligonucleotide

<400>  632
ctccatgtag caaacagtct a                                                 21


<210>  633
<211>  22
<212>  DNA
<213>  Artificial Sequence

<220>
<223>  oligonucleotide

<400>  633
caggcatcag accagcttgc ca                                                22


<210>  634
<211>  20
<212>  DNA
<213>  Artificial Sequence

<220>
<223>  oligonucleotide

<400>  634
cctcggtgtc ctacttcaaa                                                   20


<210>  635
<211>  19
<212>  DNA

<213>  Artificial Sequence

<220>
<223>  oligonucleotide

<400>  635
acttctgttc ctcgcagac                                                    19


<210>  636
<211>  22
<212>  DNA
<213>  Artificial Sequence

<220>
<223>  oligonucleotide

<400>  636
aagatcgtcg ccacctggat gc                                                22


<210>  637
<211>  22
<212>  DNA
<213>  Artificial Sequence

<220>
<223>  oligonucleotide

<400>  637
cttcaaatgt gtgcagaagg ag                                                22


<210>  638
<211>  19
<212>  DNA
<213>  Artificial Sequence

<220>
<223>  oligonucleotide

<400>  638
gaagcggtcc aggtagttc                                                    19


<210>  639
<211>  22
<212>  DNA
<213>  Artificial Sequence

<220>
<223>  oligonucleotide

<400>  639
tgcgaggaac agaagtgcga gg                                                22


<210>  640
<211>  18
<212>  DNA
<213>  Artificial Sequence

<220>

<223>  oligonucleotide

<400>  640
catgcggaag atcgtcgc                                                    18


<210>  641
<211>  17
<212>  DNA
<213>  Artificial Sequence

<220>
<223>  oligonucleotide

<400>  641
gacctcctcc tcgcact                                                     17


<210>  642
<211>  25
<212>  DNA
<213>  Artificial Sequence

<220>
<223>  oligonucleotide

<400>  642
ctggatgctg gaggtctgcg aggaa                                            25


<210>  643
<211>  22
<212>  DNA
<213>  Artificial Sequence

<220>
<223>  oligonucleotide

<400>  643
gtgtcctact tcaaatgtgt gc                                               22


<210>  644
<211>  19
<212>  DNA
<213>  Artificial Sequence

<220>
<223>  oligonucleotide

<400>  644
cctcctcgca cttctgttc                                                   19


<210>  645
<211>  22
<212>  DNA
<213>  Artificial Sequence

<220>
<223>  oligonucleotide

<400>  645

aagatcgtcg ccacctggat gc                                                22

<210>  646
<211>  20
<212>  DNA
<213>  Artificial Sequence

<220>
<223>  oligonucleotide

<400>  646
taccagatgg atgtgaaccc                                                    20

<210>  647
<211>  20
<212>  DNA
<213>  Artificial Sequence

<220>
<223>  oligonucleotide

<400>  647
ccgtgatctg tcaccacata                                                    20

<210>  648
<211>  22
<212>  DNA
<213>  Artificial Sequence

<220>
<223>  oligonucleotide

<400>  648
tgccacctgc gtgaagaagt gt                                                22

<210>  649
<211>  22
<212>  DNA
<213>  Artificial Sequence

<220>
<223>  oligonucleotide

<400>  649
gatgcattcc ttgtaaccaa ta                                                22

<210>  650
<211>  22
<212>  DNA
<213>  Artificial Sequence

<220>
<223>  oligonucleotide

<400>  650
atttcttcac caataccctt tg                                                22

<210>  651
<211>  23
<212>  DNA
<213>  Artificial Sequence

<220>
<223>  oligonucleotide

<400>  651
tcctgctttc aaacgggtct gga                                              23


<210>  652
<211>  21
<212>  DNA
<213>  Artificial Sequence

<220>
<223>  oligonucleotide

<400>  652
gagtgaaatc aggaacatct c                                                21


<210>  653
<211>  20
<212>  DNA
<213>  Artificial Sequence

<220>
<223>  oligonucleotide

<400>  653
cttgttgatt ctcagacgtg                                                  20


<210>  654
<211>  25
<212>  DNA
<213>  Artificial Sequence

<220>
<223>  oligonucleotide

<400>  654
atcgacaaga aggcacctga ctttg                                            25


<210>  655
<211>  21
<212>  DNA
<213>  Artificial Sequence

<220>
<223>  oligonucleotide

<400>  655
cagtcaattg cccatcaaac a                                                21


<210>  656
<211>  22
<212>  DNA

<213>  Artificial Sequence

<220>
<223>  oligonucleotide

<400>  656
gtgagctgtt gcaaataatc tt                                                22


<210>  657
<211>  24
<212>  DNA
<213>  Artificial Sequence

<220>
<223>  oligonucleotide

<400>  657
cacagccacc aaccacacta acga                                              24


<210>  658
<211>  23
<212>  DNA
<213>  Artificial Sequence

<220>
<223>  oligonucleotide

<400>  658
gtgtgacagt aagctaacct ttg                                               23


<210>  659
<211>  19
<212>  DNA
<213>  Artificial Sequence

<220>
<223>  oligonucleotide

<400>  659
gctcaacttc cgggtagaa                                                    19


<210>  660
<211>  28
<212>  DNA
<213>  Artificial Sequence

<220>
<223>  oligonucleotide

<400>  660
agacagattc tacatgcgca caaatccc                                          28


<210>  661
<211>  20
<212>  DNA
<213>  Artificial Sequence

<220>

<223>  oligonucleotide

<400>  661
cgatgcagcc atttctgata                                                    20


<210>  662
<211>  22
<212>  DNA
<213>  Artificial Sequence

<220>
<223>  oligonucleotide

<400>  662
ggaaagtctt cagctatttg ct                                                 22


<210>  663
<211>  30
<212>  DNA
<213>  Artificial Sequence

<220>
<223>  oligonucleotide

<400>  663
ctggagattt gatgagaaga gaaattccat                                         30


<210>  664
<211>  19
<212>  DNA
<213>  Artificial Sequence

<220>
<223>  oligonucleotide

<400>  664
catccacacc ctaggtttc                                                     19


<210>  665
<211>  22
<212>  DNA
<213>  Artificial Sequence

<220>
<223>  oligonucleotide

<400>  665
ctccagtatt tgtcctctac aa                                                 22


<210>  666
<211>  25
<212>  DNA
<213>  Artificial Sequence

<220>
<223>  oligonucleotide

<400>  666

tcgatgcagc catttctgat aagga 25

<210> 667
<211> 19
<212> DNA
<213> Artificial Sequence

<220>
<223> oligonucleotide

<400> 667
ccctaggttt ccctccaac 19

<210> 668
<211> 22
<212> DNA
<213> Artificial Sequence

<220>
<223> oligonucleotide

<400> 668
gctccatgga atttctcttc tc 22

<210> 669
<211> 25
<212> DNA
<213> Artificial Sequence

<220>
<223> oligonucleotide

<400> 669
tcgatgcagc catttctgat aagga 25

<210> 670
<211> 22
<212> DNA
<213> Artificial Sequence

<220>
<223> oligonucleotide

<400> 670
gtagaggaca aatactggag at 22

<210> 671
<211> 19
<212> DNA
<213> Artificial Sequence

<220>
<223> oligonucleotide

<400> 671
gagtcaatcc ctggaaagt 19

<210>  672
<211>  22
<212>  DNA
<213>  Artificial Sequence

<220>
<223>  oligonucleotide

<400>  672
ccatggagcc aggctttccc aa                                                22


<210>  673
<211>  20
<212>  DNA
<213>  Artificial Sequence

<220>
<223>  oligonucleotide

<400>  673
tggagacctg agaaccaatc                                                   20


<210>  674
<211>  19
<212>  DNA
<213>  Artificial Sequence

<220>
<223>  oligonucleotide

<400>  674
acccgagtgt aaccatagc                                                    19


<210>  675
<211>  23
<212>  DNA
<213>  Artificial Sequence

<220>
<223>  oligonucleotide

<400>  675
aggcagctgg cagaggaata cct                                               23


<210>  676
<211>  19
<212>  DNA
<213>  Artificial Sequence

<220>
<223>  oligonucleotide

<400>  676
ggagacctga gaaccaatc                                                    19


<210>  677
<211>  19
<212>  DNA

<213>  Artificial Sequence

<220>
<223>  oligonucleotide

<400>  677
gactctccac gcatctctg                                                    19


<210>  678
<211>  23
<212>  DNA
<213>  Artificial Sequence

<220>
<223>  oligonucleotide

<400>  678
aggcagctgg cagaggaata cct                                               23


<210>  679
<211>  20
<212>  DNA
<213>  Artificial Sequence

<220>
<223>  oligonucleotide

<400>  679
agaaccaatc tcaccgacag                                                   20


<210>  680
<211>  21
<212>  DNA
<213>  Artificial Sequence

<220>
<223>  oligonucleotide

<400>  680
ccagagattt cgactctcca c                                                 21


<210>  681
<211>  23
<212>  DNA
<213>  Artificial Sequence

<220>
<223>  oligonucleotide

<400>  681
tggttacact cgggtggcag aga                                               23


<210>  682
<211>  17
<212>  DNA
<213>  Artificial Sequence

<220>

<223>  oligonucleotide

<400>  682
tccacccttg tgctctt                                                    17


<210>  683
<211>  18
<212>  DNA
<213>  Artificial Sequence

<220>
<223>  oligonucleotide

<400>  683
actctccacg catctctg                                                   18


<210>  684
<211>  23
<212>  DNA
<213>  Artificial Sequence

<220>
<223>  oligonucleotide

<400>  684
aaccaatctc accgacaggc agc                                             23


<210>  685
<211>  19
<212>  DNA
<213>  Artificial Sequence

<220>
<223>  oligonucleotide

<400>  685
tgtgtgggtt agacttgtg                                                  19


<210>  686
<211>  19
<212>  DNA
<213>  Artificial Sequence

<220>
<223>  oligonucleotide

<400>  686
gtaacggctt cgggaatag                                                  19


<210>  687
<211>  20
<212>  DNA
<213>  Artificial Sequence

<220>
<223>  oligonucleotide

<400>  687

aaccagggca actctggccg                                                    20

<210>  688
<211>  19
<212>  DNA
<213>  Artificial Sequence

<220>
<223>  oligonucleotide

<400>  688
tgtgtgggtt agacttgtg                                                     19

<210>  689
<211>  19
<212>  DNA
<213>  Artificial Sequence

<220>
<223>  oligonucleotide

<400>  689
cacagctcat gtctgatga                                                     19

<210>  690
<211>  23
<212>  DNA
<213>  Artificial Sequence

<220>
<223>  oligonucleotide

<400>  690
ccgaagccgt tacctcgaat gca                                                23

<210>  691
<211>  19
<212>  DNA
<213>  Artificial Sequence

<220>
<223>  oligonucleotide

<400>  691
ctcagagaag accaacagg                                                     19

<210>  692
<211>  19
<212>  DNA
<213>  Artificial Sequence

<220>
<223>  oligonucleotide

<400>  692
cttcgggaat aggtgacag                                                     19

<210>  693
<211>  23
<212>  DNA
<213>  Artificial Sequence

<220>
<223>  oligonucleotide

<400>  693
acttgtgcaa ccagggcaac tct                                              23


<210>  694
<211>  19
<212>  DNA
<213>  Artificial Sequence

<220>
<223>  oligonucleotide

<400>  694
gaggttgtgt gtgggttag                                                   19


<210>  695
<211>  19
<212>  DNA
<213>  Artificial Sequence

<220>
<223>  oligonucleotide

<400>  695
tgatgagcca caggaaatg                                                   19


<210>  696
<211>  23
<212>  DNA
<213>  Artificial Sequence

<220>
<223>  oligonucleotide

<400>  696
acttgtgcaa ccagggcaac tct                                              23


<210>  697
<211>  20
<212>  DNA
<213>  Artificial Sequence

<220>
<223>  oligonucleotide

<400>  697
ttgacagtcc accaacttac                                                  20


<210>  698
<211>  21
<212>  DNA

<213>  Artificial Sequence

<220>
<223>  oligonucleotide

<400>  698
ggaggaaggg ctctagtata a                                                21


<210>  699
<211>  26
<212>  DNA
<213>  Artificial Sequence

<220>
<223>  oligonucleotide

<400>  699
aagctgggaa gccttctcta acctct                                           26


<210>  700
<211>  22
<212>  DNA
<213>  Artificial Sequence

<220>
<223>  oligonucleotide

<400>  700
gtgaataaca ttcccttcct tc                                               22


<210>  701
<211>  21
<212>  DNA
<213>  Artificial Sequence

<220>
<223>  oligonucleotide

<400>  701
tagccatagt cagcattgta a                                                21


<210>  702
<211>  29
<212>  DNA
<213>  Artificial Sequence

<220>
<223>  oligonucleotide

<400>  702
ccagatcaca tttgattgac agtccacca                                        29


<210>  703
<211>  20
<212>  DNA
<213>  Artificial Sequence

<220>

<223>  oligonucleotide

<400>  703
tgtgttgaca tccagatcac                                                    20


<210>  704
<211>  20
<212>  DNA
<213>  Artificial Sequence

<220>
<223>  oligonucleotide

<400>  704
taggagaggt tagagaaggc                                                    20


<210>  705
<211>  28
<212>  DNA
<213>  Artificial Sequence

<220>
<223>  oligonucleotide

<400>  705
ccaccaactt acaatgctga ctatggct                                           28


<210>  706
<211>  22
<212>  DNA
<213>  Artificial Sequence

<220>
<223>  oligonucleotide

<400>  706
ccaacttaca atgctgacta tg                                                 22


<210>  707
<211>  19
<212>  DNA
<213>  Artificial Sequence

<220>
<223>  oligonucleotide

<400>  707
caatcatcag gcacaggag                                                     19


<210>  708
<211>  26
<212>  DNA
<213>  Artificial Sequence

<220>
<223>  oligonucleotide

<400>  708

aagctgggaa gccttctcta acctct                                          26

<210>  709
<211>  18
<212>  DNA
<213>  Artificial Sequence

<220>
<223>  oligonucleotide

<400>  709
tgtctctgtg cccatgat                                                   18

<210>  710
<211>  20
<212>  DNA
<213>  Artificial Sequence

<220>
<223>  oligonucleotide

<400>  710
cagttccagg atgtcgtagt                                                 20

<210>  711
<211>  22
<212>  DNA
<213>  Artificial Sequence

<220>
<223>  oligonucleotide

<400>  711
actgagttca ccacgcccga tg                                              22

<210>  712
<211>  19
<212>  DNA
<213>  Artificial Sequence

<220>
<223>  oligonucleotide

<400>  712
cgcctcttcc acaaatcag                                                  19

<210>  713
<211>  21
<212>  DNA
<213>  Artificial Sequence

<220>
<223>  oligonucleotide

<400>  713
tccaggatgt cgtagtaatg g                                               21

<210>  714
<211>  22
<212>  DNA
<213>  Artificial Sequence

<220>
<223>  oligonucleotide

<400>  714
atgggcacag agacagtgct gc                                                22


<210>  715
<211>  18
<212>  DNA
<213>  Artificial Sequence

<220>
<223>  oligonucleotide

<400>  715
tggctcagac caacaagt                                                     18


<210>  716
<211>  20
<212>  DNA
<213>  Artificial Sequence

<220>
<223>  oligonucleotide

<400>  716
cagcaatgaa catgctgagg                                                   20


<210>  717
<211>  24
<212>  DNA
<213>  Artificial Sequence

<220>
<223>  oligonucleotide

<400>  717
actacgacat cctggaactg ccct                                              24


<210>  718
<211>  20
<212>  DNA
<213>  Artificial Sequence

<220>
<223>  oligonucleotide

<400>  718
ccacaaatca gacggcagca                                                   20


<210>  719
<211>  20
<212>  DNA

<213>  Artificial Sequence

<220>
<223>  oligonucleotide

<400>  719
gtcgtagtaa tggccatcgg                                                    20


<210>  720
<211>  25
<212>  DNA
<213>  Artificial Sequence

<220>
<223>  oligonucleotide

<400>  720
cccatgatgg ctcagaccaa caagt                                              25


<210>  721
<211>  19
<212>  DNA
<213>  Artificial Sequence

<220>
<223>  oligonucleotide

<400>  721
ccagagagac accagagaa                                                     19


<210>  722
<211>  19
<212>  DNA
<213>  Artificial Sequence

<220>
<223>  oligonucleotide

<400>  722
gaggtcggaa ttgcagaag                                                     19


<210>  723
<211>  24
<212>  DNA
<213>  Artificial Sequence

<220>
<223>  oligonucleotide

<400>  723
tctggcatcc tgttgttgct gtgg                                               24


<210>  724
<211>  20
<212>  DNA
<213>  Artificial Sequence

<220>

<223>  oligonucleotide

<400>  724
cccagagaga caccagagaa                                                   20


<210>  725
<211>  19
<212>  DNA
<213>  Artificial Sequence

<220>
<223>  oligonucleotide

<400>  725
gctatcagcc acagcaaca                                                    19


<210>  726
<211>  20
<212>  DNA
<213>  Artificial Sequence

<220>
<223>  oligonucleotide

<400>  726
cctggcttct ggcatcctgt                                                   20


<210>  727
<211>  19
<212>  DNA
<213>  Artificial Sequence

<220>
<223>  oligonucleotide

<400>  727
agagacacca gagaaccca                                                    19


<210>  728
<211>  17
<212>  DNA
<213>  Artificial Sequence

<220>
<223>  oligonucleotide

<400>  728
gtgggacaca ggtgcag                                                      17


<210>  729
<211>  24
<212>  DNA
<213>  Artificial Sequence

<220>
<223>  oligonucleotide

<400>  729

tctggcatcc tgttgttgct gtgg                                              24

<210>  730
<211>  17
<212>  DNA
<213>  Artificial Sequence

<220>
<223>  oligonucleotide

<400>  730
catcgccgca gatccag                                                     17

<210>  731
<211>  18
<212>  DNA
<213>  Artificial Sequence

<220>
<223>  oligonucleotide

<400>  731
tcagccacag caacaaca                                                    18

<210>  732
<211>  24
<212>  DNA
<213>  Artificial Sequence

<220>
<223>  oligonucleotide

<400>  732
cccagagaga caccagagaa ccca                                             24

<210>  733
<211>  22
<212>  DNA
<213>  Artificial Sequence

<220>
<223>  oligonucleotide

<400>  733
aaactcatgt tcaagacaga ag                                               22

<210>  734
<211>  19
<212>  DNA
<213>  Artificial Sequence

<220>
<223>  oligonucleotide

<400>  734
caagcaaggg ttcaaagac                                                   19

<210>  735
<211>  26
<212>  DNA
<213>  Artificial Sequence

<220>
<223>  oligonucleotide

<400>  735
tggagaatgt cagtctgagt caggcc                                            26


<210>  736
<211>  19
<212>  DNA
<213>  Artificial Sequence

<220>
<223>  oligonucleotide

<400>  736
gtctacctcc cgccataaa                                                    19


<210>  737
<211>  21
<212>  DNA
<213>  Artificial Sequence

<220>
<223>  oligonucleotide

<400>  737
gggaacaaga agtggagaat g                                                 21


<210>  738
<211>  26
<212>  DNA
<213>  Artificial Sequence

<220>
<223>  oligonucleotide

<400>  738
tgttcaagac agaagggcct gactca                                            26


<210>  739
<211>  19
<212>  DNA
<213>  Artificial Sequence

<220>
<223>  oligonucleotide

<400>  739
caagacagaa gggcctgac                                                    19


<210>  740
<211>  20
<212>  DNA

<213>  Artificial Sequence

<220>
<223>  oligonucleotide

<400>  740
cacacctatt gcaagcaagg                                                   20


<210>  741
<211>  30
<212>  DNA
<213>  Artificial Sequence

<220>
<223>  oligonucleotide

<400>  741
tcagactgac attctccact tcttgttccc                                        30


<210>  742
<211>  19
<212>  DNA
<213>  Artificial Sequence

<220>
<223>  oligonucleotide

<400>  742
cagccacctg aagtccaaa                                                    19


<210>  743
<211>  22
<212>  DNA
<213>  Artificial Sequence

<220>
<223>  oligonucleotide

<400>  743
gtggagaatg tcagtctgag tc                                                22


<210>  744
<211>  24
<212>  DNA
<213>  Artificial Sequence

<220>
<223>  oligonucleotide

<400>  744
ttatggcggg aggtagactg accc                                              24


<210>  745
<211>  21
<212>  DNA
<213>  Artificial Sequence

<220>

<223>  oligonucleotide

<400>  745
gccagaagca caagctattt c                                                21


<210>  746
<211>  19
<212>  DNA
<213>  Artificial Sequence

<220>
<223>  oligonucleotide

<400>  746
tgctttgcac atgctgttt                                                   19


<210>  747
<211>  22
<212>  DNA
<213>  Artificial Sequence

<220>
<223>  oligonucleotide

<400>  747
accagaccat gtgcaagtgg ca                                               22


<210>  748
<211>  20
<212>  DNA
<213>  Artificial Sequence

<220>
<223>  oligonucleotide

<400>  748
cctttcatac cagaccatgt                                                  20


<210>  749
<211>  21
<212>  DNA
<213>  Artificial Sequence

<220>
<223>  oligonucleotide

<400>  749
cgctgaatca aggattcttt c                                                21


<210>  750
<211>  22
<212>  DNA
<213>  Artificial Sequence

<220>
<223>  oligonucleotide

<400>  750

tgcaaagcat tgccgctttc ca                                              22

<210>  751
<211>  20
<212>  DNA
<213>  Artificial Sequence

<220>
<223>  oligonucleotide

<400>  751
ctatttcacc cagacacctg                                                 20

<210>  752
<211>  19
<212>  DNA
<213>  Artificial Sequence

<220>
<223>  oligonucleotide

<400>  752
aaggattctt tcggctgtg                                                  19

<210>  753
<211>  22
<212>  DNA
<213>  Artificial Sequence

<220>
<223>  oligonucleotide

<400>  753
accagaccat gtgcaagtgg ca                                              22

<210>  754
<211>  21
<212>  DNA
<213>  Artificial Sequence

<220>
<223>  oligonucleotide

<400>  754
tttgagtgca aagaaagtct g                                               21

<210>  755
<211>  20
<212>  DNA
<213>  Artificial Sequence

<220>
<223>  oligonucleotide

<400>  755
acatggtctg gtatgaaagg                                                 20

<210>  756
<211>  22
<212>  DNA
<213>  Artificial Sequence

<220>
<223>  oligonucleotide

<400>  756
agacctgctg ccagaagcac aa                                                22


<210>  757
<211>  19
<212>  DNA
<213>  Artificial Sequence

<220>
<223>  oligonucleotide

<400>  757
gacaggatgt tgacaatgc                                                    19


<210>  758
<211>  19
<212>  DNA
<213>  Artificial Sequence

<220>
<223>  oligonucleotide

<400>  758
cctcttcgtg gagtttctt                                                    19


<210>  759
<211>  23
<212>  DNA
<213>  Artificial Sequence

<220>
<223>  oligonucleotide

<400>  759
tgcgttcaag gtcaagacgt gcc                                               23


<210>  760
<211>  22
<212>  DNA
<213>  Artificial Sequence

<220>
<223>  oligonucleotide

<400>  760
caatctttca gacaggatgt tg                                                22


<210>  761
<211>  19
<212>  DNA

<213>  Artificial Sequence

<220>
<223>  oligonucleotide

<400>  761
gctcctggat ttcctcttc                                                    19


<210>  762
<211>  23
<212>  DNA
<213>  Artificial Sequence

<220>
<223>  oligonucleotide

<400>  762
acaatgcgtc tctggcacgt ctt                                               23


<210>  763
<211>  19
<212>  DNA
<213>  Artificial Sequence

<220>
<223>  oligonucleotide

<400>  763
cctccgggag aaattgcag                                                    19


<210>  764
<211>  19
<212>  DNA
<213>  Artificial Sequence

<220>
<223>  oligonucleotide

<400>  764
gtcaagacgt gccagagac                                                    19


<210>  765
<211>  26
<212>  DNA
<213>  Artificial Sequence

<220>
<223>  oligonucleotide

<400>  765
agatgcttca gagagaggaa gccgaa                                            26


<210>  766
<211>  19
<212>  DNA
<213>  Artificial Sequence

<220>

<223>  oligonucleotide

<400>  766
ggaggagatg cttcagaga                                                    19


<210>  767
<211>  19
<212>  DNA
<213>  Artificial Sequence

<220>
<223>  oligonucleotide

<400>  767
attccacttt gcgttcaag                                                    19


<210>  768
<211>  27
<212>  DNA
<213>  Artificial Sequence

<220>
<223>  oligonucleotide

<400>  768
tctttcagac aggatgttga caatgcg                                           27


<210>  769
<211>  22
<212>  DNA
<213>  Artificial Sequence

<220>
<223>  oligonucleotide

<400>  769
tgtaacacct acaccattat ct                                                22


<210>  770
<211>  21
<212>  DNA
<213>  Artificial Sequence

<220>
<223>  oligonucleotide

<400>  770
tcgcaatgaa acaatcagta g                                                 21


<210>  771
<211>  24
<212>  DNA
<213>  Artificial Sequence

<220>
<223>  oligonucleotide

<400>  771

accattacag ggagctggga cact                                              24

<210>  772
<211>  20
<212>  DNA
<213>  Artificial Sequence

<220>
<223>  oligonucleotide

<400>  772
actccagacc agtttatgac                                                    20

<210>  773
<211>  19
<212>  DNA
<213>  Artificial Sequence

<220>
<223>  oligonucleotide

<400>  773
ccaacggttg tttctttcc                                                     19

<210>  774
<211>  24
<212>  DNA
<213>  Artificial Sequence

<220>
<223>  oligonucleotide

<400>  774
agctgatgag acaaggaacc tgca                                               24

<210>  775
<211>  19
<212>  DNA
<213>  Artificial Sequence

<220>
<223>  oligonucleotide

<400>  775
gcatttggtg aacaaggag                                                     19

<210>  776
<211>  21
<212>  DNA
<213>  Artificial Sequence

<220>
<223>  oligonucleotide

<400>  776
ccaatcttca tgtccacatt c                                                  21

<210>  777
<211>  24
<212>  DNA
<213>  Artificial Sequence

<220>
<223>  oligonucleotide

<400>  777
agctgatgag acaaggaacc tgca                                              24


<210>  778
<211>  19
<212>  DNA
<213>  Artificial Sequence

<220>
<223>  oligonucleotide

<400>  778
tgacagctga tgagacaag                                                    19


<210>  779
<211>  20
<212>  DNA
<213>  Artificial Sequence

<220>
<223>  oligonucleotide

<400>  779
agctccctgt aatggttatg                                                   20


<210>  780
<211>  28
<212>  DNA
<213>  Artificial Sequence

<220>
<223>  oligonucleotide

<400>  780
cctgcagaat gtggacatga agattggg                                          28


<210>  781
<211>  22
<212>  DNA
<213>  Artificial Sequence

<220>
<223>  oligonucleotide

<400>  781
tcactgagtt catggaaaac tg                                                22


<210>  782
<211>  21
<212>  DNA

<213>  Artificial Sequence

<220>
<223>  oligonucleotide

<400>  782
gttcatctcg gacaggtact t                                                21


<210>  783
<211>  23
<212>  DNA
<213>  Artificial Sequence

<220>
<223>  oligonucleotide

<400>  783
ccttcctccg gctcaacgat ggg                                              23


<210>  784
<211>  19
<212>  DNA
<213>  Artificial Sequence

<220>
<223>  oligonucleotide

<400>  784
ctgttcgcca ctatgagaa                                                   19


<210>  785
<211>  20
<212>  DNA
<213>  Artificial Sequence

<220>
<223>  oligonucleotide

<400>  785
ggatagcact gagacttgag                                                  20


<210>  786
<211>  24
<212>  DNA
<213>  Artificial Sequence

<220>
<223>  oligonucleotide

<400>  786
aaactgaaga gctcttccaa gccc                                             24


<210>  787
<211>  22
<212>  DNA
<213>  Artificial Sequence

<220>

<223>  oligonucleotide

<400>  787
tgtaagagaa attaaggatg gc                                                22


<210>  788
<211>  20
<212>  DNA
<213>  Artificial Sequence

<220>
<223>  oligonucleotide

<400>  788
ctgagacttg aggaaacaga                                                   20


<210>  789
<211>  23
<212>  DNA
<213>  Artificial Sequence

<220>
<223>  oligonucleotide

<400>  789
tgatgtggtt gctgttcgcc act                                               23


<210>  790
<211>  21
<212>  DNA
<213>  Artificial Sequence

<220>
<223>  oligonucleotide

<400>  790
tttctgtttc ctcaagtctc a                                                 21


<210>  791
<211>  22
<212>  DNA
<213>  Artificial Sequence

<220>
<223>  oligonucleotide

<400>  791
acaccaagga taaccttcta at                                                22


<210>  792
<211>  29
<212>  DNA
<213>  Artificial Sequence

<220>
<223>  oligonucleotide

<400>  792

tgatcagcca tgttgtaata ggatagcac                                          29

<210>  793
<211>  20
<212>  DNA
<213>  Artificial Sequence

<220>
<223>  oligonucleotide

<400>  793
gccactatga gaaggcataa                                                   20

<210>  794
<211>  19
<212>  DNA
<213>  Artificial Sequence

<220>
<223>  oligonucleotide

<400>  794
cctccacacc aaggataac                                                    19

<210>  795
<211>  29
<212>  DNA
<213>  Artificial Sequence

<220>
<223>  oligonucleotide

<400>  795
tctgtttcct caagtctcag tgctatcct                                         29

<210>  796
<211>  19
<212>  DNA
<213>  Artificial Sequence

<220>
<223>  oligonucleotide

<400>  796
gtctacctct ctccttggt                                                    19

<210>  797
<211>  21
<212>  DNA
<213>  Artificial Sequence

<220>
<223>  oligonucleotide

<400>  797
caagtgctga gggtttatta t                                                 21

<210>  798
<211>  24
<212>  DNA
<213>  Artificial Sequence

<220>
<223>  oligonucleotide

<400>  798
tggaacagcg ctactgatca ccaa                                              24


<210>  799
<211>  16
<212>  DNA
<213>  Artificial Sequence

<220>
<223>  oligonucleotide

<400>  799
agagctcccg ccactc                                                       16


<210>  800
<211>  20
<212>  DNA
<213>  Artificial Sequence

<220>
<223>  oligonucleotide

<400>  800
aggtgtctca cggtcatctc                                                   20


<210>  801
<211>  24
<212>  DNA
<213>  Artificial Sequence

<220>
<223>  oligonucleotide

<400>  801
aggatgtccg ccttctccag cttc                                              24


<210>  802
<211>  19
<212>  DNA
<213>  Artificial Sequence

<220>
<223>  oligonucleotide

<400>  802
tgggtgcctc cactatgat                                                    19


<210>  803
<211>  20
<212>  DNA

<213>  Artificial Sequence

<220>
<223>  oligonucleotide

<400>  803
cttccacgtg actgagagtt                                                    20


<210>  804
<211>  22
<212>  DNA
<213>  Artificial Sequence

<220>
<223>  oligonucleotide

<400>  804
tctgtgtggg tggatgcgtg tg                                                 22


<210>  805
<211>  21
<212>  DNA
<213>  Artificial Sequence

<220>
<223>  oligonucleotide

<400>  805
gcctccacta tgatccttaa a                                                  21


<210>  806
<211>  21
<212>  DNA
<213>  Artificial Sequence

<220>
<223>  oligonucleotide

<400>  806
cgtgactgag agttcaattt c                                                  21


<210>  807
<211>  24
<212>  DNA
<213>  Artificial Sequence

<220>
<223>  oligonucleotide

<400>  807
atgcgtgtgg gcacgacttt gtac                                               24


<210>  808
<211>  19
<212>  DNA
<213>  Artificial Sequence

<220>

<223>  oligonucleotide

<400>  808
tttgagaagc agggatctg                                                    19


<210>  809
<211>  21
<212>  DNA
<213>  Artificial Sequence

<220>
<223>  oligonucleotide

<400>  809
ccaaactccg atagtccata g                                                 21


<210>  810
<211>  22
<212>  DNA
<213>  Artificial Sequence

<220>
<223>  oligonucleotide

<400>  810
ttactttgac gcgcacgccc tt                                                22


<210>  811
<211>  19
<212>  DNA
<213>  Artificial Sequence

<220>
<223>  oligonucleotide

<400>  811
gaccgtggat cacctgaaa                                                    19


<210>  812
<211>  21
<212>  DNA
<213>  Artificial Sequence

<220>
<223>  oligonucleotide

<400>  812
ccaaactccg atagtccata g                                                 21


<210>  813
<211>  22
<212>  DNA
<213>  Artificial Sequence

<220>
<223>  oligonucleotide

<400>  813

ttactttgac gcgcacgccc tt                                                22

<210>  814
<211>  19
<212>  DNA
<213>  Artificial Sequence

<220>
<223>  oligonucleotide

<400>  814
gcccttgcta tggactatc                                                    19

<210>  815
<211>  21
<212>  DNA
<213>  Artificial Sequence

<220>
<223>  oligonucleotide

<400>  815
tctagtcctt caatgatgct c                                                 21

<210>  816
<211>  24
<212>  DNA
<213>  Artificial Sequence

<220>
<223>  oligonucleotide

<400>  816
cctggcagaa gttgcgcgtt atct                                              24

<210>  817
<211>  19
<212>  DNA
<213>  Artificial Sequence

<220>
<223>  oligonucleotide

<400>  817
caggagggaa aggttactt                                                    19

<210>  818
<211>  22
<212>  DNA
<213>  Artificial Sequence

<220>
<223>  oligonucleotide

<400>  818
ccttcaatga tgctcagata ac                                                22

<210>  819
<211>  24
<212>  DNA
<213>  Artificial Sequence

<220>
<223>  oligonucleotide

<400>  819
acgcccttgc tatggactat cgga                                              24


<210>  820
<211>  22
<212>  DNA
<213>  Artificial Sequence

<220>
<223>  oligonucleotide

<400>  820
atgacagtgg atcatttgaa ga                                                22


<210>  821
<211>  20
<212>  DNA
<213>  Artificial Sequence

<220>
<223>  oligonucleotide

<400>  821
cggaatccta tgctcatgaa                                                   20


<210>  822
<211>  24
<212>  DNA
<213>  Artificial Sequence

<220>
<223>  oligonucleotide

<400>  822
actttgacgc acacgctctt gcca                                              24


<210>  823
<211>  19
<212>  DNA
<213>  Artificial Sequence

<220>
<223>  oligonucleotide

<400>  823
aagatgcttc aggcaacag                                                    19


<210>  824
<211>  21
<212>  DNA

<213>  Artificial Sequence

<220>
<223>  oligonucleotide

<400>  824
caacttctgt taggcactct c                                                21


<210>  825
<211>  23
<212>  DNA
<213>  Artificial Sequence

<220>
<223>  oligonucleotide

<400>  825
aaggctactt tgacgcacac gct                                              23


<210>  826
<211>  19
<212>  DNA
<213>  Artificial Sequence

<220>
<223>  oligonucleotide

<400>  826
gacttgtgcc aactgcttt                                                   19


<210>  827
<211>  20
<212>  DNA
<213>  Artificial Sequence

<220>
<223>  oligonucleotide

<400>  827
gcgtcaaagt agcctttacc                                                  20


<210>  828
<211>  28
<212>  DNA
<213>  Artificial Sequence

<220>
<223>  oligonucleotide

<400>  828
tcatttgaag atgcttcagg caacaggg                                         28


<210>  829
<211>  21
<212>  DNA
<213>  Artificial Sequence

<220>

<223>  oligonucleotide

<400>  829
acaaggatct gcaaagttag a                                                21


<210>  830
<211>  19
<212>  DNA
<213>  Artificial Sequence

<220>
<223>  oligonucleotide

<400>  830
atgaagtcca tggcaagag                                                   19


<210>  831
<211>  26
<212>  DNA
<213>  Artificial Sequence

<220>
<223>  oligonucleotide

<400>  831
cgtgtgcgtc aaagtagcct ttaccc                                           26


<210>  832
<211>  18
<212>  DNA
<213>  Artificial Sequence

<220>
<223>  oligonucleotide

<400>  832
tgcttagacg ctggattt                                                    18


<210>  833
<211>  19
<212>  DNA
<213>  Artificial Sequence

<220>
<223>  oligonucleotide

<400>  833
tcgtagtcga ggtcatagt                                                   19


<210>  834
<211>  26
<212>  DNA
<213>  Artificial Sequence

<220>
<223>  oligonucleotide

<400>  834

ccctcaacgt tagcttcacc aacagg                                          26

<210>  835
<211>  18
<212>  DNA
<213>  Artificial Sequence

<220>
<223>  oligonucleotide

<400>  835
tctctgaaag gctctcct                                                    18

<210>  836
<211>  19
<212>  DNA
<213>  Artificial Sequence

<220>
<223>  oligonucleotide

<400>  836
tcctgttggt gaagctaac                                                   19

<210>  837
<211>  24
<212>  DNA
<213>  Artificial Sequence

<220>
<223>  oligonucleotide

<400>  837
tgcagctgct tagacgctgg attt                                             24

<210>  838
<211>  17
<212>  DNA
<213>  Artificial Sequence

<220>
<223>  oligonucleotide

<400>  838
gacccgcttc tctgaaa                                                     17

<210>  839
<211>  19
<212>  DNA
<213>  Artificial Sequence

<220>
<223>  oligonucleotide

<400>  839
aggtcatagt tcctgttgg                                                   19

<210>  840
<211>  24
<212>  DNA
<213>  Artificial Sequence

<220>
<223>  oligonucleotide

<400>  840
tgcagctgct tagacgctgg attt                                              24


<210>  841
<211>  19
<212>  DNA
<213>  Artificial Sequence

<220>
<223>  oligonucleotide

<400>  841
ttcgggtagt ggaaaacca                                                    19


<210>  842
<211>  21
<212>  DNA
<213>  Artificial Sequence

<220>
<223>  oligonucleotide

<400>  842
catagttcct gttggtgaag c                                                 21


<210>  843
<211>  22
<212>  DNA
<213>  Artificial Sequence

<220>
<223>  oligonucleotide

<400>  843
ctcccgcgac gatgcccctc aa                                                22


<210>  844
<211>  19
<212>  DNA
<213>  Artificial Sequence

<220>
<223>  oligonucleotide

<400>  844
ctgttcgcca ctatgagaa                                                    19


<210>  845
<211>  20
<212>  DNA

<213>  Artificial Sequence

<220>
<223>  oligonucleotide

<400>  845
ggatagcact gagacttgag                                                   20


<210>  846
<211>  24
<212>  DNA
<213>  Artificial Sequence

<220>
<223>  oligonucleotide

<400>  846
aaactgaaga gctcttccaa gccc                                              24


<210>  847
<211>  22
<212>  DNA
<213>  Artificial Sequence

<220>
<223>  oligonucleotide

<400>  847
tgtaagagaa attaaggatg gc                                                22


<210>  848
<211>  20
<212>  DNA
<213>  Artificial Sequence

<220>
<223>  oligonucleotide

<400>  848
ctgagacttg aggaaacaga                                                   20


<210>  849
<211>  23
<212>  DNA
<213>  Artificial Sequence

<220>
<223>  oligonucleotide

<400>  849
tgatgtggtt gctgttcgcc act                                               23


<210>  850
<211>  21
<212>  DNA
<213>  Artificial Sequence

<220>

<223>  oligonucleotide

<400>  850
tttctgtttc ctcaagtctc a                                                21


<210>  851
<211>  22
<212>  DNA
<213>  Artificial Sequence

<220>
<223>  oligonucleotide

<400>  851
acaccaagga taaccttcta at                                               22


<210>  852
<211>  29
<212>  DNA
<213>  Artificial Sequence

<220>
<223>  oligonucleotide

<400>  852
tgatcagcca tgttgtaata ggatagcac                                        29


<210>  853
<211>  20
<212>  DNA
<213>  Artificial Sequence

<220>
<223>  oligonucleotide

<400>  853
gccactatga gaaggcataa                                                  20


<210>  854
<211>  19
<212>  DNA
<213>  Artificial Sequence

<220>
<223>  oligonucleotide

<400>  854
cctccacacc aaggataac                                                   19


<210>  855
<211>  29
<212>  DNA
<213>  Artificial Sequence

<220>
<223>  oligonucleotide

<400>  855

tctgtttcct caagtctcag tgctatcct 29

<210> 856
<211> 22
<212> DNA
<213> Artificial Sequence

<220>
<223> oligonucleotide

<400> 856
gttgctggtg ttctaaacta tt 22

<210> 857
<211> 19
<212> DNA
<213> Artificial Sequence

<220>
<223> oligonucleotide

<400> 857
cccataacca cattgacca 19

<210> 858
<211> 22
<212> DNA
<213> Artificial Sequence

<220>
<223> oligonucleotide

<400> 858
tttgtgggtg gagtttgtgc gc 22

<210> 859
<211> 19
<212> DNA
<213> Artificial Sequence

<220>
<223> oligonucleotide

<400> 859
tgtgtgtaca tttgtgggt 19

<210> 860
<211> 22
<212> DNA
<213> Artificial Sequence

<220>
<223> oligonucleotide

<400> 860
caagaaatgg tagactcaag tt 22

<210>  861
<211>  25
<212>  DNA
<213>  Artificial Sequence

<220>
<223>  oligonucleotide

<400>  861
actgcgtggt caatgtggtt atggg                                          25


<210>  862
<211>  19
<212>  DNA
<213>  Artificial Sequence

<220>
<223>  oligonucleotide

<400>  862
catttgtggg tggagtttg                                                 19


<210>  863
<211>  20
<212>  DNA
<213>  Artificial Sequence

<220>
<223>  oligonucleotide

<400>  863
gcaaccaaga aatggtagac                                                20


<210>  864
<211>  25
<212>  DNA
<213>  Artificial Sequence

<220>
<223>  oligonucleotide

<400>  864
actgcgtggt caatgtggtt atggg                                          25


<210>  865
<211>  19
<212>  DNA
<213>  Artificial Sequence

<220>
<223>  oligonucleotide

<400>  865
cgctgttgct ggtgttcta                                                 19


<210>  866
<211>  19
<212>  DNA

<213>  Artificial Sequence

<220>
<223>  oligonucleotide

<400>  866
cattgaccac gcagtgttt                                                    19


<210>  867
<211>  22
<212>  DNA
<213>  Artificial Sequence

<220>
<223>  oligonucleotide

<400>  867
tttgtgggtg gagtttgtgc gc                                                22


<210>  868
<211>  18
<212>  DNA
<213>  Artificial Sequence

<220>
<223>  oligonucleotide

<400>  868
acagttcagc gactgcag                                                     18


<210>  869
<211>  19
<212>  DNA
<213>  Artificial Sequence

<220>
<223>  oligonucleotide

<400>  869
aacgaggcgt cttcaaatg                                                    19


<210>  870
<211>  24
<212>  DNA
<213>  Artificial Sequence

<220>
<223>  oligonucleotide

<400>  870
tcagcagagg tcagatgcag aagc                                              24


<210>  871
<211>  19
<212>  DNA
<213>  Artificial Sequence

<220>

<223>  oligonucleotide

<400>  871
gctgatcaac ggctacatc                                                    19


<210>  872
<211>  19
<212>  DNA
<213>  Artificial Sequence

<220>
<223>  oligonucleotide

<400>  872
gcgtcttcaa atggcttct                                                    19


<210>  873
<211>  22
<212>  DNA
<213>  Artificial Sequence

<220>
<223>  oligonucleotide

<400>  873
tgcagtcgct gaactgtgag gc                                                22


<210>  874
<211>  20
<212>  DNA
<213>  Artificial Sequence

<220>
<223>  oligonucleotide

<400>  874
gcagaggtca gatgcagaag                                                   20


<210>  875
<211>  19
<212>  DNA
<213>  Artificial Sequence

<220>
<223>  oligonucleotide

<400>  875
gcggaggatg atgtggatg                                                    19


<210>  876
<211>  22
<212>  DNA
<213>  Artificial Sequence

<220>
<223>  oligonucleotide

<400>  876

ttgaagacgc ctcgtttgcg ct 22

<210> 877
<211> 19
<212> DNA
<213> Artificial Sequence

<220>
<223> oligonucleotide

<400> 877
agaggaggac tttgagtct 19

<210> 878
<211> 19
<212> DNA
<213> Artificial Sequence

<220>
<223> oligonucleotide

<400> 878
caaatggctt ctgcatctg 19

<210> 879
<211> 22
<212> DNA
<213> Artificial Sequence

<220>
<223> oligonucleotide

<400> 879
tgcagtcgct gaactgtgag gc 22

<210> 880
<211> 19
<212> DNA
<213> Artificial Sequence

<220>
<223> oligonucleotide

<400> 880
cctgttcgtc ttctgtacc 19

<210> 881
<211> 20
<212> DNA
<213> Artificial Sequence

<220>
<223> oligonucleotide

<400> 881
gattccatct cctccatctg 20

<210>  882
<211>  22
<212>  DNA
<213>  Artificial Sequence

<220>
<223>  oligonucleotide

<400>  882
acgtgctgag cgttactggc ag                                                22


<210>  883
<211>  19
<212>  DNA
<213>  Artificial Sequence

<220>
<223>  oligonucleotide

<400>  883
catcgtgtcc atcgtcatc                                                    19


<210>  884
<211>  18
<212>  DNA
<213>  Artificial Sequence

<220>
<223>  oligonucleotide

<400>  884
agtaacgctc agcacgtc                                                     18


<210>  885
<211>  24
<212>  DNA
<213>  Artificial Sequence

<220>
<223>  oligonucleotide

<400>  885
tggtggccct gttcgtcttc tgta                                              24


<210>  886
<211>  19
<212>  DNA
<213>  Artificial Sequence

<220>
<223>  oligonucleotide

<400>  886
catcgtgtcc atcgtcatc                                                    19


<210>  887
<211>  20
<212>  DNA

<213>  Artificial Sequence

<220>
<223>  oligonucleotide

<400>  887
ctcggatctg agattccatc                                                   20


<210>  888
<211>  22
<212>  DNA
<213>  Artificial Sequence

<220>
<223>  oligonucleotide

<400>  888
acgtgctgag cgttactggc ag                                                22


<210>  889
<211>  19
<212>  DNA
<213>  Artificial Sequence

<220>
<223>  oligonucleotide

<400>  889
cacaatccag gtagggaac                                                    19


<210>  890
<211>  20
<212>  DNA
<213>  Artificial Sequence

<220>
<223>  oligonucleotide

<400>  890
cttggtacag aagacgaaca                                                   20


<210>  891
<211>  22
<212>  DNA
<213>  Artificial Sequence

<220>
<223>  oligonucleotide

<400>  891
aaccagacca tcgccacact gc                                                22


<210>  892
<211>  19
<212>  DNA
<213>  Artificial Sequence

<220>

<223>  oligonucleotide

<400>  892
cctgttcgtc ttctgtacc                                                    19


<210>  893
<211>  20
<212>  DNA
<213>  Artificial Sequence

<220>
<223>  oligonucleotide

<400>  893
gattccatct cctccatctg                                                   20


<210>  894
<211>  22
<212>  DNA
<213>  Artificial Sequence

<220>
<223>  oligonucleotide

<400>  894
acgtgctgag cgttactggc ag                                                22


<210>  895
<211>  19
<212>  DNA
<213>  Artificial Sequence

<220>
<223>  oligonucleotide

<400>  895
cacaatccag gtagggaac                                                    19


<210>  896
<211>  20
<212>  DNA
<213>  Artificial Sequence

<220>
<223>  oligonucleotide

<400>  896
cttggtacag aagacgaaca                                                   20


<210>  897
<211>  22
<212>  DNA
<213>  Artificial Sequence

<220>
<223>  oligonucleotide

<400>  897

aaccagacca tcgccacact gc                                              22

<210>  898
<211>  17
<212>  DNA
<213>  Artificial Sequence

<220>
<223>  oligonucleotide

<400>  898
gaccagtacc cgcactt                                                   17

<210>  899
<211>  17
<212>  DNA
<213>  Artificial Sequence

<220>
<223>  oligonucleotide

<400>  899
cgcttctcgc tctcgtt                                                   17

<210>  900
<211>  22
<212>  DNA
<213>  Artificial Sequence

<220>
<223>  oligonucleotide

<400>  900
cgctgggcaa gctctggaga ct                                              22

<210>  901
<211>  19
<212>  DNA
<213>  Artificial Sequence

<220>
<223>  oligonucleotide

<400>  901
catcgtgtcc atcgtcatc                                                 19

<210>  902
<211>  20
<212>  DNA
<213>  Artificial Sequence

<220>
<223>  oligonucleotide

<400>  902
ctcggatctg agattccatc                                                20

<210>  903
<211>  22
<212>  DNA
<213>  Artificial Sequence

<220>
<223>  oligonucleotide

<400>  903
acgtgctgag cgttactggc ag                                                22


<210>  904
<211>  25
<212>  DNA
<213>  Artificial Sequence

<220>
<223>  oligonucleotide

<400>  904
ggtgaggatt tggataaatc tgatg                                             25


<210>  905
<211>  25
<212>  DNA
<213>  Artificial Sequence

<220>
<223>  oligonucleotide

<400>  905
acatgggccg aggcaagata agcaa                                             25


<210>  906
<211>  22
<212>  DNA
<213>  Artificial Sequence

<220>
<223>  oligonucleotide

<400>  906
tcaaccacaa ggtccaagaa ac                                                22


<210>  907
<211>  19
<212>  DNA
<213>  Artificial Sequence

<220>
<223>  oligonucleotide

<400>  907
gcagagattg gtgaggatt                                                    19


<210>  908
<211>  22
<212>  DNA

<213>  Artificial Sequence

<220>
<223>  oligonucleotide

<400>  908
tgggccgagg caagataagc aa                                                22


<210>  909
<211>  18
<212>  DNA
<213>  Artificial Sequence

<220>
<223>  oligonucleotide

<400>  909
tccaactcaa ccacaagg                                                     18


<210>  910
<211>  19
<212>  DNA
<213>  Artificial Sequence

<220>
<223>  oligonucleotide

<400>  910
gaggcaagat aagcaagga                                                    19


<210>  911
<211>  27
<212>  DNA
<213>  Artificial Sequence

<220>
<223>  oligonucleotide

<400>  911
tcttggacct tgtggttgag ttggaga                                           27


<210>  912
<211>  21
<212>  DNA
<213>  Artificial Sequence

<220>
<223>  oligonucleotide

<400>  912
gtggatgttc tttaggcatt t                                                 21


<210>  913
<211>  18
<212>  DNA
<213>  Artificial Sequence

<220>

<223>  oligonucleotide

<400>  913
atggcagaga ttggtgag                                                    18


<210>  914
<211>  22
<212>  DNA
<213>  Artificial Sequence

<220>
<223>  oligonucleotide

<400>  914
tgggccgagg caagataagc aa                                               22


<210>  915
<211>  18
<212>  DNA
<213>  Artificial Sequence

<220>
<223>  oligonucleotide

<400>  915
accacaaggt ccaagaaa                                                    18


<210>  916
<211>  20
<212>  DNA
<213>  Artificial Sequence

<220>
<223>  oligonucleotide

<400>  916
ggaccacttt ccctacttta                                                  20


<210>  917
<211>  22
<212>  DNA
<213>  Artificial Sequence

<220>
<223>  oligonucleotide

<400>  917
atccgccaca acctttccct gc                                               22


<210>  918
<211>  17
<212>  DNA
<213>  Artificial Sequence

<220>
<223>  oligonucleotide

<400>  918

gtctcccgga caaacat                                                    17

<210>  919
<211>  19
<212>  DNA
<213>  Artificial Sequence

<220>
<223>  oligonucleotide

<400>  919
tggattgagg accactttc                                                  19

<210>  920
<211>  22
<212>  DNA
<213>  Artificial Sequence

<220>
<223>  oligonucleotide

<400>  920
atccgccaca acctttccct gc                                              22

<210>  921
<211>  18
<212>  DNA
<213>  Artificial Sequence

<220>
<223>  oligonucleotide

<400>  921
gaaggagacc ttgccatt                                                   18

<210>  922
<211>  18
<212>  DNA
<213>  Artificial Sequence

<220>
<223>  oligonucleotide

<400>  922
caacagcact gagaggaa                                                   18

<210>  923
<211>  22
<212>  DNA
<213>  Artificial Sequence

<220>
<223>  oligonucleotide

<400>  923
acattgccaa gccaggctgg aa                                              22

<210>  924
<211>  17
<212>  DNA
<213>  Artificial Sequence

<220>
<223>  oligonucleotide

<400>  924
atgtcgtgca gggaaag                                                    17


<210>  925
<211>  19
<212>  DNA
<213>  Artificial Sequence

<220>
<223>  oligonucleotide

<400>  925
gtgtgaactc ttcgtctca                                                  19


<210>  926
<211>  24
<212>  DNA
<213>  Artificial Sequence

<220>
<223>  oligonucleotide

<400>  926
acgagaggag gaacaggagt gaca                                            24


<210>  927
<211>  18
<212>  DNA
<213>  Artificial Sequence

<220>
<223>  oligonucleotide

<400>  927
ctgtccgtgc ttcatagt                                                   18


<210>  928
<211>  18
<212>  DNA
<213>  Artificial Sequence

<220>
<223>  oligonucleotide

<400>  928
aaggactcac cgtctttg                                                   18


<210>  929
<211>  24
<212>  DNA

<213>  Artificial Sequence

<220>
<223>  oligonucleotide

<400>  929
acgagaggag gaacaggagt gaca                                              24


<210>  930
<211>  20
<212>  DNA
<213>  Artificial Sequence

<220>
<223>  oligonucleotide

<400>  930
gtccgtgctt catagtcata                                                   20


<210>  931
<211>  19
<212>  DNA
<213>  Artificial Sequence

<220>
<223>  oligonucleotide

<400>  931
ctttggaggt gtgaactct                                                    19


<210>  932
<211>  24
<212>  DNA
<213>  Artificial Sequence

<220>
<223>  oligonucleotide

<400>  932
acgagaggag gaacaggagt gaca                                              24


<210>  933
<211>  18
<212>  DNA
<213>  Artificial Sequence

<220>
<223>  oligonucleotide

<400>  933
atcttctgtc cgtgcttc                                                     18


<210>  934
<211>  20
<212>  DNA
<213>  Artificial Sequence

<220>

<223>  oligonucleotide

<400>  934
cgtctttgga ggtgtgaact                                                   20


<210>  935
<211>  31
<212>  DNA
<213>  Artificial Sequence

<220>
<223>  oligonucleotide

<400>  935
cgagaggagg aacaggagtg acactctttg t                                      31


<210>  936
<211>  23
<212>  DNA
<213>  Artificial Sequence

<220>
<223>  oligonucleotide

<400>  936
tccgtgcttc atagtcataa agg                                               23


<210>  937
<211>  18
<212>  DNA
<213>  Artificial Sequence

<220>
<223>  oligonucleotide

<400>  937
ccttcaaagc ccagtaca                                                     18


<210>  938
<211>  22
<212>  DNA
<213>  Artificial Sequence

<220>
<223>  oligonucleotide

<400>  938
acgtttgaag ggtgccggaa gt                                                22


<210>  939
<211>  18
<212>  DNA
<213>  Artificial Sequence

<220>
<223>  oligonucleotide

<400>  939

tttcgaggcg tgagtatg                                                    18

<210>  940
<211>  21
<212>  DNA
<213>  Artificial Sequence

<220>
<223>  oligonucleotide

<400>  940
cagtacatgc tggtggttca c                                               21

<210>  941
<211>  24
<212>  DNA
<213>  Artificial Sequence

<220>
<223>  oligonucleotide

<400>  941
actggcgaga agccacacaa gtgc                                            24

<210>  942
<211>  21
<212>  DNA
<213>  Artificial Sequence

<220>
<223>  oligonucleotide

<400>  942
ttcgaggcgt gagtatgact t                                               21

<210>  943
<211>  18
<212>  DNA
<213>  Artificial Sequence

<220>
<223>  oligonucleotide

<400>  943
caaagcccag tacatgct                                                   18

<210>  944
<211>  22
<212>  DNA
<213>  Artificial Sequence

<220>
<223>  oligonucleotide

<400>  944
acgtttgaag ggtgccggaa gt                                              22

<210>  945
<211>  18
<212>  DNA
<213>  Artificial Sequence

<220>
<223>  oligonucleotide

<400>  945
gtacatgctg gtggttca                                                    18


<210>  946
<211>  23
<212>  DNA
<213>  Artificial Sequence

<220>
<223>  oligonucleotide

<400>  946
cacactggcg agaagccaca caa                                              23


<210>  947
<211>  19
<212>  DNA
<213>  Artificial Sequence

<220>
<223>  oligonucleotide

<400>  947
gaggcgtgag tatgacttc                                                   19


<210>  948
<211>  17
<212>  DNA
<213>  Artificial Sequence

<220>
<223>  oligonucleotide

<400>  948
ggtgcgtctt caggttt                                                     17


<210>  949
<211>  20
<212>  DNA
<213>  Artificial Sequence

<220>
<223>  oligonucleotide

<400>  949
gaggaccagc atctaactac                                                  20


<210>  950
<211>  26
<212>  DNA

<213>  Artificial Sequence

<220>
<223>  oligonucleotide

<400>  950
tcagcagaaa gcacaaacac aactgc                                          26


<210>  951
<211>  20
<212>  DNA
<213>  Artificial Sequence

<220>
<223>  oligonucleotide

<400>  951
actcacaacc tcctgaatac                                                 20


<210>  952
<211>  22
<212>  DNA
<213>  Artificial Sequence

<220>
<223>  oligonucleotide

<400>  952
cagcagaaag cacaaacaca ac                                              22


<210>  953
<211>  23
<212>  DNA
<213>  Artificial Sequence

<220>
<223>  oligonucleotide

<400>  953
attcaggagg ttgtgagtgg gct                                             23


<210>  954
<211>  19
<212>  DNA
<213>  Artificial Sequence

<220>
<223>  oligonucleotide

<400>  954
cactatgcag ggcaccaac                                                  19


<210>  955
<211>  19
<212>  DNA
<213>  Artificial Sequence

<220>

<223>  oligonucleotide

<400>  955
cttggaccag atggaagaa                                                    19


<210>  956
<211>  26
<212>  DNA
<213>  Artificial Sequence

<220>
<223>  oligonucleotide

<400>  956
tcagcagaaa gcacaaacac aactgc                                            26


<210>  957
<211>  17
<212>  DNA
<213>  Artificial Sequence

<220>
<223>  oligonucleotide

<400>  957
ccactcacaa cctcctg                                                      17


<210>  958
<211>  18
<212>  DNA
<213>  Artificial Sequence

<220>
<223>  oligonucleotide

<400>  958
tcagaggacc agcatcta                                                     18


<210>  959
<211>  29
<212>  DNA
<213>  Artificial Sequence

<220>
<223>  oligonucleotide

<400>  959
tggaagagat cagcagaaag cacaaacac                                         29


<210>  960
<211>  20
<212>  DNA
<213>  Artificial Sequence

<220>
<223>  oligonucleotide

<400>  960

tcctgaatac agaagcagtt                                                    20

<210>  961
<211>  17
<212>  DNA
<213>  Artificial Sequence

<220>
<223>  oligonucleotide

<400>  961
aaggccctca gtacctc                                                       17

<210>  962
<211>  24
<212>  DNA
<213>  Artificial Sequence

<220>
<223>  oligonucleotide

<400>  962
tgaatcgctc agggtgtcct ctcc                                               24

<210>  963
<211>  19
<212>  DNA
<213>  Artificial Sequence

<220>
<223>  oligonucleotide

<400>  963
gtgaccacgt cgatttctt                                                     19

<210>  964
<211>  17
<212>  DNA
<213>  Artificial Sequence

<220>
<223>  oligonucleotide

<400>  964
ccacaaggcc ctcagta                                                       17

<210>  965
<211>  24
<212>  DNA
<213>  Artificial Sequence

<220>
<223>  oligonucleotide

<400>  965
tgaatcgctc agggtgtcct ctcc                                               24

<210>  966
<211>  21
<212>  DNA
<213>  Artificial Sequence

<220>
<223>  oligonucleotide

<400>  966
cacgtcgatt tcttcctctt c                                                21


<210>  967
<211>  17
<212>  DNA
<213>  Artificial Sequence

<220>
<223>  oligonucleotide

<400>  967
gacaccctga gcgattc                                                     17


<210>  968
<211>  29
<212>  DNA
<213>  Artificial Sequence

<220>
<223>  oligonucleotide

<400>  968
tgaagatgat gaagaggaag atgaagagg                                        29


<210>  969
<211>  19
<212>  DNA
<213>  Artificial Sequence

<220>
<223>  oligonucleotide

<400>  969
gaatgtggtg acagccttg                                                   19


<210>  970
<211>  17
<212>  DNA
<213>  Artificial Sequence

<220>
<223>  oligonucleotide

<400>  970
gacaccctga gcgattc                                                     17


<210>  971
<211>  30
<212>  DNA

<213>  Artificial Sequence

<220>
<223>  oligonucleotide

<400>  971
agaggaagat gaagaggaag aaatcgacgt                                        30


<210>  972
<211>  17
<212>  DNA
<213>  Artificial Sequence

<220>
<223>  oligonucleotide

<400>  972
gcttctccac agtgacc                                                      17


<210>  973
<211>  17
<212>  DNA
<213>  Artificial Sequence

<220>
<223>  oligonucleotide

<400>  973
cccttctacg acagcag                                                      17


<210>  974
<211>  22
<212>  DNA
<213>  Artificial Sequence

<220>
<223>  oligonucleotide

<400>  974
agggccttca gtactccctg ca                                                22


<210>  975
<211>  17
<212>  DNA
<213>  Artificial Sequence

<220>
<223>  oligonucleotide

<400>  975
gggacttgga gcttgag                                                      17


<210>  976
<211>  17
<212>  DNA
<213>  Artificial Sequence

<220>

<223>  oligonucleotide

<400>  976
gtggcagatt ccaccca                                                      17


<210>  977
<211>  24
<212>  DNA
<213>  Artificial Sequence

<220>
<223>  oligonucleotide

<400>  977
tctgcccatg cctctccttc tgaa                                              24


<210>  978
<211>  19
<212>  DNA
<213>  Artificial Sequence

<220>
<223>  oligonucleotide

<400>  978
cgtagagttc agccctctc                                                    19


<210>  979
<211>  17
<212>  DNA
<213>  Artificial Sequence

<220>
<223>  oligonucleotide

<400>  979
agcgacaacc cgtacac                                                      17


<210>  980
<211>  22
<212>  DNA
<213>  Artificial Sequence

<220>
<223>  oligonucleotide

<400>  980
agggccttca gtactccctg ca                                                22


<210>  981
<211>  18
<212>  DNA
<213>  Artificial Sequence

<220>
<223>  oligonucleotide

<400>  981

cattgtccgg tgactcgt                                                    18

<210>  982
<211>  17
<212>  DNA
<213>  Artificial Sequence

<220>
<223>  oligonucleotide

<400>  982
tacgacagca gcgacaa                                                     17

<210>  983
<211>  22
<212>  DNA
<213>  Artificial Sequence

<220>
<223>  oligonucleotide

<400>  983
agggccttca gtactccctg ca                                               22

<210>  984
<211>  19
<212>  DNA
<213>  Artificial Sequence

<220>
<223>  oligonucleotide

<400>  984
ttggagcttg agtcctgag                                                   19

<210>  985
<211>  21
<212>  DNA
<213>  Artificial Sequence

<220>
<223>  oligonucleotide

<400>  985
cttcttcatg gccgcgttaa t                                                21

<210>  986
<211>  24
<212>  DNA
<213>  Artificial Sequence

<220>
<223>  oligonucleotide

<400>  986
tccaggcagc ggtagtagtg gtgt                                             24

<210>  987
<211>  21
<212>  DNA
<213>  Artificial Sequence

<220>
<223>  oligonucleotide

<400>  987
aatgagcaga accatggcaa a                                                21


<210>  988
<211>  17
<212>  DNA
<213>  Artificial Sequence

<220>
<223>  oligonucleotide

<400>  988
acgtccatca gcaatgt                                                     17


<210>  989
<211>  23
<212>  DNA
<213>  Artificial Sequence

<220>
<223>  oligonucleotide

<400>  989
ccgcgttaat cccaattccc gct                                              23


<210>  990
<211>  19
<212>  DNA
<213>  Artificial Sequence

<220>
<223>  oligonucleotide

<400>  990
ttgaacacca ctactaccg                                                   19


<210>  991
<211>  17
<212>  DNA
<213>  Artificial Sequence

<220>
<223>  oligonucleotide

<400>  991
ttcatggccg cgttaat                                                     17


<210>  992
<211>  22
<212>  DNA

<213>  Artificial Sequence

<220>
<223>  oligonucleotide

<400>  992
tccaggcagc ggtagtagtg gt                                                22


<210>  993
<211>  17
<212>  DNA
<213>  Artificial Sequence

<220>
<223>  oligonucleotide

<400>  993
agcagaacca tggcaaa                                                      17


<210>  994
<211>  18
<212>  DNA
<213>  Artificial Sequence

<220>
<223>  oligonucleotide

<400>  994
cgcgttaatc ccaattcc                                                     18


<210>  995
<211>  22
<212>  DNA
<213>  Artificial Sequence

<220>
<223>  oligonucleotide

<400>  995
tccaggcagc ggtagtagtg gt                                                22


<210>  996
<211>  19
<212>  DNA
<213>  Artificial Sequence

<220>
<223>  oligonucleotide

<400>  996
gtcctcgcgt cgatataaa                                                    19


<210>  997
<211>  20
<212>  DNA
<213>  Artificial Sequence

<220>

<223>  oligonucleotide

<400>  997
ctgctacaag tcaggagttc                                                    20


<210>  998
<211>  29
<212>  DNA
<213>  Artificial Sequence

<220>
<223>  oligonucleotide

<400>  998
tggaatgatt gagcggatga ttgagaagc                                          29


<210>  999
<211>  17
<212>  DNA
<213>  Artificial Sequence

<220>
<223>  oligonucleotide

<400>  999
gatcacgcac ggaaaca                                                       17


<210>  1000
<211>  20
<212>  DNA
<213>  Artificial Sequence

<220>
<223>  oligonucleotide

<400>  1000
aaatctgcta caagtcagga                                                    20


<210>  1001
<211>  23
<212>  DNA
<213>  Artificial Sequence

<220>
<223>  oligonucleotide

<400>  1001
aagctgtttc cgtgcgtgat cct                                                23


<210>  1002
<211>  17
<212>  DNA
<213>  Artificial Sequence

<220>
<223>  oligonucleotide

<400>  1002

tcccagaagc agtcgag                                                    17


<210>  1003
<211>  20
<212>  DNA
<213>  Artificial Sequence

<220>
<223>  oligonucleotide

<400>  1003
tgattgagcg gatgattgag                                                 20


<210>  1004
<211>  23
<212>  DNA
<213>  Artificial Sequence

<220>
<223>  oligonucleotide

<400>  1004
aagctgtttc cgtgcgtgat cct                                             23


<210>  1005
<211>  17
<212>  DNA
<213>  Artificial Sequence

<220>
<223>  oligonucleotide

<400>  1005
ctccctccca gaagcag                                                    17


<210>  1006
<211>  20
<212>  DNA
<213>  Artificial Sequence

<220>
<223>  oligonucleotide

<400>  1006
tgagcggatg attgagaagc                                                 20


<210>  1007
<211>  23
<212>  DNA
<213>  Artificial Sequence

<220>
<223>  oligonucleotide

<400>  1007
tgtttccgtg cgtgatcctc acc                                             23

<210>  1008
<211>  17
<212>  DNA
<213>  Artificial Sequence

<220>
<223>  oligonucleotide

<400>  1008
ttggctccct cccagaa                                                      17


<210>  1009
<211>  18
<212>  DNA
<213>  Artificial Sequence

<220>
<223>  oligonucleotide

<400>  1009
ggcaggagag gttcaaat                                                     18


<210>  1010
<211>  28
<212>  DNA
<213>  Artificial Sequence

<220>
<223>  oligonucleotide

<400>  1010
tcaacctgaa tgatgtggca tctctgga                                          28


<210>  1011
<211>  17
<212>  DNA
<213>  Artificial Sequence

<220>
<223>  oligonucleotide

<400>  1011
agccactgct tggtatg                                                      17


<210>  1012
<211>  20
<212>  DNA
<213>  Artificial Sequence

<220>
<223>  oligonucleotide

<400>  1012
gcattgcatc aacctactat                                                   20


<210>  1013
<211>  22
<212>  DNA

<213>  Artificial Sequence

<220>
<223>  oligonucleotide

<400>  1013
acataccaag cagtggctgg cc                                                22


<210>  1014
<211>  18
<212>  DNA
<213>  Artificial Sequence

<220>
<223>  oligonucleotide

<400>  1014
attctccttc agggcatc                                                     18


<210>  1015
<211>  17
<212>  DNA
<213>  Artificial Sequence

<220>
<223>  oligonucleotide

<400>  1015
ctgggcagga gaggttc                                                      17


<210>  1016
<211>  28
<212>  DNA
<213>  Artificial Sequence

<220>
<223>  oligonucleotide

<400>  1016
agaggagctc aagccatcat cattgtct                                          28


<210>  1017
<211>  20
<212>  DNA
<213>  Artificial Sequence

<220>
<223>  oligonucleotide

<400>  1017
ccagagatgc cacatcattc                                                   20


<210>  1018
<211>  20
<212>  DNA
<213>  Artificial Sequence

<220>

<223>  oligonucleotide

<400>  1018
gggcaggaga ggttcaaatg                                                   20


<210>  1019
<211>  30
<212>  DNA
<213>  Artificial Sequence

<220>
<223>  oligonucleotide

<400>  1019
tcttcaacct gaatgatgtg gcatctctgg                                        30


<210>  1020
<211>  20
<212>  DNA
<213>  Artificial Sequence

<220>
<223>  oligonucleotide

<400>  1020
cagccactgc ttggtatgtt                                                   20


<210>  1021
<211>  17
<212>  DNA
<213>  Artificial Sequence

<220>
<223>  oligonucleotide

<400>  1021
ccagttgcag ccttctc                                                      17


<210>  1022
<211>  22
<212>  DNA
<213>  Artificial Sequence

<220>
<223>  oligonucleotide

<400>  1022
agccaaacgc cgaccaagga aa                                                22


<210>  1023
<211>  18
<212>  DNA
<213>  Artificial Sequence

<220>
<223>  oligonucleotide

<400>  1023

ggtatggcac aggtgatg                                                    18

<210>  1024
<211>  17
<212>  DNA
<213>  Artificial Sequence

<220>
<223>  oligonucleotide

<400>  1024
agccttctca gccaaac                                                     17

<210>  1025
<211>  25
<212>  DNA
<213>  Artificial Sequence

<220>
<223>  oligonucleotide

<400>  1025
taggcatcac ctgtgccata ccagt                                            25

<210>  1026
<211>  20
<212>  DNA
<213>  Artificial Sequence

<220>
<223>  oligonucleotide

<400>  1026
cctcagaact tccagaatca                                                  20

<210>  1027
<211>  17
<212>  DNA
<213>  Artificial Sequence

<220>
<223>  oligonucleotide

<400>  1027
gagggcttgg ttgtcag                                                     17

<210>  1028
<211>  22
<212>  DNA
<213>  Artificial Sequence

<220>
<223>  oligonucleotide

<400>  1028
agccaaacgc cgaccaagga aa                                               22

<210>  1029
<211>  19
<212>  DNA
<213>  Artificial Sequence

<220>
<223>  oligonucleotide

<400>  1029
tcactgcaat tctcatggt                                                    19


<210>  1030
<211>  22
<212>  DNA
<213>  Artificial Sequence

<220>
<223>  oligonucleotide

<400>  1030
agccaaacgc cgaccaagga aa                                                22


<210>  1031
<211>  20
<212>  DNA
<213>  Artificial Sequence

<220>
<223>  oligonucleotide

<400>  1031
agcaaatcac tgcaattctc                                                   20


<210>  1032
<211>  17
<212>  DNA
<213>  Artificial Sequence

<220>
<223>  oligonucleotide

<400>  1032
ggaccagact cgtctca                                                      17


<210>  1033
<211>  18
<212>  DNA
<213>  Artificial Sequence

<220>
<223>  oligonucleotide

<400>  1033
tcggatgagg aggtcatt                                                     18


<210>  1034
<211>  26
<212>  DNA

<213>  Artificial Sequence

<220>
<223>  oligonucleotide

<400>  1034
actggccaag tctctcatca agtcct                                          26


<210>  1035
<211>  17
<212>  DNA
<213>  Artificial Sequence

<220>
<223>  oligonucleotide

<400>  1035
tttggcatcg gaagcat                                                    17


<210>  1036
<211>  18
<212>  DNA
<213>  Artificial Sequence

<220>
<223>  oligonucleotide

<400>  1036
attgcactgg ccaagtct                                                   18


<210>  1037
<211>  24
<212>  DNA
<213>  Artificial Sequence

<220>
<223>  oligonucleotide

<400>  1037
tcctggaaga agctcctgga tgct                                            24


<210>  1038
<211>  17
<212>  DNA
<213>  Artificial Sequence

<220>
<223>  oligonucleotide

<400>  1038
tccctggctt tggcatc                                                    17


<210>  1039
<211>  17
<212>  DNA
<213>  Artificial Sequence

<220>

<223>  oligonucleotide

<400>  1039
agctcggatg aggaggt                                                    17


<210>  1040
<211>  26
<212>  DNA
<213>  Artificial Sequence

<220>
<223>  oligonucleotide

<400>  1040
actggccaag tctctcatca agtcct                                          26


<210>  1041
<211>  17
<212>  DNA
<213>  Artificial Sequence

<220>
<223>  oligonucleotide

<400>  1041
tggctttggc atcggaa                                                    17


<210>  1042
<211>  19
<212>  DNA
<213>  Artificial Sequence

<220>
<223>  oligonucleotide

<400>  1042
ctggtccaca gagtattcc                                                  19


<210>  1043
<211>  30
<212>  DNA
<213>  Artificial Sequence

<220>
<223>  oligonucleotide

<400>  1043
accacagagt atttctcagt cacagatctc                                      30


<210>  1044
<211>  21
<212>  DNA
<213>  Artificial Sequence

<220>
<223>  oligonucleotide

<400>  1044

agatagctca gttcttgaga c                                                21

<210>  1045
<211>  21
<212>  DNA
<213>  Artificial Sequence

<220>
<223>  oligonucleotide

<400>  1045
cagagtattc cagcagttag t                                                21

<210>  1046
<211>  30
<212>  DNA
<213>  Artificial Sequence

<220>
<223>  oligonucleotide

<400>  1046
accacagagt atttctcagt cacagatctc                                       30

<210>  1047
<211>  22
<212>  DNA
<213>  Artificial Sequence

<220>
<223>  oligonucleotide

<400>  1047
tcatggtaag atagctcagt tc                                               22

<210>  1048
<211>  19
<212>  DNA
<213>  Artificial Sequence

<220>
<223>  oligonucleotide

<400>  1048
cacagagtat tccagcagt                                                   19

<210>  1049
<211>  30
<212>  DNA
<213>  Artificial Sequence

<220>
<223>  oligonucleotide

<400>  1049
accacagagt atttctcagt cacagatctc                                       30

<210>  1050
<211>  21
<212>  DNA
<213>  Artificial Sequence

<220>
<223>  oligonucleotide

<400>  1050
accttcatgg taagatagct c                                                21


<210>  1051
<211>  20
<212>  DNA
<213>  Artificial Sequence

<220>
<223>  oligonucleotide

<400>  1051
ctcagtcaca gatctcacaa                                                  20


<210>  1052
<211>  30
<212>  DNA
<213>  Artificial Sequence

<220>
<223>  oligonucleotide

<400>  1052
agaactgagc tatcttacca tgaaggttgt                                       30


<210>  1053
<211>  24
<212>  DNA
<213>  Artificial Sequence

<220>
<223>  oligonucleotide

<400>  1053
acctaaatag tagttacagt cctc                                             24


<210>  1054
<211>  17
<212>  DNA
<213>  Artificial Sequence

<220>
<223>  oligonucleotide

<400>  1054
caccgacctc ccgttag                                                     17


<210>  1055
<211>  22
<212>  DNA

<213>  Artificial Sequence

<220>
<223>  oligonucleotide

<400>  1055
accctgaggt ccttcacagc ct                                                22


<210>  1056
<211>  20
<212>  DNA
<213>  Artificial Sequence

<220>
<223>  oligonucleotide

<400>  1056
gttctgagcc ttgagttgtg                                                   20


<210>  1057
<211>  18
<212>  DNA
<213>  Artificial Sequence

<220>
<223>  oligonucleotide

<400>  1057
cagcctgcag acacaact                                                     18


<210>  1058
<211>  21
<212>  DNA
<213>  Artificial Sequence

<220>
<223>  oligonucleotide

<400>  1058
gctttgcaga tgctgaattc g                                                 21


<210>  1059
<211>  23
<212>  DNA
<213>  Artificial Sequence

<220>
<223>  oligonucleotide

<400>  1059
agcaactctt ccacaaggtg gcc                                               23


<210>  1060
<211>  17
<212>  DNA
<213>  Artificial Sequence

<220>

<223>  oligonucleotide

<400>  1060
gaggtccttc acagcct                                                    17


<210>  1061
<211>  25
<212>  DNA
<213>  Artificial Sequence

<220>
<223>  oligonucleotide

<400>  1061
acaactcaag gctcagaaca gcagg                                           25


<210>  1062
<211>  19
<212>  DNA
<213>  Artificial Sequence

<220>
<223>  oligonucleotide

<400>  1062
caccttgtgg aagagttgc                                                  19


<210>  1063
<211>  18
<212>  DNA
<213>  Artificial Sequence

<220>
<223>  oligonucleotide

<400>  1063
gaccctgagg tccttcac                                                   18


<210>  1064
<211>  23
<212>  DNA
<213>  Artificial Sequence

<220>
<223>  oligonucleotide

<400>  1064
agccttgagt tgtgtctgca ggc                                             23


<210>  1065
<211>  18
<212>  DNA
<213>  Artificial Sequence

<220>
<223>  oligonucleotide

<400>  1065

tgtggaagag ttgctgga                                                    18

<210>  1066
<211>  19
<212>  DNA
<213>  Artificial Sequence

<220>
<223>  oligonucleotide

<400>  1066
ctgtgggcgg attagggct                                                   19

<210>  1067
<211>  19
<212>  DNA
<213>  Artificial Sequence

<220>
<223>  oligonucleotide

<400>  1067
gagactctca gggtcgaaa                                                   19

<210>  1068
<211>  24
<212>  DNA
<213>  Artificial Sequence

<220>
<223>  oligonucleotide

<400>  1068
atttctacca ctccaaacgc cggc                                             24

<210>  1069
<211>  19
<212>  DNA
<213>  Artificial Sequence

<220>
<223>  oligonucleotide

<400>  1069
gagactctca gggtcgaaa                                                   19

<210>  1070
<211>  23
<212>  DNA
<213>  Artificial Sequence

<220>
<223>  oligonucleotide

<400>  1070
aatctgtcat gctggtctgc cgc                                              23

<210>  1071
<211>  17
<212>  DNA
<213>  Artificial Sequence

<220>
<223>  oligonucleotide

<400>  1071
ttcctgtggg cggatta                                                      17


<210>  1072
<211>  17
<212>  DNA
<213>  Artificial Sequence

<220>
<223>  oligonucleotide

<400>  1072
aggtggacct ggagact                                                      17


<210>  1073
<211>  23
<212>  DNA
<213>  Artificial Sequence

<220>
<223>  oligonucleotide

<400>  1073
aatctgtcat gctggtctgc cgc                                               23


<210>  1074
<211>  20
<212>  DNA
<213>  Artificial Sequence

<220>
<223>  oligonucleotide

<400>  1074
ggcttcctct tggagaagat                                                   20


<210>  1075
<211>  20
<212>  DNA
<213>  Artificial Sequence

<220>
<223>  oligonucleotide

<400>  1075
ggacctgtca ctgtcttgta                                                   20


<210>  1076
<211>  20
<212>  DNA

<213>  Artificial Sequence

<220>
<223>  oligonucleotide

<400>  1076
aaacggcggc agaccagcat                                                    20


<210>  1077
<211>  21
<212>  DNA
<213>  Artificial Sequence

<220>
<223>  oligonucleotide

<400>  1077
gcgtttggag tggtagaaat c                                                  21


<210>  1078
<211>  19
<212>  DNA
<213>  Artificial Sequence

<220>
<223>  oligonucleotide

<400>  1078
gaagctgacc tggaagaga                                                     19


<210>  1079
<211>  24
<212>  DNA
<213>  Artificial Sequence

<220>
<223>  oligonucleotide

<400>  1079
agtttgagct ttctggctgc acca                                               24


<210>  1080
<211>  22
<212>  DNA
<213>  Artificial Sequence

<220>
<223>  oligonucleotide

<400>  1080
ccacagaatt tagctcggta tg                                                 22


<210>  1081
<211>  19
<212>  DNA
<213>  Artificial Sequence

<220>

<223>  oligonucleotide

<400>  1081
gcaggctaga gaagcagag                                                    19


<210>  1082
<211>  22
<212>  DNA
<213>  Artificial Sequence

<220>
<223>  oligonucleotide

<400>  1082
ttccaggtca gcttcgcaag gc                                                22


<210>  1083
<211>  19
<212>  DNA
<213>  Artificial Sequence

<220>
<223>  oligonucleotide

<400>  1083
gggagtacgg atgcacttt                                                    19


<210>  1084
<211>  20
<212>  DNA
<213>  Artificial Sequence

<220>
<223>  oligonucleotide

<400>  1084
gctgacctgg aagagaacat                                                   20


<210>  1085
<211>  26
<212>  DNA
<213>  Artificial Sequence

<220>
<223>  oligonucleotide

<400>  1085
tcaagtttga gctttctggc tgcacc                                            26


<210>  1086
<211>  20
<212>  DNA
<213>  Artificial Sequence

<220>
<223>  oligonucleotide

<400>  1086

gctcggtatg tcttcatgct                                                    20

<210>  1087
<211>  26
<212>  DNA
<213>  Artificial Sequence

<220>
<223>  oligonucleotide

<400>  1087
cctatcaagt ttgagctttc tggctg                                              26

<210>  1088
<211>  20
<212>  DNA
<213>  Artificial Sequence

<220>
<223>  oligonucleotide

<400>  1088
gaagctgacc tggaagagaa                                                    20

<210>  1089
<211>  22
<212>  DNA
<213>  Artificial Sequence

<220>
<223>  oligonucleotide

<400>  1089
ccacagaatt tagctcggta tg                                                  22

<210>  1090
<211>  20
<212>  DNA
<213>  Artificial Sequence

<220>
<223>  oligonucleotide

<400>  1090
gcgagaacga catcaacatc                                                    20

<210>  1091
<211>  22
<212>  DNA
<213>  Artificial Sequence

<220>
<223>  oligonucleotide

<400>  1091
agctcctgct cttggagacc ga                                                  22

<210>  1092
<211>  17
<212>  DNA
<213>  Artificial Sequence

<220>
<223>  oligonucleotide

<400>  1092
tggattcgtc accagca                                                    17


<210>  1093
<211>  29
<212>  DNA
<213>  Artificial Sequence

<220>
<223>  oligonucleotide

<400>  1093
aaggatcctg ccttaagtca acttatttg                                       29


<210>  1094
<211>  20
<212>  DNA
<213>  Artificial Sequence

<220>
<223>  oligonucleotide

<400>  1094
gatccatgta gcgactttcc                                                 20


<210>  1095
<211>  22
<212>  DNA
<213>  Artificial Sequence

<220>
<223>  oligonucleotide

<400>  1095
acgaatccac attcatctca at                                              22


<210>  1096
<211>  17
<212>  DNA
<213>  Artificial Sequence

<220>
<223>  oligonucleotide

<400>  1096
cggagctcct gctcttg                                                    17


<210>  1097
<211>  22
<212>  DNA

<213>  Artificial Sequence

<220>
<223>  oligonucleotide

<400>  1097
tgtggattcg tcaccagcac gc                                                22


<210>  1098
<211>  22
<212>  DNA
<213>  Artificial Sequence

<220>
<223>  oligonucleotide

<400>  1098
aggatccttc cattgagatg aa                                                22


<210>  1099
<211>  18
<212>  DNA
<213>  Artificial Sequence

<220>
<223>  oligonucleotide

<400>  1099
gctcctgctc ttggagac                                                     18


<210>  1100
<211>  22
<212>  DNA
<213>  Artificial Sequence

<220>
<223>  oligonucleotide

<400>  1100
tgtggattcg tcaccagcac gc                                                22


<210>  1101
<211>  20
<212>  DNA
<213>  Artificial Sequence

<220>
<223>  oligonucleotide

<400>  1101
gcaggatcct tccattgaga                                                   20


<210>  1102
<211>  20
<212>  DNA
<213>  Artificial Sequence

<220>

<223>  oligonucleotide

<400>  1102
gtcggccaag ttgatgaatg                                                   20


<210>  1103
<211>  22
<212>  DNA
<213>  Artificial Sequence

<220>
<223>  oligonucleotide

<400>  1103
acagcgtggt cctctgcctc tt                                                22


<210>  1104
<211>  21
<212>  DNA
<213>  Artificial Sequence

<220>
<223>  oligonucleotide

<400>  1104
gatgagcgtg aagtggattt g                                                 21


<210>  1105
<211>  18
<212>  DNA
<213>  Artificial Sequence

<220>
<223>  oligonucleotide

<400>  1105
cgagtcggcc aagttgat                                                     18


<210>  1106
<211>  22
<212>  DNA
<213>  Artificial Sequence

<220>
<223>  oligonucleotide

<400>  1106
acagcgtggt cctctgcctc tt                                                22


<210>  1107
<211>  19
<212>  DNA
<213>  Artificial Sequence

<220>
<223>  oligonucleotide

<400>  1107

actggatgag cgtgaagtg                                                    19


<210>  1108
<211>  17
<212>  DNA
<213>  Artificial Sequence

<220>
<223>  oligonucleotide

<400>  1108
tgtacgagtc ggccaag                                                      17


<210>  1109
<211>  22
<212>  DNA
<213>  Artificial Sequence

<220>
<223>  oligonucleotide

<400>  1109
acagcgtggt cctctgcctc tt                                                22


<210>  1110
<211>  18
<212>  DNA
<213>  Artificial Sequence

<220>
<223>  oligonucleotide

<400>  1110
gatttgcagg gcgatgtc                                                     18


<210>  1111
<211>  17
<212>  DNA
<213>  Artificial Sequence

<220>
<223>  oligonucleotide

<400>  1111
caggatcgcc tcacagt                                                      17


<210>  1112
<211>  22
<212>  DNA
<213>  Artificial Sequence

<220>
<223>  oligonucleotide

<400>  1112
acagcgtggt cctctgcctc tt                                                22

<210>  1113
<211>  19
<212>  DNA
<213>  Artificial Sequence

<220>
<223>  oligonucleotide

<400>  1113
ctcctcctcc tcgtcaatg                                                    19


<210>  1114
<211>  17
<212>  DNA
<213>  Artificial Sequence

<220>
<223>  oligonucleotide

<400>  1114
cctcaacggc gagatca                                                      17


<210>  1115
<211>  23
<212>  DNA
<213>  Artificial Sequence

<220>
<223>  oligonucleotide

<400>  1115
tcgcatcttg tgtcgctgaa gcg                                               23


<210>  1116
<211>  18
<212>  DNA
<213>  Artificial Sequence

<220>
<223>  oligonucleotide

<400>  1116
acccacagag cacgtaat                                                     18


<210>  1117
<211>  19
<212>  DNA
<213>  Artificial Sequence

<220>
<223>  oligonucleotide

<400>  1117
gaatccgaag ttggaaccc                                                    19


<210>  1118
<211>  22
<212>  DNA

<213>  Artificial Sequence

<220>
<223>  oligonucleotide

<400>  1118
agcaccctca acggcgagat ca                                                22


<210>  1119
<211>  18
<212>  DNA
<213>  Artificial Sequence

<220>
<223>  oligonucleotide

<400>  1119
gcttcagcga cacaagat                                                     18


<210>  1120
<211>  20
<212>  DNA
<213>  Artificial Sequence

<220>
<223>  oligonucleotide

<400>  1120
ttggagctga actcggaatc                                                   20


<210>  1121
<211>  22
<212>  DNA
<213>  Artificial Sequence

<220>
<223>  oligonucleotide

<400>  1121
agcaccctca acggcgagat ca                                                22


<210>  1122
<211>  18
<212>  DNA
<213>  Artificial Sequence

<220>
<223>  oligonucleotide

<400>  1122
agcgacacaa gatgcgat                                                     18


<210>  1123
<211>  17
<212>  DNA
<213>  Artificial Sequence

<220>

<223>  oligonucleotide

<400>  1123
cgctcagcac cctcaac                                                      17


<210>  1124
<211>  23
<212>  DNA
<213>  Artificial Sequence

<220>
<223>  oligonucleotide

<400>  1124
tcgcatcttg tgtcgctgaa gcg                                               23


<210>  1125
<211>  20
<212>  DNA
<213>  Artificial Sequence

<220>
<223>  oligonucleotide

<400>  1125
agcacgtaat tcctcttgcc                                                   20


<210>  1126
<211>  19
<212>  DNA
<213>  Artificial Sequence

<220>
<223>  oligonucleotide

<400>  1126
agtacccgta tgggacaaa                                                    19


<210>  1127
<211>  20
<212>  DNA
<213>  Artificial Sequence

<220>
<223>  oligonucleotide

<400>  1127
ccagtttgct atggtgaaca                                                   20


<210>  1128
<211>  29
<212>  DNA
<213>  Artificial Sequence

<220>
<223>  oligonucleotide

<400>  1128

tgcaaggtca agatggttca ttatggctg                                          29

<210>  1129
<211>  19
<212>  DNA
<213>  Artificial Sequence

<220>
<223>  oligonucleotide

<400>  1129
gggaccacaa cctggattc                                                    19

<210>  1130
<211>  22
<212>  DNA
<213>  Artificial Sequence

<220>
<223>  oligonucleotide

<400>  1130
agctctacag ctcccaggtg tg                                                22

<210>  1131
<211>  17
<212>  DNA
<213>  Artificial Sequence

<220>
<223>  oligonucleotide

<400>  1131
gctcgcagcc ttgtcag                                                      17

<210>  1132
<211>  17
<212>  DNA
<213>  Artificial Sequence

<220>
<223>  oligonucleotide

<400>  1132
gggcactggg agctcta                                                      17

<210>  1133
<211>  22
<212>  DNA
<213>  Artificial Sequence

<220>
<223>  oligonucleotide

<400>  1133
acctactgtc ctacctgcgg ca                                                22

<210>  1134
<211>  17
<212>  DNA
<213>  Artificial Sequence

<220>
<223>  oligonucleotide

<400>  1134
gggctccagg gtcttca                                                      17


<210>  1135
<211>  19
<212>  DNA
<213>  Artificial Sequence

<220>
<223>  oligonucleotide

<400>  1135
ggacaaattc ccagctgac                                                    19


<210>  1136
<211>  22
<212>  DNA
<213>  Artificial Sequence

<220>
<223>  oligonucleotide

<400>  1136
tcccaggtgt gctgacaagg ct                                                22


<210>  1137
<211>  18
<212>  DNA
<213>  Artificial Sequence

<220>
<223>  oligonucleotide

<400>  1137
ccgcaggtag gacagtag                                                     18


<210>  1138
<211>  20
<212>  DNA
<213>  Artificial Sequence

<220>
<223>  oligonucleotide

<400>  1138
ctaccacgac gactcataca                                                   20


<210>  1139
<211>  23
<212>  DNA

<213>  Artificial Sequence

<220>
<223>  oligonucleotide

<400>  1139
tggtggcctc tctctacacg act                                              23


<210>  1140
<211>  19
<212>  DNA
<213>  Artificial Sequence

<220>
<223>  oligonucleotide

<400>  1140
gctcggtttc aggagtttg                                                   19


<210>  1141
<211>  19
<212>  DNA
<213>  Artificial Sequence

<220>
<223>  oligonucleotide

<400>  1141
atggaacagc ccttctacc                                                   19


<210>  1142
<211>  25
<212>  DNA
<213>  Artificial Sequence

<220>
<223>  oligonucleotide

<400>  1142
tcatacacag ctacgggata cggcc                                            25


<210>  1143
<211>  22
<212>  DNA
<213>  Artificial Sequence

<220>
<223>  oligonucleotide

<400>  1143
tcaggagttt gtagtcgtgt ag                                               22


<210>  1144
<211>  17
<212>  DNA
<213>  Artificial Sequence

<220>

<223>  oligonucleotide

<400>  1144
cccggatgtg cactaaa                                                    17


<210>  1145
<211>  21
<212>  DNA
<213>  Artificial Sequence

<220>
<223>  oligonucleotide

<400>  1145
gctcggtttc aggagtttgt a                                               21


<210>  1146
<211>  23
<212>  DNA
<213>  Artificial Sequence

<220>
<223>  oligonucleotide

<400>  1146
tcatacacag ctacgggata cgg                                             23


<210>  1147
<211>  20
<212>  DNA
<213>  Artificial Sequence

<220>
<223>  oligonucleotide

<400>  1147
cgacgactca tacacagcta                                                 20


<210>  1148
<211>  23
<212>  DNA
<213>  Artificial Sequence

<220>
<223>  oligonucleotide

<400>  1148
aaactcctga aaccgagcct ggc                                             23


<210>  1149
<211>  19
<212>  DNA
<213>  Artificial Sequence

<220>
<223>  oligonucleotide

<400>  1149

ctttgagact ccggtaggg                                                    19

<210>  1150
<211>  21
<212>  DNA
<213>  Artificial Sequence

<220>
<223>  oligonucleotide

<400>  1150
gtggccaact acaacttctt c                                                21

<210>  1151
<211>  22
<212>  DNA
<213>  Artificial Sequence

<220>
<223>  oligonucleotide

<400>  1151
cgcaagccca agtgggacaa ga                                               22

<210>  1152
<211>  19
<212>  DNA
<213>  Artificial Sequence

<220>
<223>  oligonucleotide

<400>  1152
ggcatcatcc actgtctct                                                   19

<210>  1153
<211>  20
<212>  DNA
<213>  Artificial Sequence

<220>
<223>  oligonucleotide

<400>  1153
tgggacaaga accagatcac                                                  20

<210>  1154
<211>  24
<212>  DNA
<213>  Artificial Sequence

<220>
<223>  oligonucleotide

<400>  1154
acccagagac agtggatgat gcct                                             24

<210>  1155
<211>  19
<212>  DNA
<213>  Artificial Sequence

<220>
<223>  oligonucleotide

<400>  1155
gtcacatcgc tccagactt                                                    19


<210>  1156
<211>  22
<212>  DNA
<213>  Artificial Sequence

<220>
<223>  oligonucleotide

<400>  1156
ggacaagaac cagatcacat ac                                                22


<210>  1157
<211>  24
<212>  DNA
<213>  Artificial Sequence

<220>
<223>  oligonucleotide

<400>  1157
tgggtccaga tcaggtgtgt agcc                                              24


<210>  1158
<211>  18
<212>  DNA
<213>  Artificial Sequence

<220>
<223>  oligonucleotide

<400>  1158
cacgagcaaa ggcatcat                                                     18


<210>  1159
<211>  27
<212>  DNA
<213>  Artificial Sequence

<220>
<223>  oligonucleotide

<400>  1159
cacatacagg atcattggct acacacc                                           27


<210>  1160
<211>  19
<212>  DNA

<213>  Artificial Sequence

<220>
<223>  oligonucleotide

<400>  1160
gtcacatcgc tccagactt                                                    19


<210>  1161
<211>  20
<212>  DNA
<213>  Artificial Sequence

<220>
<223>  oligonucleotide

<400>  1161
aagtgggaca agaaccagat                                                   20


<210>  1162
<211>  19
<212>  DNA
<213>  Artificial Sequence

<220>
<223>  oligonucleotide

<400>  1162
ggagacctga gaaccaatc                                                    19


<210>  1163
<211>  19
<212>  DNA
<213>  Artificial Sequence

<220>
<223>  oligonucleotide

<400>  1163
gactctccac gcatctctg                                                    19


<210>  1164
<211>  23
<212>  DNA
<213>  Artificial Sequence

<220>
<223>  oligonucleotide

<400>  1164
aggcagctgg cagaggaata cct                                               23


<210>  1165
<211>  17
<212>  DNA
<213>  Artificial Sequence

<220>

<223>  oligonucleotide

<400>  1165
tccacccttg tgctctt                                                    17


<210>  1166
<211>  23
<212>  DNA
<213>  Artificial Sequence

<220>
<223>  oligonucleotide

<400>  1166
aaccaatctc accgacaggc agc                                             23


<210>  1167
<211>  18
<212>  DNA
<213>  Artificial Sequence

<220>
<223>  oligonucleotide

<400>  1167
actctccacg catctctg                                                   18


<210>  1168
<211>  20
<212>  DNA
<213>  Artificial Sequence

<220>
<223>  oligonucleotide

<400>  1168
agaaccaatc tcaccgacag                                                 20


<210>  1169
<211>  23
<212>  DNA
<213>  Artificial Sequence

<220>
<223>  oligonucleotide

<400>  1169
tggttacact cgggtggcag aga                                             23


<210>  1170
<211>  21
<212>  DNA
<213>  Artificial Sequence

<220>
<223>  oligonucleotide

<400>  1170

ccagagattt cgactctcca c                                                21

<210>  1171
<211>  20
<212>  DNA
<213>  Artificial Sequence

<220>
<223>  oligonucleotide

<400>  1171
tggagacctg agaaccaatc                                                   20

<210>  1172
<211>  23
<212>  DNA
<213>  Artificial Sequence

<220>
<223>  oligonucleotide

<400>  1172
aggcagctgg cagaggaata cct                                               23

<210>  1173
<211>  19
<212>  DNA
<213>  Artificial Sequence

<220>
<223>  oligonucleotide

<400>  1173
acccgagtgt aaccatagc                                                    19

<210>  1174
<211>  20
<212>  DNA
<213>  Artificial Sequence

<220>
<223>  oligonucleotide

<400>  1174
cattcccgag gagctttatg                                                   20

<210>  1175
<211>  24
<212>  DNA
<213>  Artificial Sequence

<220>
<223>  oligonucleotide

<400>  1175
tgaccactcg atccgctcct ttga                                              24

<210>  1176
<211>  20
<212>  DNA
<213>  Artificial Sequence

<220>
<223>  oligonucleotide

<400>  1176
gtcatgttca ggtccaactc                                                    20


<210>  1177
<211>  20
<212>  DNA
<213>  Artificial Sequence

<220>
<223>  oligonucleotide

<400>  1177
cccgaggagc tttatgagat                                                    20


<210>  1178
<211>  24
<212>  DNA
<213>  Artificial Sequence

<220>
<223>  oligonucleotide

<400>  1178
tgaccactcg atccgctcct ttga                                               24


<210>  1179
<211>  18
<212>  DNA
<213>  Artificial Sequence

<220>
<223>  oligonucleotide

<400>  1179
gggtcatgtt caggtcca                                                      18


<210>  1180
<211>  18
<212>  DNA
<213>  Artificial Sequence

<220>
<223>  oligonucleotide

<400>  1180
tcgatccgct cctttgat                                                      18


<210>  1181
<211>  23
<212>  DNA

<213>  Artificial Sequence

<220>
<223>  oligonucleotide

<400>  1181
tgaacatgac ccgctcccac tct                                              23


<210>  1182
<211>  17
<212>  DNA
<213>  Artificial Sequence

<220>
<223>  oligonucleotide

<400>  1182
ccaggctcct tcttcca                                                     17


<210>  1183
<211>  20
<212>  DNA
<213>  Artificial Sequence

<220>
<223>  oligonucleotide

<400>  1183
atccgctcct ttgatgatct                                                  20


<210>  1184
<211>  23
<212>  DNA
<213>  Artificial Sequence

<220>
<223>  oligonucleotide

<400>  1184
tgaacatgac ccgctcccac tct                                              23


<210>  1185
<211>  17
<212>  DNA
<213>  Artificial Sequence

<220>
<223>  oligonucleotide

<400>  1185
ccttcttcca cgagcca                                                     17


<210>  1186
<211>  18
<212>  DNA
<213>  Artificial Sequence

<220>

<223>  oligonucleotide

<400>  1186
tgtctctgtg cccatgat                                                    18


<210>  1187
<211>  20
<212>  DNA
<213>  Artificial Sequence

<220>
<223>  oligonucleotide

<400>  1187
cagttccagg atgtcgtagt                                                  20


<210>  1188
<211>  19
<212>  DNA
<213>  Artificial Sequence

<220>
<223>  oligonucleotide

<400>  1188
cgcctcttcc acaaatcag                                                   19


<210>  1189
<211>  22
<212>  DNA
<213>  Artificial Sequence

<220>
<223>  oligonucleotide

<400>  1189
atgggcacag agacagtgct gc                                               22


<210>  1190
<211>  21
<212>  DNA
<213>  Artificial Sequence

<220>
<223>  oligonucleotide

<400>  1190
tccaggatgt cgtagtaatg g                                                21


<210>  1191
<211>  18
<212>  DNA
<213>  Artificial Sequence

<220>
<223>  oligonucleotide

<400>  1191

tggctcagac caacaagt 18

<210> 1192
<211> 24
<212> DNA
<213> Artificial Sequence

<220>
<223> oligonucleotide

<400> 1192
actacgacat cctggaactg ccct 24

<210> 1193
<211> 20
<212> DNA
<213> Artificial Sequence

<220>
<223> oligonucleotide

<400> 1193
cagcaatgaa catgctgagg 20

<210> 1194
<211> 20
<212> DNA
<213> Artificial Sequence

<220>
<223> oligonucleotide

<400> 1194
gtcgtagtaa tggccatcgg 20

<210> 1195
<211> 20
<212> DNA
<213> Artificial Sequence

<220>
<223> oligonucleotide

<400> 1195
ccacaaatca gacggcagca 20

<210> 1196
<211> 25
<212> DNA
<213> Artificial Sequence

<220>
<223> oligonucleotide

<400> 1196
cccatgatgg ctcagaccaa caagt 25

<210>  1197
<211>  22
<212>  DNA
<213>  Artificial Sequence

<220>
<223>  oligonucleotide

<400>  1197
actgagttca ccacgcccga tg                                                22


<210>  1198
<211>  19
<212>  DNA
<213>  Artificial Sequence

<220>
<223>  oligonucleotide

<400>  1198
ggagcctgag cttatgaat                                                    19


<210>  1199
<211>  23
<212>  DNA
<213>  Artificial Sequence

<220>
<223>  oligonucleotide

<400>  1199
gaagtgaaag tcagatggtt tag                                               23


<210>  1200
<211>  22
<212>  DNA
<213>  Artificial Sequence

<220>
<223>  oligonucleotide

<400>  1200
ttggtggagg agaagggttg gc                                                22


<210>  1201
<211>  20
<212>  DNA
<213>  Artificial Sequence

<220>
<223>  oligonucleotide

<400>  1201
tatgaatgcc aacccttctc                                                   20


<210>  1202
<211>  19
<212>  DNA

<213>  Artificial Sequence

<220>
<223>  oligonucleotide

<400>  1202
ccctttccga tcactttca                                                    19


<210>  1203
<211>  22
<212>  DNA
<213>  Artificial Sequence

<220>
<223>  oligonucleotide

<400>  1203
aatcaacctt ggcccgtcgt cc                                                22


<210>  1204
<211>  20
<212>  DNA
<213>  Artificial Sequence

<220>
<223>  oligonucleotide

<400>  1204
caggagcctg agcttatgaa                                                   20


<210>  1205
<211>  22
<212>  DNA
<213>  Artificial Sequence

<220>
<223>  oligonucleotide

<400>  1205
gatggtttag catgaggatt gg                                                22


<210>  1206
<211>  23
<212>  DNA
<213>  Artificial Sequence

<220>
<223>  oligonucleotide

<400>  1206
tcagcaaatc aaccttggcc cgt                                               23


<210>  1207
<211>  20
<212>  DNA
<213>  Artificial Sequence

<220>

<223>  oligonucleotide

<400>  1207
cttgaagatc tcccaacctc                                                    20


<210>  1208
<211>  21
<212>  DNA
<213>  Artificial Sequence

<220>
<223>  oligonucleotide

<400>  1208
caaggttgat ttgctgagaa g                                                  21


<210>  1209
<211>  22
<212>  DNA
<213>  Artificial Sequence

<220>
<223>  oligonucleotide

<400>  1209
ttggtggagg agaagggttg gc                                                 22


<210>  1210
<211>  23
<212>  DNA
<213>  Artificial Sequence

<220>
<223>  oligonucleotide

<400>  1210
gcatgagaag tggaaagaga aat                                                23


<210>  1211
<211>  27
<212>  DNA
<213>  Artificial Sequence

<220>
<223>  oligonucleotide

<400>  1211
ccaagacaga tgcaccatca ggaatgg                                            27


<210>  1212
<211>  20
<212>  DNA
<213>  Artificial Sequence

<220>
<223>  oligonucleotide

<400>  1212

tggtcacctt cctgctttat                                                    20

<210>  1213
<211>  29
<212>  DNA
<213>  Artificial Sequence

<220>
<223>  oligonucleotide

<400>  1213
acagatgcac catcaggaat ggaattaca                                          29

<210>  1214
<211>  19
<212>  DNA
<213>  Artificial Sequence

<220>
<223>  oligonucleotide

<400>  1214
ctgagaaaca tggtcacct                                                     19

<210>  1215
<211>  23
<212>  DNA
<213>  Artificial Sequence

<220>
<223>  oligonucleotide

<400>  1215
catgagaagt ggaaagagaa atc                                                23

<210>  1216
<211>  23
<212>  DNA
<213>  Artificial Sequence

<220>
<223>  oligonucleotide

<400>  1216
ggagaagttt agcatgagaa gtg                                                23

<210>  1217
<211>  27
<212>  DNA
<213>  Artificial Sequence

<220>
<223>  oligonucleotide

<400>  1217
ccaagacaga tgcaccatca ggaatgg                                            27

<210>  1218
<211>  20
<212>  DNA
<213>  Artificial Sequence

<220>
<223>  oligonucleotide

<400>  1218
tctgagaaac atggtcacct                                                   20


<210>  1219
<211>  23
<212>  DNA
<213>  Artificial Sequence

<220>
<223>  oligonucleotide

<400>  1219
gtttagcatg agaagtggaa aga                                               23


<210>  1220
<211>  27
<212>  DNA
<213>  Artificial Sequence

<220>
<223>  oligonucleotide

<400>  1220
ccaagacaga tgcaccatca ggaatgg                                           27


<210>  1221
<211>  20
<212>  DNA
<213>  Artificial Sequence

<220>
<223>  oligonucleotide

<400>  1221
gaaacatggt caccttcctg                                                   20


<210>  1222
<211>  21
<212>  DNA
<213>  Artificial Sequence

<220>
<223>  oligonucleotide

<400>  1222
acaaatggag ctcatcctag t                                                 21


<210>  1223
<211>  22
<212>  DNA

<213>  Artificial Sequence

<220>
<223>  oligonucleotide

<400>  1223
tcaggtggct ggtcggaaag ga                                                22


<210>  1224
<211>  20
<212>  DNA
<213>  Artificial Sequence

<220>
<223>  oligonucleotide

<400>  1224
gggcatagat cacatgagga                                                   20


<210>  1225
<211>  24
<212>  DNA
<213>  Artificial Sequence

<220>
<223>  oligonucleotide

<400>  1225
caaatggagc tcatcctagt aaat                                              24


<210>  1226
<211>  22
<212>  DNA
<213>  Artificial Sequence

<220>
<223>  oligonucleotide

<400>  1226
tcaggtggct ggtcggaaag ga                                                22


<210>  1227
<211>  19
<212>  DNA
<213>  Artificial Sequence

<220>
<223>  oligonucleotide

<400>  1227
agagacgggc atagatcac                                                    19


<210>  1228
<211>  23
<212>  DNA
<213>  Artificial Sequence

<220>

<223>  oligonucleotide

<400>  1228
ggagctcatc ctagtaaatg tgt                                               23


<210>  1229
<211>  22
<212>  DNA
<213>  Artificial Sequence

<220>
<223>  oligonucleotide

<400>  1229
tcaggtggct ggtcggaaag ga                                                22


<210>  1230
<211>  18
<212>  DNA
<213>  Artificial Sequence

<220>
<223>  oligonucleotide

<400>  1230
tccagagacg ggcataga                                                     18


<210>  1231
<211>  24
<212>  DNA
<213>  Artificial Sequence

<220>
<223>  oligonucleotide

<400>  1231
gctcatccta gtaaatgtgt tacc                                              24


<210>  1232
<211>  22
<212>  DNA
<213>  Artificial Sequence

<220>
<223>  oligonucleotide

<400>  1232
tttccgacca gccacctgaa gc                                                22


<210>  1233
<211>  23
<212>  DNA
<213>  Artificial Sequence

<220>
<223>  oligonucleotide

<400>  1233

ggcatagatc acatgaggaa atc 23

<210> 1234
<211> 17
<212> DNA
<213> Artificial Sequence

<220>
<223> oligonucleotide

<400> 1234
agatgctgtg ccttcct 17

<210> 1235
<211> 27
<212> DNA
<213> Artificial Sequence

<220>
<223> oligonucleotide

<400> 1235
agattcccaa cttcttctgg agcctgg 27

<210> 1236
<211> 17
<212> DNA
<213> Artificial Sequence

<220>
<223> oligonucleotide

<400> 1236
accagtgtga ccgatcc 17

<210> 1237
<211> 18
<212> DNA
<213> Artificial Sequence

<220>
<223> oligonucleotide

<400> 1237
ccttcctccg ctgaaaca 18

<210> 1238
<211> 22
<212> DNA
<213> Artificial Sequence

<220>
<223> oligonucleotide

<400> 1238
acactggtgc gtggtggcat ac 22

<210>  1239
<211>  18
<212>  DNA
<213>  Artificial Sequence

<220>
<223>  oligonucleotide

<400>  1239
tctcgtcttc tcctccca                                                    18


<210>  1240
<211>  17
<212>  DNA
<213>  Artificial Sequence

<220>
<223>  oligonucleotide

<400>  1240
gtccagatgc tgtgcct                                                     17


<210>  1241
<211>  24
<212>  DNA
<213>  Artificial Sequence

<220>
<223>  oligonucleotide

<400>  1241
tcccaacttc ttctggagcc tggg                                             24


<210>  1242
<211>  18
<212>  DNA
<213>  Artificial Sequence

<220>
<223>  oligonucleotide

<400>  1242
ctcccagtat gccaccac                                                    18


<210>  1243
<211>  18
<212>  DNA
<213>  Artificial Sequence

<220>
<223>  oligonucleotide

<400>  1243
accacgcacc agtgtgac                                                    18


<210>  1244
<211>  19
<212>  DNA

<213>  Artificial Sequence

<220>
<223>  oligonucleotide

<400>  1244
tgccttcctc cgctgaaac                                                    19


<210>  1245
<211>  24
<212>  DNA
<213>  Artificial Sequence

<220>
<223>  oligonucleotide

<400>  1245
tcccaacttc ttctggagcc tggg                                              24


<210>  1246
<211>  17
<212>  DNA
<213>  Artificial Sequence

<220>
<223>  oligonucleotide

<400>  1246
cccacactgg cgagaag                                                      17


<210>  1247
<211>  20
<212>  DNA
<213>  Artificial Sequence

<220>
<223>  oligonucleotide

<400>  1247
ttcgctgacc gctccaacct                                                   20


<210>  1248
<211>  20
<212>  DNA
<213>  Artificial Sequence

<220>
<223>  oligonucleotide

<400>  1248
ttgacatctg agtgggtctg                                                   20


<210>  1249
<211>  17
<212>  DNA
<213>  Artificial Sequence

<220>

<223>  oligonucleotide

<400>  1249
catgtccgga cccacac                                                      17


<210>  1250
<211>  22
<212>  DNA
<213>  Artificial Sequence

<220>
<223>  oligonucleotide

<400>  1250
tggcgagaag cccttctcct gt                                                22


<210>  1251
<211>  20
<212>  DNA
<213>  Artificial Sequence

<220>
<223>  oligonucleotide

<400>  1251
ggcactggta cttcttgaca                                                   20


<210>  1252
<211>  17
<212>  DNA
<213>  Artificial Sequence

<220>
<223>  oligonucleotide

<400>  1252
ttctctaggc cctggct                                                      17


<210>  1253
<211>  22
<212>  DNA
<213>  Artificial Sequence

<220>
<223>  oligonucleotide

<400>  1253
tacaaggcca tgtccggacc ca                                                22


<210>  1254
<211>  23
<212>  DNA
<213>  Artificial Sequence

<220>
<223>  oligonucleotide

<400>  1254

ggtacttctt gacatctgag tgg                                                23

<210>  1255
<211>  17
<212>  DNA
<213>  Artificial Sequence

<220>
<223>  oligonucleotide

<400>  1255
gccctggctg ctacaag                                                      17

<210>  1256
<211>  22
<212>  DNA
<213>  Artificial Sequence

<220>
<223>  oligonucleotide

<400>  1256
actggcgaga agcccttctc ct                                                22

<210>  1257
<211>  18
<212>  DNA
<213>  Artificial Sequence

<220>
<223>  oligonucleotide

<400>  1257
ctgagtgggt ctggaggt                                                     18

<210>  1258
<211>  20
<212>  DNA
<213>  Artificial Sequence

<220>
<223>  oligonucleotide

<400>  1258
cccagagaga caccagagaa                                                   20

<210>  1259
<211>  19
<212>  DNA
<213>  Artificial Sequence

<220>
<223>  oligonucleotide

<400>  1259
gctatcagcc acagcaaca                                                    19

<210>  1260
<211>  20
<212>  DNA
<213>  Artificial Sequence

<220>
<223>  oligonucleotide

<400>  1260
cctggcttct ggcatcctgt                                                    20


<210>  1261
<211>  17
<212>  DNA
<213>  Artificial Sequence

<220>
<223>  oligonucleotide

<400>  1261
catcgccgca gatccag                                                       17


<210>  1262
<211>  24
<212>  DNA
<213>  Artificial Sequence

<220>
<223>  oligonucleotide

<400>  1262
cccagagaga caccagagaa ccca                                               24


<210>  1263
<211>  18
<212>  DNA
<213>  Artificial Sequence

<220>
<223>  oligonucleotide

<400>  1263
tcagccacag caacaaca                                                      18


<210>  1264
<211>  19
<212>  DNA
<213>  Artificial Sequence

<220>
<223>  oligonucleotide

<400>  1264
agagacacca gagaaccca                                                     19


<210>  1265
<211>  24
<212>  DNA

<213>  Artificial Sequence

<220>
<223>  oligonucleotide

<400>  1265
tctggcatcc tgttgttgct gtgg                                              24


<210>  1266
<211>  17
<212>  DNA
<213>  Artificial Sequence

<220>
<223>  oligonucleotide

<400>  1266
gtgggacaca ggtgcag                                                      17


<210>  1267
<211>  19
<212>  DNA
<213>  Artificial Sequence

<220>
<223>  oligonucleotide

<400>  1267
ccagagagac accagagaa                                                    19


<210>  1268
<211>  24
<212>  DNA
<213>  Artificial Sequence

<220>
<223>  oligonucleotide

<400>  1268
tctggcatcc tgttgttgct gtgg                                              24


<210>  1269
<211>  19
<212>  DNA
<213>  Artificial Sequence

<220>
<223>  oligonucleotide

<400>  1269
gaggtcggaa ttgcagaag                                                    19


<210>  1270
<211>  19
<212>  DNA
<213>  Artificial Sequence

<220>

<223>  oligonucleotide

<400>  1270
gaggagggca gaatcatca                                                    19


<210>  1271
<211>  28
<212>  DNA
<213>  Artificial Sequence

<220>
<223>  oligonucleotide

<400>  1271
tgcgctgata gacatccatg aacttcac                                          28


<210>  1272
<211>  20
<212>  DNA
<213>  Artificial Sequence

<220>
<223>  oligonucleotide

<400>  1272
tctcgattgg atggcagtag                                                   20


<210>  1273
<211>  19
<212>  DNA
<213>  Artificial Sequence

<220>
<223>  oligonucleotide

<400>  1273
gggcagaatc atcacgaag                                                    19


<210>  1274
<211>  20
<212>  DNA
<213>  Artificial Sequence

<220>
<223>  oligonucleotide

<400>  1274
gtctcgattg gatggcagta                                                   20


<210>  1275
<211>  26
<212>  DNA
<213>  Artificial Sequence

<220>
<223>  oligonucleotide

<400>  1275

agttcatgga tgtctatcag cgcagc                                          26

<210>  1276
<211>  17
<212>  DNA
<213>  Artificial Sequence

<220>
<223>  oligonucleotide

<400>  1276
acccatggca gaaggag                                                   17

<210>  1277
<211>  24
<212>  DNA
<213>  Artificial Sequence

<220>
<223>  oligonucleotide

<400>  1277
agggcagaat catcacgaag tggt                                           24

<210>  1278
<211>  19
<212>  DNA
<213>  Artificial Sequence

<220>
<223>  oligonucleotide

<400>  1278
cagtagctgc gctgataga                                                 19

<210>  1279
<211>  18
<212>  DNA
<213>  Artificial Sequence

<220>
<223>  oligonucleotide

<400>  1279
gaaggaggag ggcagaat                                                  18

<210>  1280
<211>  22
<212>  DNA
<213>  Artificial Sequence

<220>
<223>  oligonucleotide

<400>  1280
cagcgcagct actgccatcc aa                                             22

<210>  1281
<211>  20
<212>  DNA
<213>  Artificial Sequence

<220>
<223>  oligonucleotide

<400>  1281
tactcctgga agatgtccac                                                   20


<210>  1282
<211>  18
<212>  DNA
<213>  Artificial Sequence

<220>
<223>  oligonucleotide

<400>  1282
ccaaccgcga gaagatga                                                     18


<210>  1283
<211>  23
<212>  DNA
<213>  Artificial Sequence

<220>
<223>  oligonucleotide

<400>  1283
ccatgtacgt tgctatccag gct                                               23


<210>  1284
<211>  20
<212>  DNA
<213>  Artificial Sequence

<220>
<223>  oligonucleotide

<400>  1284
ccagaggcgt acagggatag                                                   20


<210>  1285
<211>  23
<212>  DNA
<213>  Artificial Sequence

<220>
<223>  oligonucleotide

<400>  1285
cccagatcat gtttgagacc ttc                                               23


<210>  1286
<211>  22
<212>  DNA

<213>  Artificial Sequence

<220>
<223>  oligonucleotide

<400>  1286
acgttgctat ccaggctgtg ct                                                22


<210>  1287
<211>  19
<212>  DNA
<213>  Artificial Sequence

<220>
<223>  oligonucleotide

<400>  1287
gtccatcacg atgccagtg                                                    19


<210>  1288
<211>  19
<212>  DNA
<213>  Artificial Sequence

<220>
<223>  oligonucleotide

<400>  1288
ccaaccgcga gaagatgac                                                    19


<210>  1289
<211>  29
<212>  DNA
<213>  Artificial Sequence

<220>
<223>  oligonucleotide

<400>  1289
ccagatcatg tttgagacct tcaacaccc                                         29


<210>  1290
<211>  20
<212>  DNA
<213>  Artificial Sequence

<220>
<223>  oligonucleotide

<400>  1290
ggatagcaca gcctggatag                                                   20


<210>  1291
<211>  21
<212>  DNA
<213>  Artificial Sequence

<220>

<223>  oligonucleotide

<400>  1291
gatgacccag atcatgtttg a                                                21


<210>  1292
<211>  25
<212>  DNA
<213>  Artificial Sequence

<220>
<223>  oligonucleotide

<400>  1292
ccatgtacgt tgctatccag gctgt                                            25


<210>  1293
<211>  19
<212>  DNA
<213>  Artificial Sequence

<220>
<223>  oligonucleotide

<400>  1293
cagaggcgta cagggatag                                                   19


<210>  1294
<211>  22
<212>  DNA
<213>  Artificial Sequence

<220>
<223>  oligonucleotide

<400>  1294
atgagacaga tgctaatgga tg                                               22


<210>  1295
<211>  22
<212>  DNA
<213>  Artificial Sequence

<220>
<223>  oligonucleotide

<400>  1295
tttagcagca tctgcaaccc gc                                               22


<210>  1296
<211>  20
<212>  DNA
<213>  Artificial Sequence

<220>
<223>  oligonucleotide

<400>  1296

ttgcttgcac gtagatgtta                                                    20

<210>  1297
<211>  22
<212>  DNA
<213>  Artificial Sequence

<220>
<223>  oligonucleotide

<400>  1297
caaactcatg agacagatgc ta                                                  22

<210>  1298
<211>  22
<212>  DNA
<213>  Artificial Sequence

<220>
<223>  oligonucleotide

<400>  1298
tttagcagca tctgcaaccc gc                                                  22

<210>  1299
<211>  22
<212>  DNA
<213>  Artificial Sequence

<220>
<223>  oligonucleotide

<400>  1299
gctcattagt tcatcacaga ct                                                  22

<210>  1300
<211>  19
<212>  DNA
<213>  Artificial Sequence

<220>
<223>  oligonucleotide

<400>  1300
cagccacatc atccctgtg                                                      19

<210>  1301
<211>  27
<212>  DNA
<213>  Artificial Sequence

<220>
<223>  oligonucleotide

<400>  1301
agcagacata acatctacgt gcaagca                                             27

<210>  1302
<211>  20
<212>  DNA
<213>  Artificial Sequence

<220>
<223>  oligonucleotide

<400>  1302
ggcaccgtag ggtaattgat                                                    20


<210>  1303
<211>  17
<212>  DNA
<213>  Artificial Sequence

<220>
<223>  oligonucleotide

<400>  1303
agccacatca tccctgt                                                       17


<210>  1304
<211>  22
<212>  DNA
<213>  Artificial Sequence

<220>
<223>  oligonucleotide

<400>  1304
tttagcagca tctgcaaccc gc                                                 22


<210>  1305
<211>  22
<212>  DNA
<213>  Artificial Sequence

<220>
<223>  oligonucleotide

<400>  1305
cgtagatgtt atgtctgctc at                                                 22


<210>  1306
<211>  22
<212>  DNA
<213>  Artificial Sequence

<220>
<223>  oligonucleotide

<400>  1306
gtcacagccc aagatagtta ag                                                 22


<210>  1307
<211>  24
<212>  DNA

<213>  Artificial Sequence

<220>
<223>  oligonucleotide

<400>  1307
tcatggaggt ttgaagatgc cgca                                              24


<210>  1308
<211>  19
<212>  DNA
<213>  Artificial Sequence

<220>
<223>  oligonucleotide

<400>  1308
gcaagcaagc agaatttgg                                                    19


<210>  1309
<211>  19
<212>  DNA
<213>  Artificial Sequence

<220>
<223>  oligonucleotide

<400>  1309
cgtgtgaacc atgtgactt                                                    19


<210>  1310
<211>  28
<212>  DNA
<213>  Artificial Sequence

<220>
<223>  oligonucleotide

<400>  1310
cacagcccaa gatagttaag tgggatcg                                          28


<210>  1311
<211>  19
<212>  DNA
<213>  Artificial Sequence

<220>
<223>  oligonucleotide

<400>  1311
cctccatgat gctgcttac                                                    19


<210>  1312
<211>  19
<212>  DNA
<213>  Artificial Sequence

<220>

<223>  oligonucleotide

<400>  1312
ctttgtcaca gcccaagat                                                    19


<210>  1313
<211>  24
<212>  DNA
<213>  Artificial Sequence

<220>
<223>  oligonucleotide

<400>  1313
tgggatcgag acatgtaagc agca                                              24


<210>  1314
<211>  22
<212>  DNA
<213>  Artificial Sequence

<220>
<223>  oligonucleotide

<400>  1314
tggaattcat ccaatccaaa tg                                                22


<210>  1315
<211>  19
<212>  DNA
<213>  Artificial Sequence

<220>
<223>  oligonucleotide

<400>  1315
gtatgcctgc cgtgtgaac                                                    19


<210>  1316
<211>  26
<212>  DNA
<213>  Artificial Sequence

<220>
<223>  oligonucleotide

<400>  1316
aagtgggatc gagacatgta agcagc                                            26


<210>  1317
<211>  22
<212>  DNA
<213>  Artificial Sequence

<220>
<223>  oligonucleotide

<400>  1317

ggcatcttca aacctccatg at                                                22

<210>  1318
<211>  19
<212>  DNA
<213>  Artificial Sequence

<220>
<223>  oligonucleotide

<400>  1318
caaagcagtg gacaagaag                                                   19

<210>  1319
<211>  22
<212>  DNA
<213>  Artificial Sequence

<220>
<223>  oligonucleotide

<400>  1319
tgggcagact tggtgacctt gc                                                22

<210>  1320
<211>  20
<212>  DNA
<213>  Artificial Sequence

<220>
<223>  oligonucleotide

<400>  1320
gtggcaggta ttagggataa                                                  20

<210>  1321
<211>  21
<212>  DNA
<213>  Artificial Sequence

<220>
<223>  oligonucleotide

<400>  1321
ctttgctgtt cgtgatatga g                                                21

<210>  1322
<211>  22
<212>  DNA
<213>  Artificial Sequence

<220>
<223>  oligonucleotide

<400>  1322
tgcggtgggt gtcatcaaag ca                                                22

<210>  1323
<211>  21
<212>  DNA
<213>  Artificial Sequence

<220>
<223>  oligonucleotide

<400>  1323
catttagcct tctgagcttt c                                                21


<210>  1324
<211>  21
<212>  DNA
<213>  Artificial Sequence

<220>
<223>  oligonucleotide

<400>  1324
gaaagctcag aaggctaaat g                                                21


<210>  1325
<211>  26
<212>  DNA
<213>  Artificial Sequence

<220>
<223>  oligonucleotide

<400>  1325
tcagtggtgg aagaacggtc tcagaa                                           26


<210>  1326
<211>  19
<212>  DNA
<213>  Artificial Sequence

<220>
<223>  oligonucleotide

<400>  1326
tggccaattg aaacaaaca                                                   19


<210>  1327
<211>  21
<212>  DNA
<213>  Artificial Sequence

<220>
<223>  oligonucleotide

<400>  1327
gttcgtgata tgagacagac a                                                21


<210>  1328
<211>  22
<212>  DNA

<213>  Artificial Sequence

<220>
<223>  oligonucleotide

<400>  1328
tgcggtgggt gtcatcaaag ca                                                22


<210>  1329
<211>  25
<212>  DNA
<213>  Artificial Sequence

<220>
<223>  oligonucleotide

<400>  1329
gggataatat tcatttagcc ttctg                                             25


<210>  1330
<211>  21
<212>  DNA
<213>  Artificial Sequence

<220>
<223>  oligonucleotide

<400>  1330
caagtacatc atccgagaca a                                                 21


<210>  1331
<211>  23
<212>  DNA
<213>  Artificial Sequence

<220>
<223>  oligonucleotide

<400>  1331
tcgcattgac ttgcgtttcc acc                                               23


<210>  1332
<211>  19
<212>  DNA
<213>  Artificial Sequence

<220>
<223>  oligonucleotide

<400>  1332
gtgccgttcc accttatag                                                    19


<210>  1333
<211>  19
<212>  DNA
<213>  Artificial Sequence

<220>

<223>  oligonucleotide

<400>  1333
atggtgatcg cattgactt                                                    19


<210>  1334
<211>  22
<212>  DNA
<213>  Artificial Sequence

<220>
<223>  oligonucleotide

<400>  1334
accggctata aggtggaacg gc                                                22


<210>  1335
<211>  19
<212>  DNA
<213>  Artificial Sequence

<220>
<223>  oligonucleotide

<400>  1335
ctgccggttg aagataaca                                                    19


<210>  1336
<211>  19
<212>  DNA
<213>  Artificial Sequence

<220>
<223>  oligonucleotide

<400>  1336
tcgcattgac ttgcgtttc                                                    19


<210>  1337
<211>  22
<212>  DNA
<213>  Artificial Sequence

<220>
<223>  oligonucleotide

<400>  1337
ccaagcccag tgaccttcac ct                                                22


<210>  1338
<211>  19
<212>  DNA
<213>  Artificial Sequence

<220>
<223>  oligonucleotide

<400>  1338

catcacacat gtgccgttc                                                    19

<210>  1339
<211>  19
<212>  DNA
<213>  Artificial Sequence

<220>
<223>  oligonucleotide

<400>  1339
cattgacttg cgtttccac                                                    19

<210>  1340
<211>  25
<212>  DNA
<213>  Artificial Sequence

<220>
<223>  oligonucleotide

<400>  1340
ttcacctgca gaccggctat aaggt                                             25

<210>  1341
<211>  22
<212>  DNA
<213>  Artificial Sequence

<220>
<223>  oligonucleotide

<400>  1341
taacaatgtc cccatcacac at                                                22

<210>  1342
<211>  22
<212>  DNA
<213>  Artificial Sequence

<220>
<223>  oligonucleotide

<400>  1342
gcttgtcttc aatgaaatcc tc                                                22

<210>  1343
<211>  23
<212>  DNA
<213>  Artificial Sequence

<220>
<223>  oligonucleotide

<400>  1343
tccaccatga gttggtaggc agc                                               23

<210>  1344
<211>  21
<212>  DNA
<213>  Artificial Sequence

<220>
<223>  oligonucleotide

<400>  1344
ctgttcaaga ctgccaaatt c                                                21


<210>  1345
<211>  19
<212>  DNA
<213>  Artificial Sequence

<220>
<223>  oligonucleotide

<400>  1345
ccaactcatg gtggatgtg                                                   19


<210>  1346
<211>  22
<212>  DNA
<213>  Artificial Sequence

<220>
<223>  oligonucleotide

<400>  1346
aatccgttct gccaaagcca gc                                               22


<210>  1347
<211>  22
<212>  DNA
<213>  Artificial Sequence

<220>
<223>  oligonucleotide

<400>  1347
catacatctg tagtgccaat ga                                               22


<210>  1348
<211>  22
<212>  DNA
<213>  Artificial Sequence

<220>
<223>  oligonucleotide

<400>  1348
gccagcttgt cttcaatgaa at                                               22


<210>  1349
<211>  24
<212>  DNA

<213>  Artificial Sequence

<220>
<223>  oligonucleotide

<400>  1349
atccaccatg agttggtagg cagc                                              24


<210>  1350
<211>  21
<212>  DNA
<213>  Artificial Sequence

<220>
<223>  oligonucleotide

<400>  1350
caaagccagc ttctgttcaa g                                                 21


<210>  1351
<211>  22
<212>  DNA
<213>  Artificial Sequence

<220>
<223>  oligonucleotide

<400>  1351
catggtggat gtgtttggta at                                                22


<210>  1352
<211>  24
<212>  DNA
<213>  Artificial Sequence

<220>
<223>  oligonucleotide

<400>  1352
ttggcagtct tgaacagaag ctgg                                              24


<210>  1353
<211>  19
<212>  DNA
<213>  Artificial Sequence

<220>
<223>  oligonucleotide

<400>  1353
cgaatccgtt ctgccaaag                                                    19


<210>  1354
<211>  20
<212>  DNA
<213>  Artificial Sequence

<220>

<223>  oligonucleotide

<400>  1354
caaccctgaa gtgcttgata                                                   20


<210>  1355
<211>  22
<212>  DNA
<213>  Artificial Sequence

<220>
<223>  oligonucleotide

<400>  1355
tgcattctcg cttcctggag gg                                                22


<210>  1356
<211>  22
<212>  DNA
<213>  Artificial Sequence

<220>
<223>  oligonucleotide

<400>  1356
gtttgtaccc gttgatgata ga                                                22


<210>  1357
<211>  21
<212>  DNA
<213>  Artificial Sequence

<220>
<223>  oligonucleotide

<400>  1357
cacagaggaa actctgcatt c                                                 21


<210>  1358
<211>  23
<212>  DNA
<213>  Artificial Sequence

<220>
<223>  oligonucleotide

<400>  1358
agggtgtccg caatgttgcc agt                                               23


<210>  1359
<211>  19
<212>  DNA
<213>  Artificial Sequence

<220>
<223>  oligonucleotide

<400>  1359

gatgcaacag ttgggtagc                                                    19

<210>  1360
<211>  19
<212>  DNA
<213>  Artificial Sequence

<220>
<223>  oligonucleotide

<400>  1360
gacaatggca gcatctaca                                                    19

<210>  1361
<211>  22
<212>  DNA
<213>  Artificial Sequence

<220>
<223>  oligonucleotide

<400>  1361
tgcattctcg cttcctggag gg                                                22

<210>  1362
<211>  19
<212>  DNA
<213>  Artificial Sequence

<220>
<223>  oligonucleotide

<400>  1362
ccaatctgca gacagacac                                                    19

<210>  1363
<211>  20
<212>  DNA
<213>  Artificial Sequence

<220>
<223>  oligonucleotide

<400>  1363
cagcatctac aaccctgaag                                                   20

<210>  1364
<211>  22
<212>  DNA
<213>  Artificial Sequence

<220>
<223>  oligonucleotide

<400>  1364
tgcattctcg cttcctggag gg                                                22

<210>  1365
<211>  19
<212>  DNA
<213>  Artificial Sequence

<220>
<223>  oligonucleotide

<400>  1365
gacagacact ggcaacatt                                                    19


<210>  1366
<211>  19
<212>  DNA
<213>  Artificial Sequence

<220>
<223>  oligonucleotide

<400>  1366
aagggattca ggaagacga                                                    19


<210>  1367
<211>  24
<212>  DNA
<213>  Artificial Sequence

<220>
<223>  oligonucleotide

<400>  1367
aatggctctc atgtaccctt gcct                                              24


<210>  1368
<211>  21
<212>  DNA
<213>  Artificial Sequence

<220>
<223>  oligonucleotide

<400>  1368
ttctcacaac accaccattt a                                                 21


<210>  1369
<211>  22
<212>  DNA
<213>  Artificial Sequence

<220>
<223>  oligonucleotide

<400>  1369
ccctattcta aagggattca gg                                                22


<210>  1370
<211>  24
<212>  DNA

<213>  Artificial Sequence

<220>
<223>  oligonucleotide

<400>  1370
aatggctctc atgtaccctt gcct                                              24


<210>  1371
<211>  21
<212>  DNA
<213>  Artificial Sequence

<220>
<223>  oligonucleotide

<400>  1371
actcaacatc catcttctca c                                                 21


<210>  1372
<211>  22
<212>  DNA
<213>  Artificial Sequence

<220>
<223>  oligonucleotide

<400>  1372
gattcaggaa gacgacgtaa tg                                                22


<210>  1373
<211>  22
<212>  DNA
<213>  Artificial Sequence

<220>
<223>  oligonucleotide

<400>  1373
ctctcatgta cccttgcctc cc                                                22


<210>  1374
<211>  22
<212>  DNA
<213>  Artificial Sequence

<220>
<223>  oligonucleotide

<400>  1374
accaccattt aaaggtacca aa                                                22


<210>  1375
<211>  20
<212>  DNA
<213>  Artificial Sequence

<220>

<223>  oligonucleotide

<400>  1375
cgtaatggct ctcatgtacc                                                    20


<210>  1376
<211>  29
<212>  DNA
<213>  Artificial Sequence

<220>
<223>  oligonucleotide

<400>  1376
tggtaccttt aaatggtggt gttgtgaga                                          29


<210>  1377
<211>  19
<212>  DNA
<213>  Artificial Sequence

<220>
<223>  oligonucleotide

<400>  1377
cctgcaactc aacatccat                                                     19


<210>  1378
<211>  23
<212>  DNA
<213>  Artificial Sequence

<220>
<223>  oligonucleotide

<400>  1378
gaacagagac cagaaagagt aaa                                                23


<210>  1379
<211>  24
<212>  DNA
<213>  Artificial Sequence

<220>
<223>  oligonucleotide

<400>  1379
tgtgcttgat ttgttctgca gccc                                               24


<210>  1380
<211>  20
<212>  DNA
<213>  Artificial Sequence

<220>
<223>  oligonucleotide

<400>  1380

tcctcacggt agtccaatag                                                    20


<210>  1381
<211>  22
<212>  DNA
<213>  Artificial Sequence

<220>
<223>  oligonucleotide

<400>  1381
gggaaacttg aagaacagag ac                                                 22


<210>  1382
<211>  24
<212>  DNA
<213>  Artificial Sequence

<220>
<223>  oligonucleotide

<400>  1382
tgtgcttgat ttgttctgca gccc                                               24


<210>  1383
<211>  20
<212>  DNA
<213>  Artificial Sequence

<220>
<223>  oligonucleotide

<400>  1383
caaccatgcc atctggattc                                                    20


<210>  1384
<211>  22
<212>  DNA
<213>  Artificial Sequence

<220>
<223>  oligonucleotide

<400>  1384
caagcacatc cttgctaatt tc                                                 22


<210>  1385
<211>  25
<212>  DNA
<213>  Artificial Sequence

<220>
<223>  oligonucleotide

<400>  1385
tgaatccaga tggcatggtt gctct                                              25

<210>  1386
<211>  19
<212>  DNA
<213>  Artificial Sequence

<220>
<223>  oligonucleotide

<400>  1386
cacaccatcc tcacggtag                                                    19


<210>  1387
<211>  23
<212>  DNA
<213>  Artificial Sequence

<220>
<223>  oligonucleotide

<400>  1387
gcctacaaga agtacatcaa aga                                               23


<210>  1388
<211>  27
<212>  DNA
<213>  Artificial Sequence

<220>
<223>  oligonucleotide

<400>  1388
agggaaactt gaagaacaga gaccaga                                           27


<210>  1389
<211>  20
<212>  DNA
<213>  Artificial Sequence

<220>
<223>  oligonucleotide

<400>  1389
gcaaggatgt gcttgatttg                                                   20


<210>  1390
<211>  22
<212>  DNA
<213>  Artificial Sequence

<220>
<223>  oligonucleotide

<400>  1390
tgactccttc aacaccttct tc                                                22


<210>  1391
<211>  24
<212>  DNA

<213>  Artificial Sequence

<220>
<223>  oligonucleotide

<400>  1391
ccgggctgtg tttgtagact tgga                                              24


<210>  1392
<211>  20
<212>  DNA
<213>  Artificial Sequence

<220>
<223>  oligonucleotide

<400>  1392
ccagtgcgaa cttcatcaat                                                   20


<210>  1393
<211>  21
<212>  DNA
<213>  Artificial Sequence

<220>
<223>  oligonucleotide

<400>  1393
gactccttca acaccttctt c                                                 21


<210>  1394
<211>  24
<212>  DNA
<213>  Artificial Sequence

<220>
<223>  oligonucleotide

<400>  1394
ccgggctgtg tttgtagact tgga                                              24


<210>  1395
<211>  20
<212>  DNA
<213>  Artificial Sequence

<220>
<223>  oligonucleotide

<400>  1395
ccagtgcgaa cttcatcaat                                                   20


<210>  1396
<211>  20
<212>  DNA
<213>  Artificial Sequence

<220>

<223>  oligonucleotide

<400>  1396
ggaggagatg actccttcaa                                                    20


<210>  1397
<211>  22
<212>  DNA
<213>  Artificial Sequence

<220>
<223>  oligonucleotide

<400>  1397
cttcttcagt gagacgggcg ct                                                 22


<210>  1398
<211>  20
<212>  DNA
<213>  Artificial Sequence

<220>
<223>  oligonucleotide

<400>  1398
catcaatgac tgtgggttcc                                                    20


<210>  1399
<211>  19
<212>  DNA
<213>  Artificial Sequence

<220>
<223>  oligonucleotide

<400>  1399
cagatgccaa gtgacaaga                                                     19


<210>  1400
<211>  22
<212>  DNA
<213>  Artificial Sequence

<220>
<223>  oligonucleotide

<400>  1400
cttcttcagt gagacgggcg ct                                                 22


<210>  1401
<211>  20
<212>  DNA
<213>  Artificial Sequence

<220>
<223>  oligonucleotide

<400>  1401

gggttccaag tctacaaaca                                                20